(19) Europäisches Patentamt
European Patent Office
Office européen des brevets

(11) EP 3 875 608 A1

(12) **EUROPEAN PATENT APPLICATION**

(43) Date of publication:
**08.09.2021 Bulletin 2021/36**

(51) Int Cl.:
***C12Q 1/6886*** *(2018.01)*

(21) Application number: **20161176.1**

(22) Date of filing: **05.03.2020**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Koninklijke Philips N.V.**
**5656 AG Eindhoven (NL)**

(72) Inventors:
- **HOFFMANN, Ralf Dieter**
  **5656 AE Eindhoven (NL)**
- **ORSEL, Joukje Garrelina**
  **5656 AE Eindhoven (NL)**
- **DE KLERK - STARMANS, Maud**
  **5656 AE Eindhoven (NL)**
- **VAN LIESHOUT, on Martinus Laurentius**
  **5656 AE Eindhoven (NL)**

(74) Representative: **Philips Intellectual Property & Standards**
**High Tech Campus 5**
**5656 AE Eindhoven (NL)**

# (54) PREDICTION OF RADIOTHERAPY RESPONSE FOR PROSTATE CANCER SUBJECT BASED ON IMMUNE DEFENSE RESPONSE GENES

(57) The invention relates to a method of predicting a response of a prostate cancer subject to radiotherapy, comprising determining or receiving the result of a determination of a gene expression profile for each of one or more immune defense response genes selected from the group consisting of: APOBEC3A, AIM2, APOBEC3B, CASP1, CIAO1, CTNNB1, CXCL10, CXCL9, DDX41, DDX58, DDX60, DHX36, DHX58, DHX9, GBP1, HERC5, HIST2H2BE, IFI16, IFI44L, IFIH1, IFIT1, IFIT1B, IFIT2, IFIT3, IFITM1, IFITM3, IL1B, IRF3, IRF5, IRF7, ISG15, ITGAX, LILRB1, LRRFIP1, MAVS, MB21D1, MX1, MX2, MYD88, NLRP3, NOD2, OAS1, OAS3, OASL, OPRK1, POLR3A, PTPRC, PYCARD, RSAD2, STAT1, TBK1, TICAM1, TLR2, TLR3, TLR4, TLR7, TLR8, TLR9, TMEM173, TRIM22, and ZBP1, said gene expression profile(s) being determined in a biological sample obtained from the subject, and determining the prediction of the radiotherapy response based on the gene expression profile(s) for the one or more immune defense response genes.

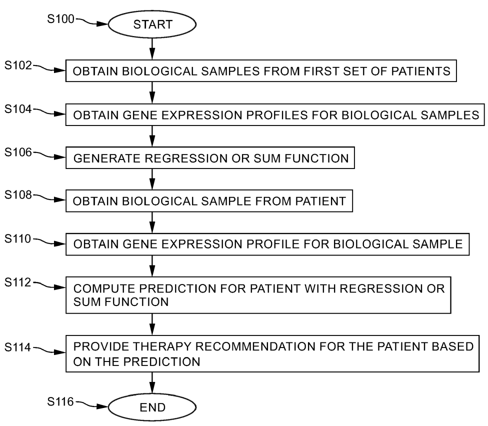


FIG. 1

EP 3 875 608 A1

SEQUENCE LISTING

<110> Koninklijke Philips N.V.

<120> Prediction of radiotherapy response for prostate cancer subject based on immune defense response genes

<130> 2019PF00711

<160> 56

<170> PatentIn version 3.5

<210> 1
<211> 1394
<212> DNA
<213> Homo sapiens

<400> 1

```
agaagtgtca gagtctttgt agctttgaaa gtcacctagg ttatttgggc atgctctcct      60

gagtcctctg ctagttaagc tctctgaaaa gaaggtggca gacccggttt gctgatcgcc     120

ccagggatca ggaggctgat cccaaagttg tcagatggag agtaaataca aggagatact     180

cttgctaaca ggcctggata acatcactga tgaggaactg gataggttta agttctttct     240

ttcagacgag tttaatattg ccacaggcaa actacatact gcaaacagaa tacaagtagc     300

taccttgatg attcaaaatg ctggggcggt gtctgcagtg atgaagacca ttcgtatttt     360

tcagaagttg aattatatgc ttttggcaaa acgtcttcag gaggagaagg agaaagttga     420

taagcaatac aaatcggtaa caaaaccaaa gccactaagt caagctgaaa tgagtcctgc     480

tgcatctgca gccatcagaa atgatgtcgc aaagcaacgt gctgcaccaa aagtctctcc     540

tcatgttaag cctgaacaga aacagatggt ggcccagcag gaatctatca gagaagggtt     600

tcagaagcgc tgtttgccag ttatggtact gaaagcaaag aagcccttca cgtttgagac     660

ccaagaaggc aagcaggaga tgtttcatgc tacagtggct acagaaaagg aattcttctt     720

tgtaaaagtt tttaatacac tgctgaaaga taaattcatt ccaaagagaa taattataat     780

agcaagatat tatcggcaca gtggtttctt agaggtaaat agcgcctcac gtgtgttaga     840

tgctgaatct gaccaaaagg ttaatgtccc gctgaacatt atcagaaaag ctggtgaaac     900

cccgaagatc aacacgcttc aaactcagcc ccttggaaca attgtgaatg gtttgtttgt     960

agtccagaag gtaacagaaa agaagaaaaa catattattt gacctaagtg acaacactgg    1020

gaaaatggaa gtactggggg ttagaaacga ggacacaatg aaatgtaagg aaggagataa    1080

ggttcgactt acattcttca cactgtcaaa aaatggagaa aaactacagc tgacatctgg    1140

agttcatagc accataaagg ttattaaggc caaaaaaaaa acatagagaa gtaaaaagga    1200

ccaattcaag ccaactggtc taagcagcat ttaattgaag aatatgtgat acagcctctt    1260

caatcagatt gtaagttacc tgaaagctgc agttcacagg ctcctctctc caccaaatta    1320
```

ggatagaata attgctggat aaacaaattc agaatatcaa cagatgatca caataaacat 1380

ctgtttctca ttca 1394

<210> 2
<211> 343
<212> PRT
<213> Homo sapiens

<400> 2

Met Glu Ser Lys Tyr Lys Glu Ile Leu Leu Leu Thr Gly Leu Asp Asn
1 5 10 15

Ile Thr Asp Glu Glu Leu Asp Arg Phe Lys Phe Phe Leu Ser Asp Glu
20 25 30

Phe Asn Ile Ala Thr Gly Lys Leu His Thr Ala Asn Arg Ile Gln Val
35 40 45

Ala Thr Leu Met Ile Gln Asn Ala Gly Ala Val Ser Ala Val Met Lys
50 55 60

Thr Ile Arg Ile Phe Gln Lys Leu Asn Tyr Met Leu Leu Ala Lys Arg
65 70 75 80

Leu Gln Glu Glu Lys Glu Lys Val Asp Lys Gln Tyr Lys Ser Val Thr
85 90 95

Lys Pro Lys Pro Leu Ser Gln Ala Glu Met Ser Pro Ala Ala Ser Ala
100 105 110

Ala Ile Arg Asn Asp Val Ala Lys Gln Arg Ala Ala Pro Lys Val Ser
115 120 125

Pro His Val Lys Pro Glu Gln Lys Gln Met Val Ala Gln Gln Glu Ser
130 135 140

Ile Arg Glu Gly Phe Gln Lys Arg Cys Leu Pro Val Met Val Leu Lys
145 150 155 160

Ala Lys Lys Pro Phe Thr Phe Glu Thr Gln Glu Gly Lys Gln Glu Met
165 170 175

Phe His Ala Thr Val Ala Thr Glu Lys Glu Phe Phe Phe Val Lys Val
180 185 190

Phe Asn Thr Leu Leu Lys Asp Lys Phe Ile Pro Lys Arg Ile Ile Ile
195 200 205

Ile Ala Arg Tyr Tyr Arg His Ser Gly Phe Leu Glu Val Asn Ser Ala
    210                 215                 220

Ser Arg Val Leu Asp Ala Glu Ser Asp Gln Lys Val Asn Val Pro Leu
225                 230                 235                 240

Asn Ile Ile Arg Lys Ala Gly Glu Thr Pro Lys Ile Asn Thr Leu Gln
                245                 250                 255

Thr Gln Pro Leu Gly Thr Ile Val Asn Gly Leu Phe Val Val Gln Lys
            260                 265                 270

Val Thr Glu Lys Lys Lys Asn Ile Leu Phe Asp Leu Ser Asp Asn Thr
        275                 280                 285

Gly Lys Met Glu Val Leu Gly Val Arg Asn Glu Asp Thr Met Lys Cys
    290                 295                 300

Lys Glu Gly Asp Lys Val Arg Leu Thr Phe Phe Thr Leu Ser Lys Asn
305                 310                 315                 320

Gly Glu Lys Leu Gln Leu Thr Ser Gly Val His Ser Thr Ile Lys Val
                325                 330                 335

Ile Lys Ala Lys Lys Lys Thr
            340

<210> 3
<211> 1358
<212> DNA
<213> Homo sapiens

<400> 3
gttggtgaag atcttaacac cacgccttga gcaagtcgca agagcgggag gacacagacc 60

aggaaccgag aagggacaag cacatggaag ccagcccagc atccgggccc agacacttga 120

tggatccaca catattcact tccaacttta acaatggcat tggaaggcat aagacctacc 180

tgtgctacga agtggagcgc ctggacaatg gcacctcggt caagatggac cagcacaggg 240

gctttctaca caaccaggct aagaatcttc tctgtggctt ttacggccgc catgcggagc 300

tgcgcttctt ggacctggtt ccttctttgc agttggaccc ggcccagatc tacagggtca 360

cttggttcat ctcctggagc ccctgcttct cctggggctg tgccggggaa gtgcgtgcgt 420

tccttcagga gaacacacac gtgagactgc gtatcttcgc tgcccgcatc tatgattacg 480

acccccctata taaggaggca ctgcaaatgc tgcgggatgc tggggcccaa gtctccatca 540

tgacctacga tgaatttaag cactgctggg acacctttgt ggaccaccag ggatgtccct 600

tccagccctg ggatggacta gatgagcaca gccaagccct gagtgggagg ctgcgggcca 660

ttctccagaa tcagggaaac tgaaggatgg gcctcagtct ctaaggaagg cagagacctg 720

ggttgagcag cagaataaaa gatcttcttc caagaaatgc aaacagaccg ttcaccacca 780

tctccagctg ctcacagacg ccagcaaagc agtatgctcc cgatcaagta gatttttaaa 840

aaatcagagt gggccgggcg cggtggctca cgcctgtaat cccagcactt tggaggccaa 900

ggcgggtgga tcacgaggtc aggagatcga gaccatcctg gctaacacgg tgaaaccctg 960

tctctactaa aaatacaaaa aattagccag gcgtggtggc gggcgcctgt agtcccagct 1020

actctggagg ctgaggcagg agagtagcgt gaacccggga ggcagagctt gcggtgagcc 1080

gagattgcgc tactgcactc cagcctgggc gacagtacca gactccatct caaaaaaaaa 1140

aaaaccagac tgaattaatt ttaactgaaa atttctctta tgttccaagt acacaatagt 1200

aagattatgc tcaatattct cagaataatt ttcaatgtat taatgaaatg aaatgataat 1260

ttggcttcat atctagacta acacaaaatt aagaatcttc cataattgct tttgctcagt 1320

aactgtgtca tgaattgcaa gagtttccac aaacacta 1358

<210> 4
<211> 199
<212> PRT
<213> Homo sapiens

<400> 4

Met Glu Ala Ser Pro Ala Ser Gly Pro Arg His Leu Met Asp Pro His
1               5                   10                  15

Ile Phe Thr Ser Asn Phe Asn Asn Gly Ile Gly Arg His Lys Thr Tyr
            20                  25                  30

Leu Cys Tyr Glu Val Glu Arg Leu Asp Asn Gly Thr Ser Val Lys Met
        35                  40                  45

Asp Gln His Arg Gly Phe Leu His Asn Gln Ala Lys Asn Leu Leu Cys
    50                  55                  60

Gly Phe Tyr Gly Arg His Ala Glu Leu Arg Phe Leu Asp Leu Val Pro
65                  70                  75                  80

Ser Leu Gln Leu Asp Pro Ala Gln Ile Tyr Arg Val Thr Trp Phe Ile
                85                  90                  95

Ser Trp Ser Pro Cys Phe Ser Trp Gly Cys Ala Gly Glu Val Arg Ala
            100                 105                 110

Phe Leu Gln Glu Asn Thr His Val Arg Leu Arg Ile Phe Ala Ala Arg

115 120 125

Ile Tyr Asp Tyr Asp Pro Leu Tyr Lys Glu Ala Leu Gln Met Leu Arg
130 135 140

Asp Ala Gly Ala Gln Val Ser Ile Met Thr Tyr Asp Glu Phe Lys His
145 150 155 160

Cys Trp Asp Thr Phe Val Asp His Gln Gly Cys Pro Phe Gln Pro Trp
165 170 175

Asp Gly Leu Asp Glu His Ser Gln Ala Leu Ser Gly Arg Leu Arg Ala
180 185 190

Ile Leu Gln Asn Gln Gly Asn
195

<210> 5
<211> 3968
<212> DNA
<213> Homo sapiens

<400> 5
ggcggaagcg ggagcctctg tcggccgcgg aagcctggag tgggcggtac gcagacgcgc 60

gcggtgagac ccgctgtctg ctcagcggac tctgcccgcc cccacctccc cctgcgtcgg 120

gccgacatga aggactcgct ggtgctgctg ggccgtgtcc cggcgcaccc ggactcccgc 180

tgctggttcc tggcctggaa ccccgcgggg accctgctgg cctcgtgcgg cggcgaccgg 240

agaatccgca tctggggcac ggagggtgac agctggatct gcaagtctgt cctttctgaa 300

ggccaccagc gcaccgtgcg gaaggtagcc tggtccccct gcggtaatta cctggcctct 360

gccagctttg atgctaccac ttgcatttgg aagaagaacc aggatgactt tgagtgtgta 420

accactctcg agggccatga aaatgaggtc aagtcagtgg cttgggcccc atctggcaac 480

ctcctggcca cttgcagccg agataagagc gtttgggtct gggaagttga tgaagaggat 540

gagtatgaat gtgtcagtgt tctcaactcc cacacacagg atgtcaagca tgtggtttgg 600

cacccaagtc aggagctctt agcttctgcc agctatgatg acacagtgaa gctgtaccgg 660

gaggaagagg atgactgggt atgctgtgcc acccttgagg gccatgaatc cactgtgtgg 720

agcttggcct ttgacccgag tggccagcgc ctggcgtctt gtagtgatga ccgtactgtg 780

cgtatctggc gtcagtatct accaggcaat gaacaagggg tggcatgcag cggctctgac 840

cccagttgga aatgtatctg tactttgtcc ggcttccact caaggaccat ttatgacatt 900

gcttggtgtc agctgacagg ggctctggcc acagcttgtg ggatgacgc gatccgcgtg 960

tttcaggagg atcccaactc ggatccacag cagcccacct tctccctgac agcccacttg 1020

```
catcaggccc attcccagga tgtcaactgt gtggcctgga accccaagga gccagggcta     1080
ctggcctcct gcagtgatga tggggaggtg gccttctgga agtatcagcg gcctgaaggc     1140
ctctgagcta cctcgacttt ggacagagta atgactcccc agaaaacgtc atataagact     1200
ttaccagccc ctgagaggac caggaggagc atccttgacc ttcatttaac ttggctcact     1260
tctcttcaga cttgggtaga agtgcagagc cacagaattg ctttccttcc ccgcctttga     1320
catgaggcct tcagtaaaga gctacagaac atgagtacat tgttatacca cagatttttc     1380
ttgcattagg gcacagtgtt aaatttttg gaggtaaata tactatttat aatcactata     1440
tatagtagga gggggtatgt gtctcaggct tttctgaagt tgcaagactt aaagaaataa     1500
tccatctgca tcccaagtcc tattttataa ggatattcat aaaaattcca tggtgaatcc     1560
ttgtctgaaa taggtcctcc cttcccagtt tctgtgtaag tctttgcatt taagacatcc     1620
aatcaataat gaaggaaatt tttttctgaa tgtaggtttg agtgaggggc acctctgctt     1680
tcccttagca accctcatat acctccctgc accgttacgc tgtgatggca actggggata     1740
gaaaaaaaat ggggaaagac aggaatccta aaagggagag ttattactgg ccacaagccc     1800
tgtattctca acagggatgc aaattggttc ttcagtaagg ataaaaaaaa atcacaagca     1860
gttgtttgtg gccctcctaa ggcccacagc acatatagtg tgtctgtgat attccatttt     1920
catggcaggg agtgatcagg aagaaggctt cctaggggac tggcgattta aaccagttga     1980
gaaacactgc catcagcagg cagtttcaga ctcactcaag ttgtctcttg acagtcactt     2040
ctaaatgggt tctaatgtga caatggcctc caaaactaca gccttccctg aagtttaagc     2100
tgtgacctta gattttagaa ggacagtggg gctgtaccta gaatagtggt tctcgaagaa     2160
tgcggcctgc agatcctggg agtcccaaga ccctttcagg gaggatctgt gaggtcaact     2220
gttggcactg tggcatgaat caaggtggtg gcagcaaact tctagtagtt ttgatatgtc     2280
cttgatagaa caaatagcaa tggttaacta ttaaatgttg acctagccag cgcagtggct     2340
catgcctgta atcccagcac tttgggaggc tgaggcgggc ggatcacctg aggtcgggag     2400
ttcgaggcca gcctgaccaa catggagaaa ccccgtctct tctaaaaata caaaattagc     2460
tgggcatggt ggtgcatgcc tgtaattcca gctactcggg aggctgaggc aagagaatcg     2520
cttgaatccg gtaggtggag gttgcagtga gccgagatca taccattgca ctccagccca     2580
ggcaacaaga gtgaaaccct gtctcaaaaa gaaaaaaaaa gttgaccttg agaatttata     2640
atattctgag aaaactggaa gcatgcataa agcccctctg ctgtgcactg aagtatgggt     2700
gccttgagga aaagcagtta cacagttgag ttgcaagctg aattggctgt gttcaaggca     2760
tgccctttag aattgaaaga actagcagat tacggtattt agacttgaat atttggctga     2820
tattttctgg aaattaatgg aatgagcctc tcacctcaag ggaaacaact gatagtgttg     2880
ccagtgataa agctttcaag caaaaattgg aatttccgaa aatctgtact ccaccatgag     2940
```

ctttattgtt ggggatatta acaaatgtga tttgtataat gaaatgcatt tcatttggaa 3000

gaattcaatg aaccattttt ccaagtgacc agtacgtgat gttacaaaat catgcatggc 3060

tcaaagattg attcaaaagt gtaagacagg ccagtggatt ttaatgtatt aacaatataa 3120

gagtgcatta agttttcgga gtctacattg cctttaagaa actatgactt gtagtaagcc 3180

gggcgcggtg gctcacgcct gtaatcccaa cactttggga ggccaaggtg ggtggatcac 3240

aaggtcagga gttcaagacc agcctggcca atatggtgaa agtccgtctc tactaaaatt 3300

acaaaaatta gccgggcgtg gtggcagatc ccttgtagtc ccagctactc gggaggctga 3360

ggcaggagaa tagcttgaac ccgggaggtg gaggttgcag tgagtcgaga tcgtgccact 3420

ggactccagc ctgggtgaca gagcgagact ccatttcaaa aaaaaaaaaa aaaaaaaaaa 3480

atcacttgta gtcttggtgt ggtatcaaag aatagccaca attagctgaa aaggctattt 3540

taaaaacttt tccaactgcg tatctgtgtg aagtcaactt acttcaacaa aaaagtttgg 3600

atgtagaagc agctgtaaga attcaactgt ttattataac aagatactaa agagactgta 3660

aaatgccacc cttctccttg gattgttttg gaagttattc ttcataaaaa atgttaacgt 3720

gggctgggca tggtggctca tgcctgtaat cccagcactc tgggaggctg aggtgggcgg 3780

atcacttgag ctcaggaatt caaggtcagc ctgggcaaca tggctaaact ctgtctctat 3840

taagaaaaaa aatgttaaca ttatgattta aaagtgcatt aaccttaatc tagataataa 3900

aagctttttg gggcaacctc cagaactgtg aaaaataaat ttgttattta aaaagaaaaa 3960

aaaaaaaa 3968

<210> 6
<211> 339
<212> PRT
<213> Homo sapiens

<400> 6

Met Lys Asp Ser Leu Val Leu Leu Gly Arg Val Pro Ala His Pro Asp
1 5 10 15

Ser Arg Cys Trp Phe Leu Ala Trp Asn Pro Ala Gly Thr Leu Leu Ala
20 25 30

Ser Cys Gly Gly Asp Arg Arg Ile Arg Ile Trp Gly Thr Glu Gly Asp
35 40 45

Ser Trp Ile Cys Lys Ser Val Leu Ser Glu Gly His Gln Arg Thr Val
50 55 60

Arg Lys Val Ala Trp Ser Pro Cys Gly Asn Tyr Leu Ala Ser Ala Ser
65 70 75 80

```
Phe Asp Ala Thr Thr Cys Ile Trp Lys Lys Asn Gln Asp Asp Phe Glu
                85                  90                  95

Cys Val Thr Thr Leu Glu Gly His Glu Asn Glu Val Lys Ser Val Ala
            100                 105                 110

Trp Ala Pro Ser Gly Asn Leu Leu Ala Thr Cys Ser Arg Asp Lys Ser
        115                 120                 125

Val Trp Val Trp Glu Val Asp Glu Glu Asp Glu Tyr Glu Cys Val Ser
    130                 135                 140

Val Leu Asn Ser His Thr Gln Asp Val Lys His Val Val Trp His Pro
145                 150                 155                 160

Ser Gln Glu Leu Leu Ala Ser Ala Ser Tyr Asp Asp Thr Val Lys Leu
                165                 170                 175

Tyr Arg Glu Glu Glu Asp Asp Trp Val Cys Cys Ala Thr Leu Glu Gly
            180                 185                 190

His Glu Ser Thr Val Trp Ser Leu Ala Phe Asp Pro Ser Gly Gln Arg
        195                 200                 205

Leu Ala Ser Cys Ser Asp Asp Arg Thr Val Arg Ile Trp Arg Gln Tyr
    210                 215                 220

Leu Pro Gly Asn Glu Gln Gly Val Ala Cys Ser Gly Ser Asp Pro Ser
225                 230                 235                 240

Trp Lys Cys Ile Cys Thr Leu Ser Gly Phe His Ser Arg Thr Ile Tyr
                245                 250                 255

Asp Ile Ala Trp Cys Gln Leu Thr Gly Ala Leu Ala Thr Ala Cys Gly
            260                 265                 270

Asp Asp Ala Ile Arg Val Phe Gln Glu Asp Pro Asn Ser Asp Pro Gln
        275                 280                 285

Gln Pro Thr Phe Ser Leu Thr Ala His Leu His Gln Ala His Ser Gln
    290                 295                 300

Asp Val Asn Cys Val Ala Trp Asn Pro Lys Glu Pro Gly Leu Leu Ala
305                 310                 315                 320

Ser Cys Ser Asp Asp Gly Glu Val Ala Phe Trp Lys Tyr Gln Arg Pro
```

325 330 335

Glu Gly Leu

<210> 7
<211> 4628
<212> DNA
<213> Homo sapiens

<400> 7
gaacgtagct agctgcaagc agaggccggc atgaccaccg agcagcgacg cagcctgcaa 60

gccttccagg attatatccg gaagaccctg gaccctacct acatcctgag ctacatggcc 120

ccctggttta gggaggaaga ggtgcagtat attcaggctg agaaaaacaa caagggccca 180

atggaggctg ccacactttt tctcaagttc ctgttggagc tccaggagga aggctggttc 240

cgtggctttt tggatgccct agaccatgca ggttattctg gactttatga agccattgaa 300

agttgggatt tcaaaaaaat tgaaaagttg gaggagtata gattactttt aaaacgttta 360

caaccagaat ttaaaaccag aattatccca accgatatca tttctgatct gtctgaatgt 420

ttaattaatc aggaatgtga agaaattcta cagatttgct ctactaaggg gatgatggca 480

ggtgcagaga aattggtgga atgccttctc agatcagaca aggaaaactg gcccaaaact 540

ttgaaacttg ctttggagaa agaaaggaac aagttcagtg aactgtggat tgtagagaaa 600

ggtataaaag atgttgaaac agaagatctt gaggataaga tggaaacttc tgacatacag 660

attttctacc aagaagatcc agaatgccag aatcttagtg agaattcatg tccaccttca 720

gaagtgtctg atacaaactt gtacagccca tttaaaccaa gaaattacca attagagctt 780

gctttgcctg ctatgaaagg aaaaaacaca ataatatgtg ctcctacagg ttgtggaaaa 840

acctttgttt cactgcttat atgtgaacat catcttaaaa aattcccaca aggacaaaag 900

gggaaagttg tctttttgc gaatcagatc ccagtgtatg aacagcagaa atctgtattc 960

tcaaaatact ttgaaagaca tgggtataga gttacaggca tttctggagc aacagctgag 1020

aatgtcccag tggaacagat tgttgagaac aatgacatca tcattttaac tccacagatt 1080

cttgtgaaca accttaaaaa gggaacgatt ccatcactat ccatctttac tttgatgata 1140

tttgatgaat gccacaacac tagtaaacaa cacccgtaca atatgatcat gtttaattat 1200

ctagatcaga aacttggagg atcttcaggc ccactgcccc aggtcattgg gctgactgcc 1260

tcggttggtg ttggggatgc caaaaacaca gatgaagcct tggattatat ctgcaagctg 1320

tgtgcttctc ttgatgcgtc agtgatagca acagtcaaac acaatctgga ggaactggag 1380

caagttgttt ataagcccca gaagtttttc aggaaagtgg aatcacggat tagcgacaaa 1440

tttaaataca tcatagctca gctgatgagg gacacagaga gtctggcaaa gagaatctgc 1500

```
aaagacctcg aaaacttatc tcaaattcaa aatagggaat ttggaacaca gaaatatgaa     1560
caatggattg ttacagttca gaaagcatgc atggtgttcc agatgccaga caaagatgaa     1620
gagagcagga tttgtaaagc cctgttttta tacacttcac atttgcggaa atataatgat     1680
gccctcatta tcagtgagca tgcacgaatg aaagatgctc tggattactt gaaagacttc     1740
ttcagcaatg tccgagcagc aggattcgat gagattgagc aagatcttac tcagagattt     1800
gaagaaaagc tgcaggaact agaaagtgtt tccagggatc ccagcaatga gaatcctaaa     1860
cttgaagacc tctgcttcat cttacaagaa gagtaccact taaacccaga gacaataaca     1920
attctctttg tgaaaaccag agcacttgtg gacgctttaa aaaattggat tgaaggaaat     1980
cctaaactca gttttctaaa acctggcata ttgactggac gtggcaaaac aaatcagaac     2040
acaggaatga ccctcccggc acagaagtgt atattggatg cattcaaagc cagtggagat     2100
cacaatattc tgattgccac ctcagttgct gatgaaggca ttgacattgc acagtgcaat     2160
cttgtcatcc tttatgagta tgtgggcaat gtcatcaaaa tgatccaaac cagaggcaga     2220
ggaagagcaa gaggtagcaa gtgcttcctt ctgactagta atgctggtgt aattgaaaaa     2280
gaacaaataa acatgtacaa agaaaaaatg atgaatgact ctattttacg ccttcagaca     2340
tgggacgaag cagtatttag ggaaaagatt ctgcatatac agactcatga aaaattcatc     2400
agagatagtc aagaaaaacc aaaacctgta cctgataagg aaaataaaaa actgctctgc     2460
agaaagtgca aagccttggc atgttacaca gctgacgtaa gagtgataga ggaatgccat     2520
tacactgtgc ttggagatgc ttttaaggaa tgctttgtga gtagaccaca tcccaagcca     2580
aagcagtttt caagttttga aaaaagagca aagatattct gtgcccgaca gaactgcagc     2640
catgactggg gaatccatgt gaagtacaag acatttgaga ttccagttat aaaaattgaa     2700
agttttgtgg tggaggatat tgcaactgga gttcagacac tgtactcgaa gtggaaggac     2760
tttcattttg agaagatacc atttgatcca gcagaaatgt ccaaatgata tcaggtcctc     2820
aatcttcagc tacagggaat gagtaacttt gagtggagaa gaaacaaaca tagtgggtat     2880
aatcatggat cgcttgtacc cctgtgaaaa tatatttttt aaaaatatct ttagcagttt     2940
gtactatatt atatatgcaa agcacaaatg agtgaatcac agcactgagt attttgtagg     3000
ccaacagagc tcatagtact tgggaaaaat taaaaagcct catttctagc cttcttttta     3060
gagtcaactg ccaacaaaca cacagtaatc actctgtaca cactgggata gatgaatgaa     3120
tggaatgttg ggaattttta tctccctttg tctccttaac ctactgtaaa ctggcttttg     3180
cccttaacaa tctactgaaa ttgttctttt gaaggttacc agtgactctg gttgccaaat     3240
ccactgggca cttcttaacc ttctatttga cctctgcgca tttggccctg ttgagcactc     3300
ttcttgaagc tctccctggg cttctctctc ttctagttct attctagtct tttttattg      3360
agtcctcctc tttgctgatc ccttccaagg gttcaatata tatacatgta tatactgtac     3420
```

atatgtatat gtaactaata tacatacata caggtatgta tatgtaatgg ttatatgtac 3480

tcatgttcct ggtgtagcaa cgtgtggtat ggctacacag agaacatgag aacataaagc 3540

catttttatg cttactacta aaagctgtcc actgtagagt tgctgtatgt agcaatgtgt 3600

atccactcta cagtggtcag cttttagtag agagcataaa aatgataaaa tacttcttga 3660

aaacttagtt tactatacat cttgccctat taatatgttc tcttaacgtg tgccattgtt 3720

ctctttgacc attttcctat aatgatgttg atgttcaaca cctggactga atgtctgttc 3780

tcagatccct tggatgttac agatgaggca gtctgactgt cctttctact tgaaagatta 3840

gaatatgtat ccaaatggca ttcacgtgtc acttagcaag gtttgctgat gcttcaaaga 3900

gcttagtttg cggtttcctg gacgtggaaa caagtatctg agttccctgg agatcaacgg 3960

gatgaggtgt tacagctgcc tccctcttca tgcaatctgg tgagcagtgg tgcaggcggg 4020

gagccagaga aacttgccag ttatataact tctctttggc ttttcttcat ctgtaaaaca 4080

aggataatac tgaactgtaa gggttagtgg agagttttta attaaaagaa tgtgtgaaaa 4140

gtacatgaca cagtagttgc ttgataatag ttactagtag tagtattctt actaagaccc 4200

aatacaaatg gattatttaa accaagttta tgagttggtt ttttttcatt ttctatttgt 4260

attttattaa gagtgtcttt tcttatgtga tttttttaa ttgctatttg atatggtttg 4320

gctatatgtc cccacccaaa tctcatcttg aattataatc cccatgtgtc aagggaggga 4380

cctgacggga ggtgattgga tcacgggggc agttgtcccc atgctgttct tgggatagtg 4440

agttagttct catgagatct gatggtttta taagtgtttg acaattcctc ctttacacac 4500

actctctctc tcatctgctg ccatgtaaga cttgcctgct tccccttctg ccatgattgt 4560

aagtttcctg aggcctcctc agccatgtgg aactgtgaat ctattaagcc tcttttcttt 4620

ataaatga 4628

<210> 8
<211> 925
<212> PRT
<213> Homo sapiens

<400> 8

Met Thr Thr Glu Gln Arg Arg Ser Leu Gln Ala Phe Gln Asp Tyr Ile
1 5 10 15

Arg Lys Thr Leu Asp Pro Thr Tyr Ile Leu Ser Tyr Met Ala Pro Trp
20 25 30

Phe Arg Glu Glu Glu Val Gln Tyr Ile Gln Ala Glu Lys Asn Asn Lys
35 40 45

```
Gly Pro Met Glu Ala Ala Thr Leu Phe Leu Lys Phe Leu Leu Glu Leu
    50                  55                  60

Gln Glu Glu Gly Trp Phe Arg Gly Phe Leu Asp Ala Leu Asp His Ala
65                  70                  75                  80

Gly Tyr Ser Gly Leu Tyr Glu Ala Ile Glu Ser Trp Asp Phe Lys Lys
                85                  90                  95

Ile Glu Lys Leu Glu Glu Tyr Arg Leu Leu Leu Lys Arg Leu Gln Pro
            100                 105                 110

Glu Phe Lys Thr Arg Ile Ile Pro Thr Asp Ile Ile Ser Asp Leu Ser
        115                 120                 125

Glu Cys Leu Ile Asn Gln Glu Cys Glu Glu Ile Leu Gln Ile Cys Ser
    130                 135                 140

Thr Lys Gly Met Met Ala Gly Ala Glu Lys Leu Val Glu Cys Leu Leu
145                 150                 155                 160

Arg Ser Asp Lys Glu Asn Trp Pro Lys Thr Leu Lys Leu Ala Leu Glu
                165                 170                 175

Lys Glu Arg Asn Lys Phe Ser Glu Leu Trp Ile Val Glu Lys Gly Ile
            180                 185                 190

Lys Asp Val Glu Thr Glu Asp Leu Glu Asp Lys Met Glu Thr Ser Asp
        195                 200                 205

Ile Gln Ile Phe Tyr Gln Glu Asp Pro Glu Cys Gln Asn Leu Ser Glu
    210                 215                 220

Asn Ser Cys Pro Pro Ser Glu Val Ser Asp Thr Asn Leu Tyr Ser Pro
225                 230                 235                 240

Phe Lys Pro Arg Asn Tyr Gln Leu Glu Leu Ala Leu Pro Ala Met Lys
                245                 250                 255

Gly Lys Asn Thr Ile Ile Cys Ala Pro Thr Gly Cys Gly Lys Thr Phe
            260                 265                 270

Val Ser Leu Leu Ile Cys Glu His His Leu Lys Lys Phe Pro Gln Gly
        275                 280                 285

Gln Lys Gly Lys Val Val Phe Phe Ala Asn Gln Ile Pro Val Tyr Glu
    290                 295                 300
```

Gln Gln Lys Ser Val Phe Ser Lys Tyr Phe Glu Arg His Gly Tyr Arg
305 310 315 320

Val Thr Gly Ile Ser Gly Ala Thr Ala Glu Asn Val Pro Val Glu Gln
325 330 335

Ile Val Glu Asn Asn Asp Ile Ile Ile Leu Thr Pro Gln Ile Leu Val
340 345 350

Asn Asn Leu Lys Lys Gly Thr Ile Pro Ser Leu Ser Ile Phe Thr Leu
355 360 365

Met Ile Phe Asp Glu Cys His Asn Thr Ser Lys Gln His Pro Tyr Asn
370 375 380

Met Ile Met Phe Asn Tyr Leu Asp Gln Lys Leu Gly Gly Ser Ser Gly
385 390 395 400

Pro Leu Pro Gln Val Ile Gly Leu Thr Ala Ser Val Gly Val Gly Asp
405 410 415

Ala Lys Asn Thr Asp Glu Ala Leu Asp Tyr Ile Cys Lys Leu Cys Ala
420 425 430

Ser Leu Asp Ala Ser Val Ile Ala Thr Val Lys His Asn Leu Glu Glu
435 440 445

Leu Glu Gln Val Val Tyr Lys Pro Gln Lys Phe Phe Arg Lys Val Glu
450 455 460

Ser Arg Ile Ser Asp Lys Phe Lys Tyr Ile Ile Ala Gln Leu Met Arg
465 470 475 480

Asp Thr Glu Ser Leu Ala Lys Arg Ile Cys Lys Asp Leu Glu Asn Leu
485 490 495

Ser Gln Ile Gln Asn Arg Glu Phe Gly Thr Gln Lys Tyr Glu Gln Trp
500 505 510

Ile Val Thr Val Gln Lys Ala Cys Met Val Phe Gln Met Pro Asp Lys
515 520 525

Asp Glu Glu Ser Arg Ile Cys Lys Ala Leu Phe Leu Tyr Thr Ser His
530 535 540

Leu Arg Lys Tyr Asn Asp Ala Leu Ile Ile Ser Glu His Ala Arg Met
545 550 555 560

Lys Asp Ala Leu Asp Tyr Leu Lys Asp Phe Phe Ser Asn Val Arg Ala
565 570 575

Ala Gly Phe Asp Glu Ile Glu Gln Asp Leu Thr Gln Arg Phe Glu Glu
580 585 590

Lys Leu Gln Glu Leu Glu Ser Val Ser Arg Asp Pro Ser Asn Glu Asn
595 600 605

Pro Lys Leu Glu Asp Leu Cys Phe Ile Leu Gln Glu Glu Tyr His Leu
610 615 620

Asn Pro Glu Thr Ile Thr Ile Leu Phe Val Lys Thr Arg Ala Leu Val
625 630 635 640

Asp Ala Leu Lys Asn Trp Ile Glu Gly Asn Pro Lys Leu Ser Phe Leu
645 650 655

Lys Pro Gly Ile Leu Thr Gly Arg Gly Lys Thr Asn Gln Asn Thr Gly
660 665 670

Met Thr Leu Pro Ala Gln Lys Cys Ile Leu Asp Ala Phe Lys Ala Ser
675 680 685

Gly Asp His Asn Ile Leu Ile Ala Thr Ser Val Ala Asp Glu Gly Ile
690 695 700

Asp Ile Ala Gln Cys Asn Leu Val Ile Leu Tyr Glu Tyr Val Gly Asn
705 710 715 720

Val Ile Lys Met Ile Gln Thr Arg Gly Arg Gly Arg Ala Arg Gly Ser
725 730 735

Lys Cys Phe Leu Leu Thr Ser Asn Ala Gly Val Ile Glu Lys Glu Gln
740 745 750

Ile Asn Met Tyr Lys Glu Lys Met Met Asn Asp Ser Ile Leu Arg Leu
755 760 765

Gln Thr Trp Asp Glu Ala Val Phe Arg Glu Lys Ile Leu His Ile Gln
770 775 780

Thr His Glu Lys Phe Ile Arg Asp Ser Gln Glu Lys Pro Lys Pro Val
785 790 795 800

Pro Asp Lys Glu Asn Lys Lys Leu Leu Cys Arg Lys Cys Lys Ala Leu

805 810 815

Ala Cys Tyr Thr Ala Asp Val Arg Val Ile Glu Glu Cys His Tyr Thr
820 825 830

Val Leu Gly Asp Ala Phe Lys Glu Cys Phe Val Ser Arg Pro His Pro
835 840 845

Lys Pro Lys Gln Phe Ser Ser Phe Glu Lys Arg Ala Lys Ile Phe Cys
850 855 860

Ala Arg Gln Asn Cys Ser His Asp Trp Gly Ile His Val Lys Tyr Lys
865 870 875 880

Thr Phe Glu Ile Pro Val Ile Lys Ile Glu Ser Phe Val Val Glu Asp
885 890 895

Ile Ala Thr Gly Val Gln Thr Leu Tyr Ser Lys Trp Lys Asp Phe His
900 905 910

Phe Glu Lys Ile Pro Phe Asp Pro Ala Glu Met Ser Lys
915 920 925

<210> 9
<211> 4493
<212> DNA
<213> Homo sapiens

<400> 9
gccatttcgc cgattcctcc atgcgagttg ctgtgcgttt ctctgttgtc tcggtagaag 60

gccagagtca cacacggtcc taagagctgg gcaccaggaa gcgaaggctg atctgaagaa 120

gacacttgaa tcatgggtga cgttaaaaat tttctgtatg cctggtgtgg caaaaggaag 180

atgaccccat cctatgaaat tagagcagtg gggaacaaaa acaggcagaa attcatgtgt 240

gaggttcagg tggaaggtta taattacact ggcatgggaa attccaccaa taaaaaagat 300

gcacaaagca atgctgccag agactttgtt aactatttgg ttcgaataaa tgaaataaag 360

agtgaagaag ttccagcttt tggggtagca tctccgcccc cacttactga tactcctgac 420

actacagcaa atgctgaagg agatttacca acaaccatgg gaggacctct tcctccacat 480

ctggctctca aagcagaaaa taattctgag gtaggggcct ctggctatgg tgttcctggg 540

cccacctggg accgaggagc caacttgaag gattactact caagaaagga agaacaagaa 600

gtgcaagcga ctctagaatc agaagaagtg gatttaaatg ctgggcttca tggaaactgg 660

accttggaaa atgctaaagc tcgtctaaac caatattttc agaaagaaaa gatccaagga 720

gaatataagt acacccaagt gggtcctgat cacaacagga gctttattgc agaaatgacc 780

atttatatca agcagctggg cagaaggatt tttgcacgag aacatggatc aaataagaaa 840
ttggcagcac agtcctgtgc cctgtcactt gtcagacaac tgtaccatct tggagtggtt 900
gaagcttact ccggacttac aaagaagaag gaaggagaga cagtggagcc ttacaaagta 960
aacctctctc aagatttaga gcatcagctg caaaacatca ttcaagagct aaatcttgag 1020
attttgcccc cgcctgaaga tccttctgtg ccagttgcac tcaacattgg caaattggct 1080
cagttcgaac catctcagcg acaaaaccaa gtgggtgtgg ttccttggtc acctccacaa 1140
tccaactgga atccttggac tagtagcaac attgatgagg ggcctctggc ttttgctact 1200
ccagagcaaa taagcatgga cctcaagaat gaattgatgt accagttgga acaggatcat 1260
gatttgcaag caatcttgca ggagagagag ttactgcctg tgaagaaatt tgaaagtgag 1320
attctggaag caatcagcca aaattcagtt gtcattatta gaggggctac tggatgtggg 1380
aaaaccacac aggttcccca gttcattcta gatgacttta tccagaatga ccgagcagca 1440
gagtgtaaca tcgtagtaac tcagcccaga agaatcagtg cggtttctgt ggcagagcga 1500
gttgcatttg aaagaggaga agagcctgga aaaagctgtg gctacagcgt tcgatttgag 1560
tctatacttc ctcgtcctca tgccagtata atgttttgta ctgtaggtgt gctcctgaga 1620
aaattagaag caggcattcg aggaatcagt catgtaattg tagatgaaat acatgaaaga 1680
gatattaata ctgacttcct tttggtagta ctgcgtgatg ttgttcaggc ttatcctgaa 1740
gttcgcattg ttcttatgtc tgctactatt gataccagca tgttttgtga atatttcttc 1800
aattgcccca tcattgaagt ttatgggagg acttacccag ttcaagaata ttttctggaa 1860
gactgcattc agatgaccca ctttgttcct ccaccaaaag acaaaaagaa gaaggataag 1920
gatgatgatg gtggtgagga tgatgatgca aattgcaact tgatctgtgg tgatgaatat 1980
ggtccagaaa caaggttgag catgtctcaa ttgaacgaaa aggaaactcc ttttgaactc 2040
atcgaggctc tacttaagta cattgaaacc cttaatgttc ctggagctgt gttggttttt 2100
ttgcctggct ggaatctgat ttatactatg cagaagcatt tggaaatgaa tccacatttt 2160
ggaagccatc ggtatcagat tctacccctg cattctcaga ttcctcgaga ggaacagcgc 2220
aaagtgtttg atccagtacc agttggagta accaaggtta ttttgtccac aaatattgct 2280
gaaacaagca ttaccataaa cgatgttgtt tatgtcattg actcctgcaa gcagaaagtg 2340
aaactcttca ctgctcacaa caatatgacc aactatgcta ccgtatgggc atcaaaaaca 2400
aaccttgagc aacggaaagg gcgagctggc cgagtacggc ctggattctg ctttcacctg 2460
tgcagccgag ctcgttttga gagacttgaa acccacatga caccagagat gttccgaaca 2520
ccattgcatg aaattgctct tagcataaaa cttctgcgtc taggaggaat tggccaattt 2580
ctggccaaag caattgaacc tccccctttg gatgctgtga ttgaagcaga acacactctt 2640
agagagcttg atgcattaga tgccaatgat gagttgactc ctttgggacg aatcctggct 2700

```
aaactcccca ttgagcctcg ttttggcaaa atgatgataa tggggtgtat tttctacgtg      2760
ggagatgcta tctgtaccat tgctgctgct acctgctttc cagagccttt catcaatgaa      2820
ggaaagcggc tgggctatat ccatcgaaat tttgctggaa acagattttc tgatcacgta      2880
gccctttat cagtattcca agcctgggat gatgctagaa tgggtggaga agaagcagag       2940
atacgttttt gtgagcacaa aagacttaat atggctacac taagaatgac ttgggaagcc      3000
aaagttcagc tcaaagagat tttgattaat tctgggtttc cagaagattg tttgttgaca      3060
caagtgttta ctaacactgg accagataat aatttggatg ttgttatctc cctcctggcc      3120
tttggtgtgt accccaatgt atgctatcat aaggaaaaga ggaagattct caccactgaa      3180
gggcgtaatg cacttatcca caaatcatct gttaattgtc cttttagtag ccaagacatg      3240
aagtacccat ctcccttctt tgtatttggt gaaaagattc gaactcgagc catctctgct      3300
aaaggcatga ctttagtcac ccccctgcag ttgcttctct ttgcctccaa gaaagtccaa      3360
tctgatgggc agattgtgct tgtagatgac tggattaaac tgcaaatatc tcatgaagct      3420
gctgcctgta tcactggtct ccgggcagcc atggaggctt tggttgttga agtaaccaaa      3480
caacctgcta tcatcagcca gttggacccc gtaaatgaac gtatgctgaa catgatccgt      3540
cagatctcta gaccctcagc tgctggtatc aaccttatga ttggcagtac acggtatgga      3600
gatggtccac gtcctcccaa gatggcccga tacgacaatg gaagcggata tagaagggga      3660
ggttctagtt acagtggtgg aggctatggc ggtggctata gcagtggagg ctatggtagc      3720
ggaggctatg gtggcagcgc caactccttt cgggcaggat atggtgcagg tgttggtgga      3780
ggctatagag gagtttcccg aggtggcttt agaggcaact ctggaggaga ctacagaggg      3840
cctagtggag gctacagagg atctggggga ttccagcgag gaggtggtag gggggcctat      3900
ggaactggct actttggaca gggaagagga ggtggcggct attaaaactt ggttatgtca      3960
gttcctgtgt gtagacagta aggaaaaaaa ggcatgctat gtgttacgtg tttttccag       4020
tatgtttatt tgccaccaaa aagtaaatgc attttcaccc attctgtggt tcattgtagt      4080
ttaaggaaac caagcatata gatgcattag tgattttgtt tatattatgt aaaatataac      4140
gatctcttaa aaataccaca gtttgtattt tttctttaag gagtaaagat ttgcctttaa      4200
ataacttggt attttcctgg ctttcgttta atacaataga aaataaagta ttacaccgaa      4260
tacttgccgt gtagtttgtt tgttgacctc gtatgttaga aaattttaca atgccagcta      4320
catctgttga ttttaaatgt cagagaagtt gtaccctgtt tcaaaagtat actaagtgat      4380
actacttgta atagaataaa tcatcttgga attgaattgt taccttttga agtaaatact      4440
ggcaagtgca caagccacat aaacctgaat aaaactttg acctagggtt gaa             4493
```

<210> 10

<211> 1270
<212> PRT
<213> Homo sapiens

<400> 10

Met Gly Asp Val Lys Asn Phe Leu Tyr Ala Trp Cys Gly Lys Arg Lys
1 5 10 15

Met Thr Pro Ser Tyr Glu Ile Arg Ala Val Gly Asn Lys Asn Arg Gln
20 25 30

Lys Phe Met Cys Glu Val Gln Val Glu Gly Tyr Asn Tyr Thr Gly Met
35 40 45

Gly Asn Ser Thr Asn Lys Lys Asp Ala Gln Ser Asn Ala Ala Arg Asp
50 55 60

Phe Val Asn Tyr Leu Val Arg Ile Asn Glu Ile Lys Ser Glu Glu Val
65 70 75 80

Pro Ala Phe Gly Val Ala Ser Pro Pro Pro Leu Thr Asp Thr Pro Asp
85 90 95

Thr Thr Ala Asn Ala Glu Gly Asp Leu Pro Thr Thr Met Gly Gly Pro
100 105 110

Leu Pro Pro His Leu Ala Leu Lys Ala Glu Asn Asn Ser Glu Val Gly
115 120 125

Ala Ser Gly Tyr Gly Val Pro Gly Pro Thr Trp Asp Arg Gly Ala Asn
130 135 140

Leu Lys Asp Tyr Tyr Ser Arg Lys Glu Glu Gln Glu Val Gln Ala Thr
145 150 155 160

Leu Glu Ser Glu Glu Val Asp Leu Asn Ala Gly Leu His Gly Asn Trp
165 170 175

Thr Leu Glu Asn Ala Lys Ala Arg Leu Asn Gln Tyr Phe Gln Lys Glu
180 185 190

Lys Ile Gln Gly Glu Tyr Lys Tyr Thr Gln Val Gly Pro Asp His Asn
195 200 205

Arg Ser Phe Ile Ala Glu Met Thr Ile Tyr Ile Lys Gln Leu Gly Arg
210 215 220

Arg Ile Phe Ala Arg Glu His Gly Ser Asn Lys Lys Leu Ala Ala Gln

225 230 235 240

Ser Cys Ala Leu Ser Leu Val Arg Gln Leu Tyr His Leu Gly Val Val
245 250 255

Glu Ala Tyr Ser Gly Leu Thr Lys Lys Lys Glu Gly Glu Thr Val Glu
260 265 270

Pro Tyr Lys Val Asn Leu Ser Gln Asp Leu Glu His Gln Leu Gln Asn
275 280 285

Ile Ile Gln Glu Leu Asn Leu Glu Ile Leu Pro Pro Pro Glu Asp Pro
290 295 300

Ser Val Pro Val Ala Leu Asn Ile Gly Lys Leu Ala Gln Phe Glu Pro
305 310 315 320

Ser Gln Arg Gln Asn Gln Val Gly Val Val Pro Trp Ser Pro Pro Gln
325 330 335

Ser Asn Trp Asn Pro Trp Thr Ser Ser Asn Ile Asp Glu Gly Pro Leu
340 345 350

Ala Phe Ala Thr Pro Glu Gln Ile Ser Met Asp Leu Lys Asn Glu Leu
355 360 365

Met Tyr Gln Leu Glu Gln Asp His Asp Leu Gln Ala Ile Leu Gln Glu
370 375 380

Arg Glu Leu Leu Pro Val Lys Lys Phe Glu Ser Glu Ile Leu Glu Ala
385 390 395 400

Ile Ser Gln Asn Ser Val Val Ile Ile Arg Gly Ala Thr Gly Cys Gly
405 410 415

Lys Thr Thr Gln Val Pro Gln Phe Ile Leu Asp Asp Phe Ile Gln Asn
420 425 430

Asp Arg Ala Ala Glu Cys Asn Ile Val Val Thr Gln Pro Arg Arg Ile
435 440 445

Ser Ala Val Ser Val Ala Glu Arg Val Ala Phe Glu Arg Gly Glu Glu
450 455 460

Pro Gly Lys Ser Cys Gly Tyr Ser Val Arg Phe Glu Ser Ile Leu Pro
465 470 475 480

Arg Pro His Ala Ser Ile Met Phe Cys Thr Val Gly Val Leu Leu Arg
485 490 495

Lys Leu Glu Ala Gly Ile Arg Gly Ile Ser His Val Ile Val Asp Glu
500 505 510

Ile His Glu Arg Asp Ile Asn Thr Asp Phe Leu Leu Val Val Leu Arg
515 520 525

Asp Val Val Gln Ala Tyr Pro Glu Val Arg Ile Val Leu Met Ser Ala
530 535 540

Thr Ile Asp Thr Ser Met Phe Cys Glu Tyr Phe Phe Asn Cys Pro Ile
545 550 555 560

Ile Glu Val Tyr Gly Arg Thr Tyr Pro Val Gln Glu Tyr Phe Leu Glu
565 570 575

Asp Cys Ile Gln Met Thr His Phe Val Pro Pro Pro Lys Asp Lys Lys
580 585 590

Lys Lys Asp Lys Asp Asp Asp Gly Gly Glu Asp Asp Asp Ala Asn Cys
595 600 605

Asn Leu Ile Cys Gly Asp Glu Tyr Gly Pro Glu Thr Arg Leu Ser Met
610 615 620

Ser Gln Leu Asn Glu Lys Glu Thr Pro Phe Glu Leu Ile Glu Ala Leu
625 630 635 640

Leu Lys Tyr Ile Glu Thr Leu Asn Val Pro Gly Ala Val Leu Val Phe
645 650 655

Leu Pro Gly Trp Asn Leu Ile Tyr Thr Met Gln Lys His Leu Glu Met
660 665 670

Asn Pro His Phe Gly Ser His Arg Tyr Gln Ile Leu Pro Leu His Ser
675 680 685

Gln Ile Pro Arg Glu Glu Gln Arg Lys Val Phe Asp Pro Val Pro Val
690 695 700

Gly Val Thr Lys Val Ile Leu Ser Thr Asn Ile Ala Glu Thr Ser Ile
705 710 715 720

Thr Ile Asn Asp Val Val Tyr Val Ile Asp Ser Cys Lys Gln Lys Val
725 730 735

Lys Leu Phe Thr Ala His Asn Asn Met Thr Asn Tyr Ala Thr Val Trp
740 745 750

Ala Ser Lys Thr Asn Leu Glu Gln Arg Lys Gly Arg Ala Gly Arg Val
755 760 765

Arg Pro Gly Phe Cys Phe His Leu Cys Ser Arg Ala Arg Phe Glu Arg
770 775 780

Leu Glu Thr His Met Thr Pro Glu Met Phe Arg Thr Pro Leu His Glu
785 790 795 800

Ile Ala Leu Ser Ile Lys Leu Leu Arg Leu Gly Gly Ile Gly Gln Phe
805 810 815

Leu Ala Lys Ala Ile Glu Pro Pro Pro Leu Asp Ala Val Ile Glu Ala
820 825 830

Glu His Thr Leu Arg Glu Leu Asp Ala Leu Asp Ala Asn Asp Glu Leu
835 840 845

Thr Pro Leu Gly Arg Ile Leu Ala Lys Leu Pro Ile Glu Pro Arg Phe
850 855 860

Gly Lys Met Met Ile Met Gly Cys Ile Phe Tyr Val Gly Asp Ala Ile
865 870 875 880

Cys Thr Ile Ala Ala Ala Thr Cys Phe Pro Glu Pro Phe Ile Asn Glu
885 890 895

Gly Lys Arg Leu Gly Tyr Ile His Arg Asn Phe Ala Gly Asn Arg Phe
900 905 910

Ser Asp His Val Ala Leu Leu Ser Val Phe Gln Ala Trp Asp Asp Ala
915 920 925

Arg Met Gly Gly Glu Glu Ala Glu Ile Arg Phe Cys Glu His Lys Arg
930 935 940

Leu Asn Met Ala Thr Leu Arg Met Thr Trp Glu Ala Lys Val Gln Leu
945 950 955 960

Lys Glu Ile Leu Ile Asn Ser Gly Phe Pro Glu Asp Cys Leu Leu Thr
965 970 975

Gln Val Phe Thr Asn Thr Gly Pro Asp Asn Asn Leu Asp Val Val Ile
980 985 990

Ser Leu Leu Ala Phe Gly Val Tyr Pro Asn Val Cys Tyr His Lys Glu
995 1000 1005

Lys Arg Lys Ile Leu Thr Thr Glu Gly Arg Asn Ala Leu Ile His
1010 1015 1020

Lys Ser Ser Val Asn Cys Pro Phe Ser Ser Gln Asp Met Lys Tyr
1025 1030 1035

Pro Ser Pro Phe Phe Val Phe Gly Glu Lys Ile Arg Thr Arg Ala
1040 1045 1050

Ile Ser Ala Lys Gly Met Thr Leu Val Thr Pro Leu Gln Leu Leu
1055 1060 1065

Leu Phe Ala Ser Lys Lys Val Gln Ser Asp Gly Gln Ile Val Leu
1070 1075 1080

Val Asp Asp Trp Ile Lys Leu Gln Ile Ser His Glu Ala Ala Ala
1085 1090 1095

Cys Ile Thr Gly Leu Arg Ala Ala Met Glu Ala Leu Val Val Glu
1100 1105 1110

Val Thr Lys Gln Pro Ala Ile Ile Ser Gln Leu Asp Pro Val Asn
1115 1120 1125

Glu Arg Met Leu Asn Met Ile Arg Gln Ile Ser Arg Pro Ser Ala
1130 1135 1140

Ala Gly Ile Asn Leu Met Ile Gly Ser Thr Arg Tyr Gly Asp Gly
1145 1150 1155

Pro Arg Pro Pro Lys Met Ala Arg Tyr Asp Asn Gly Ser Gly Tyr
1160 1165 1170

Arg Arg Gly Gly Ser Ser Tyr Ser Gly Gly Gly Tyr Gly Gly Gly
1175 1180 1185

Tyr Ser Ser Gly Gly Tyr Gly Ser Gly Gly Tyr Gly Gly Ser Ala
1190 1195 1200

Asn Ser Phe Arg Ala Gly Tyr Gly Ala Gly Val Gly Gly Gly Tyr
1205 1210 1215

Arg Gly Val Ser Arg Gly Gly Phe Arg Gly Asn Ser Gly Gly Asp

```
    1220                1225                1230

Tyr Arg  Gly Pro Ser Gly Gly  Tyr Arg Gly Ser Gly  Gly Phe Gln
    1235                1240                1245

Arg Gly  Gly Gly Arg Gly Ala  Tyr Gly Thr Gly Tyr  Phe Gly Gln
    1250                1255                1260

Gly Arg  Gly Gly Gly Gly Tyr
    1265                1270
```

<210> 11
<211> 2750
<212> DNA
<213> Homo sapiens

<400> 11

```
attgtttcct actccatttt ctctggggca atagcagaat aggagcaagc cagcactagt      60

cagctaacta agtgactcaa ccaaggcctt ttttccttgt tatctttgca gatacttcat     120

tttcttagcg tttctggaga ttacaacatc ctgcggttcc gtttctggga actttactga     180

tttatctccc ccctcacaca aataagcatt gattcctgca tttctgaaga tctcaagatc     240

tggactactg ttgaaaaaat ttccagtgag gctcacttat gtctgtaaag atgggaaaaa     300

aatacaagaa cattgttcta ctaaaaggat tagaggtcat caatgattat cattttagaa     360

tggttaagtc cttactgagc aacgatttaa aacttaattt aaaaatgaga gaagagtatg     420

acaaaattca gattgctgac ttgatggaag aaaagttccg aggtgatgct ggtttgggca     480

aactaataaa aattttcgaa gatataccaa cgcttgaaga cctggctgaa actcttaaaa     540

aagaaaagtt aaaagtaaaa ggaccagccc tatcaagaaa gaggaagaag gaagtggatg     600

ctacttcacc tgcaccctcc acaagcagca ctgtcaaaac tgaaggagca gaggcaactc     660

ctggagctca gaaaagaaaa aaatcaacca aagaaaaggc tggacccaaa gggagtaagg     720

tgtccgagga acagactcag cctccctctc ctgcaggagc cggcatgtcc acagccatgg     780

gccgttcccc atctcccaag acctcattgt cagctccacc caacagttct tcaactgaga     840

acccgaaaac agtggccaaa tgtcaggtaa ctcccagaag aaatgttctc caaaaacgcc     900

cagtgatagt gaaggtactg agtacaacaa agccatttga atatgagacc ccagaaatgg     960

agaaaaaaat aatgtttcat gctacagtgg ctacacagac acagttcttc catgtgaagg    1020

ttttaaacac cagcttgaag gagaaattca atggaaagaa aatcatcatc atatcagatt    1080

atttggaata tgatagtctc ctagaggtca atgaagaatc tactgtatct gaagctggtc    1140

ctaaccaaac gtttgaggtt ccaaataaaa tcatcaacag agcaaaggaa actctgaaga    1200

ttgatattct tcacaaacaa gcttcaggaa atattgtata tggggtattt atgctacata    1260
```

```
agaaaacagt aaatcagaag accacaatct acgaaattca ggatgataga ggaaaaatgg    1320
atgtagtggg gacaggacaa tgtcacaata tcccctgtga agaaggagat aagctccaac    1380
ttttctgctt tcgacttaga aaaaagaacc agatgtcaaa actgatttca gaaatgcata    1440
gttttatcca gataaagaaa aaaacaaacc cgagaaacaa tgaccccaag agcatgaagc    1500
taccccagga acagcgtcag cttccatatc cttcagaggc cagcacaacc ttccctgaga    1560
gccatcttcg gactcctcag atgccaccaa caactccatc cagcagtttc ttcaccaaga    1620
aaagtgaaga cacaatctcc aaaatgaatg acttcatgag gatgcagata ctgaaggaag    1680
ggagtcattt tccaggaccg ttcatgacca gcataggccc agctgagagc catccccaca    1740
ctcctcagat gcctccatca acaccaagca gcagtttctt aaccacgttg aaaccaagac    1800
tgaagactga acctgaagaa gtttccatag aagacagtgc ccagagtgac ctcaaagaag    1860
tgatggtgct gaacgcaaca gaatcatttg tatatgagcc caaagagcag aagaaaatgt    1920
ttcatgccac agtggcaact gagaatgaag tcttccgagt gaaggttttt aatattgacc    1980
taaaggagaa gttcacccca aagaagatca ttgccatagc aaattatgtt tgccgcaatg    2040
ggttcctgga ggtatatcct ttcacacttg tggctgatgt gaatgctgac cgaaacatgg    2100
agatcccaaa aggattgatt agaagtgcca gcgtaactcc taaaatcaat cagctttgct    2160
cacaaactaa aggaagtttt gtgaatgggg tgtttgaggt acataagaaa aatgtaaggg    2220
gtgaattcac ttattatgaa atacaagata atacagggaa gatggaagtg gtggtgcatg    2280
gacgactgac cacaatcaac tgtgaggaag gagataaact gaaactcacc tgctttgaat    2340
tggcaccgaa aagtgggaat accggggagt tgagatctgt aattcatagt cacatcaagg    2400
tcatcaagac caggaaaaac aagaaagaca tactcaatcc tgattcaagt atggaaactt    2460
caccagactt tttcttctaa aatctggatg tcattgacga taatgtttat ggagataagg    2520
tctaagtgcc taaaaaaatg tacatatacc tggttgaaat acaacactat acatacacac    2580
caccatatat actagctgtt aatcctatgg aatggggtat tgggagtgct tttttaattt    2640
ttcatagttt ttttttaata aaatggcata ttttgcatct acaacttcta taatttgaaa    2700
aaataaataa acattatctt ttttgtgaaa ggaaaaaaaa aaaaaaaaaa               2750
```

<210> 12
<211> 729
<212> PRT
<213> Homo sapiens

<400> 12

```
Met Gly Lys Lys Tyr Lys Asn Ile Val Leu Leu Lys Gly Leu Glu Val
1               5                   10                  15

Ile Asn Asp Tyr His Phe Arg Met Val Lys Ser Leu Leu Ser Asn Asp
```

20 25 30

Leu Lys Leu Asn Leu Lys Met Arg Glu Glu Tyr Asp Lys Ile Gln Ile
35 40 45

Ala Asp Leu Met Glu Glu Lys Phe Arg Gly Asp Ala Gly Leu Gly Lys
50 55 60

Leu Ile Lys Ile Phe Glu Asp Ile Pro Thr Leu Glu Asp Leu Ala Glu
65 70 75 80

Thr Leu Lys Lys Glu Lys Leu Lys Val Lys Gly Pro Ala Leu Ser Arg
85 90 95

Lys Arg Lys Lys Glu Val Asp Ala Thr Ser Pro Ala Pro Ser Thr Ser
100 105 110

Ser Thr Val Lys Thr Glu Gly Ala Glu Ala Thr Pro Gly Ala Gln Lys
115 120 125

Arg Lys Lys Ser Thr Lys Glu Lys Ala Gly Pro Lys Gly Ser Lys Val
130 135 140

Ser Glu Glu Gln Thr Gln Pro Pro Ser Pro Ala Gly Ala Gly Met Ser
145 150 155 160

Thr Ala Met Gly Arg Ser Pro Ser Pro Lys Thr Ser Leu Ser Ala Pro
165 170 175

Pro Asn Ser Ser Ser Thr Glu Asn Pro Lys Thr Val Ala Lys Cys Gln
180 185 190

Val Thr Pro Arg Arg Asn Val Leu Gln Lys Arg Pro Val Ile Val Lys
195 200 205

Val Leu Ser Thr Thr Lys Pro Phe Glu Tyr Glu Thr Pro Glu Met Glu
210 215 220

Lys Lys Ile Met Phe His Ala Thr Val Ala Thr Gln Thr Gln Phe Phe
225 230 235 240

His Val Lys Val Leu Asn Thr Ser Leu Lys Glu Lys Phe Asn Gly Lys
245 250 255

Lys Ile Ile Ile Ile Ser Asp Tyr Leu Glu Tyr Asp Ser Leu Leu Glu
260 265 270

Val Asn Glu Glu Ser Thr Val Ser Glu Ala Gly Pro Asn Gln Thr Phe
275 280 285

Glu Val Pro Asn Lys Ile Ile Asn Arg Ala Lys Glu Thr Leu Lys Ile
290 295 300

Asp Ile Leu His Lys Gln Ala Ser Gly Asn Ile Val Tyr Gly Val Phe
305 310 315 320

Met Leu His Lys Lys Thr Val Asn Gln Lys Thr Thr Ile Tyr Glu Ile
325 330 335

Gln Asp Asp Arg Gly Lys Met Asp Val Val Gly Thr Gly Gln Cys His
340 345 350

Asn Ile Pro Cys Glu Glu Gly Asp Lys Leu Gln Leu Phe Cys Phe Arg
355 360 365

Leu Arg Lys Lys Asn Gln Met Ser Lys Leu Ile Ser Glu Met His Ser
370 375 380

Phe Ile Gln Ile Lys Lys Lys Thr Asn Pro Arg Asn Asn Asp Pro Lys
385 390 395 400

Ser Met Lys Leu Pro Gln Glu Gln Arg Gln Leu Pro Tyr Pro Ser Glu
405 410 415

Ala Ser Thr Thr Phe Pro Glu Ser His Leu Arg Thr Pro Gln Met Pro
420 425 430

Pro Thr Thr Pro Ser Ser Ser Phe Phe Thr Lys Lys Ser Glu Asp Thr
435 440 445

Ile Ser Lys Met Asn Asp Phe Met Arg Met Gln Ile Leu Lys Glu Gly
450 455 460

Ser His Phe Pro Gly Pro Phe Met Thr Ser Ile Gly Pro Ala Glu Ser
465 470 475 480

His Pro His Thr Pro Gln Met Pro Pro Ser Thr Pro Ser Ser Ser Phe
485 490 495

Leu Thr Thr Leu Lys Pro Arg Leu Lys Thr Glu Pro Glu Glu Val Ser
500 505 510

Ile Glu Asp Ser Ala Gln Ser Asp Leu Lys Glu Val Met Val Leu Asn
515 520 525

Ala Thr Glu Ser Phe Val Tyr Glu Pro Lys Glu Gln Lys Lys Met Phe
530 535 540

His Ala Thr Val Ala Thr Glu Asn Glu Val Phe Arg Val Lys Val Phe
545 550 555 560

Asn Ile Asp Leu Lys Glu Lys Phe Thr Pro Lys Lys Ile Ile Ala Ile
565 570 575

Ala Asn Tyr Val Cys Arg Asn Gly Phe Leu Glu Val Tyr Pro Phe Thr
580 585 590

Leu Val Ala Asp Val Asn Ala Asp Arg Asn Met Glu Ile Pro Lys Gly
595 600 605

Leu Ile Arg Ser Ala Ser Val Thr Pro Lys Ile Asn Gln Leu Cys Ser
610 615 620

Gln Thr Lys Gly Ser Phe Val Asn Gly Val Phe Glu Val His Lys Lys
625 630 635 640

Asn Val Arg Gly Glu Phe Thr Tyr Tyr Glu Ile Gln Asp Asn Thr Gly
645 650 655

Lys Met Glu Val Val Val His Gly Arg Leu Thr Thr Ile Asn Cys Glu
660 665 670

Glu Gly Asp Lys Leu Lys Leu Thr Cys Phe Glu Leu Ala Pro Lys Ser
675 680 685

Gly Asn Thr Gly Glu Leu Arg Ser Val Ile His Ser His Ile Lys Val
690 695 700

Ile Lys Thr Arg Lys Asn Lys Lys Asp Ile Leu Asn Pro Asp Ser Ser
705 710 715 720

Met Glu Thr Ser Pro Asp Phe Phe Phe
725

<210> 13
<211> 3617
<212> DNA
<213> Homo sapiens

<400> 13
atcgaaacag aaaccaaagt caggcaaact ctgtaagaac tgcctgacag aaagctggac 60

tcaaagctcc tacccgagtg tgcagcagga tcgccccggt ccgggacccc aggcgcacac 120

cgcagagtcc aaagtgccgc gcctgccggc cgcacctgcc tgccgcggcc ccgcgcgccg 180
ccccgctgcc cacctgcccg cctgcccacc tgcccaggtg cgagtgcagc cccgcgcgcc 240
ggcctgagag ccctgtggac aacctcgtca ttgtcaggca cagagcggta gaccctgctt 300
ctctaagtgg gcagcggaca gcggcacgca catttcacct gtcccgcaga caacagcacc 360
atctgcttgg gagaaccctc tcccttctct gagaaagaaa gatgtcgaat gggtattcca 420
cagacgagaa tttccgctat ctcatctcgt gcttcagggc cagggtgaaa atgtacatcc 480
aggtggagcc tgtgctggac tacctgacct ttctgcctgc agaggtgaag gagcagattc 540
agaggacagt cgccacctcc gggaacatgc aggcagttga actgctgctg agcaccttgg 600
agaagggagt ctggcacctt ggttggactc gggaattcgt ggaggccctc cggagaaccg 660
gcagccctct ggccgcccgc tacatgaacc ctgagctcac ggacttgccc tctccatcgt 720
ttgagaacgc tcatgatgaa tatctccaac tgctgaacct ccttcagccc actctggtgg 780
acaagcttct agttagagac gtcttggata agtgcatgga ggaggaactg ttgacaattg 840
aagacagaaa ccggattgct gctgcagaaa acaatggaaa tgaatcaggt gtaagagagc 900
tactaaaaag gattgtgcag aaagaaaact ggttctctgc atttctgaat gttcttcgtc 960
aaacaggaaa caatgaactt gtccaagagt taacaggctc tgattgctca gaaagcaatg 1020
cagagattga gaatttatca caagttgatg gtcctcaagt ggaagagcaa cttctttcaa 1080
ccacagttca gccaaatctg gagaaggagg tctggggcat ggagaataac tcatcagaat 1140
catcttttgc agattcttct gtagtttcag aatcagacac aagtttggca gaaggaagtg 1200
tcagctgctt agatgaaagt cttggacata acagcaacat gggcagtgat tcaggcacca 1260
tgggaagtga ttcagatgaa gagaatgtgg cagcaagagc atccccggag ccagaactcc 1320
agctcaggcc ttaccaaatg gaagttgccc agccagcctt ggaagggaag aatatcatca 1380
tctgcctccc tacagggagt ggaaaaacca gagtggctgt ttacattgcc aaggatcact 1440
tagacaagaa gaaaaaagca tctgagcctg gaaaagttat agttcttgtc aataaggtac 1500
tgctagttga acagctcttc cgcaaggagt tccaaccatt tttgaagaaa tggtatcgtg 1560
ttattggatt aagtggtgat acccaactga aaatatcatt tccagaagtt gtcaagtcct 1620
gtgatattat tatcagtaca gctcaaatcc ttgaaaactc cctcttaaac ttggaaaatg 1680
gagaagatgc tggtgttcaa ttgtcagact tttccctcat tatcattgat gaatgtcatc 1740
acaccaacaa agaagcagtg tataataaca tcatgaggca ttatttgatg cagaagttga 1800
aaaacaatag actcaagaaa gaaaacaaac cagtgattcc ccttcctcag atactgggac 1860
taacagcttc acctggtgtt ggaggggcca cgaagcaagc caaagctgaa gaacacattt 1920
taaaactatg tgccaatctt gatgcattta ctattaaaac tgttaaagaa aaccttgatc 1980
aactgaaaaa ccaaatacag gagccatgca agaagtttgc cattgcagat gcaaccagag 2040

aagatccatt taaagagaaa cttctagaaa taatgacaag gattcaaact tattgtcaaa 2100

tgagtccaat gtcagatttt ggaactcaac cctatgaaca atgggccatt caaatggaaa 2160

aaaaagctgc aaaagaagga aatcgcaaag aacgtgtttg tgcagaacat ttgaggaagt 2220

acaatgaggc cctacaaatt aatgacacaa ttcgaatgat agatgcgtat actcatcttg 2280

aaactttcta taatgaagag aaagataaga agtttgcagt catagaagat gatagtgatg 2340

agggtggtga tgatgagtat tgtgatggtg atgaagatga ggatgattta aagaaacctt 2400

tgaaactgga tgaaacagat agatttctca tgactttatt ttttgaaaac aataaaatgt 2460

tgaaaaggct ggctgaaaac ccagaatatg aaaatgaaaa gctgaccaaa ttaagaaata 2520

ccataatgga gcaatatact aggactgagg aatcagcacg aggaataatc tttacaaaaa 2580

cacgacagag tgcatatgcg ctttcccagt ggattactga aaatgaaaaa tttgctgaag 2640

taggagtcaa agcccaccat ctgattggag ctggacacag cagtgagttc aaacccatga 2700

cacagaatga acaaaaagaa gtcattagta aatttcgcac tggaaaaata aatctgctta 2760

tcgctaccac agtggcagaa gaaggtctgg atattaaaga atgtaacatt gttatccgtt 2820

atggtctcgt caccaatgaa atagccatgg tccaggcccg tggtcgagcc agagctgatg 2880

agagcaccta cgtcctggtt gctcacagtg gttcaggagt tatcgaacat gagacagtta 2940

atgatttccg agagaagatg atgtataaag ctatacattg tgttcaaaat atgaaaccag 3000

aggagtatgc tcataagatt ttggaattac agatgcaaag tataatggaa aagaaaatga 3060

aaaccaagag aaatattgcc aagcattaca agaataaccc atcactaata actttccttt 3120

gcaaaaactg cagtgtgcta gcctgttctg gggaagatat ccatgtaatt gagaaaatgc 3180

atcacgtcaa tatgacccca gaattcaagg aactttacat tgtaagagaa aacaaagcac 3240

tgcaaaagaa gtgtgccgac tatcaaataa atggtgaaat catctgcaaa tgtggccagg 3300

cttggggaac aatgatggtg cacaaaggct tagatttgcc ttgtctcaaa ataaggaatt 3360

ttgtagtggt tttcaaaaat aattcaacaa agaaacaata caaaaagtgg gtagaattac 3420

ctatcacatt tcccaatctt gactattcag aatgctgttt atttagtgat gaggattagc 3480

acttgattga agattctttt aaaatactat cagttaaaca tttaatatga ttatgattaa 3540

tgtattcatt atgctacaga actgacataa gaatcaataa aatgattgtt ttactctgca 3600

aaaaaaaaaa aaaaaaa 3617

<210> 14
<211> 1025
<212> PRT
<213> Homo sapiens

<400> 14

```
Met Ser Asn Gly Tyr Ser Thr Asp Glu Asn Phe Arg Tyr Leu Ile Ser
1               5                   10                  15

Cys Phe Arg Ala Arg Val Lys Met Tyr Ile Gln Val Glu Pro Val Leu
            20                  25                  30

Asp Tyr Leu Thr Phe Leu Pro Ala Glu Val Lys Glu Gln Ile Gln Arg
        35                  40                  45

Thr Val Ala Thr Ser Gly Asn Met Gln Ala Val Glu Leu Leu Leu Ser
    50                  55                  60

Thr Leu Glu Lys Gly Val Trp His Leu Gly Trp Thr Arg Glu Phe Val
65                  70                  75                  80

Glu Ala Leu Arg Arg Thr Gly Ser Pro Leu Ala Ala Arg Tyr Met Asn
                85                  90                  95

Pro Glu Leu Thr Asp Leu Pro Ser Pro Ser Phe Glu Asn Ala His Asp
            100                 105                 110

Glu Tyr Leu Gln Leu Leu Asn Leu Leu Gln Pro Thr Leu Val Asp Lys
        115                 120                 125

Leu Leu Val Arg Asp Val Leu Asp Lys Cys Met Glu Glu Glu Leu Leu
    130                 135                 140

Thr Ile Glu Asp Arg Asn Arg Ile Ala Ala Ala Glu Asn Asn Gly Asn
145                 150                 155                 160

Glu Ser Gly Val Arg Glu Leu Leu Lys Arg Ile Val Gln Lys Glu Asn
                165                 170                 175

Trp Phe Ser Ala Phe Leu Asn Val Leu Arg Gln Thr Gly Asn Asn Glu
            180                 185                 190

Leu Val Gln Glu Leu Thr Gly Ser Asp Cys Ser Glu Ser Asn Ala Glu
        195                 200                 205

Ile Glu Asn Leu Ser Gln Val Asp Gly Pro Gln Val Glu Glu Gln Leu
    210                 215                 220

Leu Ser Thr Thr Val Gln Pro Asn Leu Glu Lys Glu Val Trp Gly Met
225                 230                 235                 240

Glu Asn Asn Ser Ser Glu Ser Ser Phe Ala Asp Ser Ser Val Val Ser
                245                 250                 255
```

Glu Ser Asp Thr Ser Leu Ala Glu Gly Ser Val Ser Cys Leu Asp Glu
260 265 270

Ser Leu Gly His Asn Ser Asn Met Gly Ser Asp Ser Gly Thr Met Gly
275 280 285

Ser Asp Ser Asp Glu Glu Asn Val Ala Ala Arg Ala Ser Pro Glu Pro
290 295 300

Glu Leu Gln Leu Arg Pro Tyr Gln Met Glu Val Ala Gln Pro Ala Leu
305 310 315 320

Glu Gly Lys Asn Ile Ile Ile Cys Leu Pro Thr Gly Ser Gly Lys Thr
325 330 335

Arg Val Ala Val Tyr Ile Ala Lys Asp His Leu Asp Lys Lys Lys Lys
340 345 350

Ala Ser Glu Pro Gly Lys Val Ile Val Leu Val Asn Lys Val Leu Leu
355 360 365

Val Glu Gln Leu Phe Arg Lys Glu Phe Gln Pro Phe Leu Lys Lys Trp
370 375 380

Tyr Arg Val Ile Gly Leu Ser Gly Asp Thr Gln Leu Lys Ile Ser Phe
385 390 395 400

Pro Glu Val Val Lys Ser Cys Asp Ile Ile Ile Ser Thr Ala Gln Ile
405 410 415

Leu Glu Asn Ser Leu Leu Asn Leu Glu Asn Gly Glu Asp Ala Gly Val
420 425 430

Gln Leu Ser Asp Phe Ser Leu Ile Ile Ile Asp Glu Cys His His Thr
435 440 445

Asn Lys Glu Ala Val Tyr Asn Asn Ile Met Arg His Tyr Leu Met Gln
450 455 460

Lys Leu Lys Asn Asn Arg Leu Lys Lys Glu Asn Lys Pro Val Ile Pro
465 470 475 480

Leu Pro Gln Ile Leu Gly Leu Thr Ala Ser Pro Gly Val Gly Gly Ala
485 490 495

Thr Lys Gln Ala Lys Ala Glu Glu His Ile Leu Lys Leu Cys Ala Asn
500 505 510

Leu Asp Ala Phe Thr Ile Lys Thr Val Lys Glu Asn Leu Asp Gln Leu
515 520 525

Lys Asn Gln Ile Gln Glu Pro Cys Lys Lys Phe Ala Ile Ala Asp Ala
530 535 540

Thr Arg Glu Asp Pro Phe Lys Glu Lys Leu Leu Glu Ile Met Thr Arg
545 550 555 560

Ile Gln Thr Tyr Cys Gln Met Ser Pro Met Ser Asp Phe Gly Thr Gln
565 570 575

Pro Tyr Glu Gln Trp Ala Ile Gln Met Glu Lys Lys Ala Ala Lys Glu
580 585 590

Gly Asn Arg Lys Glu Arg Val Cys Ala Glu His Leu Arg Lys Tyr Asn
595 600 605

Glu Ala Leu Gln Ile Asn Asp Thr Ile Arg Met Ile Asp Ala Tyr Thr
610 615 620

His Leu Glu Thr Phe Tyr Asn Glu Glu Lys Asp Lys Lys Phe Ala Val
625 630 635 640

Ile Glu Asp Asp Ser Asp Glu Gly Gly Asp Asp Glu Tyr Cys Asp Gly
645 650 655

Asp Glu Asp Glu Asp Asp Leu Lys Lys Pro Leu Lys Leu Asp Glu Thr
660 665 670

Asp Arg Phe Leu Met Thr Leu Phe Phe Glu Asn Asn Lys Met Leu Lys
675 680 685

Arg Leu Ala Glu Asn Pro Glu Tyr Glu Asn Glu Lys Leu Thr Lys Leu
690 695 700

Arg Asn Thr Ile Met Glu Gln Tyr Thr Arg Thr Glu Glu Ser Ala Arg
705 710 715 720

Gly Ile Ile Phe Thr Lys Thr Arg Gln Ser Ala Tyr Ala Leu Ser Gln
725 730 735

Trp Ile Thr Glu Asn Glu Lys Phe Ala Glu Val Gly Val Lys Ala His
740 745 750

His Leu Ile Gly Ala Gly His Ser Ser Glu Phe Lys Pro Met Thr Gln

755 760 765

Asn Glu Gln Lys Glu Val Ile Ser Lys Phe Arg Thr Gly Lys Ile Asn
770 775 780

Leu Leu Ile Ala Thr Thr Val Ala Glu Glu Gly Leu Asp Ile Lys Glu
785 790 795 800

Cys Asn Ile Val Ile Arg Tyr Gly Leu Val Thr Asn Glu Ile Ala Met
805 810 815

Val Gln Ala Arg Gly Arg Ala Arg Ala Asp Glu Ser Thr Tyr Val Leu
820 825 830

Val Ala His Ser Gly Ser Gly Val Ile Glu His Glu Thr Val Asn Asp
835 840 845

Phe Arg Glu Lys Met Met Tyr Lys Ala Ile His Cys Val Gln Asn Met
850 855 860

Lys Pro Glu Glu Tyr Ala His Lys Ile Leu Glu Leu Gln Met Gln Ser
865 870 875 880

Ile Met Glu Lys Lys Met Lys Thr Lys Arg Asn Ile Ala Lys His Tyr
885 890 895

Lys Asn Asn Pro Ser Leu Ile Thr Phe Leu Cys Lys Asn Cys Ser Val
900 905 910

Leu Ala Cys Ser Gly Glu Asp Ile His Val Ile Glu Lys Met His His
915 920 925

Val Asn Met Thr Pro Glu Phe Lys Glu Leu Tyr Ile Val Arg Glu Asn
930 935 940

Lys Ala Leu Gln Lys Lys Cys Ala Asp Tyr Gln Ile Asn Gly Glu Ile
945 950 955 960

Ile Cys Lys Cys Gly Gln Ala Trp Gly Thr Met Met Val His Lys Gly
965 970 975

Leu Asp Leu Pro Cys Leu Lys Ile Arg Asn Phe Val Val Val Phe Lys
980 985 990

Asn Asn Ser Thr Lys Lys Gln Tyr Lys Lys Trp Val Glu Leu Pro Ile
995 1000 1005

Thr Phe Pro Asn Leu Asp Tyr Ser Glu Cys Cys Leu Phe Ser Asp
1010 1015 1020

Glu Asp
1025

<210> 15
<211> 4411
<212> DNA
<213> Homo sapiens

<400> 15
aactgaaaat ccacaagaca gaatagccag atctcagagg agcctggcta agcaaaaccc 60

tgcagaacgg ctgcctaatt tacagcaacc atgaggccac ttaaggatgc agcaagaagg 120

agccatctgc aatccaggaa gaaattcctt gccaggaacc aaattggttg tcaccttcat 180

ctaggacttc tagcctcgag aacttacaaa tggtgatgat catcaggtca aggatagtct 240

ggagcaattg agatgtcact ttacatggga gttatccatt gatgacgatg aaatgcctga 300

tttagaaaac agagtcttgg atcagattga attcctagac accaaataca gtgtgggaat 360

acacaaccta ctagcctatg tgaaacacct gaaaggccag aatgaggaag ccctgaagag 420

cttaaaagaa gctgaaaact taatgcagga agaacatgac aaccaagcaa atgtgaggag 480

tctggtgacc tggggcaact ttgcctggat gtattaccac atgggcagac tggcagaagc 540

ccagacttac ctggacaagg tggagaacat ttgcaagaag ctttcaaatc ccttccgcta 600

tagaatggag tgtccagaaa tagactgtga ggaaggatgg gccttgctga agtgtggagg 660

aaaaaattat gaacgggcca aggcctgctt tgaaaaggtg cttgaagtgg accctgaaaa 720

ccctgaatcc agcgctgggt atgcgatctc tgcctatcgc ctggatggct ttaaattagc 780

cacaaaaaat cacaagccat ttctttgct tcccctaagg caggctgtcc gcttaaatcc 840

agacaatgga tatattaagg ttctccttgc cctgaagctt caggatgaag gacaggaagc 900

tgaaggagaa aagtacattg aagaagctct agccaacatg tcctcacaga cctatgtctt 960

tcgatatgca gccaagtttt accgaagaaa aggctctgtg gataaagctc ttgagttatt 1020

aaaaaaggcc ttgcaggaaa cacccacttc tgtcttactg catcaccaga tagggctttg 1080

ctacaaggca caaatgatcc aaatcaagga ggctacaaaa gggcagccta gagggcagaa 1140

cagagaaaag ctagacaaaa tgataagatc agccatattt catttgaat ctgcagtgga 1200

aaaaaagccc acatttgagg tggctcatct agacctggca agaatgtata tagaagcagg 1260

caatcacaga aaagctgaag agaattttca aaaattgtta tgcatgaaac cagtggtaga 1320

agaaacaatg caagacatac atttccacta tggtcggttt caggaatttc aaaagaaatc 1380

tgacgtcaat gcaattatcc attatttaaa agctataaaa atagaacagg catcattaac 1440

aagggataaa agtatcaatt ctttgaagaa attggtttta aggaaacttc ggagaaaggc 1500

attagatctg gaaagcttga gcctccttgg gttcgtctac aaattggaag gaaatatgaa 1560
tgaagccctg gagtactatg agcgggccct gagactggct gctgactttg agaactctgt 1620
gagacaaggt ccttaggcac ccagatatca gccactttca catttcattt cattttatgc 1680
taacatttac taatcatctt ttctgcttac tgttttcaga aacattataa ttcactgtaa 1740
tgatgtaatt cttgaataat aaatctgaca aaatattagt tgtgttcaac aattagtgaa 1800
acagaatgtg tgtatgcatg taagaaagag aaatcatttg tatgagtgct atgtagtaga 1860
gaaaaaatgt tagttaactt tgtaggaaat aaaacattgg acttacacta aatgtttaat 1920
tcattcattt tattgtgaaa taaaaataaa atccttagct cctccaccaa ctgaacagac 1980
cctcttggcc aaggagaccc cagaaacctt aaaaactaag tttcccaacc atgacaagat 2040
gagagatcat tcacacctca ttatattccc tcccttgcta actgccattg gactttttcc 2100
actgagttaa acagaaaccc atggaaaaca aagaacagaa gactcactcc ttggctgact 2160
tcacctagct cactccacgt agcgccacag ccagactccc ctcccctctt gcggtttcca 2220
catgacaact gatcagcctt ccctcctgat aagtgaccac tgcccacaga ctggttctgg 2280
ccagtccatg gaggctgcac acagggtgcc tctatgtcct ttgtttcacc ttttgatata 2340
gaaaggctaa ttttgctgta ttttaatgtt aagtctccac cacagagtga acacagaatg 2400
catgtgacat acatgtttac ataccactat tgtgtgactg cccctcatga atattcatag 2460
ccccccataa cctgttaact atgtgtgtct agccaatcca ccaaccataa aacttctgta 2520
ataccctccc ttcctccaag agcctgcttt tggttgctgt ggtaggctct gcttcccagg 2580
ctgcaggttg caggagagga ggctgcagtg gctcacgcct gtaatctcag cacttcgatg 2640
ggacgaggca ggcagatcac ctgaacccag gagttcgaga gcagccttgg caatggcaaa 2700
accaaccgtc tctacaaaaa atgcaaaaac ttagctgggt gtggtggcat gcacctgtag 2760
cttcagttcc agctactcag gaggctgagg tgagtggact gctggagcca gggagttcga 2820
ggctgcagtg tcgagatctt gccactgcac tccattctgg atgatagaac gagaccccat 2880
ctcaaaaaaa aaaaaagttc tctccaattg tatatagctt gtgattttat gtcaacacta 2940
tcaataaata gctttcagtg caagaaacca aaaatactgt aataaacagg cacatattct 3000
tcccaaacct catgcagttt acaatctagt gagagacaca gatagcagta cagagtcaat 3060
taaaggttag ttttcttcat gaagatgttt taattttaat tcaatgtgaa agggttccaa 3120
ggagtttatc ttgttttatg ccattttatt tgaagcacta cttactaagt catttgctga 3180
tattaatcta gttaaatcaa gaaatattac atgaaaatgt tgctaaatca gagatcatgg 3240
gtaacaatca cctttgatta tgaataatca tattttattg aaaggcaagg cacaacaaat 3300
aataagaagg aaaaaataaa taagcaatgt tattgatctt tcattctgta tatgttttgg 3360

ggggaatata ctagtttctt ttagtggctg taacaaatta ccacaaactt ggtgacttaa 3420

aatttcacag atttactctt tcttacagtt ctggaggtca gaagtctgaa atgggtttca 3480

atgagccaaa gtcaaggtat tgatgacgct acactcctcc ggaggctcta ggcagatagc 3540

cttttccagc ttccagaggc tgcctgaatt ctttcatcca tcttaaaaac caacagtgta 3600

gtagcctcaa atctctctct ctgcttcctt cttcacatct ccttctctcc tctgactctt 3660

ttgcctcttt cttctaagga cgcaccaggt ccacctgcat aatccagaat aattgcccca 3720

tccgcaaatc cttaatttaa taacatctgc aaagtccctt ttgctatgta aagtagcatg 3780

ttcacaggtt ctggagactt ggccatggat acgattgcgg gggggcatt attcttacca 3840

cagagcaccc caagaaaatc tccaaatttt gggcttccaa tccattttgc ttcaattatt 3900

taatattttt actccttcca gtagatactg atttcatcca ttgcccttaa gaaggtagga 3960

cagagattat ggcacatctc acattaaatg ctatattttc gttggaaata catttttttgc 4020

ttcaactttt attttaaatt caaggtaca tgtgcaggat gttcaggttt gttacacagg 4080

taaacgtgtg ccatggcggt ttgctgaaca gatcatccca tcaccaacag atcatcccat 4140

tgagaggtga agccggctgg gcttctgggt tgggtgggga cttggagaac ttttctgtct 4200

agctaaagta ttgtaaaatg gaccagtcaa cactctgtaa aatggaccaa tcagctctct 4260

gtaaaatgga ccaatcagca ggatgtgggt ggggccaagt aagggaataa aagcaggcca 4320

cccgagctgg cagcggcaac ccgctcgggt ccccttccat gctgtggaag ttttgttctt 4380

tcgctctttc aataaatctt gctgctgctc a 4411

<210> 16
<211> 4302
<212> DNA
<213> Homo sapiens

<400> 16
aactgaaaat ccacaagaca gaatagccag atctcagagg agcctggcta agcaaaaccc 60

tgcagaacgg ctgcctaatt tacagcaacc atgagtacaa atggtgatga tcatcaggtc 120

aaggatagtc tggagcaatt gagatgtcac tttacatggg agttatccat tgatgacgat 180

gaaatgcctg atttagaaaa cagagtcttg gatcagattg aattcctaga caccaaatac 240

agtgtgggaa tacacaacct actagcctat gtgaaacacc tgaaaggcca gaatgaggaa 300

gccctgaaga gcttaaaaga agctgaaaac ttaatgcagg aagaacatga caaccaagca 360

aatgtgagga gtctggtgac ctggggcaac tttgcctgga tgtattacca catgggcaga 420

ctggcagaag cccagactta cctggacaag gtggagaaca tttgcaagaa gctttcaaat 480

cccttccgct atagaatgga gtgtccagaa atagactgtg aggaaggatg ggccttgctg 540

aagtgtggag gaaaaaatta tgaacgggcc aaggcctgct ttgaaaaggt gcttgaagtg 600

gaccctgaaa accctgaatc cagcgctggg tatgcgatct ctgcctatcg cctggatggc 660
tttaaattag ccacaaaaaa tcacaagcca ttttctttgc ttcccctaag gcaggctgtc 720
cgcttaaatc cagacaatgg atatattaag gttctccttg ccctgaagct tcaggatgaa 780
ggacaggaag ctgaaggaga aaagtacatt gaagaagctc tagccaacat gtcctcacag 840
acctatgtct ttcgatatgc agccaagttt taccgaagaa aaggctctgt ggataaagct 900
cttgagttat taaaaaaggc cttgcaggaa acacccactt ctgtcttact gcatcaccag 960
atagggcttt gctacaaggc acaaatgatc caaatcaagg aggctacaaa agggcagcct 1020
agagggcaga acagagaaaa gctagacaaa atgataagat cagccatatt tcattttgaa 1080
tctgcagtgg aaaaaaagcc cacatttgag gtggctcatc tagacctggc aagaatgtat 1140
atagaagcag gcaatcacag aaaagctgaa gagaattttc aaaaattgtt atgcatgaaa 1200
ccagtggtag aagaaacaat gcaagacata catttccact atggtcggtt tcaggaattt 1260
caaaagaaat ctgacgtcaa tgcaattatc cattatttaa aagctataaa aatagaacag 1320
gcatcattaa caagggataa aagtatcaat tctttgaaga aattggtttt aaggaaactt 1380
cggagaaagg cattagatct ggaaagcttg agcctccttg ggttcgtcta caaattggaa 1440
ggaaatatga atgaagccct ggagtactat gagcgggccc tgagactggc tgctgacttt 1500
gagaactctg tgagacaagg tccttaggca cccagatatc agccactttc acatttcatt 1560
tcattttatg ctaacattta ctaatcatct tttctgctta ctgttttcag aaacattata 1620
attcactgta atgatgtaat tcttgaataa taaatctgac aaaatattag ttgtgttcaa 1680
caattagtga aacagaatgt gtgtatgcat gtaagaaaga gaaatcattt gtatgagtgc 1740
tatgtagtag agaaaaaatg ttagttaact ttgtaggaaa taaaacattg gacttacact 1800
aaatgtttaa ttcattcatt ttattgtgaa ataaaaataa aatccttagc tcctccacca 1860
actgaacaga ccctcttggc caaggagacc ccagaaacct taaaaactaa gtttcccaac 1920
catgacaaga tgagagatca ttcacacctc attatattcc ctcccttgct aactgccatt 1980
ggactttttc cactgagtta aacagaaacc catggaaaac aaagaacaga agactcactc 2040
cttggctgac ttcacctagc tcactccacg tagcgccaca gccagactcc cctcccctct 2100
tgcggtttcc acatgacaac tgatcagcct tccctcctga taagtgacca ctgcccacag 2160
actggttctg gccagtccat ggaggctgca cacagggtgc ctctatgtcc tttgtttcac 2220
cttttgatat agaaaggcta attttgctgt attttaatgt taagtctcca ccacagagtg 2280
aacacagaat gcatgtgaca tacatgttta cataccacta ttgtgtgact gcccctcatg 2340
aatattcata gccccccata acctgttaac tatgtgtgtc tagccaatcc accaaccata 2400
aaacttctgt aataccctcc cttcctccaa gagcctgctt ttggttgctg tggtaggctc 2460
tgcttcccag gctgcaggtt gcaggagagg aggctgcagt ggctcacgcc tgtaatctca 2520

gcacttcgat gggacgaggc aggcagatca cctgaaccca ggagttcgag agcagccttg 2580

gcaatggcaa aaccaaccgt ctctacaaaa aatgcaaaaa cttagctggg tgtggtggca 2640

tgcacctgta gcttcagttc cagctactca ggaggctgag gtgagtggac tgctggagcc 2700

agggagttcg aggctgcagt gtcgagatct tgccactgca ctccattctg gatgatagaa 2760

cgagacccca tctcaaaaaa aaaaaaagtt ctctccaatt gtatatagct tgtgatttta 2820

tgtcaacact atcaataaat agctttcagt gcaagaaacc aaaaatactg taataaacag 2880

gcacatattc ttcccaaacc tcatgcagtt tacaatctag tgagagacac agatagcagt 2940

acagagtcaa ttaaaggtta gttttcttca tgaagatgtt ttaattttaa ttcaatgtga 3000

aagggttcca aggagtttat cttgttttat gccattttat ttgaagcact acttactaag 3060

tcatttgctg atattaatct agttaaatca agaaatatta catgaaaatg ttgctaaatc 3120

agagatcatg ggtaacaatc acctttgatt atgaataatc atattttatt gaaaggcaag 3180

gcacaacaaa taataagaag gaaaaaataa ataagcaatg ttattgatct ttcattctgt 3240

atatgttttg gggggaatat actagtttct tttagtggct gtaacaaatt accacaaact 3300

tggtgactta aaatttcaca gatttactct ttcttacagt tctggaggtc agaagtctga 3360

aatgggtttc aatgagccaa agtcaaggta ttgatgacgc tacactcctc cggaggctct 3420

aggcagatag ccttttccag cttccagagg ctgcctgaat tctttcatcc atcttaaaaa 3480

ccaacagtgt agtagcctca aatctctctc tctgcttcct tcttcacatc tccttctctc 3540

ctctgactct tttgcctctt tcttctaagg acgcaccagg tccacctgca taatccagaa 3600

taattgcccc atccgcaaat ccttaattta ataacatctg caaagtccct tttgctatgt 3660

aaagtagcat gttcacaggt tctggagact tggccatgga tacgattgcg ggggggggcat 3720

tattcttacc acagagcacc ccaagaaaat ctccaaattt tgggcttcca atccattttg 3780

cttcaattat ttaatatttt tactccttcc agtagatact gatttcatcc attgccctta 3840

agaaggtagg acagagatta tggcacatct cacattaaat gctatatttt cgttggaaat 3900

acatttttttg cttcaacttt tattttaaat tcaagggtac atgtgcagga tgttcaggtt 3960

tgttacacag gtaaacgtgt gccatggcgg tttgctgaac agatcatccc atcaccaaca 4020

gatcatccca ttgagaggtg aagccggctg ggcttctggg ttgggtgggg acttggagaa 4080

cttttctgtc tagctaaagt attgtaaaat ggaccagtca acactctgta aaatggacca 4140

atcagctctc tgtaaaatgg accaatcagc aggatgtggg tggggccaag taagggaata 4200

aaagcaggcc acccgagctg gcagcggcaa cccgctcggg tccccttcca tgctgtggaa 4260

gttttgttct ttcgctcttt caataaatct tgctgctgct ca 4302

<210> 17

<211> 447
<212> PRT
<213> Homo sapiens

<400> 17

Met Pro Asp Leu Glu Asn Arg Val Leu Asp Gln Ile Glu Phe Leu Asp
1 5 10 15

Thr Lys Tyr Ser Val Gly Ile His Asn Leu Leu Ala Tyr Val Lys His
20 25 30

Leu Lys Gly Gln Asn Glu Glu Ala Leu Lys Ser Leu Lys Glu Ala Glu
35 40 45

Asn Leu Met Gln Glu Glu His Asp Asn Gln Ala Asn Val Arg Ser Leu
50 55 60

Val Thr Trp Gly Asn Phe Ala Trp Met Tyr Tyr His Met Gly Arg Leu
65 70 75 80

Ala Glu Ala Gln Thr Tyr Leu Asp Lys Val Glu Asn Ile Cys Lys Lys
85 90 95

Leu Ser Asn Pro Phe Arg Tyr Arg Met Glu Cys Pro Glu Ile Asp Cys
100 105 110

Glu Glu Gly Trp Ala Leu Leu Lys Cys Gly Gly Lys Asn Tyr Glu Arg
115 120 125

Ala Lys Ala Cys Phe Glu Lys Val Leu Glu Val Asp Pro Glu Asn Pro
130 135 140

Glu Ser Ser Ala Gly Tyr Ala Ile Ser Ala Tyr Arg Leu Asp Gly Phe
145 150 155 160

Lys Leu Ala Thr Lys Asn His Lys Pro Phe Ser Leu Leu Pro Leu Arg
165 170 175

Gln Ala Val Arg Leu Asn Pro Asp Asn Gly Tyr Ile Lys Val Leu Leu
180 185 190

Ala Leu Lys Leu Gln Asp Glu Gly Gln Glu Ala Glu Gly Glu Lys Tyr
195 200 205

Ile Glu Glu Ala Leu Ala Asn Met Ser Ser Gln Thr Tyr Val Phe Arg
210 215 220

Tyr Ala Ala Lys Phe Tyr Arg Arg Lys Gly Ser Val Asp Lys Ala Leu

225 230 235 240

Glu Leu Leu Lys Lys Ala Leu Gln Glu Thr Pro Thr Ser Val Leu Leu
245 250 255

His His Gln Ile Gly Leu Cys Tyr Lys Ala Gln Met Ile Gln Ile Lys
260 265 270

Glu Ala Thr Lys Gly Gln Pro Arg Gly Gln Asn Arg Glu Lys Leu Asp
275 280 285

Lys Met Ile Arg Ser Ala Ile Phe His Phe Glu Ser Ala Val Glu Lys
290 295 300

Lys Pro Thr Phe Glu Val Ala His Leu Asp Leu Ala Arg Met Tyr Ile
305 310 315 320

Glu Ala Gly Asn His Arg Lys Ala Glu Glu Asn Phe Gln Lys Leu Leu
325 330 335

Cys Met Lys Pro Val Val Glu Glu Thr Met Gln Asp Ile His Phe His
340 345 350

Tyr Gly Arg Phe Gln Glu Phe Gln Lys Lys Ser Asp Val Asn Ala Ile
355 360 365

Ile His Tyr Leu Lys Ala Ile Lys Ile Glu Gln Ala Ser Leu Thr Arg
370 375 380

Asp Lys Ser Ile Asn Ser Leu Lys Lys Leu Val Leu Arg Lys Leu Arg
385 390 395 400

Arg Lys Ala Leu Asp Leu Glu Ser Leu Ser Leu Leu Gly Phe Val Tyr
405 410 415

Lys Leu Glu Gly Asn Met Asn Glu Ala Leu Glu Tyr Tyr Glu Arg Ala
420 425 430

Leu Arg Leu Ala Ala Asp Phe Glu Asn Ser Val Arg Gln Gly Pro
435 440 445

<210> 18
<211> 478
<212> PRT
<213> Homo sapiens

<400> 18

Met Ser Thr Asn Gly Asp Asp His Gln Val Lys Asp Ser Leu Glu Gln

```
1               5                   10                  15

Leu Arg Cys His Phe Thr Trp Glu Leu Ser Ile Asp Asp Asp Glu Met
            20                  25                  30

Pro Asp Leu Glu Asn Arg Val Leu Asp Gln Ile Glu Phe Leu Asp Thr
        35                  40                  45

Lys Tyr Ser Val Gly Ile His Asn Leu Leu Ala Tyr Val Lys His Leu
    50                  55                  60

Lys Gly Gln Asn Glu Glu Ala Leu Lys Ser Leu Lys Glu Ala Glu Asn
65                  70                  75                  80

Leu Met Gln Glu Glu His Asp Asn Gln Ala Asn Val Arg Ser Leu Val
                85                  90                  95

Thr Trp Gly Asn Phe Ala Trp Met Tyr Tyr His Met Gly Arg Leu Ala
            100                 105                 110

Glu Ala Gln Thr Tyr Leu Asp Lys Val Glu Asn Ile Cys Lys Lys Leu
        115                 120                 125

Ser Asn Pro Phe Arg Tyr Arg Met Glu Cys Pro Glu Ile Asp Cys Glu
    130                 135                 140

Glu Gly Trp Ala Leu Leu Lys Cys Gly Gly Lys Asn Tyr Glu Arg Ala
145                 150                 155                 160

Lys Ala Cys Phe Glu Lys Val Leu Glu Val Asp Pro Glu Asn Pro Glu
                165                 170                 175

Ser Ser Ala Gly Tyr Ala Ile Ser Ala Tyr Arg Leu Asp Gly Phe Lys
            180                 185                 190

Leu Ala Thr Lys Asn His Lys Pro Phe Ser Leu Leu Pro Leu Arg Gln
        195                 200                 205

Ala Val Arg Leu Asn Pro Asp Asn Gly Tyr Ile Lys Val Leu Leu Ala
    210                 215                 220

Leu Lys Leu Gln Asp Glu Gly Gln Glu Ala Glu Gly Glu Lys Tyr Ile
225                 230                 235                 240

Glu Glu Ala Leu Ala Asn Met Ser Ser Gln Thr Tyr Val Phe Arg Tyr
                245                 250                 255
```

```
Ala Ala Lys Phe Tyr Arg Arg Lys Gly Ser Val Asp Lys Ala Leu Glu
            260                 265                 270

Leu Leu Lys Lys Ala Leu Gln Glu Thr Pro Thr Ser Val Leu Leu His
        275                 280                 285

His Gln Ile Gly Leu Cys Tyr Lys Ala Gln Met Ile Gln Ile Lys Glu
    290                 295                 300

Ala Thr Lys Gly Gln Pro Arg Gly Gln Asn Arg Glu Lys Leu Asp Lys
305                 310                 315                 320

Met Ile Arg Ser Ala Ile Phe His Phe Glu Ser Ala Val Glu Lys Lys
                325                 330                 335

Pro Thr Phe Glu Val Ala His Leu Asp Leu Ala Arg Met Tyr Ile Glu
            340                 345                 350

Ala Gly Asn His Arg Lys Ala Glu Glu Asn Phe Gln Lys Leu Leu Cys
        355                 360                 365

Met Lys Pro Val Val Glu Glu Thr Met Gln Asp Ile His Phe His Tyr
    370                 375                 380

Gly Arg Phe Gln Glu Phe Gln Lys Lys Ser Asp Val Asn Ala Ile Ile
385                 390                 395                 400

His Tyr Leu Lys Ala Ile Lys Ile Glu Gln Ala Ser Leu Thr Arg Asp
                405                 410                 415

Lys Ser Ile Asn Ser Leu Lys Lys Leu Val Leu Arg Lys Leu Arg Arg
            420                 425                 430

Lys Ala Leu Asp Leu Glu Ser Leu Ser Leu Leu Gly Phe Val Tyr Lys
        435                 440                 445

Leu Glu Gly Asn Met Asn Glu Ala Leu Glu Tyr Tyr Glu Arg Ala Leu
    450                 455                 460

Arg Leu Ala Ala Asp Phe Glu Asn Ser Val Arg Gln Gly Pro
465                 470                 475
```

<210> 19
<211> 2407
<212> DNA
<213> Homo sapiens

<400> 19
gtggaaacct cttcagcatt tgcttggaat cagtaagcta aaaacaaaat caaccgggac 60

cccagctttt cagaactgca gggaaacagc catcatgagt gaggtcacca agaattccct 120

ggagaaaatc cttccacagc tgaaatgcca tttcacctgg aacttattca aggaagacag 180

tgtctcaagg gatctagaag atagagtgtg taaccagatt gaatttttaa acactgagtt 240

caaagctaca atgtacaact tgttggccta cataaaacac ctagatggta acaacgaggc 300

agccctggaa tgcttacggc aagctgaaga gttaatccag caagaacatg ctgaccaagc 360

agaaatcaga agtctagtca cttggggaaa ctacgcctgg gtctactatc acttgggcag 420

actctcagat gctcagattt atgtagataa ggtgaaacaa acctgcaaga aattttcaaa 480

tccatacagt attgagtatt ctgaacttga ctgtgaggaa gggtggacac aactgaagtg 540

tggaagaaat gaaagggcga aggtgtgttt tgagaaggct ctggaagaaa agcccaacaa 600

cccagaattc tcctctggac tggcaattgc gatgtaccat ctggataatc acccagagaa 660

acagttctct actgatgttt tgaagcaggc cattgagctg agtcctgata accaatacgt 720

caaggttctc ttgggcctga aactgcagaa gatgaataaa gaagctgaag gagagcagtt 780

tgttgaagaa gccttggaaa agtctccttg ccaaacagat gtcctccgca gtgcagccaa 840

attttacaga agaaaaggtg acctagacaa agctattgaa ctgtttcaac gggtgttgga 900

atccacacca aacaatggct acctctatca ccagattggg tgctgctaca aggcaaaagt 960

aagacaaatg cagaatacag gagaatctga agctagtgga aataaagaga tgattgaagc 1020

actaaagcaa tatgctatgg actattcgaa taaagctctt gagaagggac tgaatcctct 1080

gaatgcatac tccgatctcg ctgagttcct ggagacggaa tgttatcaga caccattcaa 1140

taaggaagtc cctgatgctg aaaagcaaca atcccatcag cgctactgca accttcagaa 1200

atataatggg aagtctgaag acactgctgt gcaacatggt ttagagggtt tgtccataag 1260

caaaaaatca actgacaagg aagagatcaa agaccaacca cagaatgtat ctgaaaatct 1320

gcttccacaa aatgcaccaa attattggta tcttcaagga ttaattcata agcagaatgg 1380

agatctgctg caagcagcca aatgttatga gaaggaactg ggccgcctgc taagggatgc 1440

cccttcaggc ataggcagta ttttcctgtc agcatctgag cttgaggatg gtagtgagga 1500

aatgggccag ggcgcagtca gctccagtcc cagagagctc ctctctaact cagagcaact 1560

gaactgagac agaggaggaa aacagagcat cagaagcctg cagtggtggt tgtgacgggt 1620

aggacgatag gaagacaggg ggccccaacc tgggattgct gagcagggaa gctttgcatg 1680

ttgctctaag gtacattttt aaagagttgt tttttggccg ggcgcagtgg ctcatgcctg 1740

taatcccagc actttgggag gccgaggtgg gcggatcacg aggtctggag tttgagacca 1800

tcctggctaa cacagtgaaa tcccgtctct actaaaaata caaaaaatta gccaggcgtg 1860

gtggctggca cctgtagtcc cagctacttg ggaggctgag gcaggagaat ggcgtgaacc 1920

tggaaggaag aggttgcagt gagccaagat tgcgcccctg cactccagcc tgggcaacag 1980

agcaagactc catctcaaaa aaaaaaaaaa aaaaaaaaaa gagttgtttt ctcatgttca 2040

ttatagttca ttacagttac atagtccgaa ggtcttacaa ctaatcactg gtagcaataa 2100

atgcttcagg cccacatgat gctgattagt tctcagtttt cattcagttc acaatataac 2160

caccattcct gccctccctg ccaagggtca taaatggtga ctgcctaaca acaaaatttg 2220

cagtctcatc tcattttcat ccagacttct ggaactcaaa gattaacttt tgactaaccc 2280

tggaatatct cttatctcac ttatagcttc aggcatgtat ttatatgtat tcttgatagc 2340

aataccataa tcaatgtgta ttcctgatag taatgctaca ataaatccaa acatttcaac 2400

tctgtta 2407

<210> 20
<211> 490
<212> PRT
<213> Homo sapiens

<400> 20

Met Ser Glu Val Thr Lys Asn Ser Leu Glu Lys Ile Leu Pro Gln Leu
1 5 10 15

Lys Cys His Phe Thr Trp Asn Leu Phe Lys Glu Asp Ser Val Ser Arg
20 25 30

Asp Leu Glu Asp Arg Val Cys Asn Gln Ile Glu Phe Leu Asn Thr Glu
35 40 45

Phe Lys Ala Thr Met Tyr Asn Leu Leu Ala Tyr Ile Lys His Leu Asp
50 55 60

Gly Asn Asn Glu Ala Ala Leu Glu Cys Leu Arg Gln Ala Glu Glu Leu
65 70 75 80

Ile Gln Gln Glu His Ala Asp Gln Ala Glu Ile Arg Ser Leu Val Thr
85 90 95

Trp Gly Asn Tyr Ala Trp Val Tyr Tyr His Leu Gly Arg Leu Ser Asp
100 105 110

Ala Gln Ile Tyr Val Asp Lys Val Lys Gln Thr Cys Lys Lys Phe Ser
115 120 125

Asn Pro Tyr Ser Ile Glu Tyr Ser Glu Leu Asp Cys Glu Glu Gly Trp
130 135 140

Thr Gln Leu Lys Cys Gly Arg Asn Glu Arg Ala Lys Val Cys Phe Glu

145 150 155 160

Lys Ala Leu Glu Glu Lys Pro Asn Asn Pro Glu Phe Ser Ser Gly Leu
165 170 175

Ala Ile Ala Met Tyr His Leu Asp Asn His Pro Glu Lys Gln Phe Ser
180 185 190

Thr Asp Val Leu Lys Gln Ala Ile Glu Leu Ser Pro Asp Asn Gln Tyr
195 200 205

Val Lys Val Leu Leu Gly Leu Lys Leu Gln Lys Met Asn Lys Glu Ala
210 215 220

Glu Gly Glu Gln Phe Val Glu Glu Ala Leu Glu Lys Ser Pro Cys Gln
225 230 235 240

Thr Asp Val Leu Arg Ser Ala Ala Lys Phe Tyr Arg Arg Lys Gly Asp
245 250 255

Leu Asp Lys Ala Ile Glu Leu Phe Gln Arg Val Leu Glu Ser Thr Pro
260 265 270

Asn Asn Gly Tyr Leu Tyr His Gln Ile Gly Cys Cys Tyr Lys Ala Lys
275 280 285

Val Arg Gln Met Gln Asn Thr Gly Glu Ser Glu Ala Ser Gly Asn Lys
290 295 300

Glu Met Ile Glu Ala Leu Lys Gln Tyr Ala Met Asp Tyr Ser Asn Lys
305 310 315 320

Ala Leu Glu Lys Gly Leu Asn Pro Leu Asn Ala Tyr Ser Asp Leu Ala
325 330 335

Glu Phe Leu Glu Thr Glu Cys Tyr Gln Thr Pro Phe Asn Lys Glu Val
340 345 350

Pro Asp Ala Glu Lys Gln Gln Ser His Gln Arg Tyr Cys Asn Leu Gln
355 360 365

Lys Tyr Asn Gly Lys Ser Glu Asp Thr Ala Val Gln His Gly Leu Glu
370 375 380

Gly Leu Ser Ile Ser Lys Lys Ser Thr Asp Lys Glu Glu Ile Lys Asp
385 390 395 400

Gln Pro Gln Asn Val Ser Glu Asn Leu Leu Pro Gln Asn Ala Pro Asn
405 410 415

Tyr Trp Tyr Leu Gln Gly Leu Ile His Lys Gln Asn Gly Asp Leu Leu
420 425 430

Gln Ala Ala Lys Cys Tyr Glu Lys Glu Leu Gly Arg Leu Leu Arg Asp
435 440 445

Ala Pro Ser Gly Ile Gly Ser Ile Phe Leu Ser Ala Ser Glu Leu Glu
450 455 460

Asp Gly Ser Glu Glu Met Gly Gln Gly Ala Val Ser Ser Ser Pro Arg
465 470 475 480

Glu Leu Leu Ser Asn Ser Glu Gln Leu Asn
485 490

<210> 21
<211> 4438
<212> DNA
<213> Homo sapiens

<400> 21
ccgtgggacg ggcggaggcg cccgagtccc gcttccccgg cgcccttcca ccccgagccc 60

gactcagccc gcggccacct gcgccccgcc cctgtcggcc gcgcccgagc ccagcgccgc 120

gagccgctcc ccggcgggct ggctcctggc cccggaagcg cgagcgttca cttagcggcg 180

agtggctccg tctccgcgga cagagcgcgc gccccctggc ccggcccgcg aggggctccc 240

ggcgcggtcc ccgagcattt cccgccgggt ggagcgggcc gagcccggca ggatgaccag 300

ccccgcggcc gctcaaagcc gggagatcga ctgtttgagc ccggaagcgc agaagctggc 360

ggaagcccgg ctcgctgcaa aacgggcggc ccgcgcggag gctcgcgaga tccgcatgaa 420

ggagctggag cggcagcaga aggaggaaga cagtgagcgc tactctcgta gatccagaag 480

aaacacatcg gcttctgatg aagacgagcg catgtcagtg ggtagtcgtg gaagcctgag 540

ggtagaagag agaccagaaa aagattttac tgagaagggg tctcgtaaca tgccgggcct 600

gtctgcagcc acgctggcct ctctgggtgg gacttcctct cggagaggca gcggagacac 660

ctccatctcc atcgacaccg aggcatccat cagggaaatc aaggactctc tagcagaagt 720

tgaagagaaa tataagaagg ctatggtttc caatgctcag ctagacaatg aaaagacaaa 780

cttcatgtac caggttgata ccctaaaaga tatgttgctg gagcttgaag aacagctggc 840

tgaatctagg cggcagtacg aagagaaaaa caaagaattt gaaagggaaa aacacgccca 900

cagtatactg caatttcagt ttgctgaagt caaggaggcc ctgaagcaaa gagaggaaat 960

gctcgagaaa catggaataa tcctaaattc agaaatagct accaatggag agacttccga 1020

caccctcaat aatgttggat accaaggtcc taccaagatg acaaaagaag agttaaatgc 1080
cctcaagtcg acaggggatg ggaccctagg aagagccagt gaagtggagg tgaaaaatga 1140
aatcgtggcg aatgtgggga aaagagaaat cttgcacaat actgagaaag aacaacacac 1200
agaggacaca gtgaaggact gtgtggacat agaggtattc cctgctggtg agaataccga 1260
ggaccagaaa tcctctgaag acactgcccc attcctagga accttagcag gtgctaccta 1320
tgaggaacag gttcaaagcc aaattcttga gagcagttct ctccctgaaa acacagtaca 1380
ggttgagtca aatgaggtca tgggtgcacc agatgacagg accagaactc cccttgagcc 1440
atccaactgt tggagtgact tagatggtgg gaaccacaca gagaatgtgg gagaggcagc 1500
agtgactcag gttgaagagc aggcaggcac agtggcctcg tgtcctttag ggcatagtga 1560
tgacacagtt tatcatgatg acaaatgtat ggtagaggtc ccccaagagt tagagacaag 1620
cacagggcat agtttagaga aagaattcac caaccaggaa gcagctgagc ccaaggaggt 1680
tccagcgcac agtacagaag taggtaggga tcacaacgaa gaagagggtg aagaaacagg 1740
attaagggac gagaaaccaa tcaagacaga agttcctggt tctccagcag gaactgaggg 1800
caactgtcag gaagcgacag gtccaagtac agtagacact caaaatgaac ccttagatat 1860
gaaagagccc gatgaagaaa agagtgacca acagggagag gcattggact catcgcagaa 1920
gaagacaaag aacaagaaaa agaaaaacaa gaagaaaaaa tccccagtac ccgtagaaac 1980
ccttaaagat gttaaaaaag agttaacgta tcagaacaca gatttaagtg aaattaagga 2040
agaagagcag gtaaagtcta ctgacagaaa gtcagcagtg gaagcccaaa acgaggtgac 2100
tgaaaatcca aaacagaaaa ttgcagcaga aagcagtgaa aatgttgatt gtccggagaa 2160
tcctaaaatt aagttggatg gaaaacttga ccaagaaggt gatgatgtac aaacagcagc 2220
tgaggaggta ctagctgatg gagacacatt agattttgag gatgacaccg ttcaatcatc 2280
aggcccgagg gctggtggtg aagaattaga tgaaggtgtt gcaaaagata atgctaaaat 2340
agatggtgcc actcaaagca gtcctgcaga accaaagagc gaagacgcag atcgctgcac 2400
cctgcccgaa catgaaagtc cctcacagga cattagtgat gcctgtgaag cagaaagtac 2460
agagaggtgt gagatgtcag aacatccaag tcagaccgtc aggaaagctt tagacagcaa 2520
tagcctagag aacgatgact tgtcggcacc aggaagagag ccagggcact tcaatccaga 2580
aagcagagaa gataccagag gagggaatga gaagggcaaa agcaaagaag actgtaccat 2640
gtcctaagct gaggcaggcg gcaggcgcgg tgcacaggaa gtctcagtgt gaaggggtct 2700
tttctctcca ctgccaatgt aagtagaatg ttctaaattc atagagaggc actgtatgac 2760
aattaccagg tgctctactg ctttaagtta tagactgtta cttgtagatt tccatgtaat 2820
cattgaggtt atcacccaga ttagaaagac atatttgtta tcagtgtacg ttctaattga 2880

gagcattcca gtagtatcaa acaataatgt ctactgttta tagtccactt aataaaaata 2940

gaggcattta ctatttgcct taggctgata ggaatgtggg ttttcttgac caaatatatc 3000

agcatctaat tgaaatgacc aaatagcatt cttagacttc tgtattatga atataattga 3060

tatttaaatt aatgtcttgt tcacatatgt gtactttcat atttgatttt aaaatgtaca 3120

ttataacctg tatggtattt tatttaaagg agataaacag ccaaatagca aataggtcac 3180

tgaatgataa gatttgcacc ttagaacaat aatcatttta aggataacaa gtaaatgtct 3240

gaaagcatga ggggctttat ttgcctttac ctcatatgag tctttgatct tgaaccgata 3300

cttttggatc tcattgttga tatacctgaa tttactttgt aagagatttt aacttcactt 3360

catgctgatg atgtatcaaa ttcattttat agaaagattt aaagtttttt tctggaagtg 3420

atatatgtca aattacattt cctactgcag tatttgagca gggacagtca ttttttaaat 3480

gtttttggcc gggcgtggtg gctcatgcct gtaatctcag tacattggga ggccaaggca 3540

ggtggatcac ctgaggtcaa gagttcgagg ccagcctggc caacatggtg aaaccctgtc 3600

tctactaaaa atacaaaaaa ttggccgggc gtgatggtgg gcgcctgtaa tcccagccac 3660

tccagaggct gaggcaggag aatcgcttga acctgcgagg cagagattgc agtgagccaa 3720

gatcaagcca ttgtactcca gcctggacaa caagagcgaa actctgtcta aaaaaaaaaa 3780

aaaaaacaca cacacacaca acacaatgtt ttcacgcctg taaacctagc acattgggaa 3840

gccaaggtgg gaggattgct tgaggccagg agttcaaggc tgcagtgagc tatgattgca 3900

cactgtactc tagcctggga gacagagtga gacactgtct ctaaaaaaaa aaaaaaaaaa 3960

aaaaaaagtt tttgaacctt aaaatacttt gtttgaattt ctaatcatca ttcaaaagag 4020

cagtaaaaaa tggttacttg ttcttgtaca agctactaat tagactatag taggatattt 4080

taaagagctg aatcactttt ggtattttgg tataaatatt ttcatttgtt atgtcccagt 4140

atattcttac tggaaaattc ttgttttgat ctgcctgaag aaaatatctg ttttctatat 4200

aaaaaaattt tttaaaataa ttgtaaagtt agatttaaaa ttgtaaaata taaaatcaca 4260

aaggaatgta ccttatgaat gttgttgaca ttttatgaaa ttatgtggat tcatattact 4320

gttacaagat agaattgaat gcaaaaagac caaaacctca ataaaatttg aggaaaacgt 4380

gttattatgt aattgaaata aaaacatttt ataattgtgc aaaaaaaaaa aaaaaaaa 4438

<210> 22
<211> 4109
<212> DNA
<213> Homo sapiens

<400> 22
cgggccgggg cggcgcgagc ggctggagca acgggccccg cggcagctgc gggcgacgcg 60

gtcgatggac atgggcaccc agggatcggg gcgcaagcgg ctccccaacc gggagcggct 120

cacggcggag gacgacgcgc tcaaccagat cgcgcgggag gcggaagccc ggctcgctgc 180
aaaacgggcg gcccgcgcgg aggctcgcga gatccgcatg aaggagctgg agcggcagca 240
gaaggagatc tatcaggtcc aaaagaaata ttatgggctg gatacaaaat ggggtgacat 300
cgagcagtgg atggaagaca gtgagcgcta ctctcgtaga tccagaagaa acacatcggc 360
ttctgatgaa gacgagcgca tgtcagtggg tagtcgtgga agcctgaggt cgcagcctga 420
cttggagtat ggggggtcctt acgcctggac aaatggttat gatggagaat tgtatggatc 480
acagtccctg aatagaagat ctggcaggcc ctcctgtctg tacagcgctg cccggccttc 540
ggggagttac cgggcgtctg tgttggatga aggcagcttc ggtgggaccc gacggggcag 600
cacctccggc tcccgtgctc cctcggagta cagcggccac ctcaactcca gctcccgcgc 660
ctcctccagg gccagctcgg cccgggccag ccctgtggta gaagagagac cagaaaaaga 720
ttttactgag aaggggtctc gtaacatgcc gggcctgtct gcagccacgc tggcctctct 780
gggtgggact tcctctcgga gaggcagcgg agacacctcc atctccatcg acaccgaggc 840
atccatcagg gaaatcaagg aactcaatga gttaaaggac cagattcagg atgtagaagg 900
caaatacatg cagggattga aagagatgaa ggactctcta gcagaagttg aagagaaata 960
taagaaggct atggtttcca atgctcagct agacaatgaa aagacaaact tcatgtacca 1020
ggttgatacc ctaaaagata tgttgctgga gcttgaagaa cagctggctg aatctaggcg 1080
gcagtacgaa gagaaaaaca aagaatttga aagggaaaaa cacgcccaca gtatactgca 1140
atttcagttt gctgaagtca aggaggccct gaagcaaaga gaggaaatgc tcgaggaaat 1200
ccgacagcta cagcagaaac aggcgagttc tatcagggag atttctgatc ttcaggaaac 1260
aatagagtgg aaagacaaaa agataggggc attagagagg cagaaagagt tctttgattc 1320
cgtaaggagt gaacgggatg atcttagaga agaagtagtc atgctgaaag aggaattaaa 1380
gaaacatgga ataatcctaa attcagaaat agctaccaat ggagagactt ccgacacccct 1440
caataatgtt ggataccaag gtcctaccaa gatgacaaaa gaagagttaa atgccctcaa 1500
gtcgacaggg gatgggaccc tagatattag gttgaaaaag ctggttgatg aacgggaatg 1560
cttattggaa cagattaaga aactcaaagg gcagctggag gagagacaga agattggcaa 1620
actagacaat cttcgatctg aagatgatgt cttggaaaac gggacagaca tgcatgtaat 1680
ggacctacaa agggatgcca acagacagat cagcgacctc aaatttaaac ttgcaaaatc 1740
tgagcaagag ataactgcat tagaacaaaa tgtaataagg ttagagagtc aagtatcacg 1800
ttacaaatca gcggctgaaa atgcagaaaa aatagaagat gaacttaagg cagaaaaacg 1860
gaaactccaa agagagctcc gctctgcatt ggataaaaca gaagagctcg aggtgagcaa 1920
cggccactta gtgaagcgtc tggaaaaaat gaaagcaaat cggagtgcac tcttgtccca 1980
gcagtaaatt ccagctctga tcaggcaact ggttggtgac tggagagcat tgtttcatag 2040

gcttttctct gtcctatctg ggagcgctgc ttcttcccct gccttccgag agacgaagac 2100
cgtggcgagc ttggcgctta ggggctcccg tgccatggct caccccaggg agccccagca 2160
gccaccaggt gcctctgtct gcagacccct ggcccgggct ggcgccgacg ctcagaacct 2220
gcaggtactt cataagcaca caggggcctc gagggagctc tgtgtctgac cgcacagcag 2280
cctctgaatg ccgctggaag tgatgatcaa agtaaagatt cagttgggac ttgagttttt 2340
tttttttttc atgtgtcttg ctgaagatta aggggaaatg ttacagtgtt gggacttcct 2400
ttcatggcag aatctacaat ttgagcgact tcagtagtat ctcttagtct acgcttttca 2460
tacacaaaac actgtggaac cacaagccat taccaagcaa aactctttca ctggaaacaa 2520
gggggcagtc tagaagtaaa agtgacctta agaagactct ttacaggcaa caaatgaagc 2580
ttttctaagg gatttttgca tcagttcagt cataagaata ctttttttcca gggtaattag 2640
gcaatagctt cactgaaaat gacagctttt cattgcatta tttaatcctt atatttggaa 2700
ttgaagtcgt taacttcttt taaagaatgt actattagaa aaattaaaaa tgaaatgttg 2760
agagacttca gcaatgtggt tctaattttt ttccactgag aaagaagatc tttaatttca 2820
tattaatggt tctgtatatt ttgggtcatc tttttatttt ttaagaatat caagtcaatt 2880
catttttctt tccctattta aaaaaaaagg tgttttcaca gaatgagtgc acttaaaaag 2940
tgaagtgaag gaggaggtaa cagtagagac gatggcaata tcatcaagga caaaagtaaa 3000
aacgtttagc tacctgctga tttttagtga ctgttcatat atgttgtatt tcaagtatgg 3060
ctggtgaagc cagtcagctt ttcgggacgt tagcaagtgg aaactgagtc agtatcatcc 3120
aaaaccatat ctagtcttaa cacatggaga atgctggagt gagggttgtg agttcagggt 3180
atataatcaa gaaaacactc ccagcataat gctaggggtc accagtgtcc atcccccaga 3240
actgtatgga tctaggatat acacagctgc gttgcattaa gaaagagatg aaatctctat 3300
taaaatacac aagatttttg tatctccttg tgcagaggat atttgccact gcccattggg 3360
aagcagacaa gttatagggg gctgggggcc aacactggca agtaggaaac cacgggtcgg 3420
acaggtgagc aaaatgtgct ggcaggtggg caccactggg agacccacac tgcacacccg 3480
ggcaccgtat gaacaggaaa gaggaaggaa gctggacgaa gcccctcagg gaccctgtgc 3540
tgaccatgct gggcccaccg gcaaaaggga gatattcagt tccttgtctc atccttaagg 3600
tttcttccac aacatctgaa tacaagcatg tttaactggg aaaatgtcta tgtcatgcgt 3660
gaataacacc agcagcaaac actcacacat cacgcagaca cggccggcag catgctgacg 3720
cttttaggta tttttcactc atgcaatttt cacatatttt cactcatttc atttgcacgg 3780
aaattctatg aggtagatgc tgttatcaaa tccacattac agatgaggga cccagggtcc 3840
aggaaggtga actggcagaa gtctcccagc tggtagaaca gggctgcaag gcatcgattc 3900

ccaggtgtct cacagccctg agaagatggc gttttcccta tcagtggctc tgaggaagtc 3960

aagccttcag tctctacctc tcccaccaat tcttttggaa acagcaaacc aatgttacac 4020

acacttccta atccagagga agctagaaca cgatttttaa atttatttag taaaataaaa 4080

ctttttttgc agatgtaacg aaaaaaaaa 4109

<210> 23
<211> 4285
<212> DNA
<213> Homo sapiens

<400> 23
gcccttccac cccgagcccg actcagcccg cggccacctg cgccccgccc ctgtcggccg 60

cgcccgagcc cagcgccgcg agccgctccc cggcgggctg gctcctggcc ccggaagcgc 120

gagcgttcac ttagcggcga gtggctccgt ctccgcggac agagcgcgcg ccccctggcc 180

cggcccgcga ggggctcccg gcgcggtccc cgagcatttc ccgccgggtg gagcgggccg 240

agcccggcag gatgaccagc cccgcggccg ctcaaagccg ggagatcgac tgtttgagcc 300

cggaagcgca gaagctggcg gaagcccggc tcgctgcaaa acgggcggcc cgcgcggagg 360

ctcgcgagat ccgcatgaag gagctggagc ggcagcagaa ggaggtagaa gagagaccag 420

aaaaagattt tactgagaag gggtctcgta acatgccggg cctgtctgca gccacgctgg 480

cctctctggg tgggacttcc tctcggagag gcagcggaga cacctccatc tccatcgaca 540

ccgaggcatc catcagggaa atcaaggact ctctagcaga agttgaagag aaatataaga 600

aggctatggt ttccaatgct cagctagaca atgaaaagac aaacttcatg taccaggttg 660

ataccctaaa agatatgttg ctggagcttg aagaacagct ggctgaatct aggcggcagt 720

acgaagagaa aaacaaagaa tttgaaaggg aaaaacacgc ccacagtata ctgcaatttc 780

agtttgctga agtcaaggag gccctgaagc aaagagagga aatgctcgag aaacatggaa 840

taatcctaaa ttcagaaata gctaccaatg gagagacttc cgacaccctc aataatgttg 900

gataccaagg tcctaccaag atgacaaaag aagagttaaa tgccctcaag tcgacagggg 960

atgggaccct aggaagagcc agtgaagtgg aggtgaaaaa tgaaatcgtg gcgaatgtgg 1020

ggaaaagaga aatcttgcac aatactgaga aagaacaaca cacagaggac acagtgaagg 1080

actgtgtgga catagaggta ttccctgctg gtgagaatac cgaggaccag aaatcctctg 1140

aagacactgc cccattccta ggaaccttag caggtgctac ctatgaggaa caggttcaaa 1200

gccaaattct tgagagcagt tctctccctg aaaacacagt acaggttgag tcaaatgagg 1260

tcatgggtgc accagatgac aggaccagaa ctccccttga gccatccaac tgttggagtg 1320

acttagatgg tgggaaccac acagagaatg tgggagaggc agcagtgact caggttgaag 1380

agcaggcagg cacagtggcc tcgtgtcctt tagggcatag tgatgacaca gtttatcatg 1440

atgacaaatg tatggtagag gtcccccaag agttagagac aagcacaggg catagtttag 1500
agaaagaatt caccaaccag gaagcagctg agcccaagga ggttccagcg cacagtacag 1560
aagtaggtag ggatcacaac gaagaagagg gtgaagaaac aggattaagg gacgagaaac 1620
caatcaagac agaagttcct ggttctccag caggaactga gggcaactgt caggaagcga 1680
caggtccaag tacagtagac actcaaaatg aacccttaga tatgaaagag cccgatgaag 1740
aaaagagtga ccaacaggga gaggcattgg actcatcgca gaagaagaca aagaacaaga 1800
aaaagaaaaa caagaagaaa aaatccccag tacccgtaga aacccttaaa gatgttaaaa 1860
aagagttaac gtatcagaac acagatttaa gtgaaattaa ggaagaagag caggtaaagt 1920
ctactgacag aaagtcagca gtggaagccc aaaacgaggt gactgaaaat ccaaaacaga 1980
aaattgcagc agaaagcagt gaaaatgttg attgtccgga gaatcctaaa attaagttgg 2040
atggaaaact tgaccaagaa ggtgatgatg tacaaacagc agctgaggag gtactagctg 2100
atggagacac attagatttt gaggatgaca ccgttcaatc atcaggcccg agggctggtg 2160
gtgaagaatt agatgaaggt gttgcaaaag ataatgctaa aatagatggt gccactcaaa 2220
gcagtcctgc agaaccaaag agcgaagacg cagatcgctg caccctgccc gaacatgaaa 2280
gtccctcaca ggacattagt gatgcctgtg aagcagaaag tacagagagg tgtgagatgt 2340
cagaacatcc aagtcagacc gtcaggaaag ctttagacag caatagccta gagaacgatg 2400
acttgtcggc accaggaaga gagccagggc acttcaatcc agaaagcaga gaagatacca 2460
gaggagggaa tgagaagggc aaaagcaaag aagactgtac catgtcctaa gctgaggcag 2520
gcggcaggcg cggtgcacag gaagtctcag tgtgaagggg tcttttctct ccactgccaa 2580
tgtaagtaga atgttctaaa ttcatagaga ggcactgtat gacaattacc aggtgctcta 2640
ctgctttaag ttatagactg ttacttgtag atttccatgt aatcattgag gttatcaccc 2700
agattagaaa gacatatttg ttatcagtgt acgttctaat tgagagcatt ccagtagtat 2760
caaacaataa tgtctactgt ttatagtcca cttaataaaa atagaggcat ttactatttg 2820
ccttaggctg ataggaatgt gggttttctt gaccaaatat atcagcatct aattgaaatg 2880
accaaatagc attcttagac ttctgtatta tgaatataat tgatatttaa attaatgtct 2940
tgttcacata tgtgtacttt catatttgat tttaaaatgt acattataac ctgtatggta 3000
ttttatttaa aggagataaa cagccaaata gcaaataggt cactgaatga taagatttgc 3060
accttagaac aataatcatt ttaaggataa caagtaaatg tctgaaagca tgaggggctt 3120
tatttgcctt tacctcatat gagtctttga tcttgaaccg atacttttgg atctcattgt 3180
tgatatacct gaatttactt tgtaagagat tttaacttca cttcatgctg atgatgtatc 3240
aaattcattt tatagaaaga tttaaagttt ttttctggaa gtgatatatg tcaaattaca 3300
tttcctactg cagtatttga gcagggacag tcattttttta aatgtttttg gccgggcgtg 3360

gtggctcatg cctgtaatct cagtacattg ggaggccaag gcaggtggat cacctgaggt 3420

caagagttcg aggccagcct ggccaacatg gtgaaaccct gtctctacta aaaatacaaa 3480

aaattggccg ggcgtgatgg tgggcgcctg taatcccagc cactccagag gctgaggcag 3540

gagaatcgct tgaacctgcg aggcagagat tgcagtgagc caagatcaag ccattgtact 3600

ccagcctgga caacaagagc gaaactctgt ctaaaaaaaa aaaaaaaaac acacacacac 3660

acaacacaat gttttcacgc ctgtaaacct agcacattgg gaagccaagg tgggaggatt 3720

gcttgaggcc aggagttcaa ggctgcagtg agctatgatt gcacactgta ctctagcctg 3780

ggagacagag tgagacactg tctctaaaaa aaaaaaaaaa aaaaaaaaaa gtttttgaac 3840

cttaaaatac tttgtttgaa tttctaatca tcattcaaaa gagcagtaaa aaatggttac 3900

ttgttcttgt acaagctact aattagacta tagtaggata ttttaaagag ctgaatcact 3960

tttggtattt tggtataaat attttcattt gttatgtccc agtatattct tactggaaaa 4020

ttcttgtttt gatctgcctg aagaaaatat ctgttttcta tataaaaaaa ttttttaaaa 4080

taattgtaaa gttagattta aaattgtaaa atataaaatc acaaaggaat gtaccttatg 4140

aatgttgttg acattttatg aaattatgtg gattcatatt actgttacaa gatagaattg 4200

aatgcaaaaa gaccaaaacc tcaataaaat ttgaggaaaa cgtgttatta tgtaattgaa 4260

ataaaaacat tttataattg tgcaa 4285

<210> 24
<211> 4453
<212> DNA
<213> Homo sapiens

<400> 24
gcccttccac cccgagcccg actcagcccg cggccacctg cgccccgccc ctgtcggccg 60

cgcccgagcc cagcgccgcg agccgctccc cggcgggctg gctcctggcc ccggaagcgc 120

gagcgttcac ttagcggcga gtggctccgt ctccgcggac agagcgcgcg ccccctggcc 180

cggcccgcga ggggctcccg gcgcggtccc cgagcatttc ccgccgggtg gagcgggccg 240

agcccggcag gatgaccagc cccgcggccg ctcaaagccg ggagatcgac tgtttgagcc 300

cggaagcgca gaagctggcg gaagcccggc tcgctgcaaa acgggcggcc cgcgcggagg 360

ctcgcgagat ccgcatgaag gagctggagc ggcagcagaa ggaggaagac agtgagcgct 420

actctcgtag atccagaaga aacacatcgg cttctgatga agacgagcgc atgtcagtgg 480

gtagtcgtgg aagcctgagg gtagaagaga gaccagaaaa agattttact gagaaggggt 540

ctcgtaacat gccgggcctg tctgcagcca cgctggcctc tctgggtggg acttcctctc 600

ggagaggcag cggagacacc tccatctcca tcgacaccga ggcatccatc agggaaatca 660

aggaactcaa tgagttaaag gaccagattc aggatgtaga aggcaaatac atgcagggat 720

tgaaagagat gaaggactct ctagcagaag ttgaagagaa atataagaag gctatggttt 780

ccaatgctca gctagacaat gaaaagacaa acttcatgta ccaggttgat accctaaaag 840

atatgttgct ggagcttgaa gaacagctgg ctgaatctag gcggcagtac gaagagaaaa 900

acaaagaatt tgaaagggaa aaacacgccc acagtatact gcaatttcag tttgctgaag 960

tcaaggaggc cctgaagcaa agagaggaaa tgctcgagaa acatggaata atcctaaatt 1020

cagaaatagc taccaatgga gagacttccg acaccctcaa taatgttgga taccaaggtc 1080

ctaccaagat gacaaaagaa gagttaaatg ccctcaagtc gacaggggat gggaccctag 1140

gaagagccag tgaagtggag gtgaaaaatg aaatcgtggc gaatgtgggg aaaagagaaa 1200

tcttgcacaa tactgagaaa gaacaacaca cagaggacac agtgaaggac tgtgtggaca 1260

tagaggtatt ccctgctggt gagaataccg aggaccagaa atcctctgaa gacactgccc 1320

cattcctagg aaccttagca ggtgctacct atgaggaaca ggttcaaagc caaattcttg 1380

agagcagttc tctccctgaa aacacagtac aggttgagtc aaatgaggtc atgggtgcac 1440

cagatgacag gaccagaact ccccttgagc catccaactg ttggagtgac ttagatggtg 1500

ggaaccacac agagaatgtg ggagaggcag cagtgactca ggttgaagag caggcaggca 1560

cagtggcctc gtgtccttta gggcatagtg atgacacagt ttatcatgat gacaaatgta 1620

tggtagaggt cccccaagag ttagagacaa gcacagggca tagtttagag aaagaattca 1680

ccaaccagga agcagctgag cccaaggagg ttccagcgca cagtacagaa gtaggtaggg 1740

atcacaacga agaagagggt gaagaaacag gattaaggga cgagaaacca atcaagacag 1800

aagttcctgg ttctccagca ggaactgagg gcaactgtca ggaagcgaca ggtccaagta 1860

cagtagacac tcaaaatgaa cccttagata tgaaagagcc cgatgaagaa aagagtgacc 1920

aacagggaga ggcattggac tcatcgcaga agaagacaaa gaacaagaaa aagaaaaaca 1980

agaagaaaaa atccccagta cccgtagaaa cccttaaaga tgttaaaaaa gagttaacgt 2040

atcagaacac agatttaagt gaaattaagg aagaagagca ggtaaagtct actgacagaa 2100

agtcagcagt ggaagcccaa aacgaggtga ctgaaaatcc aaaacagaaa attgcagcag 2160

aaagcagtga aaatgttgat tgtccggaga atcctaaaat taagttggat ggaaaacttg 2220

accaagaagg tgatgatgta caaacagcag ctgaggaggt actagctgat ggagacacat 2280

tagattttga ggatgacacc gttcaatcat caggcccgag ggctggtggt gaagaattag 2340

atgaaggtgt tgcaaaagat aatgctaaaa tagatggtgc cactcaaagc agtcctgcag 2400

aaccaaagag cgaagacgca gatcgctgca ccctgcccga acatgaaagt ccctcacagg 2460

acattagtga tgcctgtgaa gcagaaagta cagagaggtg tgagatgtca gaacatccaa 2520

gtcagaccgt caggaaagct ttagacagca atagcctaga gaacgatgac ttgtcggcac 2580

caggaagaga gccagggcac ttcaatccag aaagcagaga agataccaga ggagggaatg 2640
agaagggcaa aagcaaagaa gactgtacca tgtcctaagc tgaggcaggc ggcaggcgcg 2700
gtgcacagga agtctcagtg tgaaggggtc ttttctctcc actgccaatg taagtagaat 2760
gttctaaatt catagagagg cactgtatga caattaccag gtgctctact gctttaagtt 2820
atagactgtt acttgtagat ttccatgtaa tcattgaggt tatcacccag attagaaaga 2880
catatttgtt atcagtgtac gttctaattg agagcattcc agtagtatca aacaataatg 2940
tctactgttt atagtccact taataaaaat agaggcattt actatttgcc ttaggctgat 3000
aggaatgtgg gttttcttga ccaaatatat cagcatctaa ttgaaatgac caaatagcat 3060
tcttagactt ctgtattatg aatataattg atatttaaat taatgtcttg ttcacatatg 3120
tgtactttca tatttgattt taaaatgtac attataacct gtatggtatt ttatttaaag 3180
gagataaaca gccaaatagc aaataggtca ctgaatgata agatttgcac cttagaacaa 3240
taatcatttt aaggataaca agtaaatgtc tgaaagcatg aggggcttta tttgccttta 3300
cctcatatga gtctttgatc ttgaaccgat acttttggat ctcattgttg atatacctga 3360
atttactttg taagagattt taacttcact tcatgctgat gatgtatcaa attcatttta 3420
tagaaagatt taaagttttt ttctggaagt gatatatgtc aaattacatt tcctactgca 3480
gtatttgagc agggacagtc attttttaaa tgtttttggc cgggcgtggt ggctcatgcc 3540
tgtaatctca gtacattggg aggccaaggc aggtggatca cctgaggtca agagttcgag 3600
gccagcctgg ccaacatggt gaaaccctgt ctctactaaa aatacaaaaa attggccggg 3660
cgtgatggtg ggcgcctgta atcccagcca ctccagaggc tgaggcagga gaatcgcttg 3720
aacctgcgag gcagagattg cagtgagcca agatcaagcc attgtactcc agcctggaca 3780
acaagagcga aactctgtct aaaaaaaaaa aaaaaaacac acacacacac aacacaatgt 3840
tttcacgcct gtaaacctag cacattggga agccaaggtg ggaggattgc ttgaggccag 3900
gagttcaagg ctgcagtgag ctatgattgc acactgtact ctagcctggg agacagagtg 3960
agacactgtc tctaaaaaaa aaaaaaaaaa aaaaaaaagt ttttgaacct taaaatactt 4020
tgtttgaatt tctaatcatc attcaaaaga gcagtaaaaa atggttactt gttcttgtac 4080
aagctactaa ttagactata gtaggatatt ttaaagagct gaatcacttt tggtattttg 4140
gtataaatat tttcatttgt tatgtcccag tatattctta ctggaaaatt cttgttttga 4200
tctgcctgaa gaaaatatct gttttctata taaaaaaatt ttttaaaata attgtaaagt 4260
tagatttaaa attgtaaaat ataaaatcac aaaggaatgt accttatgaa tgttgttgac 4320
attttatgaa attatgtgga ttcatattac tgttacaaga tagaattgaa tgcaaaaaga 4380
ccaaaacctc aataaaattt gaggaaaacg tgttattatg taattgaaat aaaaacattt 4440
tataattgtg caa 4453

<210> 25
<211> 784
<212> PRT
<213> Homo sapiens

<400> 25

```
Met Thr Ser Pro Ala Ala Ala Gln Ser Arg Glu Ile Asp Cys Leu Ser
1               5                   10                  15

Pro Glu Ala Gln Lys Leu Ala Glu Ala Arg Leu Ala Ala Lys Arg Ala
            20                  25                  30

Ala Arg Ala Glu Ala Arg Glu Ile Arg Met Lys Glu Leu Glu Arg Gln
        35                  40                  45

Gln Lys Glu Glu Asp Ser Glu Arg Tyr Ser Arg Arg Ser Arg Arg Asn
    50                  55                  60

Thr Ser Ala Ser Asp Glu Asp Glu Arg Met Ser Val Gly Ser Arg Gly
65                  70                  75                  80

Ser Leu Arg Val Glu Glu Arg Pro Glu Lys Asp Phe Thr Glu Lys Gly
                85                  90                  95

Ser Arg Asn Met Pro Gly Leu Ser Ala Ala Thr Leu Ala Ser Leu Gly
            100                 105                 110

Gly Thr Ser Ser Arg Arg Gly Ser Gly Asp Thr Ser Ile Ser Ile Asp
        115                 120                 125

Thr Glu Ala Ser Ile Arg Glu Ile Lys Asp Ser Leu Ala Glu Val Glu
    130                 135                 140

Glu Lys Tyr Lys Lys Ala Met Val Ser Asn Ala Gln Leu Asp Asn Glu
145                 150                 155                 160

Lys Thr Asn Phe Met Tyr Gln Val Asp Thr Leu Lys Asp Met Leu Leu
                165                 170                 175

Glu Leu Glu Glu Gln Leu Ala Glu Ser Arg Arg Gln Tyr Glu Glu Lys
            180                 185                 190

Asn Lys Glu Phe Glu Arg Glu Lys His Ala His Ser Ile Leu Gln Phe
        195                 200                 205

Gln Phe Ala Glu Val Lys Glu Ala Leu Lys Gln Arg Glu Glu Met Leu
    210                 215                 220
```

```
Glu Lys His Gly Ile Ile Leu Asn Ser Glu Ile Ala Thr Asn Gly Glu
225                 230                 235                 240

Thr Ser Asp Thr Leu Asn Asn Val Gly Tyr Gln Gly Pro Thr Lys Met
                245                 250                 255

Thr Lys Glu Glu Leu Asn Ala Leu Lys Ser Thr Gly Asp Gly Thr Leu
            260                 265                 270

Gly Arg Ala Ser Glu Val Glu Val Lys Asn Glu Ile Val Ala Asn Val
        275                 280                 285

Gly Lys Arg Glu Ile Leu His Asn Thr Glu Lys Glu Gln His Thr Glu
    290                 295                 300

Asp Thr Val Lys Asp Cys Val Asp Ile Glu Val Phe Pro Ala Gly Glu
305                 310                 315                 320

Asn Thr Glu Asp Gln Lys Ser Ser Glu Asp Thr Ala Pro Phe Leu Gly
                325                 330                 335

Thr Leu Ala Gly Ala Thr Tyr Glu Glu Gln Val Gln Ser Gln Ile Leu
            340                 345                 350

Glu Ser Ser Ser Leu Pro Glu Asn Thr Val Gln Val Glu Ser Asn Glu
        355                 360                 365

Val Met Gly Ala Pro Asp Asp Arg Thr Arg Thr Pro Leu Glu Pro Ser
    370                 375                 380

Asn Cys Trp Ser Asp Leu Asp Gly Gly Asn His Thr Glu Asn Val Gly
385                 390                 395                 400

Glu Ala Ala Val Thr Gln Val Glu Glu Gln Ala Gly Thr Val Ala Ser
                405                 410                 415

Cys Pro Leu Gly His Ser Asp Asp Thr Val Tyr His Asp Asp Lys Cys
            420                 425                 430

Met Val Glu Val Pro Gln Glu Leu Glu Thr Ser Thr Gly His Ser Leu
        435                 440                 445

Glu Lys Glu Phe Thr Asn Gln Glu Ala Ala Glu Pro Lys Glu Val Pro
    450                 455                 460

Ala His Ser Thr Glu Val Gly Arg Asp His Asn Glu Glu Glu Gly Glu
```

465 470 475 480

Glu Thr Gly Leu Arg Asp Glu Lys Pro Ile Lys Thr Glu Val Pro Gly
485 490 495

Ser Pro Ala Gly Thr Glu Gly Asn Cys Gln Glu Ala Thr Gly Pro Ser
500 505 510

Thr Val Asp Thr Gln Asn Glu Pro Leu Asp Met Lys Glu Pro Asp Glu
515 520 525

Glu Lys Ser Asp Gln Gln Gly Glu Ala Leu Asp Ser Ser Gln Lys Lys
530 535 540

Thr Lys Asn Lys Lys Lys Lys Asn Lys Lys Lys Lys Ser Pro Val Pro
545 550 555 560

Val Glu Thr Leu Lys Asp Val Lys Lys Glu Leu Thr Tyr Gln Asn Thr
565 570 575

Asp Leu Ser Glu Ile Lys Glu Glu Glu Gln Val Lys Ser Thr Asp Arg
580 585 590

Lys Ser Ala Val Glu Ala Gln Asn Glu Val Thr Glu Asn Pro Lys Gln
595 600 605

Lys Ile Ala Ala Glu Ser Ser Glu Asn Val Asp Cys Pro Glu Asn Pro
610 615 620

Lys Ile Lys Leu Asp Gly Lys Leu Asp Gln Glu Gly Asp Asp Val Gln
625 630 635 640

Thr Ala Ala Glu Glu Val Leu Ala Asp Gly Asp Thr Leu Asp Phe Glu
645 650 655

Asp Asp Thr Val Gln Ser Ser Gly Pro Arg Ala Gly Gly Glu Glu Leu
660 665 670

Asp Glu Gly Val Ala Lys Asp Asn Ala Lys Ile Asp Gly Ala Thr Gln
675 680 685

Ser Ser Pro Ala Glu Pro Lys Ser Glu Asp Ala Asp Arg Cys Thr Leu
690 695 700

Pro Glu His Glu Ser Pro Ser Gln Asp Ile Ser Asp Ala Cys Glu Ala
705 710 715 720

Glu Ser Thr Glu Arg Cys Glu Met Ser Glu His Pro Ser Gln Thr Val
725 730 735

Arg Lys Ala Leu Asp Ser Asn Ser Leu Glu Asn Asp Asp Leu Ser Ala
740 745 750

Pro Gly Arg Glu Pro Gly His Phe Asn Pro Glu Ser Arg Glu Asp Thr
755 760 765

Arg Gly Gly Asn Glu Lys Gly Lys Ser Lys Glu Asp Cys Thr Met Ser
770 775 780

<210> 26
<211> 640
<212> PRT
<213> Homo sapiens

<400> 26

Met Asp Met Gly Thr Gln Gly Ser Gly Arg Lys Arg Leu Pro Asn Arg
1 5 10 15

Glu Arg Leu Thr Ala Glu Asp Asp Ala Leu Asn Gln Ile Ala Arg Glu
20 25 30

Ala Glu Ala Arg Leu Ala Ala Lys Arg Ala Ala Arg Ala Glu Ala Arg
35 40 45

Glu Ile Arg Met Lys Glu Leu Glu Arg Gln Gln Lys Glu Ile Tyr Gln
50 55 60

Val Gln Lys Lys Tyr Tyr Gly Leu Asp Thr Lys Trp Gly Asp Ile Glu
65 70 75 80

Gln Trp Met Glu Asp Ser Glu Arg Tyr Ser Arg Arg Ser Arg Arg Asn
85 90 95

Thr Ser Ala Ser Asp Glu Asp Glu Arg Met Ser Val Gly Ser Arg Gly
100 105 110

Ser Leu Arg Ser Gln Pro Asp Leu Glu Tyr Gly Gly Pro Tyr Ala Trp
115 120 125

Thr Asn Gly Tyr Asp Gly Glu Leu Tyr Gly Ser Gln Ser Leu Asn Arg
130 135 140

Arg Ser Gly Arg Pro Ser Cys Leu Tyr Ser Ala Ala Arg Pro Ser Gly
145 150 155 160

```
Ser Tyr Arg Ala Ser Val Leu Asp Glu Gly Ser Phe Gly Gly Thr Arg
                165                 170                 175

Arg Gly Ser Thr Ser Gly Ser Arg Ala Pro Ser Glu Tyr Ser Gly His
            180                 185                 190

Leu Asn Ser Ser Ser Arg Ala Ser Ser Arg Ala Ser Ser Ala Arg Ala
        195                 200                 205

Ser Pro Val Val Glu Glu Arg Pro Glu Lys Asp Phe Thr Glu Lys Gly
    210                 215                 220

Ser Arg Asn Met Pro Gly Leu Ser Ala Ala Thr Leu Ala Ser Leu Gly
225                 230                 235                 240

Gly Thr Ser Ser Arg Arg Gly Ser Gly Asp Thr Ser Ile Ser Ile Asp
                245                 250                 255

Thr Glu Ala Ser Ile Arg Glu Ile Lys Glu Leu Asn Glu Leu Lys Asp
            260                 265                 270

Gln Ile Gln Asp Val Glu Gly Lys Tyr Met Gln Gly Leu Lys Glu Met
        275                 280                 285

Lys Asp Ser Leu Ala Glu Val Glu Glu Lys Tyr Lys Lys Ala Met Val
    290                 295                 300

Ser Asn Ala Gln Leu Asp Asn Glu Lys Thr Asn Phe Met Tyr Gln Val
305                 310                 315                 320

Asp Thr Leu Lys Asp Met Leu Leu Glu Leu Glu Glu Gln Leu Ala Glu
                325                 330                 335

Ser Arg Arg Gln Tyr Glu Glu Lys Asn Lys Glu Phe Glu Arg Glu Lys
            340                 345                 350

His Ala His Ser Ile Leu Gln Phe Gln Phe Ala Glu Val Lys Glu Ala
        355                 360                 365

Leu Lys Gln Arg Glu Glu Met Leu Glu Glu Ile Arg Gln Leu Gln Gln
    370                 375                 380

Lys Gln Ala Ser Ser Ile Arg Glu Ile Ser Asp Leu Gln Glu Thr Ile
385                 390                 395                 400

Glu Trp Lys Asp Lys Lys Ile Gly Ala Leu Glu Arg Gln Lys Glu Phe
                405                 410                 415
```

```
Phe Asp Ser Val Arg Ser Glu Arg Asp Asp Leu Arg Glu Glu Val Val
            420                 425                 430

Met Leu Lys Glu Glu Leu Lys Lys His Gly Ile Ile Leu Asn Ser Glu
        435                 440                 445

Ile Ala Thr Asn Gly Glu Thr Ser Asp Thr Leu Asn Asn Val Gly Tyr
    450                 455                 460

Gln Gly Pro Thr Lys Met Thr Lys Glu Glu Leu Asn Ala Leu Lys Ser
465                 470                 475                 480

Thr Gly Asp Gly Thr Leu Asp Ile Arg Leu Lys Lys Leu Val Asp Glu
                485                 490                 495

Arg Glu Cys Leu Leu Glu Gln Ile Lys Lys Leu Lys Gly Gln Leu Glu
            500                 505                 510

Glu Arg Gln Lys Ile Gly Lys Leu Asp Asn Leu Arg Ser Glu Asp Asp
        515                 520                 525

Val Leu Glu Asn Gly Thr Asp Met His Val Met Asp Leu Gln Arg Asp
    530                 535                 540

Ala Asn Arg Gln Ile Ser Asp Leu Lys Phe Lys Leu Ala Lys Ser Glu
545                 550                 555                 560

Gln Glu Ile Thr Ala Leu Glu Gln Asn Val Ile Arg Leu Glu Ser Gln
                565                 570                 575

Val Ser Arg Tyr Lys Ser Ala Ala Glu Asn Ala Glu Lys Ile Glu Asp
            580                 585                 590

Glu Leu Lys Ala Glu Lys Arg Lys Leu Gln Arg Glu Leu Arg Ser Ala
        595                 600                 605

Leu Asp Lys Thr Glu Glu Leu Glu Val Ser Asn Gly His Leu Val Lys
    610                 615                 620

Arg Leu Glu Lys Met Lys Ala Asn Arg Ser Ala Leu Leu Ser Gln Gln
625                 630                 635                 640
```

<210> 27
<211> 752
<212> PRT
<213> Homo sapiens

<400> 27

```
Met Thr Ser Pro Ala Ala Ala Gln Ser Arg Glu Ile Asp Cys Leu Ser
1               5                   10                  15

Pro Glu Ala Gln Lys Leu Ala Glu Ala Arg Leu Ala Ala Lys Arg Ala
            20                  25                  30

Ala Arg Ala Glu Ala Arg Glu Ile Arg Met Lys Glu Leu Glu Arg Gln
        35                  40                  45

Gln Lys Glu Val Glu Glu Arg Pro Glu Lys Asp Phe Thr Glu Lys Gly
    50                  55                  60

Ser Arg Asn Met Pro Gly Leu Ser Ala Ala Thr Leu Ala Ser Leu Gly
65                  70                  75                  80

Gly Thr Ser Ser Arg Arg Gly Ser Gly Asp Thr Ser Ile Ser Ile Asp
                85                  90                  95

Thr Glu Ala Ser Ile Arg Glu Ile Lys Asp Ser Leu Ala Glu Val Glu
            100                 105                 110

Glu Lys Tyr Lys Lys Ala Met Val Ser Asn Ala Gln Leu Asp Asn Glu
        115                 120                 125

Lys Thr Asn Phe Met Tyr Gln Val Asp Thr Leu Lys Asp Met Leu Leu
    130                 135                 140

Glu Leu Glu Glu Gln Leu Ala Glu Ser Arg Arg Gln Tyr Glu Glu Lys
145                 150                 155                 160

Asn Lys Glu Phe Glu Arg Glu Lys His Ala His Ser Ile Leu Gln Phe
                165                 170                 175

Gln Phe Ala Glu Val Lys Glu Ala Leu Lys Gln Arg Glu Glu Met Leu
            180                 185                 190

Glu Lys His Gly Ile Ile Leu Asn Ser Glu Ile Ala Thr Asn Gly Glu
        195                 200                 205

Thr Ser Asp Thr Leu Asn Asn Val Gly Tyr Gln Gly Pro Thr Lys Met
    210                 215                 220

Thr Lys Glu Glu Leu Asn Ala Leu Lys Ser Thr Gly Asp Gly Thr Leu
225                 230                 235                 240

Gly Arg Ala Ser Glu Val Glu Val Lys Asn Glu Ile Val Ala Asn Val
                245                 250                 255
```

Gly Lys Arg Glu Ile Leu His Asn Thr Glu Lys Glu Gln His Thr Glu
260 265 270

Asp Thr Val Lys Asp Cys Val Asp Ile Glu Val Phe Pro Ala Gly Glu
275 280 285

Asn Thr Glu Asp Gln Lys Ser Ser Glu Asp Thr Ala Pro Phe Leu Gly
290 295 300

Thr Leu Ala Gly Ala Thr Tyr Glu Glu Gln Val Gln Ser Gln Ile Leu
305 310 315 320

Glu Ser Ser Ser Leu Pro Glu Asn Thr Val Gln Val Glu Ser Asn Glu
325 330 335

Val Met Gly Ala Pro Asp Asp Arg Thr Arg Thr Pro Leu Glu Pro Ser
340 345 350

Asn Cys Trp Ser Asp Leu Asp Gly Gly Asn His Thr Glu Asn Val Gly
355 360 365

Glu Ala Ala Val Thr Gln Val Glu Glu Gln Ala Gly Thr Val Ala Ser
370 375 380

Cys Pro Leu Gly His Ser Asp Asp Thr Val Tyr His Asp Asp Lys Cys
385 390 395 400

Met Val Glu Val Pro Gln Glu Leu Glu Thr Ser Thr Gly His Ser Leu
405 410 415

Glu Lys Glu Phe Thr Asn Gln Glu Ala Ala Glu Pro Lys Glu Val Pro
420 425 430

Ala His Ser Thr Glu Val Gly Arg Asp His Asn Glu Glu Glu Gly Glu
435 440 445

Glu Thr Gly Leu Arg Asp Glu Lys Pro Ile Lys Thr Glu Val Pro Gly
450 455 460

Ser Pro Ala Gly Thr Glu Gly Asn Cys Gln Glu Ala Thr Gly Pro Ser
465 470 475 480

Thr Val Asp Thr Gln Asn Glu Pro Leu Asp Met Lys Glu Pro Asp Glu
485 490 495

Glu Lys Ser Asp Gln Gln Gly Glu Ala Leu Asp Ser Ser Gln Lys Lys

500 505 510

Thr Lys Asn Lys Lys Lys Lys Asn Lys Lys Lys Lys Ser Pro Val Pro
515 520 525

Val Glu Thr Leu Lys Asp Val Lys Lys Glu Leu Thr Tyr Gln Asn Thr
530 535 540

Asp Leu Ser Glu Ile Lys Glu Glu Glu Gln Val Lys Ser Thr Asp Arg
545 550 555 560

Lys Ser Ala Val Glu Ala Gln Asn Glu Val Thr Glu Asn Pro Lys Gln
565 570 575

Lys Ile Ala Ala Glu Ser Ser Glu Asn Val Asp Cys Pro Glu Asn Pro
580 585 590

Lys Ile Lys Leu Asp Gly Lys Leu Asp Gln Glu Gly Asp Asp Val Gln
595 600 605

Thr Ala Ala Glu Glu Val Leu Ala Asp Gly Asp Thr Leu Asp Phe Glu
610 615 620

Asp Asp Thr Val Gln Ser Ser Gly Pro Arg Ala Gly Gly Glu Glu Leu
625 630 635 640

Asp Glu Gly Val Ala Lys Asp Asn Ala Lys Ile Asp Gly Ala Thr Gln
645 650 655

Ser Ser Pro Ala Glu Pro Lys Ser Glu Asp Ala Asp Arg Cys Thr Leu
660 665 670

Pro Glu His Glu Ser Pro Ser Gln Asp Ile Ser Asp Ala Cys Glu Ala
675 680 685

Glu Ser Thr Glu Arg Cys Glu Met Ser Glu His Pro Ser Gln Thr Val
690 695 700

Arg Lys Ala Leu Asp Ser Asn Ser Leu Glu Asn Asp Asp Leu Ser Ala
705 710 715 720

Pro Gly Arg Glu Pro Gly His Phe Asn Pro Glu Ser Arg Glu Asp Thr
725 730 735

Arg Gly Gly Asn Glu Lys Gly Lys Ser Lys Glu Asp Cys Thr Met Ser
740 745 750

<210> 28
<211> 808
<212> PRT
<213> Homo sapiens

<400> 28

Met Thr Ser Pro Ala Ala Ala Gln Ser Arg Glu Ile Asp Cys Leu Ser
1 5 10 15

Pro Glu Ala Gln Lys Leu Ala Glu Ala Arg Leu Ala Ala Lys Arg Ala
20 25 30

Ala Arg Ala Glu Ala Arg Glu Ile Arg Met Lys Glu Leu Glu Arg Gln
35 40 45

Gln Lys Glu Glu Asp Ser Glu Arg Tyr Ser Arg Arg Ser Arg Arg Asn
50 55 60

Thr Ser Ala Ser Asp Glu Asp Glu Arg Met Ser Val Gly Ser Arg Gly
65 70 75 80

Ser Leu Arg Val Glu Glu Arg Pro Glu Lys Asp Phe Thr Glu Lys Gly
85 90 95

Ser Arg Asn Met Pro Gly Leu Ser Ala Ala Thr Leu Ala Ser Leu Gly
100 105 110

Gly Thr Ser Ser Arg Arg Gly Ser Gly Asp Thr Ser Ile Ser Ile Asp
115 120 125

Thr Glu Ala Ser Ile Arg Glu Ile Lys Glu Leu Asn Glu Leu Lys Asp
130 135 140

Gln Ile Gln Asp Val Glu Gly Lys Tyr Met Gln Gly Leu Lys Glu Met
145 150 155 160

Lys Asp Ser Leu Ala Glu Val Glu Glu Lys Tyr Lys Lys Ala Met Val
165 170 175

Ser Asn Ala Gln Leu Asp Asn Glu Lys Thr Asn Phe Met Tyr Gln Val
180 185 190

Asp Thr Leu Lys Asp Met Leu Leu Glu Leu Glu Glu Gln Leu Ala Glu
195 200 205

Ser Arg Arg Gln Tyr Glu Glu Lys Asn Lys Glu Phe Glu Arg Glu Lys
210 215 220

His Ala His Ser Ile Leu Gln Phe Gln Phe Ala Glu Val Lys Glu Ala
225 230 235 240

Leu Lys Gln Arg Glu Glu Met Leu Glu Lys His Gly Ile Ile Leu Asn
245 250 255

Ser Glu Ile Ala Thr Asn Gly Glu Thr Ser Asp Thr Leu Asn Asn Val
260 265 270

Gly Tyr Gln Gly Pro Thr Lys Met Thr Lys Glu Glu Leu Asn Ala Leu
275 280 285

Lys Ser Thr Gly Asp Gly Thr Leu Gly Arg Ala Ser Glu Val Glu Val
290 295 300

Lys Asn Glu Ile Val Ala Asn Val Gly Lys Arg Glu Ile Leu His Asn
305 310 315 320

Thr Glu Lys Glu Gln His Thr Glu Asp Thr Val Lys Asp Cys Val Asp
325 330 335

Ile Glu Val Phe Pro Ala Gly Glu Asn Thr Glu Asp Gln Lys Ser Ser
340 345 350

Glu Asp Thr Ala Pro Phe Leu Gly Thr Leu Ala Gly Ala Thr Tyr Glu
355 360 365

Glu Gln Val Gln Ser Gln Ile Leu Glu Ser Ser Ser Leu Pro Glu Asn
370 375 380

Thr Val Gln Val Glu Ser Asn Glu Val Met Gly Ala Pro Asp Asp Arg
385 390 395 400

Thr Arg Thr Pro Leu Glu Pro Ser Asn Cys Trp Ser Asp Leu Asp Gly
405 410 415

Gly Asn His Thr Glu Asn Val Gly Glu Ala Ala Val Thr Gln Val Glu
420 425 430

Glu Gln Ala Gly Thr Val Ala Ser Cys Pro Leu Gly His Ser Asp Asp
435 440 445

Thr Val Tyr His Asp Asp Lys Cys Met Val Glu Val Pro Gln Glu Leu
450 455 460

Glu Thr Ser Thr Gly His Ser Leu Glu Lys Glu Phe Thr Asn Gln Glu
465 470 475 480

Ala Ala Glu Pro Lys Glu Val Pro Ala His Ser Thr Glu Val Gly Arg
485 490 495

Asp His Asn Glu Glu Glu Gly Glu Glu Thr Gly Leu Arg Asp Glu Lys
500 505 510

Pro Ile Lys Thr Glu Val Pro Gly Ser Pro Ala Gly Thr Glu Gly Asn
515 520 525

Cys Gln Glu Ala Thr Gly Pro Ser Thr Val Asp Thr Gln Asn Glu Pro
530 535 540

Leu Asp Met Lys Glu Pro Asp Glu Glu Lys Ser Asp Gln Gln Gly Glu
545 550 555 560

Ala Leu Asp Ser Ser Gln Lys Lys Thr Lys Asn Lys Lys Lys Lys Asn
565 570 575

Lys Lys Lys Lys Ser Pro Val Pro Val Glu Thr Leu Lys Asp Val Lys
580 585 590

Lys Glu Leu Thr Tyr Gln Asn Thr Asp Leu Ser Glu Ile Lys Glu Glu
595 600 605

Glu Gln Val Lys Ser Thr Asp Arg Lys Ser Ala Val Glu Ala Gln Asn
610 615 620

Glu Val Thr Glu Asn Pro Lys Gln Lys Ile Ala Ala Glu Ser Ser Glu
625 630 635 640

Asn Val Asp Cys Pro Glu Asn Pro Lys Ile Lys Leu Asp Gly Lys Leu
645 650 655

Asp Gln Glu Gly Asp Asp Val Gln Thr Ala Ala Glu Glu Val Leu Ala
660 665 670

Asp Gly Asp Thr Leu Asp Phe Glu Asp Asp Thr Val Gln Ser Ser Gly
675 680 685

Pro Arg Ala Gly Gly Glu Glu Leu Asp Glu Gly Val Ala Lys Asp Asn
690 695 700

Ala Lys Ile Asp Gly Ala Thr Gln Ser Ser Pro Ala Glu Pro Lys Ser
705 710 715 720

Glu Asp Ala Asp Arg Cys Thr Leu Pro Glu His Glu Ser Pro Ser Gln
725 730 735

Asp Ile Ser Asp Ala Cys Glu Ala Glu Ser Thr Glu Arg Cys Glu Met
740 745 750

Ser Glu His Pro Ser Gln Thr Val Arg Lys Ala Leu Asp Ser Asn Ser
755 760 765

Leu Glu Asn Asp Asp Leu Ser Ala Pro Gly Arg Glu Pro Gly His Phe
770 775 780

Asn Pro Glu Ser Arg Glu Asp Thr Arg Gly Gly Asn Glu Lys Gly Lys
785 790 795 800

Ser Lys Glu Asp Cys Thr Met Ser
805

<210> 29
<211> 2691
<212> DNA
<213> Homo sapiens

<400> 29
aatgcgaccc gaccgcgctg aggctccagg accgcccgcc atggctgcag gaggtcccgg 60

cgcggggtct gcggccccgg tctcctccac atcctccctt cccctggctg ctctcaacat 120

gcgagtgcgg cgccgcctgt ctctgttctt gaacgtgcgg acacaggtgg cggccgactg 180

gaccgcgctg gcggaggaga tggactttga gtacttggag atccggcaac tggagacaca 240

agcggacccc actggcaggc tgctggacgc ctggcaggga cgccctggcg cctctgtagg 300

ccgactgctc gagctgctta ccaagctggg ccgcgacgac gtgctgctgg agctgggacc 360

cagcattgag gaggattgcc aaaagtatat cttgaagcag cagcaggagg aggctgagaa 420

gcctttacag gtggccgctg tagacagcag tgtcccacgg acagcagagc tggcgggcat 480

caccacactt gatgaccccc tggggcatat gcctgagcgt ttcgatgcct tcatctgcta 540

ttgccccagc gacatccagt ttgtgcagga gatgatccgg caactggaac agacaaacta 600

tcgactgaag ttgtgtgtgt ctgaccgcga tgtcctgcct ggcacctgtg tctggtctat 660

tgctagtgag ctcatcgaaa agaggttggc tagaaggcca cggggtgggt gccgccggat 720

ggtggtggtt gtctctgatg attacctgca gagcaaggaa tgtgacttcc agaccaaatt 780

tgcactcagc ctctctccag gtgcccatca gaagcgactg atccccatca agtacaaggc 840

aatgaagaaa gagttcccca gcatcctgag gttcatcact gtctgcgact acaccaaccc 900

ctgcaccaaa tcttggttct ggactcgcct tgccaaggcc ttgtccctgc cctgaagact 960

gttctgaggc cctgggtgtg tgtgtatctg tctgcctgtc catgtacttc tgccctgcct 1020

cctcctttcg ttgtaggagg aatctgtgct ctacttacct ctcaattcct ggagatgcca 1080

acttcacaga cacgtctgca gcagctggac atcacatttc atgtcctgca tggaaccagt 1140

ggctgtgagt ggcatgtcca cttgctggat tatcagccag gacactatag aacaggacca 1200

gctgagacta agaaggacca gcagagccag ctcagctctg agccattcac acatcttcac 1260

cctcagtttc ctcacttgag gagtgggatg gggagaacag agagtagctg tgtttgaatc 1320

cctgtaggaa atggtgaagc atagctctgg gtctcctggg ggagaccagg cttggctgcg 1380

ggagagctgg ctgttgctgg actacatgct ggccactgct gtgaccacga cactgctggg 1440

gcagcttctt ccacagtgat gcctactgat gcttcagtgc ctctgcacac cgcccattcc 1500

acttcctcct tccccacagg gcaggtgggg aagcagtttg gcccagccca aggagacccc 1560

accttgagcc ttatttccta atgggtccac ctctcatctg catctttcac acctcccagc 1620

ttctgcccaa ccttcagcag tgacaagtcc ccaagagact cgcctgagca gcttgggctg 1680

cttttcattt ccacctgtca ggatgcctgt ggtcatgctc tcagctccac ctggcatgag 1740

aagggatcct ggcctctggc atattcatca agtatgagtt ctggggatga gtcactgtaa 1800

tgatgtgagc agggagcctt cctccctggg ccacctgcag agagctttcc caccaacttt 1860

gtaccttgat tgccttacaa agttatttgt ttacaaacag cgaccatata aaagcctcct 1920

gccccaaagc ttgtgggcac atgggcacat acagactcac atacagacac acacatatat 1980

gtacagacat gtactctcac acacacaggc accagcatac acacgttttt ctaggtacag 2040

ctcccaggaa cagctaggtg ggaaagtccc atcactgagg gagcctaacc atgtccctga 2100

acaaaaattg ggcactcatc tattcctttt ctcttgtgtc cctactcatt gaaaccaaac 2160

tctggaaagg acccaatgta ccagtattta tacctctaat gaagcacaga gagaggaaga 2220

gagctgctta aactcacaca acaatgaact gcagacacag ctgttctctc cctctctcct 2280

tcccagagca atttatactt taccctcagg ctgtcctctg gggagaaggt gccatggtct 2340

taggtgtctg tgccccagga cagaccctag gaccctaaat ccaatagaaa atgcatatct 2400

ttgctccact ttcagccagg ctggagcaag gtaccttttc ttaggatctt gggagggaat 2460

ggatgcccct ctctgcatga tcttgttgag gcatttagct gccatgcacc tgtcccctt 2520

taatactggg cattttaaag ccatctcaag aggcatcttc tacatgtttt gtacgcatta 2580

aaataatttc aaagatatct gagaaaagcc gatatttgcc attcttccta tatcctggaa 2640

tatatcttgc atcctgagtt tataataata aataatattc taccttggaa a 2691

<210> 30
<211> 2727
<212> DNA
<213> Homo sapiens

<400> 30
agattcctac ttcttacgcc ccccacatca cccgcctcga gacctcaagg gtagaggtgg 60

```
gcacccccgc ctccgcactt ttgctcgggg ctccagattg tagggcaggg cggcgcttct      120
cggaaagcga aagccggcgg ggcggggcgg gtgccgcagg agaaagagga agcgctggca      180
gacaatgcga cccgaccgcg ctgaggctcc aggaccgccc gccatggctg caggaggtcc      240
cggcgcgggg tctgcggccc cggtctcctc cacatcctcc cttcccctgg ctgctctcaa      300
catgcgagtg cggcgccgcc tgtctctgtt cttgaacgtg cggacacagg tggcggccga      360
ctggaccgcg ctggcggagg agatggactt tgagtacttg gagatccggc aactggagac      420
acaagcggac cccactggca ggctgctgga cgcctggcag ggacgccctg gcgcctctgt      480
aggccgactg ctcgagctgc ttaccaagct gggccgcgac gacgtgctgc tggagctggg      540
acccagcatt gggcatatgc ctgagcgttt cgatgccttc atctgctatt gccccagcga      600
catccagttt gtgcaggaga tgatccggca actggaacag acaaactatc gactgaagtt      660
gtgtgtgtct gaccgcgatg tcctgcctgg cacctgtgtc tggtctattg ctagtgagct      720
catcgaaaag aggtgccgcc ggatggtggt ggttgtctct gatgattacc tgcagagcaa      780
ggaatgtgac ttccagacca aatttgcact cagcctctct ccaggtgccc atcagaagcg      840
actgatcccc atcaagtaca aggcaatgaa gaaagagttc cccagcatcc tgaggttcat      900
cactgtctgc gactacacca acccctgcac caaatcttgg ttctggactc gccttgccaa      960
ggccttgtcc ctgccctgaa gactgttctg aggccctggg tgtgtgtgta tctgtctgcc     1020
tgtccatgta cttctgccct gcctcctcct ttcgttgtag gaggaatctg tgctctactt     1080
acctctcaat tcctggagat gccaacttca cagacacgtc tgcagcagct ggacatcaca     1140
tttcatgtcc tgcatggaac cagtggctgt gagtggcatg tccacttgct ggattatcag     1200
ccaggacact atagaacagg accagctgag actaagaagg accagcagag ccagctcagc     1260
tctgagccat tcacacatct tcaccctcag tttcctcact tgaggagtgg gatggggaga     1320
acagagagta gctgtgtttg aatccctgta ggaaatggtg aagcatagct ctgggtctcc     1380
tgggggagac caggcttggc tgcgggagag ctggctgttg ctggactaca tgctggccac     1440
tgctgtgacc acgacactgc tggggcagct tcttccacag tgatgcctac tgatgcttca     1500
gtgcctctgc acaccgccca ttccacttcc tccttcccca cagggcaggt ggggaagcag     1560
tttggcccag cccaaggaga ccccaccttg agccttattt cctaatgggt ccacctctca     1620
tctgcatctt tcacacctcc cagcttctgc ccaaccttca gcagtgacaa gtccccaaga     1680
gactcgcctg agcagcttgg gctgcttttc atttccacct gtcaggatgc ctgtggtcat     1740
gctctcagct ccacctggca tgagaaggga tcctggcctc tggcatattc atcaagtatg     1800
agttctgggg atgagtcact gtaatgatgt gagcagggag ccttcctccc tgggccacct     1860
gcagagagct ttcccaccaa ctttgtacct tgattgcctt acaaagttat ttgtttacaa     1920
```

acagcgacca tataaaagcc tcctgcccca aagcttgtgg gcacatgggc acatacagac 1980

tcacatacag acacacacat atatgtacag acatgtactc tcacacacac aggcaccagc 2040

atacacacgt ttttctaggt acagctccca ggaacagcta ggtgggaaag tcccatcact 2100

gagggagcct aaccatgtcc ctgaacaaaa attgggcact catctattcc ttttctcttg 2160

tgtccctact cattgaaacc aaactctgga aaggacccaa tgtaccagta tttatacctc 2220

taatgaagca cagagagagg aagagagctg cttaaactca cacaacaatg aactgcagac 2280

acagctgttc tctccctctc tccttcccag agcaatttat actttaccct caggctgtcc 2340

tctggggaga aggtgccatg gtcttaggtg tctgtgcccc aggacagacc ctaggaccct 2400

aaatccaata gaaaatgcat atctttgctc cactttcagc caggctggag caaggtacct 2460

tttcttagga tcttgggagg gaatggatgc ccctctctgc atgatcttgt tgaggcattt 2520

agctgccatg cacctgtccc cctttaatac tgggcatttt aaagccatct caagaggcat 2580

cttctacatg ttttgtacgc attaaaataa tttcaaagat atctgagaaa agccgatatt 2640

tgccattctt cctatatcct ggaatatatc ttgcatcctg agtttataat aataaataat 2700

attctacctt ggaaaaaaaa aaaaaaa 2727

<210> 31
<211> 2486
<212> DNA
<213> Homo sapiens

<400> 31
aatgcgaccc gaccgcgctg aggctccagg accgcccgcc atggctgcag gaggtcccgg 60

cgcggggtct gcggccccgg tctcctccac atcctccctt cccctggctg ctctcaacat 120

gcgagtgcgg cgccgcctgt ctctgttctt gaacgtgcgg acacaggtgg cggccgactg 180

gaccgcgctg gcggaggaga tggactttga gtacttggag atccggcaac tggagacaca 240

agcggacccc actggcaggc tgctggacgc ctggcaggga cgccctggcg cctctgtagg 300

ccgactgctc gagctgctta ccaagctggg ccgcgacgac gtgctgctgg agctgggacc 360

cagcattgag gaggattgcc aaaagtatat cttgaagcag cagcaggagg aggctgagaa 420

gcctttacag gtggccgctg tagacagcag tgtcccacgg acagcagagc tggcgggcat 480

caccacactt gatgaccccc tgggtgccgc cggatggtgg tggttgtctc tgatgattac 540

ctgcagagca aggaatgtga cttccagacc aaatttgcac tcagcctctc tccaggtgcc 600

catcagaagc gactgatccc catcaagtac aaggcaatga agaaagagtt ccccagcatc 660

ctgaggttca tcactgtctg cgactacacc aacccctgca ccaaatcttg gttctggact 720

cgccttgcca aggccttgtc cctgccctga agactgttct gaggccctgg gtgtgtgtgt 780

atctgtctgc ctgtccatgt acttctgccc tgcctcctcc tttcgttgta ggaggaatct 840

gtgctctact tacctctcaa ttcctggaga tgccaacttc acagacacgt ctgcagcagc 900

tggacatcac atttcatgtc ctgcatggaa ccagtggctg tgagtggcat gtccacttgc 960

tggattatca gccaggacac tatagaacag gaccagctga gactaagaag gaccagcaga 1020

gccagctcag ctctgagcca ttcacacatc ttcaccctca gtttcctcac ttgaggagtg 1080

ggatggggag aacagagagt agctgtgttt gaatccctgt aggaaatggt gaagcatagc 1140

tctgggtctc ctgggggaga ccaggcttgg ctgcgggaga gctggctgtt gctggactac 1200

atgctggcca ctgctgtgac cacgacactg ctggggcagc ttcttccaca gtgatgccta 1260

ctgatgcttc agtgcctctg cacaccgccc attccacttc ctccttcccc acagggcagg 1320

tggggaagca gtttggccca gcccaaggag accccacctt gagccttatt tcctaatggg 1380

tccacctctc atctgcatct ttcacacctc ccagcttctg cccaaccttc agcagtgaca 1440

agtccccaag agactcgcct gagcagcttg ggctgctttt catttccacc tgtcaggatg 1500

cctgtggtca tgctctcagc tccacctggc atgagaaggg atcctggcct ctggcatatt 1560

catcaagtat gagttctggg gatgagtcac tgtaatgatg tgagcaggga gccttcctcc 1620

ctgggccacc tgcagagagc tttcccacca actttgtacc ttgattgcct tacaaagtta 1680

tttgtttaca aacagcgacc atataaaagc ctcctgcccc aaagcttgtg ggcacatggg 1740

cacatacaga ctcacataca gacacacaca tatatgtaca gacatgtact ctcacacaca 1800

caggcaccag catacacacg tttttctagg tacagctccc aggaacagct aggtgggaaa 1860

gtcccatcac tgagggagcc taaccatgtc cctgaacaaa aattgggcac tcatctattc 1920

cttttctctt gtgtccctac tcattgaaac caaactctgg aaaggaccca atgtaccagt 1980

atttatacct ctaatgaagc acagagagag gaagagagct gcttaaactc acacaacaat 2040

gaactgcaga cacagctgtt ctctccctct ctccttccca gagcaattta tactttaccc 2100

tcaggctgtc ctctggggag aaggtgccat ggtcttaggt gtctgtgccc caggacagac 2160

cctaggaccc taaatccaat agaaaatgca tatctttgct ccactttcag ccaggctgga 2220

gcaaggtacc ttttcttagg atcttgggag ggaatggatg cccctctctg catgatcttg 2280

ttgaggcatt tagctgccat gcacctgtcc ccctttaata ctgggcattt taaagccatc 2340

tcaagaggca tcttctacat gttttgtacg cattaaaata atttcaaaga tatctgagaa 2400

aagccgatat ttgccattct tcctatatcc tggaatatat cttgcatcct gagtttataa 2460

taataaataa tattctacct tggaaa 2486

<210> 32
<211> 2351
<212> DNA
<213> Homo sapiens

<400> 32

aatgcgaccc gaccgcgctg aggctccagg accgcccgcc atggctgcag gaggtcccgg 60
cgcggggtct gcggccccgg tctcctccac atcctccctt cccctggctg ctctcaacat 120
gcgagtgcgg cgccgcctgt ctctgttctt gaacgtgcgg acacaggtgg cggccgactg 180
gaccgcgctg gcggaggaga tggactttga gtacttggag atccggcaac tggagacaca 240
agcggacccc actggcaggc tgctggacgc ctggcaggga cgccctggcg cctctgtagg 300
ccgactgctc gagctgctta ccaagctggg ccgcgacgac gtgctgctgg agctgggacc 360
cagcattggt gccgccggat ggtggtggtt gtctctgatg attacctgca gagcaaggaa 420
tgtgacttcc agaccaaatt tgcactcagc ctctctccag gtgcccatca gaagcgactg 480
atccccatca agtacaaggc aatgaagaaa gagttcccca gcatcctgag gttcatcact 540
gtctgcgact acaccaaccc ctgcaccaaa tcttggttct ggactcgcct tgccaaggcc 600
ttgtccctgc cctgaagact gttctgaggc cctgggtgtg tgtgtatctg tctgcctgtc 660
catgtacttc tgccctgcct cctcctttcg ttgtaggagg aatctgtgct ctacttacct 720
ctcaattcct ggagatgcca acttcacaga cacgtctgca gcagctggac atcacatttc 780
atgtcctgca tggaaccagt ggctgtgagt ggcatgtcca cttgctggat tatcagccag 840
gacactatag aacaggacca gctgagacta agaaggacca gcagagccag ctcagctctg 900
agccattcac acatcttcac cctcagtttc ctcacttgag gagtgggatg gggagaacag 960
agagtagctg tgtttgaatc cctgtaggaa atggtgaagc atagctctgg gtctcctggg 1020
ggagaccagg cttggctgcg ggagagctgg ctgttgctgg actacatgct ggccactgct 1080
gtgaccacga cactgctggg gcagcttctt ccacagtgat gcctactgat gcttcagtgc 1140
ctctgcacac cgcccattcc acttcctcct tccccacagg gcaggtgggg aagcagtttg 1200
gcccagccca aggagacccc accttgagcc ttatttccta atgggtccac ctctcatctg 1260
catctttcac acctcccagc ttctgcccaa ccttcagcag tgacaagtcc ccaagagact 1320
cgcctgagca gcttgggctg cttttcattt ccacctgtca ggatgcctgt ggtcatgctc 1380
tcagctccac ctggcatgag aagggatcct ggcctctggc atattcatca agtatgagtt 1440
ctggggatga gtcactgtaa tgatgtgagc agggagcctt cctccctggg ccacctgcag 1500
agagctttcc caccaacttt gtaccttgat tgccttacaa agttatttgt ttacaaacag 1560
cgaccatata aaagcctcct gccccaaagc ttgtgggcac atgggcacat acagactcac 1620
atacagacac acacatatat gtacagacat gtactctcac acacacaggc accagcatac 1680
acacgttttt ctaggtacag ctcccaggaa cagctaggtg ggaaagtccc atcactgagg 1740
gagcctaacc atgtccctga acaaaaattg ggcactcatc tattcctttt ctcttgtgtc 1800
cctactcatt gaaaccaaac tctggaaagg acccaatgta ccagtattta tacctctaat 1860
gaagcacaga gagaggaaga gagctgctta aactcacaca acaatgaact gcagacacag 1920

ctgttctctc cctctctcct tcccagagca atttatactt taccctcagg ctgtcctctg 1980

gggagaaggt gccatggtct taggtgtctg tgccccagga cagaccctag gaccctaaat 2040

ccaatagaaa atgcatatct ttgctccact ttcagccagg ctggagcaag gtaccttttc 2100

ttaggatctt gggagggaat ggatgcccct ctctgcatga tcttgttgag gcatttagct 2160

gccatgcacc tgtcccccctt taatactggg cattttaaag ccatctcaag aggcatcttc 2220

tacatgtttt gtacgcatta aaataatttc aaagatatct gagaaaagcc gatatttgcc 2280

attcttccta tatcctggaa tatatcttgc atcctgagtt tataataata aataatattc 2340

taccttggaa a 2351

<210> 33
<211> 2862
<212> DNA
<213> Homo sapiens

<400> 33
agattcctac ttcttacgcc ccccacatca cccgcctcga gacctcaagg gtagaggtgg 60

gcaccccccgc ctccgcactt ttgctcgggg ctccagattg tagggcaggg cggcgcttct 120

cggaaagcga aagccggcgg ggcggggcgg gtgccgcagg agaaagagga agcgctggca 180

gacaatgcga cccgaccgcg ctgaggctcc aggaccgccc gccatggctg caggaggtcc 240

cggcgcgggg tctgcggccc cggtctcctc cacatcctcc cttcccctgg ctgctctcaa 300

catgcgagtg cggcgccgcc tgtctctgtt cttgaacgtg cggacacagg tggcggccga 360

ctggaccgcg ctggcggagg agatggactt tgagtacttg gagatccggc aactggagac 420

acaagcggac ccccactggca ggctgctgga cgcctggcag ggacgccctg gcgcctctgt 480

aggccgactg ctcgagctgc ttaccaagct gggccgcgac gacgtgctgc tggagctggg 540

acccagcatt gaggaggatt gccaaaagta tatcttgaag cagcagcagg aggaggctga 600

gaagccttta caggtggccg ctgtagacag cagtgtccca cggacagcag agctggcggg 660

catcaccaca cttgatgacc ccctggggca tatgcctgag cgtttcgatg ccttcatctg 720

ctattgcccc agcgacatcc agtttgtgca ggagatgatc cggcaactgg aacagacaaa 780

ctatcgactg aagttgtgtg tgtctgaccg cgatgtcctg cctggcacct gtgtctggtc 840

tattgctagt gagctcatcg aaaagaggtg ccgccggatg gtggtggttg tctctgatga 900

ttacctgcag agcaaggaat gtgacttcca gaccaaattt gcactcagcc tctctccagg 960

tgcccatcag aagcgactga tccccatcaa gtacaaggca atgaagaaag agttccccag 1020

catcctgagg ttcatcactg tctgcgacta caccaacccc tgcaccaaat cttggttctg 1080

gactcgcctt gccaaggcct tgtccctgcc ctgaagactg ttctgaggcc ctgggtgtgt 1140

gtgtatctgt ctgcctgtcc atgtacttct gccctgcctc ctcctttcgt tgtaggagga 1200

atctgtgctc tacttacctc tcaattcctg gagatgccaa cttcacagac acgtctgcag 1260
cagctggaca tcacatttca tgtcctgcat ggaaccagtg gctgtgagtg gcatgtccac 1320
ttgctggatt atcagccagg acactataga acaggaccag ctgagactaa gaaggaccag 1380
cagagccagc tcagctctga gccattcaca catcttcacc ctcagtttcc tcacttgagg 1440
agtgggatgg ggagaacaga gagtagctgt gtttgaatcc ctgtaggaaa tggtgaagca 1500
tagctctggg tctcctgggg gagaccaggc ttggctgcgg gagagctggc tgttgctgga 1560
ctacatgctg gccactgctg tgaccacgac actgctgggg cagcttcttc cacagtgatg 1620
cctactgatg cttcagtgcc tctgcacacc gcccattcca cttcctcctt ccccacaggg 1680
caggtgggga agcagtttgg cccagcccaa ggagacccca ccttgagcct tatttcctaa 1740
tgggtccacc tctcatctgc atctttcaca cctcccagct tctgcccaac cttcagcagt 1800
gacaagtccc caagagactc gcctgagcag cttgggctgc ttttcatttc cacctgtcag 1860
gatgcctgtg gtcatgctct cagctccacc tggcatgaga agggatcctg gcctctggca 1920
tattcatcaa gtatgagttc tggggatgag tcactgtaat gatgtgagca gggagccttc 1980
ctccctgggc cacctgcaga gagctttccc accaactttg taccttgatt gccttacaaa 2040
gttatttgtt tacaaacagc gaccatataa aagcctcctg ccccaaagct tgtgggcaca 2100
tgggcacata cagactcaca tacagacaca cacatatatg tacagacatg tactctcaca 2160
cacacaggca ccagcataca cacgtttttc taggtacagc tcccaggaac agctaggtgg 2220
gaaagtccca tcactgaggg agcctaacca tgtccctgaa caaaaattgg gcactcatct 2280
attccttttc tcttgtgtcc ctactcattg aaaccaaact ctggaaagga cccaatgtac 2340
cagtatttat acctctaatg aagcacagag agaggaagag agctgcttaa actcacacaa 2400
caatgaactg cagacacagc tgttctctcc ctctctcctt cccagagcaa tttatacttt 2460
accctcaggc tgtcctctgg ggagaaggtg ccatggtctt aggtgtctgt gccccaggac 2520
agaccctagg accctaaatc caatagaaaa tgcatatctt tgctccactt tcagccaggc 2580
tggagcaagg taccttttct taggatcttg ggagggaatg gatgcccctc tctgcatgat 2640
cttgttgagg catttagctg ccatgcacct gtcccccttt aatactgggc attttaaagc 2700
catctcaaga ggcatcttct acatgttttg tacgcattaa aataatttca aagatatctg 2760
agaaaagccg atatttgcca ttcttcctat atcctggaat atatcttgca tcctgagttt 2820
ataataataa ataatattct accttggaaa aaaaaaaaaa aa 2862

<210> 34
<211> 304
<212> PRT
<213> Homo sapiens

<400> 34

```
Met Ala Ala Gly Gly Pro Gly Ala Gly Ser Ala Ala Pro Val Ser Ser
1               5                   10                  15

Thr Ser Ser Leu Pro Leu Ala Ala Leu Asn Met Arg Val Arg Arg Arg
            20                  25                  30

Leu Ser Leu Phe Leu Asn Val Arg Thr Gln Val Ala Ala Asp Trp Thr
        35                  40                  45

Ala Leu Ala Glu Glu Met Asp Phe Glu Tyr Leu Glu Ile Arg Gln Leu
    50                  55                  60

Glu Thr Gln Ala Asp Pro Thr Gly Arg Leu Leu Asp Ala Trp Gln Gly
65                  70                  75                  80

Arg Pro Gly Ala Ser Val Gly Arg Leu Leu Glu Leu Leu Thr Lys Leu
                85                  90                  95

Gly Arg Asp Asp Val Leu Leu Glu Leu Gly Pro Ser Ile Glu Glu Asp
            100                 105                 110

Cys Gln Lys Tyr Ile Leu Lys Gln Gln Gln Glu Glu Ala Glu Lys Pro
        115                 120                 125

Leu Gln Val Ala Ala Val Asp Ser Ser Val Pro Arg Thr Ala Glu Leu
    130                 135                 140

Ala Gly Ile Thr Thr Leu Asp Asp Pro Leu Gly His Met Pro Glu Arg
145                 150                 155                 160

Phe Asp Ala Phe Ile Cys Tyr Cys Pro Ser Asp Ile Gln Phe Val Gln
                165                 170                 175

Glu Met Ile Arg Gln Leu Glu Gln Thr Asn Tyr Arg Leu Lys Leu Cys
            180                 185                 190

Val Ser Asp Arg Asp Val Leu Pro Gly Thr Cys Val Trp Ser Ile Ala
        195                 200                 205

Ser Glu Leu Ile Glu Lys Arg Leu Ala Arg Arg Pro Arg Gly Gly Cys
    210                 215                 220

Arg Arg Met Val Val Val Val Ser Asp Asp Tyr Leu Gln Ser Lys Glu
225                 230                 235                 240

Cys Asp Phe Gln Thr Lys Phe Ala Leu Ser Leu Ser Pro Gly Ala His
```

245 250 255

Gln Lys Arg Leu Ile Pro Ile Lys Tyr Lys Ala Met Lys Lys Glu Phe
260 265 270

Pro Ser Ile Leu Arg Phe Ile Thr Val Cys Asp Tyr Thr Asn Pro Cys
275 280 285

Thr Lys Ser Trp Phe Trp Thr Arg Leu Ala Lys Ala Leu Ser Leu Pro
290 295 300

<210> 35
<211> 251
<212> PRT
<213> Homo sapiens

<400> 35

Met Ala Ala Gly Gly Pro Gly Ala Gly Ser Ala Ala Pro Val Ser Ser
1 5 10 15

Thr Ser Ser Leu Pro Leu Ala Ala Leu Asn Met Arg Val Arg Arg Arg
20 25 30

Leu Ser Leu Phe Leu Asn Val Arg Thr Gln Val Ala Ala Asp Trp Thr
35 40 45

Ala Leu Ala Glu Glu Met Asp Phe Glu Tyr Leu Glu Ile Arg Gln Leu
50 55 60

Glu Thr Gln Ala Asp Pro Thr Gly Arg Leu Leu Asp Ala Trp Gln Gly
65 70 75 80

Arg Pro Gly Ala Ser Val Gly Arg Leu Leu Glu Leu Leu Thr Lys Leu
85 90 95

Gly Arg Asp Asp Val Leu Leu Glu Leu Gly Pro Ser Ile Gly His Met
100 105 110

Pro Glu Arg Phe Asp Ala Phe Ile Cys Tyr Cys Pro Ser Asp Ile Gln
115 120 125

Phe Val Gln Glu Met Ile Arg Gln Leu Glu Gln Thr Asn Tyr Arg Leu
130 135 140

Lys Leu Cys Val Ser Asp Arg Asp Val Leu Pro Gly Thr Cys Val Trp
145 150 155 160

Ser Ile Ala Ser Glu Leu Ile Glu Lys Arg Cys Arg Arg Met Val Val

165 170 175

Val Val Ser Asp Asp Tyr Leu Gln Ser Lys Glu Cys Asp Phe Gln Thr
180 185 190

Lys Phe Ala Leu Ser Leu Ser Pro Gly Ala His Gln Lys Arg Leu Ile
195 200 205

Pro Ile Lys Tyr Lys Ala Met Lys Lys Glu Phe Pro Ser Ile Leu Arg
210 215 220

Phe Ile Thr Val Cys Asp Tyr Thr Asn Pro Cys Thr Lys Ser Trp Phe
225 230 235 240

Trp Thr Arg Leu Ala Lys Ala Leu Ser Leu Pro
245 250

<210> 36
<211> 191
<212> PRT
<213> Homo sapiens

<400> 36

Met Ala Ala Gly Gly Pro Gly Ala Gly Ser Ala Ala Pro Val Ser Ser
1 5 10 15

Thr Ser Ser Leu Pro Leu Ala Ala Leu Asn Met Arg Val Arg Arg Arg
20 25 30

Leu Ser Leu Phe Leu Asn Val Arg Thr Gln Val Ala Ala Asp Trp Thr
35 40 45

Ala Leu Ala Glu Glu Met Asp Phe Glu Tyr Leu Glu Ile Arg Gln Leu
50 55 60

Glu Thr Gln Ala Asp Pro Thr Gly Arg Leu Leu Asp Ala Trp Gln Gly
65 70 75 80

Arg Pro Gly Ala Ser Val Gly Arg Leu Leu Glu Leu Leu Thr Lys Leu
85 90 95

Gly Arg Asp Asp Val Leu Leu Glu Leu Gly Pro Ser Ile Glu Glu Asp
100 105 110

Cys Gln Lys Tyr Ile Leu Lys Gln Gln Gln Glu Glu Ala Glu Lys Pro
115 120 125

Leu Gln Val Ala Ala Val Asp Ser Ser Val Pro Arg Thr Ala Glu Leu

130 135 140

Ala Gly Ile Thr Thr Leu Asp Asp Pro Leu Gly Ala Ala Gly Trp Trp
145 150 155 160

Trp Leu Ser Leu Met Ile Thr Cys Arg Ala Arg Asn Val Thr Ser Arg
165 170 175

Pro Asn Leu His Ser Ala Ser Leu Gln Val Pro Ile Arg Ser Asp
180 185 190

<210> 37
<211> 146
<212> PRT
<213> Homo sapiens

<400> 37

Met Ala Ala Gly Gly Pro Gly Ala Gly Ser Ala Ala Pro Val Ser Ser
1 5 10 15

Thr Ser Ser Leu Pro Leu Ala Ala Leu Asn Met Arg Val Arg Arg Arg
20 25 30

Leu Ser Leu Phe Leu Asn Val Arg Thr Gln Val Ala Ala Asp Trp Thr
35 40 45

Ala Leu Ala Glu Glu Met Asp Phe Glu Tyr Leu Glu Ile Arg Gln Leu
50 55 60

Glu Thr Gln Ala Asp Pro Thr Gly Arg Leu Leu Asp Ala Trp Gln Gly
65 70 75 80

Arg Pro Gly Ala Ser Val Gly Arg Leu Leu Glu Leu Leu Thr Lys Leu
85 90 95

Gly Arg Asp Asp Val Leu Leu Glu Leu Gly Pro Ser Ile Gly Ala Ala
100 105 110

Gly Trp Trp Trp Leu Ser Leu Met Ile Thr Cys Arg Ala Arg Asn Val
115 120 125

Thr Ser Arg Pro Asn Leu His Ser Ala Ser Leu Gln Val Pro Ile Arg
130 135 140

Ser Asp
145

<210> 38

<211> 296
<212> PRT
<213> Homo sapiens

<400> 38

Met Ala Ala Gly Gly Pro Gly Ala Gly Ser Ala Ala Pro Val Ser Ser
1 5 10 15

Thr Ser Ser Leu Pro Leu Ala Ala Leu Asn Met Arg Val Arg Arg Arg
20 25 30

Leu Ser Leu Phe Leu Asn Val Arg Thr Gln Val Ala Ala Asp Trp Thr
35 40 45

Ala Leu Ala Glu Glu Met Asp Phe Glu Tyr Leu Glu Ile Arg Gln Leu
50 55 60

Glu Thr Gln Ala Asp Pro Thr Gly Arg Leu Leu Asp Ala Trp Gln Gly
65 70 75 80

Arg Pro Gly Ala Ser Val Gly Arg Leu Leu Glu Leu Leu Thr Lys Leu
85 90 95

Gly Arg Asp Asp Val Leu Leu Glu Leu Gly Pro Ser Ile Glu Glu Asp
100 105 110

Cys Gln Lys Tyr Ile Leu Lys Gln Gln Gln Glu Glu Ala Glu Lys Pro
115 120 125

Leu Gln Val Ala Ala Val Asp Ser Ser Val Pro Arg Thr Ala Glu Leu
130 135 140

Ala Gly Ile Thr Thr Leu Asp Asp Pro Leu Gly His Met Pro Glu Arg
145 150 155 160

Phe Asp Ala Phe Ile Cys Tyr Cys Pro Ser Asp Ile Gln Phe Val Gln
165 170 175

Glu Met Ile Arg Gln Leu Glu Gln Thr Asn Tyr Arg Leu Lys Leu Cys
180 185 190

Val Ser Asp Arg Asp Val Leu Pro Gly Thr Cys Val Trp Ser Ile Ala
195 200 205

Ser Glu Leu Ile Glu Lys Arg Cys Arg Arg Met Val Val Val Val Ser
210 215 220

Asp Asp Tyr Leu Gln Ser Lys Glu Cys Asp Phe Gln Thr Lys Phe Ala

225 230 235 240

Leu Ser Leu Ser Pro Gly Ala His Gln Lys Arg Leu Ile Pro Ile Lys
245 250 255

Tyr Lys Ala Met Lys Lys Glu Phe Pro Ser Ile Leu Arg Phe Ile Thr
260 265 270

Val Cys Asp Tyr Thr Asn Pro Cys Thr Lys Ser Trp Phe Trp Thr Arg
275 280 285

Leu Ala Lys Ala Leu Ser Leu Pro
290 295

<210> 39
<211> 1614
<212> DNA
<213> Homo sapiens

<400> 39
gcatttccag agcagagttc agagaaaggc tgggctgctt gttgctggct aaaggacaaa 60
gggtaagttt caggaagcag aagagtgagc agatgaaatt cagcactggg atcaggggag 120
tgtctgattt gcaaaaggaa agtgcaaaga cagctcctcc cttctgagga aacgaaacca 180
acagcagtcc aagctcagtc agcagaagag ataaaagcaa acaggtctgg gaggcagttc 240
tgttgccact ctctctcctg tcaatgatgg atctcagaaa taccccagcc aaatctctgg 300
acaagttcat tgaagactat ctcttgccag acacgtgttt ccgcatgcaa atcaaccatg 360
ccattgacat catctgtggg ttcctgaagg aaaggtgctt ccgaggtagc tcctaccctg 420
tgtgtgtgtc caaggtggta aagggtggct cctcaggcaa gggcaccacc ctcagaggcc 480
gatctgacgc tgacctggtt gtcttcctca gtcctctcac cacttttcag gatcagttaa 540
atcgccgggg agagttcatc caggaaatta ggagacagct ggaagcctgt caaagagaga 600
gagcattttc cgtgaagttt gaggtccagg ctccacgctg gggcaacccc cgtgcgctca 660
gcttcgtact gagttcgctc cagctcgggg aggggtgga gttcgatgtg ctgcctgcct 720
ttgatgccct gggtcagttg actggcggct ataaacctaa cccccaaatc tatgtcaagc 780
tcatcgagga gtgcaccgac ctgcagaaag agggcgagtt ctccacctgc ttcacagaac 840
tacagagaga cttcctgaag cagcgcccca ccaagctcaa gagcctcatc cgcctagtca 900
agcactggta ccaaaattgt aagaagaagc ttgggaagct gccacctcag tatgccctgg 960
agctcctgac ggtctatgct tgggagcgag ggagcatgaa aacacatttc aacacagccc 1020
agggatttcg gacggtcttg gaattagtca taaactacca gcaactctgc atctactgga 1080
caaagtatta tgactttaaa aaccccatta ttgaaaagta cctgagaagg cagctcacga 1140

aacccaggcc tgtgatcctg gacccggcgg accctacagg aaacttgggt ggtggagacc 1200

caaagggttg gaggcagctg gcacaagagg ctgaggcctg gctgaattac ccatgcttta 1260

agaattggga tgggtcccca gtgagctcct ggattctgct ggtaaacctc acactggttg 1320

gcagaaggaa ctataccaat aattagtgaa catgcggtga atttgcaaca gacaagagga 1380

gcctcattat cctatagttt ccaggttgct tagggaggca gaaatcacag caaggaaaac 1440

cttcaataat aaacagacgt ctcataaaat taattgcaac ccaacctctc tctctactta 1500

aaattagcat ctatttccag ctctgctttc aatgccccat atgaatacat gtgaactccc 1560

tccctctctt cctccctgtc tccttctctc tctctctgtc cctcattaaa aaat 1614

<210> 40
<211> 1627
<212> DNA
<213> Homo sapiens

<400> 40
gcatttccag agcagagttc agagaaaggc tgggctgctt gttgctggct aaaggacaaa 60

gggtaagttt caggaagcag aagagtgagc agatgaaatt cagcactggg atcaggggag 120

tgtctgattt gcaaaaggaa agtgcaaaga cagctcctcc cttctgagga aacgaaacca 180

acagcagtcc aagctcagtc agcagaagag ataaaagcaa acaggtctgg gaggcagttc 240

tgttgccact ctctctcctg tcaatgatgg atctcagaaa taccccagcc aaatctctgg 300

acaagttcat tgaagactat ctcttgccag acacgtgttt ccgcatgcaa atcaaccatg 360

ccattgacat catctgtggg ttcctgaagg aaaggtgctt ccgaggtagc tcctaccctg 420

tgtgtgtgtc caaggtggta aagggtggct cctcaggcaa gggcaccacc ctcagaggcc 480

gatctgacgc tgacctggtt gtcttcctca gtcctctcac cacttttcag gatcagttaa 540

atcgccgggg agagttcatc caggaaatta ggagacagct ggaagcctgt caaagagaga 600

gagcattttc cgtgaagttt gaggtccagg ctccacgctg gggcaacccc cgtgcgctca 660

gcttcgtact gagttcgctc cagctcgggg aggggtgga gttcgatgtg ctgcctgcct 720

ttgatgccct gggtcagttg actggcggct ataaacctaa cccccaaatc tatgtcaagc 780

tcatcgagga gtgcaccgac ctgcagaaag agggcgagtt ctccacctgc ttcacagaac 840

tacagagaga cttcctgaag cagcgcccca ccaagctcaa gagcctcatc cgcctagtca 900

agcactggta ccaaaattgt aagaagaagc ttgggaagct gccacctcag tatgccctgg 960

agctcctgac ggtctatgct tgggagcgag ggagcatgaa aacacatttc aacacagccc 1020

agggatttcg gacggtcttg gaattagtca taaactacca gcaactctgc atctactgga 1080

caaagtatta tgactttaaa aaccccatta ttgaaaagta cctgagaagg cagctcacga 1140

aacccaggcc tgtgatcctg gacccggcgg accctacagg aaacttgggt ggtggagacc 1200

caaagggttg gaggcagctg gcacaagagg ctgaggcctg gctgaattac ccatgcttta 1260

agaattggga tgggtcccca gtgagctcct ggattctgct ggtgagacct cctgcttcct 1320

ccctgccatt catccctgcc cctctccatg aagcttgaga catatagctg gagaccattc 1380

tttccaaaga acttacctct tgccaaaggc catttatatt catatagtga caggctgtgc 1440

tccatatttt acagtcattt tggtcacaat cgagggtttc tggaattttc acatcccttg 1500

tccagaattc attcccctaa gagtaataat aaataatctc taacaccatt tattgactgt 1560

ctgcttcggg ctcaggttct gtcctaagcc ctttaatatg cactctctca ttaaatagtc 1620

acaacaa 1627

<210> 41
<211> 1533
<212> DNA
<213> Homo sapiens

<400> 41
agtccaagct cagtcagcag aagagataaa agcaaacagg tctgggaggc agttctgttg 60

ccactctctc tcctgtcaat gatggatctc agaaataccc cagccaaatc tctggacaag 120

ttcattgaag actatctctt gccagacacg tgtttccgca tgcaaatcaa ccatgccatt 180

gacatcatct gtgggttcct gaaggaaagg tgcttccgag gtagctccta ccctgtgtgt 240

gtgtccaagg tggtaaaggg tggctcctca ggcaagggca ccaccctcag aggccgatct 300

gacgctgacc tggttgtctt cctcagtcct ctcaccactt ttcaggatca gttaaatcgc 360

cggggagagt tcatccagga aattaggaga cagctggaag cctgtcaaag agagagagca 420

ttttccgtga agtttgaggt ccaggctcca cgctggggca acccccgtgc gctcagcttc 480

gtactgagtt cgctccagct cggggagggg gtggagttcg atgtgctgcc tgcctttgat 540

gccctgggtc agttgactgg cggctataaa cctaaccccc aaatctatgt caagctcatc 600

gaggagtgca ccgacctgca gaaagagggc gagttctcca cctgcttcac agaactacag 660

agagacttcc tgaagcagcg ccccaccaag ctcaagagcc tcatccgcct agtcaagcac 720

tggtaccaaa attgtaagaa gaagcttggg aagctgccac ctcagtatgc cctggagctc 780

ctgacggtct atgcttggga gcgagggagc atgaaaacac atttcaacac agcccaggga 840

tttcggacgg tcttggaatt agtcataaac taccagcaac tctgcatcta ctggacaaag 900

tattatgact ttaaaaaccc cattattgaa aagtacctga gaaggcagct cacgaaaccc 960

aggcctgtga tcctggaccc ggcggaccct acaggaaact tgggtggtgg agacccaaag 1020

ggttggaggc agctggcaca agaggctgag gcctggctga attacccatg ctttaagaat 1080

tgggatgggt ccccagtgag ctcctggatt ctgctgaccc agcacactcc aggcagcatc 1140

caccccacag gcagaagagg actggacctg caccatcctc tgaatgccag tgcatcttgg 1200

gggaaagggc tccagtgtta tctggaccag ttccttcatt ttcaggtggg actcttgatc 1260

cagagaggac aaagctcctc agtgagctgg tgtataatcc aggacagaac ccaggtctcc 1320

tgactcctgg ccttctatgc cctctatcct atcatagata acattctcca cagcctcact 1380

tcattccacc tattctctga aaatattccc tgagagagaa cagagagatt tagataagag 1440

aatgaaattc cagccttgac tttcttctgt gcacctgatg ggagggtaat gtctaatgta 1500

ttatcaataa caataaaaat aaagcaaata cca 1533

<210> 42
<211> 1631
<212> DNA
<213> Homo sapiens

<400> 42
agtccaagct cagtcagcag aagagataaa agcaaacagg tctgggaggc agttctgttg 60

ccactctctc tcctgtcaat gatggatctc agaaataccc cagccaaatc tctggacaag 120

ttcattgaag actatctctt gccagacacg tgtttccgca tgcaaatcaa ccatgccatt 180

gacatcatct gtgggttcct gaaggaaagg tgcttccgag gtagctccta ccctgtgtgt 240

gtgtccaagg tggtaaaggg tggctcctca ggcaagggca ccaccctcag aggccgatct 300

gacgctgacc tggttgtctt cctcagtcct ctcaccactt ttcaggatca gttaaatcgc 360

cggggagagt tcatccagga aattaggaga cagctggaag cctgtcaaag agagagagca 420

ttttccgtga agtttgaggt ccaggctcca cgctggggca acccccgtgc gctcagcttc 480

gtactgagtt cgctccagct cggggagggg gtggagttcg atgtgctgcc tgcctttgat 540

gccctgggtc agttgactgg cggctataaa cctaaccccc aaatctatgt caagctcatc 600

gaggagtgca ccgacctgca gaaagagggc gagttctcca cctgcttcac agaactacag 660

agagacttcc tgaagcagcg ccccaccaag ctcaagagcc tcatccgcct agtcaagcac 720

tggtaccaaa attgtaagaa gaagcttggg aagctgccac ctcagtatgc cctggagctc 780

ctgacggtct atgcttggga gcgagggagc atgaaaacac atttcaacac agcccaggga 840

tttcggacgg tcttggaatt agtcataaac taccagcaac tctgcatcta ctggacaaag 900

tattatgact ttaaaaaccc cattattgaa aagtacctga gaaggcagct cacgaaaccc 960

aggcctgtga tcctggaccc ggcggaccct acaggaaact tgggtggtgg agacccaaag 1020

ggttggaggc agctggcaca agaggctgag gcctggctga attacccatg ctttaagaat 1080

tgggatgggt ccccagtgag ctcctggatt ctgctggctg aaagcaacag tgcagacgat 1140

gagaccgacg atcccaggag gtatcagaaa tatggttaca ttggaacaca tgagtaccct 1200

catttctctc atagacccag cacactccag gcagcatcca ccccacaggc agaagaggac 1260

tggacctgca ccatcctctg aatgccagtg catcttgggg gaaagggctc cagtgttatc 1320

tggaccagtt ccttcatttt caggtgggac tcttgatcca gagaggacaa agctcctcag 1380

tgagctggtg tataatccag gacagaaccc aggtctcctg actcctggcc ttctatgccc 1440

tctatcctat catagataac attctccaca gcctcacttc attccaccta ttctctgaaa 1500

atattccctg agagagaaca gagagattta gataagagaa tgaaattcca gccttgactt 1560

tcttctgtgc acctgatggg agggtaatgt ctaatgtatt atcaataaca ataaaaataa 1620

agcaaatacc a 1631

<210> 43
<211> 360
<212> PRT
<213> Homo sapiens

<400> 43

Met Met Asp Leu Arg Asn Thr Pro Ala Lys Ser Leu Asp Lys Phe Ile
1 5 10 15

Glu Asp Tyr Leu Leu Pro Asp Thr Cys Phe Arg Met Gln Ile Asn His
20 25 30

Ala Ile Asp Ile Ile Cys Gly Phe Leu Lys Glu Arg Cys Phe Arg Gly
35 40 45

Ser Ser Tyr Pro Val Cys Val Ser Lys Val Val Lys Gly Gly Ser Ser
50 55 60

Gly Lys Gly Thr Thr Leu Arg Gly Arg Ser Asp Ala Asp Leu Val Val
65 70 75 80

Phe Leu Ser Pro Leu Thr Thr Phe Gln Asp Gln Leu Asn Arg Arg Gly
85 90 95

Glu Phe Ile Gln Glu Ile Arg Arg Gln Leu Glu Ala Cys Gln Arg Glu
100 105 110

Arg Ala Phe Ser Val Lys Phe Glu Val Gln Ala Pro Arg Trp Gly Asn
115 120 125

Pro Arg Ala Leu Ser Phe Val Leu Ser Ser Leu Gln Leu Gly Glu Gly
130 135 140

Val Glu Phe Asp Val Leu Pro Ala Phe Asp Ala Leu Gly Gln Leu Thr
145 150 155 160

Gly Gly Tyr Lys Pro Asn Pro Gln Ile Tyr Val Lys Leu Ile Glu Glu
165 170 175

Cys Thr Asp Leu Gln Lys Glu Gly Glu Phe Ser Thr Cys Phe Thr Glu
180 185 190

Leu Gln Arg Asp Phe Leu Lys Gln Arg Pro Thr Lys Leu Lys Ser Leu
195 200 205

Ile Arg Leu Val Lys His Trp Tyr Gln Asn Cys Lys Lys Lys Leu Gly
210 215 220

Lys Leu Pro Pro Gln Tyr Ala Leu Glu Leu Leu Thr Val Tyr Ala Trp
225 230 235 240

Glu Arg Gly Ser Met Lys Thr His Phe Asn Thr Ala Gln Gly Phe Arg
245 250 255

Thr Val Leu Glu Leu Val Ile Asn Tyr Gln Gln Leu Cys Ile Tyr Trp
260 265 270

Thr Lys Tyr Tyr Asp Phe Lys Asn Pro Ile Ile Glu Lys Tyr Leu Arg
275 280 285

Arg Gln Leu Thr Lys Pro Arg Pro Val Ile Leu Asp Pro Ala Asp Pro
290 295 300

Thr Gly Asn Leu Gly Gly Gly Asp Pro Lys Gly Trp Arg Gln Leu Ala
305 310 315 320

Gln Glu Ala Glu Ala Trp Leu Asn Tyr Pro Cys Phe Lys Asn Trp Asp
325 330 335

Gly Ser Pro Val Ser Ser Trp Ile Leu Leu Val Asn Leu Thr Leu Val
340 345 350

Gly Arg Arg Asn Tyr Thr Asn Asn
355 360

<210> 44
<211> 364
<212> PRT
<213> Homo sapiens

<400> 44

Met Met Asp Leu Arg Asn Thr Pro Ala Lys Ser Leu Asp Lys Phe Ile
1 5 10 15

Glu Asp Tyr Leu Leu Pro Asp Thr Cys Phe Arg Met Gln Ile Asn His
20 25 30

```
Ala Ile Asp Ile Ile Cys Gly Phe Leu Lys Glu Arg Cys Phe Arg Gly
        35                  40                  45

Ser Ser Tyr Pro Val Cys Val Ser Lys Val Val Lys Gly Gly Ser Ser
    50                  55                  60

Gly Lys Gly Thr Thr Leu Arg Gly Arg Ser Asp Ala Asp Leu Val Val
65                  70                  75                  80

Phe Leu Ser Pro Leu Thr Thr Phe Gln Asp Gln Leu Asn Arg Arg Gly
                85                  90                  95

Glu Phe Ile Gln Glu Ile Arg Arg Gln Leu Glu Ala Cys Gln Arg Glu
            100                 105                 110

Arg Ala Phe Ser Val Lys Phe Glu Val Gln Ala Pro Arg Trp Gly Asn
        115                 120                 125

Pro Arg Ala Leu Ser Phe Val Leu Ser Ser Leu Gln Leu Gly Glu Gly
    130                 135                 140

Val Glu Phe Asp Val Leu Pro Ala Phe Asp Ala Leu Gly Gln Leu Thr
145                 150                 155                 160

Gly Gly Tyr Lys Pro Asn Pro Gln Ile Tyr Val Lys Leu Ile Glu Glu
                165                 170                 175

Cys Thr Asp Leu Gln Lys Glu Gly Glu Phe Ser Thr Cys Phe Thr Glu
            180                 185                 190

Leu Gln Arg Asp Phe Leu Lys Gln Arg Pro Thr Lys Leu Lys Ser Leu
        195                 200                 205

Ile Arg Leu Val Lys His Trp Tyr Gln Asn Cys Lys Lys Lys Leu Gly
    210                 215                 220

Lys Leu Pro Pro Gln Tyr Ala Leu Glu Leu Leu Thr Val Tyr Ala Trp
225                 230                 235                 240

Glu Arg Gly Ser Met Lys Thr His Phe Asn Thr Ala Gln Gly Phe Arg
                245                 250                 255

Thr Val Leu Glu Leu Val Ile Asn Tyr Gln Gln Leu Cys Ile Tyr Trp
            260                 265                 270

Thr Lys Tyr Tyr Asp Phe Lys Asn Pro Ile Ile Glu Lys Tyr Leu Arg
        275                 280                 285
```

Arg Gln Leu Thr Lys Pro Arg Pro Val Ile Leu Asp Pro Ala Asp Pro
    290                 295                 300

Thr Gly Asn Leu Gly Gly Gly Asp Pro Lys Gly Trp Arg Gln Leu Ala
305                 310                 315                 320

Gln Glu Ala Glu Ala Trp Leu Asn Tyr Pro Cys Phe Lys Asn Trp Asp
                325                 330                 335

Gly Ser Pro Val Ser Ser Trp Ile Leu Leu Val Arg Pro Pro Ala Ser
            340                 345                 350

Ser Leu Pro Phe Ile Pro Ala Pro Leu His Glu Ala
        355                 360

<210>  45
<211>  414
<212>  PRT
<213>  Homo sapiens

<400>  45

Met Met Asp Leu Arg Asn Thr Pro Ala Lys Ser Leu Asp Lys Phe Ile
1               5                   10                  15

Glu Asp Tyr Leu Leu Pro Asp Thr Cys Phe Arg Met Gln Ile Asn His
            20                  25                  30

Ala Ile Asp Ile Ile Cys Gly Phe Leu Lys Glu Arg Cys Phe Arg Gly
        35                  40                  45

Ser Ser Tyr Pro Val Cys Val Ser Lys Val Val Lys Gly Gly Ser Ser
    50                  55                  60

Gly Lys Gly Thr Thr Leu Arg Gly Arg Ser Asp Ala Asp Leu Val Val
65                  70                  75                  80

Phe Leu Ser Pro Leu Thr Thr Phe Gln Asp Gln Leu Asn Arg Arg Gly
                85                  90                  95

Glu Phe Ile Gln Glu Ile Arg Arg Gln Leu Glu Ala Cys Gln Arg Glu
            100                 105                 110

Arg Ala Phe Ser Val Lys Phe Glu Val Gln Ala Pro Arg Trp Gly Asn
        115                 120                 125

Pro Arg Ala Leu Ser Phe Val Leu Ser Ser Leu Gln Leu Gly Glu Gly
    130                 135                 140

Val Glu Phe Asp Val Leu Pro Ala Phe Asp Ala Leu Gly Gln Leu Thr
145 150 155 160

Gly Gly Tyr Lys Pro Asn Pro Gln Ile Tyr Val Lys Leu Ile Glu Glu
165 170 175

Cys Thr Asp Leu Gln Lys Glu Gly Glu Phe Ser Thr Cys Phe Thr Glu
180 185 190

Leu Gln Arg Asp Phe Leu Lys Gln Arg Pro Thr Lys Leu Lys Ser Leu
195 200 205

Ile Arg Leu Val Lys His Trp Tyr Gln Asn Cys Lys Lys Lys Leu Gly
210 215 220

Lys Leu Pro Pro Gln Tyr Ala Leu Glu Leu Leu Thr Val Tyr Ala Trp
225 230 235 240

Glu Arg Gly Ser Met Lys Thr His Phe Asn Thr Ala Gln Gly Phe Arg
245 250 255

Thr Val Leu Glu Leu Val Ile Asn Tyr Gln Gln Leu Cys Ile Tyr Trp
260 265 270

Thr Lys Tyr Tyr Asp Phe Lys Asn Pro Ile Ile Glu Lys Tyr Leu Arg
275 280 285

Arg Gln Leu Thr Lys Pro Arg Pro Val Ile Leu Asp Pro Ala Asp Pro
290 295 300

Thr Gly Asn Leu Gly Gly Gly Asp Pro Lys Gly Trp Arg Gln Leu Ala
305 310 315 320

Gln Glu Ala Glu Ala Trp Leu Asn Tyr Pro Cys Phe Lys Asn Trp Asp
325 330 335

Gly Ser Pro Val Ser Ser Trp Ile Leu Leu Thr Gln His Thr Pro Gly
340 345 350

Ser Ile His Pro Thr Gly Arg Arg Gly Leu Asp Leu His His Pro Leu
355 360 365

Asn Ala Ser Ala Ser Trp Gly Lys Gly Leu Gln Cys Tyr Leu Asp Gln
370 375 380

Phe Leu His Phe Gln Val Gly Leu Leu Ile Gln Arg Gly Gln Ser Ser

385 390 395 400

Ser Val Ser Trp Cys Ile Ile Gln Asp Arg Thr Gln Val Ser
405 410

<210> 46
<211> 400
<212> PRT
<213> Homo sapiens

<400> 46

Met Met Asp Leu Arg Asn Thr Pro Ala Lys Ser Leu Asp Lys Phe Ile
1 5 10 15

Glu Asp Tyr Leu Leu Pro Asp Thr Cys Phe Arg Met Gln Ile Asn His
20 25 30

Ala Ile Asp Ile Ile Cys Gly Phe Leu Lys Glu Arg Cys Phe Arg Gly
35 40 45

Ser Ser Tyr Pro Val Cys Val Ser Lys Val Val Lys Gly Gly Ser Ser
50 55 60

Gly Lys Gly Thr Thr Leu Arg Gly Arg Ser Asp Ala Asp Leu Val Val
65 70 75 80

Phe Leu Ser Pro Leu Thr Thr Phe Gln Asp Gln Leu Asn Arg Arg Gly
85 90 95

Glu Phe Ile Gln Glu Ile Arg Arg Gln Leu Glu Ala Cys Gln Arg Glu
100 105 110

Arg Ala Phe Ser Val Lys Phe Glu Val Gln Ala Pro Arg Trp Gly Asn
115 120 125

Pro Arg Ala Leu Ser Phe Val Leu Ser Ser Leu Gln Leu Gly Glu Gly
130 135 140

Val Glu Phe Asp Val Leu Pro Ala Phe Asp Ala Leu Gly Gln Leu Thr
145 150 155 160

Gly Gly Tyr Lys Pro Asn Pro Gln Ile Tyr Val Lys Leu Ile Glu Glu
165 170 175

Cys Thr Asp Leu Gln Lys Glu Gly Glu Phe Ser Thr Cys Phe Thr Glu
180 185 190

Leu Gln Arg Asp Phe Leu Lys Gln Arg Pro Thr Lys Leu Lys Ser Leu

195 200 205

Ile Arg Leu Val Lys His Trp Tyr Gln Asn Cys Lys Lys Lys Leu Gly
210 215 220

Lys Leu Pro Pro Gln Tyr Ala Leu Glu Leu Leu Thr Val Tyr Ala Trp
225 230 235 240

Glu Arg Gly Ser Met Lys Thr His Phe Asn Thr Ala Gln Gly Phe Arg
245 250 255

Thr Val Leu Glu Leu Val Ile Asn Tyr Gln Gln Leu Cys Ile Tyr Trp
260 265 270

Thr Lys Tyr Tyr Asp Phe Lys Asn Pro Ile Ile Glu Lys Tyr Leu Arg
275 280 285

Arg Gln Leu Thr Lys Pro Arg Pro Val Ile Leu Asp Pro Ala Asp Pro
290 295 300

Thr Gly Asn Leu Gly Gly Gly Asp Pro Lys Gly Trp Arg Gln Leu Ala
305 310 315 320

Gln Glu Ala Glu Ala Trp Leu Asn Tyr Pro Cys Phe Lys Asn Trp Asp
325 330 335

Gly Ser Pro Val Ser Ser Trp Ile Leu Leu Ala Glu Ser Asn Ser Ala
340 345 350

Asp Asp Glu Thr Asp Asp Pro Arg Arg Tyr Gln Lys Tyr Gly Tyr Ile
355 360 365

Gly Thr His Glu Tyr Pro His Phe Ser His Arg Pro Ser Thr Leu Gln
370 375 380

Ala Ala Ser Thr Pro Gln Ala Glu Glu Asp Trp Thr Cys Thr Ile Leu
385 390 395 400

<210> 47
<211> 4216
<212> DNA
<213> Homo sapiens

<400> 47
agtttctctt ctcggccacc tcctgcatag agggtaccat tctgcgctgc tgcaagttac 60

ggaatgaaaa attagaacaa cagaaacatg gaaaacatgt tccttcagtc gtcaatgctg 120

acctgcattt tcctgctaat atctggttcc tgtgagttat gcgccgaaga aaatttttct 180

agaagctatc cttgtgatga gaaaaagcaa aatgactcag ttattgcaga gtgcagcaat 240

cgtcgactac aggaagttcc ccaaacggtg ggcaaatatg tgacagaact agacctgtct 300

gataatttca tcacacacat aacgaatgaa tcatttcaag ggctgcaaaa tctcactaaa 360

ataaatctaa accacaaccc caatgtacag caccagaacg gaaatcccgg tatacaatca 420

aatggcttga atatcacaga cggggcattc ctcaacctaa aaaacctaag ggagttactg 480

cttgaagaca accagttacc ccaaataccc tctggtttgc cagagtcttt gacagaactt 540

agtctaattc aaaacaatat atacaacata actaaagagg gcatttcaag acttataaac 600

ttgaaaaatc tctatttggc ctggaactgc tattttaaca aagtttgcga gaaaactaac 660

atagaagatg gagtatttga aacgctgaca aatttggagt tgctatcact atctttcaat 720

tctctttcac acgtgccacc caaactgcca agctccctac gcaaactttt tctgagcaac 780

acccagatca aatacattag tgaagaagat ttcaagggat tgataaattt aacattacta 840

gatttaagcg ggaactgtcc gaggtgcttc aatgccccat ttccatgcgt gccttgtgat 900

ggtggtgctt caattaatat agatcgtttt gcttttcaaa acttgaccca acttcgatac 960

ctaaacctct ctagcacttc cctcaggaag attaatgctg cctggtttaa aaatatgcct 1020

catctgaagg tgctggatct tgaattcaac tatttagtgg gagaaatagc ctctggggca 1080

tttttaacga tgctgccccg cttagaaata cttgacttgt cttttaacta tataaagggg 1140

agttatccac agcatattaa tatttccaga aacttctcta aacttttgtc tctacgggca 1200

ttgcatttaa gaggttatgt gttccaggaa ctcagagaag atgatttcca gcccctgatg 1260

cagcttccaa acttatcgac tatcaacttg ggtattaatt ttattaagca aatcgatttc 1320

aaacttttcc aaaatttctc caatctggaa attatttact tgtcagaaaa cagaatatca 1380

ccgttggtaa aagatacccg gcagagttat gcaaatagtt cctcttttca acgtcatatc 1440

cggaaacgac gctcaacaga ttttgagttt gacccacatt cgaactttta tcatttcacc 1500

cgtcctttaa taaagccaca atgtgctgct tatggaaaag ccttagattt aagcctcaac 1560

agtattttct tcattgggcc aaaccaattt gaaaatcttc ctgacattgc ctgtttaaat 1620

ctgtctgcaa atagcaatgc tcaagtgtta agtggaactg aattttcagc cattcctcat 1680

gtcaaatatt tggatttgac aaacaataga ctagactttg ataatgctag tgctcttact 1740

gaattgtccg acttggaagt tctagatctc agctataatt cacactattt cagaatagca 1800

ggcgtaacac atcatctaga atttattcaa aatttcacaa atctaaaagt tttaaacttg 1860

agccacaaca acatttatac tttaacagat aagtataacc tggaaagcaa gtccctggta 1920

gaattagttt tcagtggcaa tcgccttgac attttgtgga atgatgatga caacaggtat 1980

atctccattt tcaaaggtct caagaatctg acacgtctgg atttatccct taataggctg 2040

aagcacatcc caaatgaagc attccttaat ttgccagcga gtctcactga actacatata 2100

aatgataata tgttaaagtt ttttaactgg acattactcc agcagtttcc tcgtctcgag 2160

ttgcttgact tacgtggaaa caaactactc tttttaactg atagcctatc tgactttaca 2220

tcttcccttc ggacactgct gctgagtcat aacaggattt cccacctacc ctctggcttt 2280

ctttctgaag tcagtagtct gaagcacctc gatttaagtt ccaatctgct aaaaacaatc 2340

aacaaatccg cacttgaaac taagaccacc accaaattat ctatgttgga actacacgga 2400

aacccctttg aatgcacctg tgacattgga gatttccgaa gatggatgga tgaacatctg 2460

aatgtcaaaa ttcccagact ggtagatgtc atttgtgcca gtcctgggga tcaaagaggg 2520

aagagtattg tgagtctgga gctaacaact tgtgtttcag atgtcactgc agtgatatta 2580

tttttcttca cgttctttat caccaccatg gttatgttgg ctgccctggc tcaccatttg 2640

ttttactggg atgtttggtt tatatataat gtgtgtttag ctaaggtaaa aggctacagg 2700

tctctttcca catcccaaac tttctatgat gcttacattt cttatgacac caaagatgcc 2760

tctgttactg actgggtgat aaatgagctg cgctaccacc ttgaagagag ccgagacaaa 2820

aacgttctcc tttgtctaga ggagagggat tgggacccgg gattggccat catcgacaac 2880

ctcatgcaga gcatcaacca aagcaagaaa acagtatttg ttttaaccaa aaaatatgca 2940

aaaagctgga actttaaaac agctttttac ttggctttgc agaggctaat ggatgagaac 3000

atggatgtga ttatatttat cctgctggag ccagtgttac agcattctca gtatttgagg 3060

ctacggcagc ggatctgtaa gagctccatc ctccagtggc ctgacaaccc gaaggcagaa 3120

ggcttgtttt ggcaaactct gagaaatgtg gtcttgactg aaaatgattc acggtataac 3180

aatatgtatg tcgattccat taagcaatac taactgacgt taagtcatga tttcgcgcca 3240

taataaagat gcaaaggaat gacatttctg tattagttat ctattgctat gtaacaaatt 3300

atcccaaaac ttagtggttt aaaacaacac atttgctggc ccacagtttt tgagggtcag 3360

gagtccaggc ccagcataac tgggtcctct gctcagggtg tctcagaggc tgcaatgtag 3420

gtgttcacca gagacatagg catcactggg gtcacactca tgtggttgtt ttctggattc 3480

aattcctcct gggctattgg ccaaaggcta tactcatgta agccatgcga gcctctccca 3540

caaggcagct tgcttcatca gagctagcaa aaaagagagg ttgctagcaa gatgaagtca 3600

caatcttttg taatcgaatc aaaaaagtga tatctcatca ctttggccat attctatttg 3660

ttagaagtaa accacaggtc ccaccagctc catgggagtg accacctcag tccagggaaa 3720

acagctgaag accaagatgg tgagctctga ttgcttcagt tggtcatcaa ctattttccc 3780

ttgactgctg tcctgggatg gcctgctatc ttgatgatag attgtgaata tcaggaggca 3840

gggatcactg tggaccatct tagcagttga cctaacacat cttcttttca atatctaaga 3900

acttttgcca ctgtgactaa tggtcctaat attaagctgt tgtttatatt tatcatatat 3960

ctatggctac atggttatat tatgctgtgg ttgcgttcgg ttttatttac agttgctttt 4020

acaaatattt gctgtaacat ttgacttcta aggtttagat gccatttaag aactgagatg 4080

gatagctttt aaagcatctt ttacttctta ccatttttta aaagtatgca gctaaattcg 4140

aagcttttgg tctatattgt taattgccat tgctgtaaat cttaaaatga atgaataaaa 4200

atgtttcatt ttacaa 4216

<210> 48
<211> 4353
<212> DNA
<213> Homo sapiens

<400> 48
agtttctctt ctcggccacc tcctgcatag agggtaccat tctgcgctgc tgcaagttac 60

ggaatgaaaa attagaacaa cagaaacatg gttctcttga cacttcagtg ttagggaaca 120

tcagcaagac ccatcccagg agaccttgaa ggaagccttt gaaagggaga atgaaggagt 180

catctttgca aaatagctcc tgcagcctgg gaaaggagac taaaaaggaa aacatgttcc 240

ttcagtcgtc aatgctgacc tgcattttcc tgctaatatc tggttcctgt gagttatgcg 300

ccgaagaaaa tttttctaga agctatcctt gtgatgagaa aaagcaaaat gactcagtta 360

ttgcagagtg cagcaatcgt cgactacagg aagttcccca aacggtgggc aaatatgtga 420

cagaactaga cctgtctgat aatttcatca cacacataac gaatgaatca tttcaagggc 480

tgcaaaatct cactaaaata aatctaaacc acaaccccaa tgtacagcac cagaacggaa 540

atcccggtat acaatcaaat ggcttgaata tcacagacgg ggcattcctc aacctaaaaa 600

acctaaggga gttactgctt gaagacaacc agttacccca aataccctct ggtttgccag 660

agtctttgac agaacttagt ctaattcaaa acaatatata caacataact aaagagggca 720

tttcaagact tataaacttg aaaaatctct atttggcctg gaactgctat tttaacaaag 780

tttgcgagaa aactaacata gaagatggag tatttgaaac gctgacaaat ttggagttgc 840

tatcactatc tttcaattct ctttcacacg tgccacccaa actgccaagc tccctacgca 900

aactttttct gagcaacacc cagatcaaat acattagtga agaagatttc aagggattga 960

taaatttaac attactagat ttaagcggga actgtccgag gtgcttcaat gccccatttc 1020

catgcgtgcc ttgtgatggt ggtgcttcaa ttaatataga tcgttttgct tttcaaaact 1080

tgacccaact tcgataccta aacctctcta gcacttccct caggaagatt aatgctgcct 1140

ggtttaaaaa tatgcctcat ctgaaggtgc tggatcttga attcaactat ttagtgggag 1200

aaatagcctc tggggcattt ttaacgatgc tgccccgctt agaaatactt gacttgtctt 1260

ttaactatat aaaggggagt tatccacagc atattaatat ttccagaaac ttctctaaac 1320

ttttgtctct acgggcattg catttaagag gttatgtgtt ccaggaactc agagaagatg 1380

atttccagcc cctgatgcag cttccaaact tatcgactat caacttgggt attaatttta 1440
ttaagcaaat cgatttcaaa cttttccaaa atttctccaa tctggaaatt atttacttgt 1500
cagaaaacag aatatcaccg ttggtaaaag atacccggca gagttatgca aatagttcct 1560
cttttcaacg tcatatccgg aaacgacgct caacagattt tgagtttgac ccacattcga 1620
acttttatca tttcacccgt cctttaataa agccacaatg tgctgcttat ggaaaagcct 1680
tagatttaag cctcaacagt attttcttca ttgggccaaa ccaatttgaa aatcttcctg 1740
acattgcctg tttaaatctg tctgcaaata gcaatgctca agtgttaagt ggaactgaat 1800
tttcagccat tcctcatgtc aaatatttgg atttgacaaa caatagacta gactttgata 1860
atgctagtgc tcttactgaa ttgtccgact tggaagttct agatctcagc tataattcac 1920
actatttcag aatagcaggc gtaacacatc atctagaatt tattcaaaat ttcacaaatc 1980
taaaagtttt aaacttgagc cacaacaaca tttatacttt aacagataag tataacctgg 2040
aaagcaagtc cctggtagaa ttagttttca gtggcaatcg ccttgacatt ttgtggaatg 2100
atgatgacaa caggtatatc tccattttca aaggtctcaa gaatctgaca cgtctggatt 2160
tatcccttaa taggctgaag cacatcccaa atgaagcatt ccttaatttg ccagcgagtc 2220
tcactgaact acatataaat gataatatgt taaagttttt taactggaca ttactccagc 2280
agtttcctcg tctcgagttg cttgacttac gtggaaacaa actactcttt ttaactgata 2340
gcctatctga ctttacatct tcccttcgga cactgctgct gagtcataac aggatttccc 2400
acctaccctc tggctttctt tctgaagtca gtagtctgaa gcacctcgat ttaagttcca 2460
atctgctaaa aacaatcaac aaatccgcac ttgaaactaa gaccaccacc aaattatcta 2520
tgttggaact acacggaaac ccctttgaat gcacctgtga cattggagat ttccgaagat 2580
ggatggatga acatctgaat gtcaaaattc ccagactggt agatgtcatt tgtgccagtc 2640
ctggggatca aagagggaag agtattgtga gtctggagct aacaacttgt gtttcagatg 2700
tcactgcagt gatattattt ttcttcacgt tctttatcac caccatggtt atgttggctg 2760
ccctggctca ccatttgttt tactgggatg tttggtttat atataatgtg tgtttagcta 2820
aggtaaaagg ctacaggtct ctttccacat cccaaacttt ctatgatgct tacatttctt 2880
atgacaccaa agatgcctct gttactgact gggtgataaa tgagctgcgc taccaccttg 2940
aagagagccg agacaaaaac gttctccttt gtctagagga gagggattgg gacccgggat 3000
tggccatcat cgacaacctc atgcagagca tcaaccaaag caagaaaaca gtatttgttt 3060
taaccaaaaa atatgcaaaa agctggaact ttaaaacagc tttttacttg gctttgcaga 3120
ggctaatgga tgagaacatg gatgtgatta tatttatcct gctggagcca gtgttacagc 3180
attctcagta tttgaggcta cggcagcgga tctgtaagag ctccatcctc cagtggcctg 3240
acaacccgaa ggcagaaggc ttgttttggc aaactctgag aaatgtggtc ttgactgaaa 3300

atgattcacg gtataacaat atgtatgtcg attccattaa gcaatactaa ctgacgttaa 3360

gtcatgattt cgcgccataa taaagatgca aaggaatgac atttctgtat tagttatcta 3420

ttgctatgta acaaattatc ccaaaactta gtggtttaaa acaacacatt tgctggccca 3480

cagtttttga gggtcaggag tccaggccca gcataactgg gtcctctgct cagggtgtct 3540

cagaggctgc aatgtaggtg ttcaccagag acataggcat cactggggtc acactcatgt 3600

ggttgttttc tggattcaat tcctcctggg ctattggcca aaggctatac tcatgtaagc 3660

catgcgagcc tctcccacaa ggcagcttgc ttcatcagag ctagcaaaaa agagaggttg 3720

ctagcaagat gaagtcacaa tcttttgtaa tcgaatcaaa aaagtgatat ctcatcactt 3780

tggccatatt ctatttgtta gaagtaaacc acaggtccca ccagctccat gggagtgacc 3840

acctcagtcc agggaaaaca gctgaagacc aagatggtga gctctgattg cttcagttgg 3900

tcatcaacta ttttcccttg actgctgtcc tgggatggcc tgctatcttg atgatagatt 3960

gtgaatatca ggaggcaggg atcactgtgg accatcttag cagttgacct aacacatctt 4020

cttttcaata tctaagaact tttgccactg tgactaatgg tcctaatatt aagctgttgt 4080

ttatatttat catatatcta tggctacatg gttatattat gctgtggttg cgttcggttt 4140

tatttacagt tgcttttaca aatatttgct gtaacatttg acttctaagg tttagatgcc 4200

atttaagaac tgagatggat agcttttaaa gcatctttta cttcttacca tttttaaaa 4260

gtatgcagct aaattcgaag cttttggtct atattgttaa ttgccattgc tgtaaatctt 4320

aaaatgaatg aataaaaatg tttcatttta caa 4353

<210> 49
<211> 1041
<212> PRT
<213> Homo sapiens

<400> 49

Met Glu Asn Met Phe Leu Gln Ser Ser Met Leu Thr Cys Ile Phe Leu
1 5 10 15

Leu Ile Ser Gly Ser Cys Glu Leu Cys Ala Glu Glu Asn Phe Ser Arg
20 25 30

Ser Tyr Pro Cys Asp Glu Lys Lys Gln Asn Asp Ser Val Ile Ala Glu
35 40 45

Cys Ser Asn Arg Arg Leu Gln Glu Val Pro Gln Thr Val Gly Lys Tyr
50 55 60

Val Thr Glu Leu Asp Leu Ser Asp Asn Phe Ile Thr His Ile Thr Asn
65 70 75 80

Glu Ser Phe Gln Gly Leu Gln Asn Leu Thr Lys Ile Asn Leu Asn His
85 90 95

Asn Pro Asn Val Gln His Gln Asn Gly Asn Pro Gly Ile Gln Ser Asn
100 105 110

Gly Leu Asn Ile Thr Asp Gly Ala Phe Leu Asn Leu Lys Asn Leu Arg
115 120 125

Glu Leu Leu Leu Glu Asp Asn Gln Leu Pro Gln Ile Pro Ser Gly Leu
130 135 140

Pro Glu Ser Leu Thr Glu Leu Ser Leu Ile Gln Asn Asn Ile Tyr Asn
145 150 155 160

Ile Thr Lys Glu Gly Ile Ser Arg Leu Ile Asn Leu Lys Asn Leu Tyr
165 170 175

Leu Ala Trp Asn Cys Tyr Phe Asn Lys Val Cys Glu Lys Thr Asn Ile
180 185 190

Glu Asp Gly Val Phe Glu Thr Leu Thr Asn Leu Glu Leu Leu Ser Leu
195 200 205

Ser Phe Asn Ser Leu Ser His Val Pro Pro Lys Leu Pro Ser Ser Leu
210 215 220

Arg Lys Leu Phe Leu Ser Asn Thr Gln Ile Lys Tyr Ile Ser Glu Glu
225 230 235 240

Asp Phe Lys Gly Leu Ile Asn Leu Thr Leu Leu Asp Leu Ser Gly Asn
245 250 255

Cys Pro Arg Cys Phe Asn Ala Pro Phe Pro Cys Val Pro Cys Asp Gly
260 265 270

Gly Ala Ser Ile Asn Ile Asp Arg Phe Ala Phe Gln Asn Leu Thr Gln
275 280 285

Leu Arg Tyr Leu Asn Leu Ser Ser Thr Ser Leu Arg Lys Ile Asn Ala
290 295 300

Ala Trp Phe Lys Asn Met Pro His Leu Lys Val Leu Asp Leu Glu Phe
305 310 315 320

Asn Tyr Leu Val Gly Glu Ile Ala Ser Gly Ala Phe Leu Thr Met Leu

325 330 335

Pro Arg Leu Glu Ile Leu Asp Leu Ser Phe Asn Tyr Ile Lys Gly Ser
340 345 350

Tyr Pro Gln His Ile Asn Ile Ser Arg Asn Phe Ser Lys Leu Leu Ser
355 360 365

Leu Arg Ala Leu His Leu Arg Gly Tyr Val Phe Gln Glu Leu Arg Glu
370 375 380

Asp Asp Phe Gln Pro Leu Met Gln Leu Pro Asn Leu Ser Thr Ile Asn
385 390 395 400

Leu Gly Ile Asn Phe Ile Lys Gln Ile Asp Phe Lys Leu Phe Gln Asn
405 410 415

Phe Ser Asn Leu Glu Ile Ile Tyr Leu Ser Glu Asn Arg Ile Ser Pro
420 425 430

Leu Val Lys Asp Thr Arg Gln Ser Tyr Ala Asn Ser Ser Ser Phe Gln
435 440 445

Arg His Ile Arg Lys Arg Arg Ser Thr Asp Phe Glu Phe Asp Pro His
450 455 460

Ser Asn Phe Tyr His Phe Thr Arg Pro Leu Ile Lys Pro Gln Cys Ala
465 470 475 480

Ala Tyr Gly Lys Ala Leu Asp Leu Ser Leu Asn Ser Ile Phe Phe Ile
485 490 495

Gly Pro Asn Gln Phe Glu Asn Leu Pro Asp Ile Ala Cys Leu Asn Leu
500 505 510

Ser Ala Asn Ser Asn Ala Gln Val Leu Ser Gly Thr Glu Phe Ser Ala
515 520 525

Ile Pro His Val Lys Tyr Leu Asp Leu Thr Asn Asn Arg Leu Asp Phe
530 535 540

Asp Asn Ala Ser Ala Leu Thr Glu Leu Ser Asp Leu Glu Val Leu Asp
545 550 555 560

Leu Ser Tyr Asn Ser His Tyr Phe Arg Ile Ala Gly Val Thr His His
565 570 575

Leu Glu Phe Ile Gln Asn Phe Thr Asn Leu Lys Val Leu Asn Leu Ser
580 585 590

His Asn Asn Ile Tyr Thr Leu Thr Asp Lys Tyr Asn Leu Glu Ser Lys
595 600 605

Ser Leu Val Glu Leu Val Phe Ser Gly Asn Arg Leu Asp Ile Leu Trp
610 615 620

Asn Asp Asp Asp Asn Arg Tyr Ile Ser Ile Phe Lys Gly Leu Lys Asn
625 630 635 640

Leu Thr Arg Leu Asp Leu Ser Leu Asn Arg Leu Lys His Ile Pro Asn
645 650 655

Glu Ala Phe Leu Asn Leu Pro Ala Ser Leu Thr Glu Leu His Ile Asn
660 665 670

Asp Asn Met Leu Lys Phe Phe Asn Trp Thr Leu Leu Gln Gln Phe Pro
675 680 685

Arg Leu Glu Leu Leu Asp Leu Arg Gly Asn Lys Leu Leu Phe Leu Thr
690 695 700

Asp Ser Leu Ser Asp Phe Thr Ser Ser Leu Arg Thr Leu Leu Leu Ser
705 710 715 720

His Asn Arg Ile Ser His Leu Pro Ser Gly Phe Leu Ser Glu Val Ser
725 730 735

Ser Leu Lys His Leu Asp Leu Ser Ser Asn Leu Leu Lys Thr Ile Asn
740 745 750

Lys Ser Ala Leu Glu Thr Lys Thr Thr Thr Lys Leu Ser Met Leu Glu
755 760 765

Leu His Gly Asn Pro Phe Glu Cys Thr Cys Asp Ile Gly Asp Phe Arg
770 775 780

Arg Trp Met Asp Glu His Leu Asn Val Lys Ile Pro Arg Leu Val Asp
785 790 795 800

Val Ile Cys Ala Ser Pro Gly Asp Gln Arg Gly Lys Ser Ile Val Ser
805 810 815

Leu Glu Leu Thr Thr Cys Val Ser Asp Val Thr Ala Val Ile Leu Phe
820 825 830

Phe Phe Thr Phe Phe Ile Thr Thr Met Val Met Leu Ala Ala Leu Ala
835 840 845

His His Leu Phe Tyr Trp Asp Val Trp Phe Ile Tyr Asn Val Cys Leu
850 855 860

Ala Lys Val Lys Gly Tyr Arg Ser Leu Ser Thr Ser Gln Thr Phe Tyr
865 870 875 880

Asp Ala Tyr Ile Ser Tyr Asp Thr Lys Asp Ala Ser Val Thr Asp Trp
885 890 895

Val Ile Asn Glu Leu Arg Tyr His Leu Glu Glu Ser Arg Asp Lys Asn
900 905 910

Val Leu Leu Cys Leu Glu Glu Arg Asp Trp Asp Pro Gly Leu Ala Ile
915 920 925

Ile Asp Asn Leu Met Gln Ser Ile Asn Gln Ser Lys Lys Thr Val Phe
930 935 940

Val Leu Thr Lys Lys Tyr Ala Lys Ser Trp Asn Phe Lys Thr Ala Phe
945 950 955 960

Tyr Leu Ala Leu Gln Arg Leu Met Asp Glu Asn Met Asp Val Ile Ile
965 970 975

Phe Ile Leu Leu Glu Pro Val Leu Gln His Ser Gln Tyr Leu Arg Leu
980 985 990

Arg Gln Arg Ile Cys Lys Ser Ser Ile Leu Gln Trp Pro Asp Asn Pro
995 1000 1005

Lys Ala Glu Gly Leu Phe Trp Gln Thr Leu Arg Asn Val Val Leu
1010 1015 1020

Thr Glu Asn Asp Ser Arg Tyr Asn Asn Met Tyr Val Asp Ser Ile
1025 1030 1035

Lys Gln Tyr
1040

<210> 50
<211> 1059
<212> PRT
<213> Homo sapiens

<400> 50

```
Met Lys Glu Ser Ser Leu Gln Asn Ser Ser Cys Ser Leu Gly Lys Glu
1               5                   10                  15

Thr Lys Lys Glu Asn Met Phe Leu Gln Ser Ser Met Leu Thr Cys Ile
            20                  25                  30

Phe Leu Leu Ile Ser Gly Ser Cys Glu Leu Cys Ala Glu Glu Asn Phe
        35                  40                  45

Ser Arg Ser Tyr Pro Cys Asp Glu Lys Lys Gln Asn Asp Ser Val Ile
    50                  55                  60

Ala Glu Cys Ser Asn Arg Arg Leu Gln Glu Val Pro Gln Thr Val Gly
65                  70                  75                  80

Lys Tyr Val Thr Glu Leu Asp Leu Ser Asp Asn Phe Ile Thr His Ile
                85                  90                  95

Thr Asn Glu Ser Phe Gln Gly Leu Gln Asn Leu Thr Lys Ile Asn Leu
            100                 105                 110

Asn His Asn Pro Asn Val Gln His Gln Asn Gly Asn Pro Gly Ile Gln
        115                 120                 125

Ser Asn Gly Leu Asn Ile Thr Asp Gly Ala Phe Leu Asn Leu Lys Asn
    130                 135                 140

Leu Arg Glu Leu Leu Leu Glu Asp Asn Gln Leu Pro Gln Ile Pro Ser
145                 150                 155                 160

Gly Leu Pro Glu Ser Leu Thr Glu Leu Ser Leu Ile Gln Asn Asn Ile
                165                 170                 175

Tyr Asn Ile Thr Lys Glu Gly Ile Ser Arg Leu Ile Asn Leu Lys Asn
            180                 185                 190

Leu Tyr Leu Ala Trp Asn Cys Tyr Phe Asn Lys Val Cys Glu Lys Thr
        195                 200                 205

Asn Ile Glu Asp Gly Val Phe Glu Thr Leu Thr Asn Leu Glu Leu Leu
    210                 215                 220

Ser Leu Ser Phe Asn Ser Leu Ser His Val Pro Pro Lys Leu Pro Ser
225                 230                 235                 240

Ser Leu Arg Lys Leu Phe Leu Ser Asn Thr Gln Ile Lys Tyr Ile Ser
                245                 250                 255
```

Glu Glu Asp Phe Lys Gly Leu Ile Asn Leu Thr Leu Leu Asp Leu Ser
260 265 270

Gly Asn Cys Pro Arg Cys Phe Asn Ala Pro Phe Pro Cys Val Pro Cys
275 280 285

Asp Gly Gly Ala Ser Ile Asn Ile Asp Arg Phe Ala Phe Gln Asn Leu
290 295 300

Thr Gln Leu Arg Tyr Leu Asn Leu Ser Ser Thr Ser Leu Arg Lys Ile
305 310 315 320

Asn Ala Ala Trp Phe Lys Asn Met Pro His Leu Lys Val Leu Asp Leu
325 330 335

Glu Phe Asn Tyr Leu Val Gly Glu Ile Ala Ser Gly Ala Phe Leu Thr
340 345 350

Met Leu Pro Arg Leu Glu Ile Leu Asp Leu Ser Phe Asn Tyr Ile Lys
355 360 365

Gly Ser Tyr Pro Gln His Ile Asn Ile Ser Arg Asn Phe Ser Lys Leu
370 375 380

Leu Ser Leu Arg Ala Leu His Leu Arg Gly Tyr Val Phe Gln Glu Leu
385 390 395 400

Arg Glu Asp Asp Phe Gln Pro Leu Met Gln Leu Pro Asn Leu Ser Thr
405 410 415

Ile Asn Leu Gly Ile Asn Phe Ile Lys Gln Ile Asp Phe Lys Leu Phe
420 425 430

Gln Asn Phe Ser Asn Leu Glu Ile Ile Tyr Leu Ser Glu Asn Arg Ile
435 440 445

Ser Pro Leu Val Lys Asp Thr Arg Gln Ser Tyr Ala Asn Ser Ser Ser
450 455 460

Phe Gln Arg His Ile Arg Lys Arg Arg Ser Thr Asp Phe Glu Phe Asp
465 470 475 480

Pro His Ser Asn Phe Tyr His Phe Thr Arg Pro Leu Ile Lys Pro Gln
485 490 495

Cys Ala Ala Tyr Gly Lys Ala Leu Asp Leu Ser Leu Asn Ser Ile Phe

500 505 510

Phe Ile Gly Pro Asn Gln Phe Glu Asn Leu Pro Asp Ile Ala Cys Leu
515 520 525

Asn Leu Ser Ala Asn Ser Asn Ala Gln Val Leu Ser Gly Thr Glu Phe
530 535 540

Ser Ala Ile Pro His Val Lys Tyr Leu Asp Leu Thr Asn Asn Arg Leu
545 550 555 560

Asp Phe Asp Asn Ala Ser Ala Leu Thr Glu Leu Ser Asp Leu Glu Val
565 570 575

Leu Asp Leu Ser Tyr Asn Ser His Tyr Phe Arg Ile Ala Gly Val Thr
580 585 590

His His Leu Glu Phe Ile Gln Asn Phe Thr Asn Leu Lys Val Leu Asn
595 600 605

Leu Ser His Asn Asn Ile Tyr Thr Leu Thr Asp Lys Tyr Asn Leu Glu
610 615 620

Ser Lys Ser Leu Val Glu Leu Val Phe Ser Gly Asn Arg Leu Asp Ile
625 630 635 640

Leu Trp Asn Asp Asp Asp Asn Arg Tyr Ile Ser Ile Phe Lys Gly Leu
645 650 655

Lys Asn Leu Thr Arg Leu Asp Leu Ser Leu Asn Arg Leu Lys His Ile
660 665 670

Pro Asn Glu Ala Phe Leu Asn Leu Pro Ala Ser Leu Thr Glu Leu His
675 680 685

Ile Asn Asp Asn Met Leu Lys Phe Phe Asn Trp Thr Leu Leu Gln Gln
690 695 700

Phe Pro Arg Leu Glu Leu Leu Asp Leu Arg Gly Asn Lys Leu Leu Phe
705 710 715 720

Leu Thr Asp Ser Leu Ser Asp Phe Thr Ser Ser Leu Arg Thr Leu Leu
725 730 735

Leu Ser His Asn Arg Ile Ser His Leu Pro Ser Gly Phe Leu Ser Glu
740 745 750

```
Val Ser Ser Leu Lys His Leu Asp Leu Ser Ser Asn Leu Leu Lys Thr
        755                 760                 765

Ile Asn Lys Ser Ala Leu Glu Thr Lys Thr Thr Thr Lys Leu Ser Met
    770                 775                 780

Leu Glu Leu His Gly Asn Pro Phe Glu Cys Thr Cys Asp Ile Gly Asp
785                 790                 795                 800

Phe Arg Arg Trp Met Asp Glu His Leu Asn Val Lys Ile Pro Arg Leu
                805                 810                 815

Val Asp Val Ile Cys Ala Ser Pro Gly Asp Gln Arg Gly Lys Ser Ile
            820                 825                 830

Val Ser Leu Glu Leu Thr Thr Cys Val Ser Asp Val Thr Ala Val Ile
        835                 840                 845

Leu Phe Phe Phe Thr Phe Phe Ile Thr Thr Met Val Met Leu Ala Ala
    850                 855                 860

Leu Ala His His Leu Phe Tyr Trp Asp Val Trp Phe Ile Tyr Asn Val
865                 870                 875                 880

Cys Leu Ala Lys Val Lys Gly Tyr Arg Ser Leu Ser Thr Ser Gln Thr
                885                 890                 895

Phe Tyr Asp Ala Tyr Ile Ser Tyr Asp Thr Lys Asp Ala Ser Val Thr
            900                 905                 910

Asp Trp Val Ile Asn Glu Leu Arg Tyr His Leu Glu Glu Ser Arg Asp
        915                 920                 925

Lys Asn Val Leu Leu Cys Leu Glu Glu Arg Asp Trp Asp Pro Gly Leu
    930                 935                 940

Ala Ile Ile Asp Asn Leu Met Gln Ser Ile Asn Gln Ser Lys Lys Thr
945                 950                 955                 960

Val Phe Val Leu Thr Lys Lys Tyr Ala Lys Ser Trp Asn Phe Lys Thr
                965                 970                 975

Ala Phe Tyr Leu Ala Leu Gln Arg Leu Met Asp Glu Asn Met Asp Val
            980                 985                 990

Ile Ile Phe Ile Leu Leu Glu Pro  Val Leu Gln His Ser  Gln Tyr Leu
        995                 1000                 1005
```

Arg Leu Arg Gln Arg Ile Cys Lys Ser Ser Ile Leu Gln Trp Pro
1010 1015 1020

Asp Asn Pro Lys Ala Glu Gly Leu Phe Trp Gln Thr Leu Arg Asn
1025 1030 1035

Val Val Leu Thr Glu Asn Asp Ser Arg Tyr Asn Asn Met Tyr Val
1040 1045 1050

Asp Ser Ile Lys Gln Tyr
1055

<210> 51
<211> 2142
<212> DNA
<213> Homo sapiens

<400> 51
agagctgcaa gaagcaccag gctcggccac ttcagaagcc ccagcctcga cctagcccac 60

cctctcaggg ccacagtgca gaagcctgca cacctgccaa gtctctccga ctccttgcag 120

ctgctgtcag catggcccag gctcctgctg acccgggcag agaaggccac cttgaacaaa 180

gaatcctgca ggtgctgaca gaggctggct ccccggtgaa acttgcccag ctggtgaagg 240

aatgccaagc acccaagagg gagctcaacc aagtcctcta ccgaatgaaa aaggagttga 300

aagtctccct cacatcccct gccacctggt gcttgggcgg gactgatcct gaaggcgagg 360

gtcctgcaga gctggccttg tccagccctg ccgagaggcc ccagcaacat gcagctacaa 420

ttccagagac ccctggccct cagttcagcc aacaacggga ggaagacatc tacaggtttc 480

tcaaagacaa tggtccccag agggccctgg tcatcgccca agcactggga atgaggacag 540

caaaagatgt gaaccgagac ttgtacagga tgaagagcag gcaccttctg gacatggatg 600

agcagtccaa agcatggacg atttaccgcc cagaagattc tggaagaaga gcaaagtcag 660

cctcaattat ttaccagcac aatccaatca acatgatctg ccagaatgga cccaacagct 720

ggatttccat tgcaaactcc gaagccatcc agattggaca cgggaacatc attacaagac 780

agacagtctc cagggaggac ggttccgccg gtccacgcca cctcccttca atggcaccag 840

gtgattcctc aacttggggg accctagttg atccctgggg gccccaggac atccacatgg 900

agcagtccat actgagacgg gtgcagctgg gacacagcaa tgagatgagg ctccacggcg 960

tcccgtccga gggccctgcc cacatccccc ctggcagccc cccagtctct gccactgctg 1020

ccggcccaga agcttcgttt gaagcaagaa ttcccagtcc aggaactcac cctgaggggg 1080

aagccgccca gagaatccac atgaaatcgt gctttctcga ggacgccacc atcggcaaca 1140

gcaacaaaat gtctatcagc ccaggggtgg ctggcccagg aggagtcgca gggtctggag 1200

agggggagcc aggggaggac gcaggtcgtc gtcccgcaga cacacaatcc agaagtcact 1260

ttcctcgaga cattggtcag cccatcactc ccagccactc gaagctcacc cccaagctgg 1320

aaactatgac tcttggaaac aggagtcaca aagctgcaga aggcagccac tatgtggatg 1380

aagcctcaca cgaggggagc tggtggggag gtgggattta gtgcacagcc tcacgtgggg 1440

cttggacaca ggctgggggt gggcgcatgc tagggagact agcctgctgc tctctgcatt 1500

ccttagcgtc ttgtttgacc tgcttgcttc cagacataac ctgcatgaat cagttttggg 1560

ggaatggacc tggcatgggg atgggttcag gccaggtctt ttgatggcca ggagtagatg 1620

acagggagtt gccttgggga acctttggtg tgccaagagg aggtgggtag atgggagtgg 1680

ggctcggtcc cccaggccca ggggactctc tccactcttt cctgggctcg gggcatctgc 1740

ctggagttac cttccatcat ggctacctgc tgtggtttga atgtttgagt cccaacaaaa 1800

ttcatatcaa aacataatcc caactgggtg cagtggctca cgcctgtaat cccagcactt 1860

tgggaggccg aggcgggcgg atcaataggt caggaaatcc agaccgtcct ggctaacatg 1920

gtgaaacccc gtctctacta aaaaaaaaaa tacaaaaaat tagccgggcg ttgtggcggg 1980

cacctggagt cccagctact ccggaggctg agggaggaga atggtgtgaa cccgggaggt 2040

ggagcttcca gtgagccgag atcgcgccac tgcactccag gctgggcgac agagcgagac 2100

tccgtctcaa aaaaataaat acataaataa aaaataaacc aa 2142

<210> 52
<211> 1917
<212> DNA
<213> Homo sapiens

<400> 52
agagctgcaa gaagcaccag gctcggccac ttcagaagcc ccagcctcga cctagcccac 60

cctctcaggg ccacagtgca gaagcctgca cacctgccaa gtctctccga ctccttgcag 120

ctgctgtcag catggcccag gctcctgctg acccgggcag agaagccgag aggccccagc 180

aacatgcagc tacaattcca gagacccctg gccctcagtt cagccaacaa cgggaggaag 240

acatctacag gtttctcaaa gacaatggtc cccagagggc cctggtcatc gcccaagcac 300

tgggaatgag gacagcaaaa gatgtgaacc gagacttgta caggatgaag agcaggcacc 360

ttctggacat ggatgagcag tccaaagcat ggacgattta ccgcccagaa gattctggaa 420

gaagagcaaa gtcagcctca attatttacc agcacaatcc aatcaacatg atctgccaga 480

atggacccaa cagctggatt tccattgcaa actccgaagc catccagatt ggacacggga 540

acatcattac aagacagaca gtctccaggg aggacggttc cgccggtcca cgccacctcc 600

cttcaatggc accaggtgat tcctcaactt gggggaccct agttgatccc tgggggcccc 660

aggacatcca catggagcag tccatactga gacgggtgca gctgggacac agcaatgaga 720

tgaggctcca cggcgtcccg tccgagggcc ctgcccacat cccccctggc agcccccccag 780

tctctgccac tgctgccggc ccagaagctt cgtttgaagc aagaattccc agtccaggaa 840

ctcaccctga gggggaagcc gcccagagaa tccacatgaa atcgtgcttt ctcgaggacg 900

ccaccatcgg caacagcaac aaaatgtcta tcagcccagg ggtggctggc ccaggaggag 960

tcgcagggtc tggagagggg gagccagggg aggacgcagg tcgtcgtccc gcagacacac 1020

aatccagaag tcactttcct cgagacattg gtcagcccat cactcccagc cactcgaagc 1080

tcacccccaa gctggaaact atgactcttg gaaacaggag tcacaaagct gcagaaggca 1140

gccactatgt ggatgaagcc tcacacgagg ggagctggtg gggaggtggg atttagtgca 1200

cagcctcacg tggggcttgg acacaggctg ggggtgggcg catgctaggg agactagcct 1260

gctgctctct gcattcctta gcgtcttgtt tgacctgctt gcttccagac ataacctgca 1320

tgaatcagtt ttgggggaat ggacctggca tggggatggg ttcaggccag gtcttttgat 1380

ggccaggagt agatgacagg gagttgcctt ggggaacctt tggtgtgcca agaggaggtg 1440

ggtagatggg agtggggctc ggtcccccag gcccagggga ctctctccac tctttcctgg 1500

gctcggggca tctgcctgga gttaccttcc atcatggcta cctgctgtgg tttgaatgtt 1560

tgagtcccaa caaaattcat atcaaaacat aatcccaact gggtgcagtg gctcacgcct 1620

gtaatcccag cactttggga ggccgaggcg ggcggatcaa taggtcagga aatccagacc 1680

gtcctggcta acatggtgaa accccgtctc tactaaaaaa aaaaatacaa aaaattagcc 1740

gggcgttgtg gcgggcacct ggagtcccag ctactccgga ggctgaggga ggagaatggt 1800

gtgaacccgg gaggtggagc ttccagtgag ccgagatcgc gccactgcac tccaggctgg 1860

gcgacagagc gagactccgt ctcaaaaaaa taaatacata aataaaaaat aaaccaa 1917

<210> 53
<211> 1255
<212> DNA
<213> Homo sapiens

<400> 53
tttttttttc tttcaaaaga cgaacagaga agtttcattt tctttttctc ctgaaaccga 60

atctggccgg cctggctagg catctatttc cgggctgtaa gcagctgaca cctgcccagt 120

ggaagctggc atccctcccc ttgtgggttc agagctgcaa gaagcaccag gctcggccac 180

ttcagaagcc ccagcctcga cctagcccac cctctcaggg ccacagtgca gaagcctgca 240

cacctgccaa gtctctccga ctccttgcag ctgctgtcag catggcccag gctcctgctg 300

acccgggcag agaaggccac cttgaacaaa gaatcctgca ggtgctgaca gaggctggct 360

ccccggtgaa acttgcccag ctggtgaagg aatgccaagc acccaagagg gagctcaacc 420

aagtcctcta ccgaatgaaa aaggagttga aagtctccct cacatcccct gccacctggt 480

gcttgggcgg gactgatcct gaaggcgagg gtcctgcaga gctggccttg tccagccctg 540

ccgagaggcc ccagcaacat gcagctacaa ttccagagac ccctggccct cagttcagcc 600

aacaacggga ggaagacatc tacaggtttc tcaaagacaa tggtccccag agggccctgg 660

tcatcgccca agcactggga atgaggacag caaaagatgt gaaccgagac ttgtacagga 720

tgaagagcag gcaccttctg gacatggatg agcagtccaa agcatggacg atttaccgcc 780

cagaagattc tggaagaaga gcaaagtcag cctcaattat ttaccagcac aatccaatca 840

acatgatctg ccagaatgga cccaacagct ggatttccat tgcaaactcc gaagccatcc 900

agattggaca cgggaacatc attacaagac agacagtctc cagggaggac ggtaagtcac 960

ccaagagagc ccagggaggg gacctcggtg gggagccacc ggatcctctg ggtgggggca 1020

aagggtaggg atggggagtg gggggattct gccctccaag ggaaagggt tgcttccaga 1080

cccccacagt ccacctttac ggccgtcctg agaatgagga cacctggatc aaagctctcc 1140

tgatttccct gtactctgat actttctacc tcattttaaa gtttaattta atttttattt 1200

ttctaataaa attttaaaat taagatggga aaaaaaaaaa aaaaaaaaaa aaaaa 1255

<210> 54
<211> 429
<212> PRT
<213> Homo sapiens

<400> 54

Met Ala Gln Ala Pro Ala Asp Pro Gly Arg Glu Gly His Leu Glu Gln
1               5                   10                  15

Arg Ile Leu Gln Val Leu Thr Glu Ala Gly Ser Pro Val Lys Leu Ala
            20                  25                  30

Gln Leu Val Lys Glu Cys Gln Ala Pro Lys Arg Glu Leu Asn Gln Val
        35                  40                  45

Leu Tyr Arg Met Lys Lys Glu Leu Lys Val Ser Leu Thr Ser Pro Ala
    50                  55                  60

Thr Trp Cys Leu Gly Gly Thr Asp Pro Glu Gly Glu Gly Pro Ala Glu
65                  70                  75                  80

Leu Ala Leu Ser Ser Pro Ala Glu Arg Pro Gln Gln His Ala Ala Thr
                85                  90                  95

Ile Pro Glu Thr Pro Gly Pro Gln Phe Ser Gln Gln Arg Glu Glu Asp
            100                 105                 110

Ile Tyr Arg Phe Leu Lys Asp Asn Gly Pro Gln Arg Ala Leu Val Ile

115 120 125

Ala Gln Ala Leu Gly Met Arg Thr Ala Lys Asp Val Asn Arg Asp Leu
130 135 140

Tyr Arg Met Lys Ser Arg His Leu Leu Asp Met Asp Glu Gln Ser Lys
145 150 155 160

Ala Trp Thr Ile Tyr Arg Pro Glu Asp Ser Gly Arg Arg Ala Lys Ser
165 170 175

Ala Ser Ile Ile Tyr Gln His Asn Pro Ile Asn Met Ile Cys Gln Asn
180 185 190

Gly Pro Asn Ser Trp Ile Ser Ile Ala Asn Ser Glu Ala Ile Gln Ile
195 200 205

Gly His Gly Asn Ile Ile Thr Arg Gln Thr Val Ser Arg Glu Asp Gly
210 215 220

Ser Ala Gly Pro Arg His Leu Pro Ser Met Ala Pro Gly Asp Ser Ser
225 230 235 240

Thr Trp Gly Thr Leu Val Asp Pro Trp Gly Pro Gln Asp Ile His Met
245 250 255

Glu Gln Ser Ile Leu Arg Arg Val Gln Leu Gly His Ser Asn Glu Met
260 265 270

Arg Leu His Gly Val Pro Ser Glu Gly Pro Ala His Ile Pro Pro Gly
275 280 285

Ser Pro Pro Val Ser Ala Thr Ala Ala Gly Pro Glu Ala Ser Phe Glu
290 295 300

Ala Arg Ile Pro Ser Pro Gly Thr His Pro Glu Gly Glu Ala Ala Gln
305 310 315 320

Arg Ile His Met Lys Ser Cys Phe Leu Glu Asp Ala Thr Ile Gly Asn
325 330 335

Ser Asn Lys Met Ser Ile Ser Pro Gly Val Ala Gly Pro Gly Gly Val
340 345 350

Ala Gly Ser Gly Glu Gly Glu Pro Gly Glu Asp Ala Gly Arg Arg Pro
355 360 365

Ala Asp Thr Gln Ser Arg Ser His Phe Pro Arg Asp Ile Gly Gln Pro
370 375 380

Ile Thr Pro Ser His Ser Lys Leu Thr Pro Lys Leu Glu Thr Met Thr
385 390 395 400

Leu Gly Asn Arg Ser His Lys Ala Ala Glu Gly Ser His Tyr Val Asp
405 410 415

Glu Ala Ser His Glu Gly Ser Trp Trp Gly Gly Gly Ile
420 425

<210> 55
<211> 354
<212> PRT
<213> Homo sapiens

<400> 55

Met Ala Gln Ala Pro Ala Asp Pro Gly Arg Glu Ala Glu Arg Pro Gln
1 5 10 15

Gln His Ala Ala Thr Ile Pro Glu Thr Pro Gly Pro Gln Phe Ser Gln
20 25 30

Gln Arg Glu Glu Asp Ile Tyr Arg Phe Leu Lys Asp Asn Gly Pro Gln
35 40 45

Arg Ala Leu Val Ile Ala Gln Ala Leu Gly Met Arg Thr Ala Lys Asp
50 55 60

Val Asn Arg Asp Leu Tyr Arg Met Lys Ser Arg His Leu Leu Asp Met
65 70 75 80

Asp Glu Gln Ser Lys Ala Trp Thr Ile Tyr Arg Pro Glu Asp Ser Gly
85 90 95

Arg Arg Ala Lys Ser Ala Ser Ile Ile Tyr Gln His Asn Pro Ile Asn
100 105 110

Met Ile Cys Gln Asn Gly Pro Asn Ser Trp Ile Ser Ile Ala Asn Ser
115 120 125

Glu Ala Ile Gln Ile Gly His Gly Asn Ile Ile Thr Arg Gln Thr Val
130 135 140

Ser Arg Glu Asp Gly Ser Ala Gly Pro Arg His Leu Pro Ser Met Ala
145 150 155 160

Pro Gly Asp Ser Ser Thr Trp Gly Thr Leu Val Asp Pro Trp Gly Pro
165 170 175

Gln Asp Ile His Met Glu Gln Ser Ile Leu Arg Arg Val Gln Leu Gly
180 185 190

His Ser Asn Glu Met Arg Leu His Gly Val Pro Ser Glu Gly Pro Ala
195 200 205

His Ile Pro Pro Gly Ser Pro Pro Val Ser Ala Thr Ala Ala Gly Pro
210 215 220

Glu Ala Ser Phe Glu Ala Arg Ile Pro Ser Pro Gly Thr His Pro Glu
225 230 235 240

Gly Glu Ala Ala Gln Arg Ile His Met Lys Ser Cys Phe Leu Glu Asp
245 250 255

Ala Thr Ile Gly Asn Ser Asn Lys Met Ser Ile Ser Pro Gly Val Ala
260 265 270

Gly Pro Gly Gly Val Ala Gly Ser Gly Glu Gly Glu Pro Gly Glu Asp
275 280 285

Ala Gly Arg Arg Pro Ala Asp Thr Gln Ser Arg Ser His Phe Pro Arg
290 295 300

Asp Ile Gly Gln Pro Ile Thr Pro Ser His Ser Lys Leu Thr Pro Lys
305 310 315 320

Leu Glu Thr Met Thr Leu Gly Asn Arg Ser His Lys Ala Ala Glu Gly
325 330 335

Ser His Tyr Val Asp Glu Ala Ser His Glu Gly Ser Trp Trp Gly Gly
340 345 350

Gly Ile

<210> 56
<211> 248
<212> PRT
<213> Homo sapiens

<400> 56

Met Ala Gln Ala Pro Ala Asp Pro Gly Arg Glu Gly His Leu Glu Gln
1 5 10 15

```
Arg Ile Leu Gln Val Leu Thr Glu Ala Gly Ser Pro Val Lys Leu Ala
            20                  25                  30

Gln Leu Val Lys Glu Cys Gln Ala Pro Lys Arg Glu Leu Asn Gln Val
        35                  40                  45

Leu Tyr Arg Met Lys Lys Glu Leu Lys Val Ser Leu Thr Ser Pro Ala
    50                  55                  60

Thr Trp Cys Leu Gly Gly Thr Asp Pro Glu Gly Glu Gly Pro Ala Glu
65                  70                  75                  80

Leu Ala Leu Ser Ser Pro Ala Glu Arg Pro Gln Gln His Ala Ala Thr
                85                  90                  95

Ile Pro Glu Thr Pro Gly Pro Gln Phe Ser Gln Gln Arg Glu Glu Asp
            100                 105                 110

Ile Tyr Arg Phe Leu Lys Asp Asn Gly Pro Gln Arg Ala Leu Val Ile
        115                 120                 125

Ala Gln Ala Leu Gly Met Arg Thr Ala Lys Asp Val Asn Arg Asp Leu
    130                 135                 140

Tyr Arg Met Lys Ser Arg His Leu Leu Asp Met Asp Glu Gln Ser Lys
145                 150                 155                 160

Ala Trp Thr Ile Tyr Arg Pro Glu Asp Ser Gly Arg Arg Ala Lys Ser
                165                 170                 175

Ala Ser Ile Ile Tyr Gln His Asn Pro Ile Asn Met Ile Cys Gln Asn
            180                 185                 190

Gly Pro Asn Ser Trp Ile Ser Ile Ala Asn Ser Glu Ala Ile Gln Ile
        195                 200                 205

Gly His Gly Asn Ile Ile Thr Arg Gln Thr Val Ser Arg Glu Asp Gly
    210                 215                 220

Lys Ser Pro Lys Arg Ala Gln Gly Gly Asp Leu Gly Gly Glu Pro Pro
225                 230                 235                 240

Asp Pro Leu Gly Gly Gly Lys Gly
                245
```

## Description

FIELD OF THE INVENTION

The invention relates to a method of predicting a response of a prostate cancer subject to radiotherapy. Moreover, the invention relates to a diagnostic kit, to a use of the kit in a method of predicting a response of a prostate cancer subject to radiotherapy, to a use of a gene expression profile for each of one or more immune defense response genes in radiotherapy prediction for a prostate cancer subject, and to a corresponding computer program product.

BACKGROUND OF THE INVENTION

Cancer is a class of diseases in which a group of cells displays uncontrolled growth, invasion and sometimes metastasis. These three malignant properties of cancers differentiate them from benign tumours, which are self-limited and do not invade or metastasize. Prostate Cancer (PCa) is the second most commonly-occurring non-skin malignancy in men, with an estimated 1.3 million new cases diagnosed and 360,000 deaths world-wide in 2018 (see Bray F. et al., "Global cancer statistics 2018: GLOBOCAN estimates of incidence and mortality worldwide for 36 cancers in 185 countries", CA Cancer J Clin, Vol. 68, No. 6, pages 394-424, 2018). In the US, about 90% of the new cases concern localized cancer, meaning that metastases have not yet been formed (see ACS (American Cancer Society), "Cancer Facts & Figures 2010", 2010).

For the treatment of primary localized prostate cancer, several radical therapies are available, of which surgery (radical prostatectomy, RP) and radiation therapy (RT) are most commonly used. RT is administered via an external beam or via the implantation of radioactive seeds into the prostate (brachytherapy) or a combination of both. It is especially preferable for patients who are not eligible for surgery or have been diagnosed with a tumour in an advanced localized or regional stage. Radical RT is provided to up to 50% of patients diagnosed with localized prostate cancer in the US (see ACS, 2010, ibid).

After treatment, prostate cancer antigen (PSA) levels in the blood are measured for disease monitoring. An increase of the blood PSA level provides a biochemical surrogate measure for cancer recurrence or progression. However, the variation in reported biochemical progression-free survival (bPFS) is large (see Grimm P. et al., "Comparative analysis of prostate-specific antigen free survival outcomes for patients with low, intermediate and high risk prostate cancer treatment by radical therapy. Results from the Prostate Cancer Results Study Group", BJU Int, Suppl. 1, pages 22-29, 2012). For many patients, the bPFS at 5 or even 10 years after radical RT may lie above 90%. Unfortunately, for the group of patients at medium and especially higher risk of recurrence, the bPFS can drop to around 40% at 5 years, depending on the type of RT used (see Grimm P. et al., 2012, ibid).

A large number of the patients with primary localized prostate cancer that are not treated with RT will undergo RP (see ACS, 2010, ibid). After RP, an average of 60% of patients in the highest risk group experience biochemical recurrence after 5 and 10 years (see Grimm P. et al., 2012, ibid). In case of biochemical progression after RP, one of the main challenges is the uncertainty whether this is due to recurring localized disease, one or more metastases or even an indolent disease that will not lead to clinical disease progression (see Dal Pra A. et al., "Contemporary role of postoperative radiotherapy for prostate cancer", Transl Androl Urol, Vo. 7, No. 3, pages 399-413, 2018, and Herrera F.G. and Berthold D.R., "Radiation therapy after radical prostatectomy: Implications for clinicians", Front Oncol, Vol. 6, No. 117, 2016). RT to eradicate remaining cancer cells in the prostate bed is one of the main treatment options to salvage survival after a PSA increase following RP. The effectiveness of salvage radiotherapy (SRT) results in 5-year bPFS for 18% to 90% of patients, depending on multiple factors (see Herrera F.G. and Berthold D.R., 2016, ibid, and Pisansky T.M. et al., "Salvage radiation therapy dose response for biochemical failure of prostate cancer after prostatectomy - A multi-institutional observational study", Int J Radiat Oncol Biol Phys, Vol. 96, No. 5, pages 1046-1053, 2016).

It is clear that for certain patient groups, radical or salvage RT is not effective. Their situation is even worsened by the serious side effects that RT can cause, such as bowel inflammation and dysfunction, urinary incontinence and erectile dysfunction (see Resnick M.J. et al., "Long-term functional outcomes after treatment for localized prostate cancer", N Engl J Med, Vol. 368, No. 5, pages 436-445, 2013, and Hegarty S.E. et al., "Radiation therapy after radical prostatectomy for prostate cancer: Evaluation of complications and influence of radiation timing on outcomes in a large, population-based cohort", PLoS One, Vol. 10, No. 2, 2015). In addition, the median cost of one course of RT based on Medicare reimbursement is $ 18,000, with a wide variation up to about $ 40,000 (see Paravati A.J. et al., "Variation in the cost of radiation therapy among medicare patients with cancer", J Oncol Pract, Vol. 11, No. 5, pages 403-409, 2015). These figures do not include the considerable longitudinal costs of follow-up care after radical and salvage RT.

An improved prediction of effectiveness of RT for each patient, be it in the radical or the salvage setting, would improve therapy selection and potentially survival. This can be achieved by 1) optimizing RT for those patients where RT is predicted to be effective (e.g., by dose escalation or a different starting time) and 2) guiding patients where RT is predicted not to be effective to an alternative, potentially more effective form of treatment. Further, this would reduce suffering for those patients who would be spared ineffective therapy and would reduce costs spent on ineffective therapies.

Numerous investigations have been conducted into measures for response prediction of radical RT (see Hall W.A. et al., "Biomarkers of outcome in patients with localized prostate cancer treated with radiotherapy", Semin Radiat Oncol, Vol. 27, pages 11-20, 2016, and Raymond E. et al., "An appraisal of analytical tools used in predicting clinical outcomes following radiation therapy treatment of men with prostate cancer: A systematic review", Radiat Oncol, Vol. 12, No. 1, page 56, 2017) and SRT (see Herrera F.G. and Berthold D.R., 2016, ibid). Many of these measures depend on the concentration of the blood-based biomarker PSA. Metrics investigated for prediction of response before start of RT (radical as well as salvage) include the absolute value of the PSA concentration, its absolute value relative to the prostate volume, the absolute increase over a certain time and the doubling time. Other frequently considered factors are the Gleason score and the clinical tumour stage. For the SRT setting, additional factors are relevant, e.g., surgical margin status, time to recurrence after RP, pre-/peri-surgical PSA values and clinico-pathological parameters.

Although these clinical variables provide limited improvements in patient stratification in various risk groups, there is a need for better predictive tools.

A wide range of biomarker candidates in tissue and bodily fluids has been investigated, but validation is often limited and generally demonstrates prognostic information and not a predictive (therapy-specific) value (see Hall W.A. et al., 2016, ibid). A small number of gene expression panels is currently being validated by commercial organizations. One or a few of these may show predictive value for RT in future (see Dal Pra A. et al., 2018, ibid).

In conclusion, a strong need for better prediction of response to RT remains, for primary prostate cancer as well as for the post-surgery setting.

## SUMMARY OF THE INVENTION

It is an objective of the invention to provide a method of predicting a response of a prostate cancer subject to radiotherapy, which allows to make better treatment decisions. It is a further objective of the invention to provide a diagnostic kit, a use of the kit in a method of predicting a response of a prostate cancer subject to radiotherapy, a use of a gene expression profile for each of one or more immune defense response genes in radiotherapy prediction for a prostate cancer subject, and a corresponding computer program product.

In a first aspect of the present invention, a method of predicting a response of a prostate cancer subject to radiotherapy is presented, comprising:

- determining or receiving the result of a determination of a gene expression profile for each of one or more, for example, 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60 or all, immune defense response genes selected from the group consisting of: APOBEC3A, AIM2, APOBEC3B, CASP1, CIAO1, CTNNB1, CXCL10, CXCL9, DDX41, DDX58, DDX60, DHX36, DHX58, DHX9, GBP1, HERC5, HIST2H2BE, IFI16, IFI44L, IFIH1, IFIT1, IFIT1B, IFIT2, IFIT3, IFITM1, IFITM3, IL1B, IRF3, IRF5, IRF7, ISG15, ITGAX, LILRB1, LRRFIP1, MAVS, MB21D1, MX1, MX2, MYD88, NLRP3, NOD2, OAS1, OAS3, OASL, OPRK1, POLR3A, PTPRC, PYCARD, RSAD2, STAT1, TBK1, TICAM1, TLR2, TLR3, TLR4, TLR7, TLR8, TLR9, TMEM173, TRIM22, and ZBP1, preferably, for each of one or more, for example, 1, 2, 3, 4, 5, 6, 7, 8, 9, 10, 11, 12, 13 or all, immune defense response genes selected from the group consisting of: AIM2, APOBEC3A, CIAO1, DDX58, DHX9, IFI16, IFIH1, IFIT1, IFIT3, LRRFIP1, MYD88, OAS1, TLR8, and ZBP1, said gene expression profile(s) being determined in a biological sample obtained from the subject,
- determining the prediction of the radiotherapy response based on the gene expression profile(s) for the one or more immune defense response genes, and
- optionally, providing the prediction or a therapy recommendation based on the prediction to a medical caregiver or the subject.

In recent years, the importance of the immune system in cancer inhibition as well as in cancer initiation, promotion and metastasis has become very evident (see Mantovani A. et al., "Cancer-related inflammation", Nature, Vol. 454, No. 7203, pages 436-444, 2008, and Giraldo N.A. et al., "The clinical role of the TME in solid cancer", Br J Cancer, Vol. 120, No. 1, pages 45-53, 2019). The immune cells and the molecules they secrete form a crucial part of the tumour microenvironment and most immune cells can infiltrate the tumour tissue. The immune system and the tumour affect and shape one another. Thus, anti-tumour immunity can prevent tumour formation while an inflammatory tumour environment may promote cancer initiation and proliferation. At the same time, tumour cells that may have originated in an immune system-independent manner will shape the immune microenvironment by recruiting immune cells and can have a pro-inflammatory effect while also suppressing anti-cancer immunity.

Some of the immune cells in the tumour microenvironment will have either a general tumour-promoting or a general tumour-inhibiting effect, while other immune cells exhibit plasticity and show both tumour-promoting and tumour-inhibiting potential. Thus, the overall immune microenvironment of the tumour is a mixture of the various immune cells present, the cytokines they produce and their interactions with tumour cells and with other cells in the tumour microenvironment (see Giraldo N.A. et al., 2019, ibid).

The principles described above with regard to the role of the immune system in cancer in general also apply to prostate cancer. Chronic inflammation has been linked to the formation of benign as well as malignant prostate tissue (see Hall W.A. et al., 2016, ibid) and most prostate cancer tissue samples show immune cell infiltrates. The presence of specific immune cells with a pro-tumour effect has been correlated with worse prognosis, while tumours in which natural killer cells were more activated showed better response to therapy and longer recurrence-free periods (see Shiao S.L. et al., "Regulation of prostate cancer progression by tumor microenvironment", Cancer Lett, Vol. 380, No. 1, pages 340-348, 2016).

While a therapy will be influenced by the immune components of the tumour microenvironment, RT itself extensively affects the make-up of these components (see Barker H.E. et al., "The tumor microenvironment after radiotherapy: Mechanisms of resistance or recurrence", Nat Rev Cancer, Vol. 15, No. 7, pages 409-425, 2015). Because suppressive cell types are comparably radiation-insensitive, their relative numbers will increase. Counteractively, the inflicted radiation damage activates cell survival pathways and stimulates the immune system, triggering inflammatory responses and immune cell recruitment. Whether the net effect will be tumour-promoting or tumour-suppressing is as yet uncertain, but its potential for enhancement of cancer immunotherapies is being investigated.

The present invention is based on the idea that, since the status of the immune system and of the immune microenvironment have an impact on therapy effectiveness, the ability to identify markers predictive for this effect might help to be better able to predict overall RT response.

The integrity and stability of genomics DNA is permanently under stress induced by various cell internal and external factors like exposure to radiation, viral or bacterial infections, but also oxidation and replication stress (see Gasser S. et al., "Sensing of dangerous DNA", Mechanisms of Aging and Development, Vol. 165, pages 33-46, 2017). In order to maintain DNA structure and stability, a cell must be able to recognize all types of DNA damages like single or double strand breaks etc. induced by various factors. This process involves the participation of a multitude of specific proteins depending on the kind of damage as part of DNA recognition pathways.

Recent evidence suggests that mis-localized DNA (e.g., DNA unnaturally appearing in the cytosolic fraction of the cell in contrast to the nucleus) and damaged DNA (e.g., through mutations occurring in cancer development) is used by the immune system to identify infected or otherwise diseased cells while genomic and mitochondrial DNA present in healthy cells is ignored by DNA recognition pathways. In diseased cells, cytosolic DNA sensor proteins have been demonstrated to be involved in the detection of DNA occurring unnaturally in the cytosol of the cell. Detection of such DNA by different nucleic acid sensors translates into similar responses leading to nuclear factor kappa-B (NF-kB) and interferon type I (IFN type I) signalling followed by the activation of innate immune system components. While the recognition of viral DNA is known to induce an IFN type I response, evidence that sensing of DNA damage can initiate immune responses has only recently been accumulating.

TLR9 (Toll-like receptor 9) located in the endosomes was one of the first DNA sensors molecules identified to be involved in the immune recognition of DNA by signalling downstream via the adaptor protein myeloid differentiation primary-response protein88 (MYD88). This interaction in turn activates mitogen-activated protein kinases (MAPKs) and NF-kB. TLR9 also induces the generation of type I interferons through the activation of IRF7 via IkB Kinase alpha (IKKalpha) in plasmacytoid dendritic cells (pDCs). Various other DNA immune receptors including IFI16 (IFN-gamma-inducible protein 16), cGAS (cyclic DMP-AMP synthase, DDX41 (DEAD-box helicase 41), as well as ZBP1 (Z-DNA-binding protein 1) interact with STING (stimulator of IFN genes), which activates the IKK complex and IRF3 through TBK1 (TANK binding kinase 1). ZBP1 also activates NF-kB via recruitment of RIP1 and RIP3 (receptor-interacting protein 1 and 3, respectively). While the helicase DHX36 (DEAH-box helicase 36) interacts in a complex with TRID to induce NF-kB and IRF-3/7 the DHX9 helicase stimulates MYD88-dependent signalling in plasmacytoid dendritic cells. The DNA sensor LRRFIP1 (leucine-rich repeat flightless-interacting protein) complexes with beta-catenin to activate the transcription of IRF3 whereas AIM2 (absent in melanoma 2) recruits the adaptor protein ASC (apoptosis speck-like protein) to induce a caspase-1-activating inflammasome complex leading to the secretion of interleukin-lbeta (IL-1beta) and IL-18 (see Fig. 1 of Gasser S. et al., 2017, ibid, which provides a schematic overview of DNA damage and DNA sensor pathways leading to the production of inflammatory cytokines and the expression of ligands for activating innate immune receptors. Members of the non-homologous end joining pathway (orange), homologous recombination (red), inflammasome (dark green), NF- kB and interferon responses (light green) are shown).

The factors and mechanisms responsible for activating the DNA sensor pathways in cancer are currently not well elucidated. It will be important to identify the intratumoral DNA species, sensors and pathways implicated in the expression of IFNs in different cancer types at all stages of the disease. In addition to therapeutic targets in cancer, such factors may also have prognostic and predictive value. Novel DNA sensor pathway agonists and antagonists are currently being developed and tested in preclinical trials. Such compounds will be useful in characterizing the role of DNA sensor pathways in the pathogenesis of cancer, autoimmunity and potentially other diseases.

The identified immune defense response genes APOBEC3A, AIM2, APOBEC3B, CASP1, CIAO1, CTNNB1, CXCL10, CXCL9, DDX41, DDX58, DDX60, DHX36, DHX58, DHX9, GBP1, HERC5, HIST2H2BE, IFI16, IFI44L, IFIH1, IFIT1, IFIT1B, IFIT2, IFIT3, IFITM1, IFITM3, IL1B, IRF3, IRF5, IRF7, ISG15, ITGAX, LILRB1, LRRFIP1, MAVS, MB21D1, MX1, MX2, MYD88, NLRP3, NOD2, OAS1, OAS3, OASL, OPRK1, POLR3A, PTPRC, PYCARD, RSAD2, STAT1, TBK1, TICAM1, TLR2, TLR3, TLR4, TLR7, TLR8, TLR9, TMEM173, TRIM22, and ZBP1, and AIM2, APOBEC3A, CIAO1, DDX58, DHX9, IFI16, IFIH1, IFIT1, IFIT3, LRRFIP1, MYD88, OAS1, TLR8, and ZBP1, respectively, were identified as follows: A group of 538 prostate cancer patients were treated with RP and the prostate cancer tissue was stored together with clinical (e.g., pathological Gleason grade group (pGGG), pathology state (pT stage)) as well as relevant outcome parameters (e.g., biochemical recurrence (BCR), metastatic recurrence, prostate cancer specific death (PCa death), salvage radiation treatment (SRT), salvage androgen deprivation treatment (SADT), chemotherapy (CTX)). For each of these patients, a PDE4D7 score was calculated and categorized into four PDE4D7 score classes as described in Alves de Inda M. et al., "Validation of Cyclic Adenosine Monophosphate Phosphodiesterase-4D7 for its Independent Contribution to Risk Stratification in a Prostate Cancer Patient Cohort with Longitudinal Biological Outcomes", Eur Urol Focus, Vol. 4, No. 3, pages 376-384, 2018. PDE4D7 score class 1 represents patient samples with lowest expression levels of PDE4D7, whereas PDE4D7 score class 4 represents patient samples with highest levels of PDE4D7 expression. RNASeq expression data (TPM - Transcripts Per Million) of the 538 prostate cancer subjects was then investigated for differential gene expression between the PDE4D7 score classes 1 and 4. In particular, it was determined for around 20,000 protein coding transcripts whether the mean expression level of the PDE4D7 score class 1 patients was more than twice as high as the mean expression level of the PDE4D7 score class 4 patients. This analysis resulted in 637 genes with a ratio PDE4D7 score class 1 / PDE4D7 score class 4 of > 2 with a minimum mean expression of 1 TPM in each of the four PDE4D7 score classes. These 637 genes were then further subjected to molecular pathway analysis (www.david.ncifcrf.gov), which resulted in a range of enriched annotation clusters. The annotation cluster #2 demonstrated enrichment (enrichment score: 10.8) in 30 genes with a function in defense response to viruses, negative regulation of viral genome replication as well as type I interferon signaling. A further heat map analysis confirmed that these immune defense response genes were generally higher expressed in samples from patients in PDE4D7 score class 1 than from patients in PDE4D7 score class 4. The class of genes with a function in defense response to viruses, negative regulation of viral genome replication as well as type I interferon signaling was further enriched to 61 genes by literature search to identify additional genes with the same molecular function. A further selection from the 61 genes was made based on the combinatorial power to separate patients who died from prostate cancer vs. those who did not, resulting of a preferred set of 14 genes. It was found that the number of events (metastases, prostate cancer specific death) was enriched in sub-cohorts with a low expression of these genes compared to the total patient cohort (#538) and a sub-cohort of 151 patients undergoing salvage RT (SRT) after post-surgical disease recurrence.

The term "AIM2" refers to the Absent in Melanoma 2 gene (Ensembl: ENSG00000163568), for example, to the sequence as defined in NCBI Reference Sequence NM_004833, specifically, to the nucleotide sequence as set forth in SEQ ID NO:1, which corresponds to the sequence of the above indicated NCBI Reference Sequence of the AIM2 transcript, and also relates to the corresponding amino acid sequence for example as set forth in SEQ ID NO:2, which corresponds to the protein sequence defined in NCBI Protein Accession Reference Sequence NP_004824 encoding the AIM2 polypeptide.

The term "AIM2" also comprises nucleotide sequences showing a high degree of homology to AIM2, e.g., nucleic acid sequences being at least 75%, 80%, 85%, 90%, 91%, 92%, 93%, 94%, 95%, 96%, 97%, 98% or 99% identical to the sequence as set forth in SEQ ID NO: 1 or amino acid sequences being at least 75%, 80%, 85%, 90%, 91%, 92%, 93%, 94%, 95%, 96%, 97%, 98% or 99% identical to the sequence as set forth in SEQ ID NO:2 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:2 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:1.

The term "APOBEC3A" refers to the Apolipoprotein B mRNA Editing Enzyme Catalytic Subunit 3A gene (Ensembl: ENSG00000128383), for example, to the sequence as defined in NCBI Reference Sequence NM_145699, specifically, to the nucleotide sequence as set forth in SEQ ID NO:3, which corresponds to the sequence of the above indicated NCBI Reference Sequence of the APOBEC3A transcript, and also relates to the corresponding amino acid sequence for example as set forth in SEQ ID NO:4, which corresponds to the protein sequence defined in NCBI Protein Accession Reference Sequence NP_663745 encoding the APOBEC3A polypeptide.

The term "APOBEC3A" also comprises nucleotide sequences showing a high degree of homology to APOBEC3A, e.g., nucleic acid sequences being at least 75%, 80%, 85%, 90%, 91%, 92%, 93%, 94%, 95%, 96%, 97%, 98% or 99% identical to the sequence as set forth in SEQ ID NO:3 or amino acid sequences being at least 75%, 80%, 85%, 90%, 91%, 92%, 93%, 94%, 95%, 96%, 97%, 98% or 99% identical to the sequence as set forth in SEQ ID NO:4 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:4 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:3.

The term "CIAO1" refers to the Cytosolic Iron-Sulfur Assembly Component 1 gene (Ensembl: ENSG00000144021), for example, to the sequence as defined in NCBI Reference Sequence NM_004804, specifically, to the nucleotide sequence as set forth in SEQ ID NO:5, which corresponds to the sequence of the above indicated NCBI Reference Sequence of the CIAO1 transcript, and also relates to the corresponding amino acid sequence for example as set forth in SEQ ID NO:6, which corresponds to the protein sequence defined in NCBI Protein Accession Reference Sequence NP_663745 encoding the CIAO1 polypeptide.

The term "CIAO1" also comprises nucleotide sequences showing a high degree of homology to CIAO1, e.g., nucleic acid sequences being at least 75%, 80%, 85%, 90%, 91%, 92%, 93%, 94%, 95%, 96%, 97%, 98% or 99% identical to the sequence as set forth in SEQ ID NO:5 or amino acid sequences being at least 75%, 80%, 85%, 90%, 91%, 92%, 93%, 94%, 95%, 96%, 97%, 98% or 99% identical to the sequence as set forth in SEQ ID NO:6 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:6 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:5.

The term "DDX58" refers to the DExD/H-box Helicase 58 gene (Ensembl: ENSG00000107201), for example, to the sequence as defined in NCBI Reference Sequence NM_014314, specifically, to the nucleotide sequence as set forth in SEQ ID NO:7, which corresponds to the sequence of the above indicated NCBI Reference Sequence of the DDX58 transcript, and also relates to the corresponding amino acid sequence for example as set forth in SEQ ID NO:8, which corresponds to the protein sequence defined in NCBI Protein Accession Reference Sequence NP_055129 encoding the DDX58 polypeptide.

The term "DDX58" also comprises nucleotide sequences showing a high degree of homology to DDX58, e.g., nucleic acid sequences being at least 75%, 80%, 85%, 90%, 91%, 92%, 93%, 94%, 95%, 96%, 97%, 98% or 99% identical to the sequence as set forth in SEQ ID NO:7 or amino acid sequences being at least 75%, 80%, 85%, 90%, 91%, 92%, 93%, 94%, 95%, 96%, 97%, 98% or 99% identical to the sequence as set forth in SEQ ID NO:8 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:8 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:7.

The term "DHX9" refers to the DExD/H-box Helicase 9 gene (Ensembl: ENSG00000135829), for example, to the sequence as defined in NCBI Reference Sequence NM_001357, specifically, to the nucleotide sequence as set forth in SEQ ID NO:9, which corresponds to the sequence of the above indicated NCBI Reference Sequence of the DHX9 transcript, and also relates to the corresponding amino acid sequence for example as set forth in SEQ ID NO: 10, which corresponds to the protein sequence defined in NCBI Protein Accession Reference Sequence NP_001348 encoding the DHX9 polypeptide.

The term "DHX9" also comprises nucleotide sequences showing a high degree of homology to DHX9, e.g., nucleic acid sequences being at least 75%, 80%, 85%, 90%, 91%, 92%, 93%, 94%, 95%, 96%, 97%, 98% or 99% identical to the sequence as set forth in SEQ ID NO:9 or amino acid sequences being at least 75%, 80%, 85%, 90%, 91%, 92%, 93%, 94%, 95%, 96%, 97%, 98% or 99% identical to the sequence as set forth in SEQ ID NO:10 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:10 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:9.

The term "IFI16" refers to the Interferon Gamma Inducible Protein 16 gene (Ensembl: ENSG00000163565), for example, to the sequence as defined in NCBI Reference Sequence NM_005531, specifically, to the nucleotide sequence as set forth in SEQ ID NO:11, which corresponds to the sequence of the above indicated NCBI Reference Sequence of the IFI16 transcript, and also relates to the corresponding amino acid sequence for example as set forth in SEQ ID NO:12, which corresponds to the protein sequence defined in NCBI Protein Accession Reference Sequence NP_005522 encoding the IFI16 polypeptide.

The term "IFI16" also comprises nucleotide sequences showing a high degree of homology to IFI16, e.g., nucleic acid sequences being at least 75%, 80%, 85%, 90%, 91%, 92%, 93%, 94%, 95%, 96%, 97%, 98% or 99% identical to the sequence as set forth in SEQ ID NO:11 or amino acid sequences being at least 75%, 80%, 85%, 90%, 91%, 92%, 93%, 94%, 95%, 96%, 97%, 98% or 99% identical to the sequence as set forth in SEQ ID NO:12 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:12 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:11.

The term "IFIH1" refers to the Interferon Induced With Helicase C Domain 1 gene (Ensembl: ENSG00000115267), for example, to the sequence as defined in NCBI Reference Sequence NM_022168, specifically, to the nucleotide sequence as set forth in SEQ ID NO:13, which corresponds to the sequence of the above indicated NCBI Reference Sequence of the IFIH1 transcript, and also relates to the corresponding amino acid sequence for example as set forth in SEQ ID NO:14, which corresponds to the protein sequence defined in NCBI Protein Accession Reference Sequence NP_071451 encoding the IFIH1 polypeptide.

The term "IFIH1" also comprises nucleotide sequences showing a high degree of homology to IFIH1, e.g., nucleic acid sequences being at least 75%, 80%, 85%, 90%, 91%, 92%, 93%, 94%, 95%, 96%, 97%, 98% or 99% identical to the sequence as set forth in SEQ ID NO:13 or amino acid sequences being at least 75%, 80%, 85%, 90%, 91%, 92%, 93%, 94%, 95%, 96%, 97%, 98% or 99% identical to the sequence as set forth in SEQ ID NO: 14 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:14 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO: 13.

The term "IFIT1" refers to the Interferon Induced Protein With Tetratricopeptide Repeats 1 gene (Ensembl: ENSG00000185745), for example, to the sequence as defined in NCBI Reference Sequence NM_001270929 or in NCBI Reference Sequence NM_001548.5, specifically, to the nucleotide sequence as set forth in SEQ ID NO:15 or in SEQ ID NO:16, which correspond to the sequences of the above indicated NCBI Reference Sequences of the IFIT1 transcript, and also relates to the corresponding amino acid sequence for example as set forth in SEQ ID NO:17 or in SEQ ID NO: 18, which correspond to the protein sequences defined in NCBI Protein Accession Reference Sequence NP_001257858 and in NCBI Protein Accession Reference Sequence NP_001539 encoding the IFIT1 polypeptide.

The term "IFIT1" also comprises nucleotide sequences showing a high degree of homology to IFIT1, e.g., nucleic acid sequences being at least 75%, 80%, 85%, 90%, 91%, 92%, 93%, 94%, 95%, 96%, 97%, 98% or 99% identical to the sequence as set forth in SEQ ID NO: 15 or in SEQ ID NO:16 or amino acid sequences being at least 75%, 80%, 85%, 90%, 91%, 92%, 93%, 94%, 95%, 96%, 97%, 98% or 99% identical to the sequence as set forth in SEQ ID NO:17 or in SEQ ID NO:18 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO: 17 or SEQ ID NO:18 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:15 or in SEQ ID NO:16.

The term "IFIT3" refers to the Interferon Induced Protein With Tetratricopeptide Repeats 3 gene (Ensembl: ENSG00000119917), for example, to the sequence as defined in NCBI Reference Sequence NM_001031683, specifically, to the nucleotide sequence as set forth in SEQ ID NO: 19, which corresponds to the sequence of the above indicated NCBI Reference Sequence of the IFIT3 transcript, and also relates to the corresponding amino acid sequence for example as set forth in SEQ ID NO:20, which corresponds to the protein sequence defined in NCBI Protein Accession Reference Sequence NP_001026853 encoding the IFIT3 polypeptide.

The term "IFIT3" also comprises nucleotide sequences showing a high degree of homology to IFIT3, e.g., nucleic acid sequences being at least 75%, 80%, 85%, 90%, 91%, 92%, 93%, 94%, 95%, 96%, 97%, 98% or 99% identical to the sequence as set forth in SEQ ID NO:19 or amino acid sequences being at least 75%, 80%, 85%, 90%, 91%, 92%, 93%, 94%, 95%, 96%, 97%, 98% or 99% identical to the sequence as set forth in SEQ ID NO:20 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:20 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:19.

The term "LRRFIP1" refers to the LRR Binding FLII Interacting Protein 1 gene (Ensembl: ENSG00000124831), for example, to the sequence as defined in NCBI Reference Sequence NM_004735 or in NCBI Reference Sequence NM_001137550 or in NCBI Reference Sequence NM_001137553 or in NCBI Reference Sequence NM_001137552, specifically, to the nucleotide sequence as set forth in SEQ ID NO:21 or in SEQ ID NO:22 or in SEQ ID NO:23 or in SEQ ID NO:24, which correspond to the sequences of the above indicated NCBI Reference Sequences of the LRRFIP1 transcript, and also relates to the corresponding amino acid sequence for example as set forth in SEQ ID NO:25 or in SEQ ID NO:26 or in SEQ ID NO:27 or in SEQ ID NO:28, which correspond to the protein sequences defined in NCBI Protein Accession Reference Sequence NP_004726 and in NCBI Protein Accession Reference Sequence NP_001131022 and in NCBI Protein Accession Reference Sequence NP_001131025 and in NCBI Protein Accession Reference Sequence NP_001131024 encoding the LRRFIP1 polypeptide.

The term "LRRFIP1" also comprises nucleotide sequences showing a high degree of homology to LRRFIP1, e.g., nucleic acid sequences being at least 75%, 80%, 85%, 90%, 91%, 92%, 93%, 94%, 95%, 96%, 97%, 98% or 99% identical to the sequence as set forth in SEQ ID NO:21 or in SEQ ID NO:22 or in SEQ ID NO:23 or in SEQ ID NO:24 or amino acid sequences being at least 75%, 80%, 85%, 90%, 91%, 92%, 93%, 94%, 95%, 96%, 97%, 98% or 99% identical to the sequence as set forth in SEQ ID NO:25 or in SEQ ID NO:26 or in SEQ ID NO:27 or in SEQ ID NO:28 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:25 or in SEQ ID NO:26 or in SEQ ID NO:27 or in SEQ ID NO:28 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:21 or in SEQ ID NO:22 or in SEQ ID NO:23 or in SEQ ID NO:24.

The term "MYD88" refers to the MYD88 Innate Immune Signal Transduction Adaptor gene (Ensembl: ENSG00000172936), for example, to the sequence as defined in NCBI Reference Sequence NM_001172567 or in NCBI Reference Sequence NM_001172568 or in NCBI Reference Sequence NM_001172569 or in NCBI Reference Sequence NM_001172566 or in NCBI Reference Sequence NM_002468, specifically, to the nucleotide sequences as set forth in SEQ ID NO:29 or in SEQ ID NO:30 or in SEQ ID NO:31 or in SEQ ID NO:32 or in SEQ ID NO:33, which correspond to the sequences of the above indicated NCBI Reference Sequences of the MYD88 transcript, and also relates to the corresponding amino acid sequence for example as set forth in SEQ ID NO:34 or in SEQ ID NO:35 or in SEQ ID NO:36 or in SEQ ID NO:37 or in SEQ ID NO:38, which correspond to the protein sequences defined in NCBI Protein Accession Reference Sequence NP_001166038 and in NCBI Protein Accession Reference Sequence NP_001166039 and in NCBI Protein Accession Reference Sequence NP_001166040 and in NCBI Protein Accession Reference Sequence NP_001166037 and in NCBI Protein Accession Reference Sequence NP_002459 encoding the MYD88 polypeptide.

The term "MYD88" also comprises nucleotide sequences showing a high degree of homology to MYD88, e.g., nucleic acid sequences being at least 75%, 80%, 85%, 90%, 91%, 92%, 93%, 94%, 95%, 96%, 97%, 98% or 99% identical to the sequence as set forth in SEQ ID NO:29 or in SEQ ID NO:30 or in SEQ ID NO:31 or in SEQ ID NO:32 or in SEQ ID NO:33 or amino acid sequences being at least 75%, 80%, 85%, 90%, 91%, 92%, 93%, 94%, 95%, 96%, 97%, 98% or 99% identical to the sequence as set forth in SEQ ID NO:34 or in SEQ ID NO:35 or in SEQ ID NO:36 or in SEQ ID NO:37 or in SEQ ID NO:38 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:34 or in SEQ ID NO:35 or in SEQ ID NO:36 or in SEQ ID NO:37 or in SEQ ID NO:38 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:29 or in SEQ ID NO:30 or in SEQ ID NO:31 or in SEQ ID NO:32 or in SEQ ID NO:33.

The term "OAS1" refers to the 2'-5'-Oligoadenylate Synthetase 1 gene (Ensembl: ENSG00000089127), for example, to the sequence as defined in NCBI Reference Sequence NM_001320151 or in NCBI Reference Sequence NM_002534 or in NCBI Reference Sequence NM_001032409 or in NCBI Reference Sequence NM_016816, specifically, to the nucleotide sequences as set forth in SEQ ID NO:39 or in SEQ ID NO:40 or in SEQ ID NO:41 or in SEQ ID NO:42, which correspond to the sequences of the above indicated NCBI Reference Sequences of the OAS1 transcript, and also relates to the corresponding amino acid sequences for example as set forth in SEQ ID NO:43 or in SEQ ID NO:44 or in SEQ ID NO:45 or in SEQ ID NO:46, which correspond to the protein sequences defined in NCBI Protein Accession Reference Sequence NP_001307080 and in NCBI Protein Accession Reference Sequence NP_002525 and in NCBI Protein Accession Reference Sequence NP_001027581 and in NCBI Protein Accession Reference Sequence NP_058132 encoding the OAS1 polypeptide.

The term "OAS1" also comprises nucleotide sequences showing a high degree of homology to OAS1, e.g., nucleic acid sequences being at least 75%, 80%, 85%, 90%, 91%, 92%, 93%, 94%, 95%, 96%, 97%, 98% or 99% identical to the sequence as set forth in SEQ ID NO:39 or in SEQ ID NO:40 or in SEQ ID NO:41 or in SEQ ID NO:42 or amino acid sequences being at least 75%, 80%, 85%, 90%, 91%, 92%, 93%, 94%, 95%, 96%, 97%, 98% or 99% identical to the sequence as set forth in SEQ ID NO:43 or in SEQ ID NO:44 or in SEQ ID NO:45 or in SEQ ID NO:46 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:43 or in SEQ ID NO:44 or in SEQ ID NO:45 or in SEQ ID NO:46 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:39 or in SEQ ID NO:40 or in SEQ ID NO:41 or in SEQ ID NO:42.

The term "TLR8" refers to the Toll Like Receptor 8 gene (Ensembl: ENSG00000101916), for example, to the sequence as defined in NCBI Reference Sequence NM_138636 or in NCBI Reference Sequence NM_016610, specifically, to the nucleotide sequences as set forth in SEQ ID NO:47 or in SEQ ID NO:48, which correspond to the sequences of the above indicated NCBI Reference Sequences of the TLR8 transcript, and also relates to the corresponding amino acid sequences for example as set forth in SEQ ID NO:49 or in SEQ ID NO:50, which corresponds to the protein sequences defined in NCBI Protein Accession Reference Sequence NP_619542 and in NCBI Protein Accession Reference Sequence NP_057694 encoding the TLR8 polypeptide.

The term "TLR8" also comprises nucleotide sequences showing a high degree of homology to TLR8, e.g., nucleic acid sequences being at least 75%, 80%, 85%, 90%, 91%, 92%, 93%, 94%, 95%, 96%, 97%, 98% or 99% identical to the sequence as set forth in SEQ ID NO:47 or in SEQ ID NO:48 or amino acid sequences being at least 75%, 80%, 85%, 90%, 91%, 92%, 93%, 94%, 95%, 96%, 97%, 98% or 99% identical to the sequence as set forth in SEQ ID NO:49 or in SEQ ID NO:50 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:49 or in SEQ ID NO:50 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:47 or in SEQ ID NO:48.

The term "ZBP1" refers to the Z-DNA Binding Protein 1 gene (Ensembl: ENSG00000124256), for example, to the sequence as defined in NCBI Reference Sequence NM_030776 or in NCBI Reference Sequence NM_001160418 or in NCBI Reference Sequence NM_001160419, specifically, to the nucleotide sequence as set forth in SEQ ID NO:51 or in SEQ ID NO:52 or in SEQ ID NO:53, which corresponds to the sequence of the above indicated NCBI Reference Sequence of the ZBP1 transcript, and also relates to the corresponding amino acid sequence for example as set forth in SEQ ID NO:54 or in SEQ ID NO:55 or in SEQ ID NO:56, which corresponds to the protein sequences defined in NCBI Protein Accession Reference Sequence NP_110403 and in NCBI Protein Accession Reference Sequence NP_001153890 and in NCBI Protein Accession Reference Sequence NP_001153891 encoding the ZBP1 polypeptide.

The term "ZBP1" also comprises nucleotide sequences showing a high degree of homology to ZBP1, e.g., nucleic acid sequences being at least 75%, 80%, 85%, 90%, 91%, 92%, 93%, 94%, 95%, 96%, 97%, 98% or 99% identical to the sequence as set forth in SEQ ID NO:51 or in SEQ ID NO:52 or in SEQ ID NO:53 or amino acid sequences being at least 75%, 80%, 85%, 90%, 91%, 92%, 93%, 94%, 95%, 96%, 97%, 98% or 99% identical to the sequence as set forth in SEQ ID NO:54 or in SEQ ID NO:55 or in SEQ ID NO:56 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:54 or in SEQ ID NO:55 or in SEQ ID NO:56 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:51 or in SEQ ID NO:52 or in SEQ ID NO:53.

The term "biological sample" or "sample obtained from a subject" refers to any biological material obtained via suitable methods known to the person skilled in the art from a subject, e.g., a prostate cancer patient. The biological sample used may be collected in a clinically acceptable manner, e.g., in a way that nucleic acids (in particular RNA) or proteins are preserved.

The biological sample(s) may include body tissue and/or a fluid, such as, but not limited to, blood, sweat, saliva, and urine. Furthermore, the biological sample may contain a cell extract derived from or a cell population including an epithelial cell, such as a cancerous epithelial cell or an epithelial cell derived from tissue suspected to be cancerous. The biological sample may contain a cell population derived from a glandular tissue, e.g., the sample may be derived from the prostate of a male subject. Additionally, cells may be purified from obtained body tissues and fluids if necessary, and then used as the biological sample. In some realizations, the sample may be a tissue sample, a urine sample, a urine sediment sample, a blood sample, a saliva sample, a semen sample, a sample including circulating tumour cells, extracellular vesicles, a sample containing prostate secreted exosomes, or cell lines or cancer cell line.

In one particular realization, biopsy or resections samples may be obtained and/or used. Such samples may include cells or cell lysates.

It is also conceivable that the content of a biological sample is submitted to an enrichment step. For instance, a sample may be contacted with ligands specific for the cell membrane or organelles of certain cell types, e.g., prostate cells, functionalized for example with magnetic particles. The material concentrated by the magnetic particles may subsequently be used for detection and analysis steps as described herein above or below.

Furthermore, cells, e.g., tumour cells, may be enriched via filtration processes of fluid or liquid samples, e.g., blood, urine, etc. Such filtration processes may also be combined with enrichment steps based on ligand specific interactions as described herein above.

The term "prostate cancer" refers to a cancer of the prostate gland in the male reproductive system, which occurs when cells of the prostate mutate and begin to multiply out of control. Typically, prostate cancer is linked to an elevated level of prostate-specific antigen (PSA). In one embodiment of the present invention the term "prostate cancer" relates to a cancer showing PSA levels above 3.0. In another embodiment the term relates to cancer showing PSA levels above 2.0. The term "PSA level" refers to the concentration of PSA in the blood in ng/ml.

The term "non-progressive prostate cancer state" means that a sample of an individual does not show parameter values indicating "biochemical recurrence" and/or "clinical recurrence" and/or "metastases" and/or "castration-resistant disease" and/or "prostate cancer or disease specific death".

The term "progressive prostate cancer state" means that a sample of an individual shows parameter values indicating "biochemical recurrence" and/or "clinical recurrence" and/or "metastases" and/or "castration-resistant disease" and/or "prostate cancer or disease specific death".

The term "biochemical recurrence" generally refers to recurrent biological values of increased PSA indicating the presence of prostate cancer cells in a sample. However, it is also possible to use other markers that can be used in the detection of the presence or that rise suspicion of such presence.

The term "clinical recurrence" refers to the presence of clinical signs indicating the presence of tumour cells as measured, for example using in vivo imaging.

The term "metastases" refers to the presence of metastatic disease in organs other than the prostate.

The term "castration-resistant disease" refers to the presence of hormone-insensitive prostate cancer; i.e., a cancer in the prostate that does not any longer respond to androgen deprivation therapy (ADT).

The term "prostate cancer specific death or disease specific death" refers to death of a patient from his prostate cancer.

It is preferred that the one or more immune defense response genes comprise three or more of the immune defense response genes selected from the group consisting of: AIM2, APOBEC3A, CIAO1, DDX58, DHX9, IFI16, IFIH1, IFIT1, IFIT3, LRRFIP1, MYD88, OAS1, TLR8, and ZBP1.

It is further preferred that the one or more immune defense response genes comprise six or more of the immune defense response genes selected from the group consisting of: AIM2, APOBEC3A, CIAO1, DDX58, DHX9, IFI16, IFIH1, IFIT1, IFIT3, LRRFIP1, MYD88, OAS1, TLR8, and ZBP1.

It is yet further preferred that the one or more immune defense response genes comprise nine or more, preferably, all of the immune defense response genes selected from the group consisting of: AIM2, APOBEC3A, CIAO1, DDX58, DHX9, IFI16, IFIH1, IFIT1, IFIT3, LRRFIP1, MYD88, OAS1, TLR8, and ZBP1.

It is preferred that the determining of the prediction of the radiotherapy response comprises combining the gene expression profiles for two or more, for example, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60 or all, of the immune defense response genes selected from the group consisting of: APOBEC3A, AIM2, APOBEC3B, CASP1, CIAO1, CTNNB1, CXCL10, CXCL9, DDX41, DDX58, DDX60, DHX36, DHX58, DHX9, GBP1, HERC5, HIST2H2BE, IFI16, IFI44L, IFIH1, IFIT1, IFIT1B, IFIT2, IFIT3, IFITM1, IFITM3, IL1B, IRF3, IRF5, IRF7, ISG15, ITGAX, LILRB1, LRRFIP1, MAVS, MB21D1, MX1, MX2, MYD88, NLRP3, NOD2, OAS1, OAS3, OASL, OPRK1, POLR3A, PTPRC, PYCARD, RSAD2, STAT1, TBK1, TICAM1, TLR2, TLR3, TLR4, TLR7, TLR8, TLR9, TMEM173, TRIM22, and ZBP1, preferably, for example, 2, 3, 4, 5, 6, 7, 8, 9, 10, 11, 12, 13 or all, of the immune defense response genes selected from the group consisting of: AIM2, APOBEC3A, CIAO1, DDX58, DHX9, IFI16, IFIH1, IFIT1, IFIT3, LRRFIP1, MYD88, OAS1, TLR8, and ZBP1, with a regression function that had been derived from a population of prostate cancer subjects.

Cox proportional-hazards regression allows analyzing the effect of several risk factors on time to a tested event like survival. Thereby, the risk factors maybe dichotomous or discrete variables like a risk score or a clinical stage but may also be a continuous variable like a biomarker measurement or gene expression values. The probability of the endpoint (e.g., death or disease recurrence) is called the hazard. Next to the information on whether or not the tested endpoint was reached by e.g. subject in a patient cohort (e.g., patient did die or not) also the time to the endpoint is considered in the regression analysis. The hazard is modeled as: $H(t)=H_0(t) \cdot \exp(w_1 \cdot V_1 + w_2 \cdot V_2 + w_3 \cdot V_3 + ...)$, where $V_1$, $V_2$, $V_3$ ... are predictor variables and $H_0(t)$ is the baseline hazard while H(t) is the hazard at any time t. The hazard ratio (or the risk to reach the event) is represented by $Ln[H(t)/ H_0(t)]= w_1 \cdot V_1 + w_2 \cdot V_2 + w_3 \cdot V_3 + ...$, where the coefficients or weights $w_1$, $w_2$, $w_3$ ... are estimated by the Cox regression analysis and can be interpreted in a similar manner as for logistic regression analysis.

In one particular realization, the prediction of the radiotherapy response is determined as follows:

$$(w_1 \cdot \mathrm{AIM2}) + (w_2 \cdot \mathrm{APOBEC3A}) + (w_3 \cdot \mathrm{CIAO1}) + (w_4 \cdot \mathrm{DDX58}) + (w_5 \cdot \mathrm{DHX9}) + (w_6 \cdot \mathrm{IFI16}) + (w_7 \cdot \mathrm{IFIH1}) + (w_8 \cdot \mathrm{IFIT1}) + (w_9 \cdot \mathrm{IFIT3}) + (w_{10} \cdot \mathrm{LRRFIP1}) + (w_{11} \cdot \mathrm{MYD88}) + (w_{12} \cdot \mathrm{OAS1}) + (w_{13} \cdot \mathrm{TLR8}) + (w_{14} \cdot \mathrm{ZBP1}) \quad (1)$$

where $w_1$ to $w_{14}$ are weights and AIM2, APOBEC3A, CIAO1, DDX58, DHX9, IFI16, IFIH1, IFIT1, IFIT3, LRRFIP1, MYD88, OAS1, TLR8, and ZBP1 are the expression levels of the immune defense response genes.

In one example, $w_1$ may be about -0.5 to 0.5, such as -0.02116, $w_2$ may be about -0.5 to -0.5, such as -0.01195, $w_3$ may be about -0.5 to 0.5, such as -0.02092, $w_4$ may be about -0.5 to 0.5, such as -0.009288, $w_5$ may be about -0.5 to 0.5, such as 0.002662, $w_6$ may be about -0.5 to 0.5, such as -0.01924, $w_7$ may be about -0.5 to 0.5, such as 0.007922, $w_8$ may be about -0.5 to 0.5, such as -0.06404, $w_9$ may be about -0.5 to 0.5, such as 0.01869, $w_{10}$ may be about -0.5 to 0.5, such as -0.005425, $w_{11}$ may be about -0.5 to 0.5, such as 0.002878, $w_{12}$ may be about -0.5 to 0.5, such as 0.001348, $w_{13}$ may be about -0.5 to 0.5, such as - 0.04764, and $w_{14}$ may be about -0.5 to 0.5, such as -0.01494.

Alternatively, it is preferred that the determining of the prediction of the radiotherapy response comprises combining the gene expression profiles for two or more, for example, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60 or all, of the immune defense response genes selected from the group consisting of: APOBEC3A, AIM2, APOBEC3B, CASP1, CIAO1, CTNNB1, CXCL10, CXCL9, DDX41, DDX58, DDX60, DHX36, DHX58, DHX9, GBP1, HERC5, HIST2H2BE, IFI16, IFI44L, IFIH1, IFIT1, IFIT1B, IFIT2, IFIT3, IFITM1, IFITM3, IL1B, IRF3, IRF5, IRF7, ISG15, ITGAX, LILRB1, LRRFIP1, MAVS, MB21D1, MX1, MX2, MYD88, NLRP3, NOD2, OAS1, OAS3, OASL, OPRK1, POLR3A, PTPRC, PYCARD, RSAD2, STAT1, TBK1, TICAM1, TLR2, TLR3, TLR4, TLR7, TLR8, TLR9, TMEM173, TRIM22, and ZBP1, preferably, for example, 2, 3, 4, 5, 6, 7, 8, 9, 10, 11, 12, 13 or all, of the immune defense response genes selected from the group consisting of: AIM2, APOBEC3A, CIAO1, DDX58, DHX9, IFI16, IFIH1, IFIT1, IFIT3, LRRFIP1, MYD88, OAS1, TLR8, and ZBP1, with a sum function that sums discretized gene expression profiles of the two or more immune defense response genes, wherein the gene expression profiles are discretized using respective cut-offs that had been derived from a population of prostate cancer subjects.

In one particular realization, the prediction of the radiotherapy response is determined as follows:

```
For each immune defense response gene i
   if( EL(i) < cut-off(i) )
      BEL(i) = 0
   else
      BEL(i) = 1                                                    (2)
if ( sum( BEL(i) < thresh )
   IDR_score = 0
else
   IDR_score = 1
```

where EL(i) and cut-off(i) are the expression level and the cut-off of the i-th immune defense response gene, respectively, BEL(i) is the binarized expression level of the i-th immune defense response gene, IDR _score is the immune defense response score, and thresh is a threshold. Instead of using a binarization as in Eq. (2), other discretizations may be used, e.g., a gene expression profile may be mapped to one of three or four values using two or three cut-offs.

In one example, the cut-off for AIM2 may be about 30 to 40, such as 33.44, the cut-off for APOBEC3A may be about 5 to 15, such as 8.55, the cut-off for CIAO1 may be about 60 to 70, such as 64.49, the cut-off for DDX58 may be about 40 to 50, such as 45.87, the cut-off for DHX9 may be about 200 to 250, such as 230.03, the cut-off for IFI16 may be about 50 to 60, such as 53.56, the cut-off for IFIH1 may be about 90 to 110, such as 99.63, the cut-off for IFIT1 may be about 10 to 20, such as 14.57, the cut-off for IFIT3 may be about 5 to 15, such as 11.75, the cut-off for LRRFIP1 may be about 260 to 310, such as 283.82, the cut-off for MYD88 may be about 20 to 30, such as 23.03, the cut-off for OAS1 may be about 30 to 40, such as 33.93, the cut-off for TLR8 may be about 5 to 15, such as 9.89, the cut-off for ZBP1 may be about 15 to 25, such as 20.16, and thresh may be about 0 to 5, such as 1.

The prediction of the radiotherapy response may also be classified or categorized into one of at least two risk groups, based on the value of the prediction of the radiotherapy response. For example, there may be two risk groups, or three risk groups, or four risk groups, or more than four predefined risk groups. Each risk group covers a respective range of (non-overlapping) values of the prediction of the radiotherapy response. For example, a risk group may indicate a probability of occurrence of a specific clinical event from 0 to <0.1 or from 0.1 to <0.25 or from 0.25 to <0.5 or from 0.5 to 1.0 or the like.

It is preferred that the biological sample is obtained from the subject before the start of the radiotherapy. The gene expression profile(s) may be determined in the form of mRNA or protein in tissue of prostate cancer. Alternatively, if the immune defense response genes are present in a soluble form, the gene expression profile(s) may be determined in blood.

It is further preferred that the radiotherapy is radical radiotherapy or salvage radiotherapy.

It is preferred that the prediction of the radiotherapy response is negative or positive for the effectiveness of the radiotherapy, wherein a therapy is recommended based on the prediction and, if the prediction is negative, the recommended therapy comprises one or more of: (i) radiotherapy provided earlier than is the standard; (ii) radiotherapy with an increased radiation dose; (iii) an adjuvant therapy, such as androgen deprivation therapy; and iv) an alternative therapy that is not a radiation therapy. The degree to which the prediction is negative may determine the degree to which the recommended therapy deviates from the standard form of radiotherapy.

In a further aspect of the present invention, an apparatus for predicting a response of a prostate cancer subject to radiotherapy is presented, comprising:

- an input adapted to receive data indicative of a gene expression profile for each of one or more, for example, 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60 or all, immune defense response genes selected from the group consisting of: APOBEC3A, AIM2, APOBEC3B, CASP1, CIAO1, CTNNB1, CXCL10, CXCL9, DDX41, DDX58, DDX60, DHX36, DHX58, DHX9, GBP1, HERC5, HIST2H2BE, IFI16, IFI44L, IFIH1, IFIT1, IFIT1B, IFIT2, IFIT3, IFITM1, IFITM3, IL1B, IRF3, IRF5, IRF7, ISG15, ITGAX, LILRB1, LRRFIP1, MAVS, MB21D1, MX1, MX2, MYD88, NLRP3, NOD2, OAS1, OAS3, OASL, OPRK1, POLR3A, PTPRC, PYCARD, RSAD2, STAT1, TBK1, TICAM1, TLR2, TLR3, TLR4, TLR7, TLR8, TLR9, TMEM173, TRIM22, and ZBP1, preferably, for each of one or more, for example, 1, 2, 3, 4, 5, 6, 7, 8, 9, 10, 11, 12, 13 or all, immune defense response genes selected from the group consisting of: AIM2, APOBEC3A, CIAO1, DDX58, DHX9, IFI16, IFIH1, IFIT1, IFIT3, LRRFIP1, MYD88, OAS1, TLR8, and ZBP1, said gene expression profile(s) being determined in a biological sample obtained from the subject,
- a processor adapted to determine the prediction of the radiotherapy response based on the gene expression profile(s) for the one or more immune defense response signaling genes, and
- optionally, a providing unit adapted to provide the prediction or a therapy recommendation based on the prediction to a medical caregiver or the subject.

In a further aspect of the present invention, a computer program product is presented comprising instructions which, when the program is executed by a computer, cause the computer to carry out a method comprising:

- receiving data indicative of a gene expression profile for each of one or more, for example, 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60 or all, immune defense response genes selected from the group consisting of: APOBEC3A, AIM2, APOBEC3B, CASP1, CIAO1, CTNNB1, CXCL10, CXCL9, DDX41, DDX58, DDX60, DHX36, DHX58, DHX9, GBP1, HERC5, HIST2H2BE, IFI16, IFI44L, IFIH1, IFIT1, IFIT1B, IFIT2, IFIT3, IFITM1, IFITM3, IL1B, IRF3, IRF5, IRF7, ISG15, ITGAX, LILRB1, LRRFIP1, MAVS, MB21D1, MX1, MX2, MYD88, NLRP3, NOD2, OAS1, OAS3, OASL, OPRK1, POLR3A, PTPRC, PYCARD, RSAD2, STAT1, TBK1, TICAM1, TLR2, TLR3, TLR4, TLR7, TLR8, TLR9, TMEM173, TRIM22, and ZBP1, preferably, for each of one or more, for example, 1, 2, 3, 4, 5, 6, 7, 8, 9, 10, 11, 12, 13 or all, immune defense response genes selected from the group consisting of: AIM2, APOBEC3A, CIAO1, DDX58, DHX9, IFI16, IFIH1, IFIT1, IFIT3, LRRFIP1, MYD88, OAS1, TLR8, and ZBP1, said gene expression profile(s) being determined in a biological sample obtained from the subject,
- determining the prediction of the radiotherapy response based on the gene expression profile(s) for the one or more immune defense response genes, and
- optionally, providing the prediction or a therapy recommendation based on the prediction to a medical caregiver or the subject.

In a further aspect of the present invention, a diagnostic kit is presented, comprising:

- at least one primer and/or probe for determining the gene expression profile for each of one or more, for example, 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60 or all, immune defense response genes selected from the group consisting of: APOBEC3A, AIM2, APOBEC3B, CASP1, CIAO1, CTNNB1, CXCL10, CXCL9, DDX41, DDX58, DDX60, DHX36, DHX58, DHX9, GBP1, HERC5, HIST2H2BE, IFI16, IFI44L, IFIH1, IFIT1, IFIT1B, IFIT2, IFIT3, IFITM1, IFITM3, IL1B, IRF3, IRF5, IRF7, ISG15, ITGAX, LILRB1, LRRFIP1, MAVS, MB21D1, MX1, MX2, MYD88, NLRP3, NOD2, OAS1, OAS3, OASL, OPRK1, POLR3A, PTPRC, PYCARD, RSAD2, STAT1, TBK1, TICAM1, TLR2, TLR3, TLR4, TLR7, TLR8, TLR9, TMEM173, TRIM22, and ZBP1, preferably, for each of one or more, for example, 1, 2, 3, 4, 5, 6, 7, 8, 9, 10, 11, 12, 13 or all, immune defense response genes selected from the group consisting of: AIM2, APOBEC3A, CIAO1, DDX58, DHX9, IFI16, IFIH1, IFIT1, IFIT3, LRRFIP1, MYD88, OAS1, TLR8, and ZBP1, in a biological sample obtained from the subject, and
- optionally, an apparatus as defined in claim 11 or a computer program product as defined in claim 12.

In a further aspect of the present invention, a use of the kit as defined in claim 13 is presented.

It is preferred that the use as defined in claim 14 is in a method of predicting a response of a prostate cancer subject to radiotherapy.

In a further aspect of the present invention, a method is presented, comprising:

- receiving a biological sample obtained from a prostate cancer subject,
- using the kit as defined in claim 12 to determine a gene expression profile for each of one or more, for example, 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60 or all, immune defense response genes selected from the group consisting of: APOBEC3A, AIM2, APOBEC3B, CASP1, CIAO1, CTNNB1, CXCL10, CXCL9, DDX41, DDX58, DDX60, DHX36, DHX58, DHX9, GBP1, HERC5, HIST2H2BE, IFI16, IFI44L, IFIH1, IFIT1, IFIT1B, IFIT2, IFIT3, IFITM1, IFITM3, IL1B, IRF3, IRF5, IRF7, ISG15, ITGAX, LILRB1, LRRFIP1, MAVS, MB21D1, MX1, MX2, MYD88, NLRP3, NOD2, OAS1, OAS3, OASL, OPRK1, POLR3A, PTPRC, PYCARD, RSAD2, STAT1, TBK1, TICAM1, TLR2, TLR3, TLR4, TLR7, TLR8, TLR9, TMEM173, TRIM22, and ZBP1, preferably, for each of one or more, for example, 1, 2, 3, 4, 5, 6, 7, 8, 9, 10, 11, 12, 13 or all, immune defense response genes selected from the group consisting of: AIM2, APOBEC3A, CIAO1, DDX58, DHX9, IFI16, IFIH1, IFIT1, IFIT3, LRRFIP1, MYD88, OAS1, TLR8, and ZBP1, in the biological sample obtained from the subject.

In a further aspect of the present invention, a use of a gene expression profile for each of one or more, for example, 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60 or all, immune defense response genes selected from the group consisting of: APOBEC3A, AIM2, APOBEC3B, CASP1, CIAO1, CTNNB1, CXCL10, CXCL9, DDX41, DDX58, DDX60, DHX36, DHX58, DHX9, GBP1, HERC5, HIST2H2BE, IFI16, IFI44L, IFIH1, IFIT1, IFIT1B, IFIT2, IFIT3, IFITM1, IFITM3, IL1B, IRF3, IRF5, IRF7, ISG15, ITGAX, LILRB1, LRRFIP1, MAVS, MB21D1, MX1, MX2, MYD88, NLRP3, NOD2, OAS1, OAS3, OASL, OPRK1, POLR3A, PTPRC, PYCARD, RSAD2, STAT1, TBK1, TICAM1, TLR2, TLR3, TLR4, TLR7, TLR8, TLR9, TMEM173, TRIM22, and ZBP1, preferably, for each of one or more, for example, 1, 2, 3, 4, 5, 6, 7, 8, 9, 10, 11, 12, 13 or all, immune defense response genes selected from the group consisting of: AIM2, APOBEC3A, CIAO1, DDX58, DHX9, IFI16, IFIH1, IFIT1, IFIT3, LRRFIP1, MYD88, OAS1, TLR8, and ZBP1, in a method of predicting a response of a prostate cancer subject to radiotherapy is presented, comprising:

- determining the prediction of the radiotherapy response based on the gene expression profile(s) for the one or more immune defense response genes, and
- optionally, providing the prediction or a therapy recommendation based on the prediction to a medical caregiver or the subject.

It shall be understood that the method of claim 1, the apparatus of claim 10, the computer program product of claim 11, the diagnostic kit of claim 12, the use of the diagnostic kit of claim 13, the method of claim 15, and the use of a gene expression profile(s) of claim 16 have similar and/or identical preferred embodiments, in particular, as defined in the dependent claims.

It shall be understood that a preferred embodiment of the present invention can also be any combination of the dependent claims or above embodiments with the respective independent claim.

These and other aspects of the invention will be apparent from and elucidated with reference to the embodiments described hereinafter.

## BRIEF DESCRIPTION OF THE DRAWINGS

In the following drawings:

Fig. 1 shows schematically and exemplarily a flowchart of an embodiment of a method of predicting a response of a prostate cancer subject to radiotherapy.

Figs. 2 and 3 show a Kaplan-Meier curve analysis of the immune defense response model (IDR model). The clinical endpoint that was tested was the time to prostate cancer specific death (PCa Death) after the start of the salvage radiation therapy (SRT) due to post-surgical disease recurrence.

Fig. 4 shows a Kaplan-Meier curve analysis of the immune defense response score (IDR_score) for men with post-surgical recurrent prostate cancer undergoing salvage radiation therapy (SRT). The clinical endpoint that was tested was prostate cancer specific death (PCa Death) after the start of SRT.

Fig. 5 shows a Kaplan-Meier curve analysis of the CARPA-S score categories for men with post-surgical recurrent prostate cancer undergoing salvage radiation therapy (SRT). The clinical endpoint that was tested was prostate cancer specific death (PCa Death) after the start of SRT.

Fig. 6 shows a Kaplan-Meier curve analysis of the EAU BCR risk groups (EAU_BCR_Risk) for men with post-surgical recurrent prostate cancer undergoing salvage radiation therapy (SRT). The clinical endpoint that was tested was prostate cancer specific death (PCa Death) after the start of SRT.

Fig. 7 shows a Kaplan-Meier curve analysis of the immune defense response score (IDR score) for men with post-surgical recurrent prostate cancer undergoing salvage androgen deprivation therapy (SADT). The clinical endpoint that was tested was prostate cancer specific death (PCa Death) after the start of SADT.

Fig. 8 shows a Kaplan-Meier curve analysis of the CARPA-S score categories for men with post-surgical recurrent prostate cancer undergoing salvage androgen deprivation therapy (SADT). The clinical endpoint that was tested was prostate cancer specific death (PCa Death) after the start of SADT.

Fig. 9 shows a Kaplan-Meier curve analysis of the EAU BCR risk groups (EAU_BCR_Risk) for men with post-surgical recurrent prostate cancer undergoing salvage radiation treatment (SRT). The clinical endpoint that was tested was prostate cancer specific death (PCa Death) after the start of SRT.

Fig. 10 shows a Kaplan-Meier curve analysis of the immune defense response score (IDR score) for men with post-surgical recurrent prostate cancer undergoing cytotoxic therapy (CTX). The clinical endpoint that was tested was prostate cancer specific death (PCa Death) after the start of CTX.

Fig. 11 shows a Kaplan-Meier curve analysis of the CARPA-S score categories for men with post-surgical recurrent prostate cancer undergoing cytotoxic therapy (CTX). The clinical endpoint that was tested was prostate cancer specific death (PCa Death) after the start of CTX.

Fig. 12 shows a Kaplan-Meier curve analysis of the EAU BCR risk groups (EAU_BCR_Risk) for men with post-surgical recurrent prostate cancer undergoing cytotoxic therapy (CTX). The clinical endpoint that was tested was prostate cancer specific death (PCa Death) after the start of CTX.

Fig. 13 shows a Kaplan-Meier curve analysis of the immune defense response score (IDR_score) for men with post-surgical recurrent prostate cancer. The clinical endpoint that was tested was metastases-free survival after primary treatment.

Fig. 14 shows a Kaplan-Meier curve analysis of the CARPA-S score categories for men with post-surgical recurrent prostate cancer. The clinical endpoint that was tested was metastases-free survival after primary treatment.

Fig. 15 shows a Kaplan-Meier curve analysis of the immune defense response score (IDR score) for men with post-surgical recurrent prostate cancer. The clinical endpoint that was tested was metastases-free survival after post-surgical biochemical recurrence (BCR).

Fig. 16 shows a Kaplan-Meier curve analysis of the CARPA-S score categories for men with post-surgical recurrent prostate cancer. The clinical endpoint that was tested was metastases-free survival after post-surgical biochemical recurrence (BCR).

Fig. 17 shows a Kaplan-Meier curve analysis of the immune defense response score (IDR score) for men with post-surgical recurrent prostate cancer. The clinical endpoint that was tested was overall survival after primary treatment.

Fig. 18 shows a Kaplan-Meier curve analysis of the pathology Gleason grade group (pGGG) for men with post-surgical recurrent prostate cancer. The clinical endpoint that was tested was overall survival after primary treatment.

Fig. 19 shows a Kaplan-Meier curve analysis of the immune defense response score (IDR score) for men with post-surgical recurrent prostate cancer. The tested endpoint was overall survival after post-surgical biochemical recurrence (BCR).

Fig. 20 shows a Kaplan-Meier curve analysis of the pathology Gleason grade group (pGGG) for men with post-surgical recurrent prostate cancer. The tested endpoint was overall survival after post-surgical biochemical recurrence (BCR).

DETAILED DESCRIPTION OF EMBODIMENTS

**Overview Of Radiotherapy Response Prediction**

Fig. 1 shows schematically and exemplarily a flowchart of an embodiment of a method of predicting a response of a prostate cancer subject to radiotherapy.

The method begins at step S100.

At step S102, a biological sample is obtained from each of a first set of patients (subjects) diagnosed with prostate cancer. Preferably, monitoring prostate cancer has been performed for these prostate cancer patients over a period of time, such as at least one year, or at least two years, or about five years, after obtaining the biological sample.

At step S104, a gene expression profile

for each of two or more, for example, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60 or all, immune defense response genes selected from the group consisting of: APOBEC3A, AIM2, APOBEC3B, CASP1, CIAO1, CTNNB1, CXCL10, CXCL9, DDX41, DDX58, DDX60, DHX36, DHX58, DHX9, GBP1, HERC5, HIST2H2BE, IFI16, IFI44L, IFIH1, IFIT1, IFIT1B, IFIT2, IFIT3, IFITM1, IFITM3, IL1B, IRF3, IRF5, IRF7, ISG15, ITGAX, LILRB1, LRRFIP1, MAVS, MB21D1, MX1, MX2, MYD88, NLRP3, NOD2, OAS1, OAS3, OASL, OPRK1, POLR3A, PTPRC, PYCARD, RSAD2, STAT1, TBK1, TICAM1, TLR2, TLR3, TLR4, TLR7, TLR8, TLR9, TMEM173, TRIM22, and ZBP1, preferably, for each of two or more, for example, 2, 3, 4, 5, 6, 7, 8, 9, 10, 11, 12, 13 or all, immune defense response genes selected from the group consisting of: AIM2, APOBEC3A, CIAO1, DDX58, DHX9, IFI16, IFIH1, IFIT1, IFIT3, LRRFIP1, MYD88, OAS1, TLR8, and ZBP1, is obtained for each of the biological samples obtained from the first set of patients, e.g., by performing RT-qPCR (real-time quantitative PCR) on RNA extracted from each biological sample. The exemplary gene expression profiles include an expression level (e.g., value) for each of the two or more immune defense response genes.

At step S106, a regression function for assigning a prediction of the radiotherapy response is determined based on the gene expression profiles for the two or more immune defense response genes, APOBEC3A, AIM2, APOBEC3B, CASP1, CIAO1, CTNNB1, CXCL10, CXCL9, DDX41, DDX58, DDX60, DHX36, DHX58, DHX9, GBP1, HERC5, HIST2H2BE, IFI16, IFI44L, IFIH1, IFIT1, IFIT1B, IFIT2, IFIT3, IFITM1, IFITM3, IL1B, IRF3, IRF5, IRF7, ISG15, ITGAX, LILRB1, LRRFIP1, MAVS, MB21D1, MX1, MX2, MYD88, NLRP3, NOD2, OAS1, OAS3, OASL, OPRK1, POLR3A, PTPRC, PYCARD, RSAD2, STAT1, TBK1, TICAM1, TLR2, TLR3, TLR4, TLR7, TLR8, TLR9, TMEM173, TRIM22, and/or ZBP1, preferably, AIM2, APOBEC3A, CIAO1, DDX58, DHX9, IFI16, IFIH1, IFIT1, IFIT3, LRRFIP1, MYD88, OAS1, TLR8, and/or ZBP1, obtained for at least some of the biological samples obtained for the first set of patients and respective results obtained from the monitoring. In one particular realization, the regression function is determined as specified in Eq. (1) above.

At step S108, a biological sample is obtained from a patient (subject or individual). The patient can be a new patient or one of the first set.

At step S110, a gene expression profile is obtained for each of the two or more, for example, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60 or all, preferably, for example, 1, 2, 3, 4, 5, 6, 7, 8, 9, 10, 11, 12, 13 or all, immune defense response genes, e.g., by performing PCR on the biological sample.

At step S112, a prediction of the radiotherapy response based on the gene expression profiles for the two or more immune defense response genes is determined for the patient using the regression function. This will be described in more detail later in the description.

At S114, a therapy recommendation may be provided, e.g., to the patient or his or her guardian, to a doctor, or to another healthcare worker, based on the prediction. To this end, the prediction may be categorized into one of a predefined set of risk groups, based on the value of the prediction. In one particular realization, the prediction of the radiotherapy response may be negative or positive for the effectiveness of the radiotherapy. If the prediction is negative, the recommended therapy may comprise one or more of: (i) radiotherapy provided earlier than is the standard; (ii) radiotherapy with an increased radiation dose; (iii) an adjuvant therapy, such as androgen deprivation therapy; and iv) an alternative therapy that is not a radiation therapy.

The method ends at S116.

In one embodiment, the gene expression profiles at steps S104 and S110 are determined by detecting mRNA expression using two or more primers and/or probes and/or two or more sets thereof.

In an alternative, the function for assigning the prediction of the radiotherapy response that is determined in step S106 is not based on a regression function but the gene expression profiles of the two or more immune defense response genes are combined with a sum function that sums discretized gene expression profiles of the two or more immune defense response genes, wherein the gene expression profiles are discretized using respective cut-offs that had been derived from the population of prostate cancer subjects. In one particular realization, the sum function is determined as specified in Eq. (2) above. In step S112, the prediction of the radiotherapy response is then determined for the patient using the sum function.

The immune system interacts in a strong manner with prostate cancer, both on a systemic level and in the tumour microenvironment. Immune defense response genes play a central role in the regulation of immune activity. Immune defense response genes may therefore provide information on the effectiveness of RT. However, which immune defense response genes may have predictive value in this application is extremely difficult to deduce from existing literature due to the many factors that influence the exact function of immune defense response genes.

We investigated the extent to which the expression of immune defense response genes in prostate cancer tissue correlates with the recurrence of disease after radical RT or SRT.

We have identified 61 resp. 14 immune defense response genes for which the degree of expression in prostate cancer tissue significantly correlates with mortality after SRT, in a cohort of 151 prostate cancer patients.

Based on the significant correlation with outcome after RT, we expect that the identified molecules will provide predictive value with regard to the effectiveness of radical RT and/or SRT.

RESULTS

**Cox Regression Analysis**

We then set out to test whether the combination of these 14 immune defense response genes will exhibit more prognostic value. With Cox regression we modelled the expression levels of the 14 immune defense response genes to prostate cancer specific death after post-surgical salvage RT (IDR_model). We tested the model in Kaplan-Meier survival analysis.

The investigated patient group consisting of 538 subjects is a surgical cohort. All patients were undergoing prostatectomy to remove their primary prostate tumors. Approximately 30% of all patients experienced PSA relapse (or biochemical recurrence) within 10 years after prostate surgery. Therefore, many of those patients (#151) with PSA recurrence have undergone salvage radiation therapy (SRT) either alone or in combination with anti-androgen treatments as a secondary therapy to treat the recurring cancer cells.

Despite this treatment, some 40 to 50% of these patients developed regional or distant metastases and some 25% died from prostate cancer within 5 to 10 years after the start of SRT.

The Cox regression function was derived as follows: IDR model:

$$(w_1 \cdot \mathrm{AIM2}) + (w_2 \cdot \mathrm{APOBEC3A}) + (w_3 \cdot \mathrm{CIAO1}) + (w_4 \cdot \mathrm{DDX58}) + (w_5 \cdot \mathrm{DHX9}) + (w_6 \cdot \mathrm{IFI16}) + (w_7 \cdot \mathrm{IFIH1}) + (w_8 \cdot \mathrm{IFIT1}) + (w_9 \cdot \mathrm{IFIT3}) + (w_{10} \cdot \mathrm{LRRFIP1}) + (w_{11} \cdot \mathrm{MYD88}) + (w_{12} \cdot \mathrm{OAS1}) + (w_{13} \cdot \mathrm{TLR8}) + (w_{14} \cdot \mathrm{ZBP1})$$

The details for the weights $w_1$ to $w_{14}$ are shown in the following TABLE 1.

TABLE 1: Variables and weights for the Cox regression model, i.e., the immune defense response model (IDR model).

| Variable | Weights | |
|---|---|---|
| **AIM2** | $w_1$ | -0,02116 |
| **APOBEC3A** | $w_2$ | -0,01195 |
| **CIAO1** | $w_3$ | -0,02092 |
| **DDX58** | $w_4$ | 0,009288 |
| **DHX9** | $w_5$ | 0,002662 |
| **IFI16** | $w_6$ | -0,01924 |
| **IFIH1** | $w_7$ | 0,007922 |
| **IFIT1** | $w_8$ | -0,06404 |
| **IFIT3** | $w_9$ | 0,01869 |
| **LRRFIP1** | $w_{10}$ | -0,005425 |
| **MYD88** | $w_{11}$ | 0,002878 |
| **OAS1** | $w_{12}$ | 0,001348 |
| **TLR8** | $w_{13}$ | -0,04764 |
| **ZBP1** | $w_{14}$ | -0,01494 |

**ROC Curve Analysis**

Next, we tested the logistic regression model as outlined above for their power to predict 5-year prostate cancer specific death (PCa Death) after start of salvage radiation therapy (SRT) due to post-surgical disease recurrence. The performance of the model was compared to the EAU BCR risk groups (see Tilki D. et al., "External validation of the European Association of Urology Biochemical Recurrence Risk groups to predict metastasis and mortality after radical prostatectomy in a European cohort", Eur Urol, Vol. 75, No. 6, pages 896-900, 2019).

Fig. 2 shows a ROC curve analysis of two predictive models. The IDR_model (AUC=0,83) is the Cox regression model based on the 14 immune defense response genes. The EAU_BCR_Risk (AUC=0,76) is the EAU_BCR_Risk groups (European Association of Urology Biochemical Recurrence Risk groups).

**Kaplan-Meier Survival Analysis**

For Kaplan-Meier curve analysis, the Cox function of the risk model (TCR_signaling_model) was categorized into two sub-cohorts based on a cut-off (see description of figure below). The goal was to create patient classes by separating the classes with the median risk score as calculated from the IDR model for each patient with to some extent similar number of patients within the individual group.

The patient classes represent an increasing risk to experience the tested clinical endpoints of time to prostate cancer specific death (Fig. 3) since the start of salvage radiation therapy (SRT) for the created risk model (IDR model).

Fig. 3 shows a Kaplan-Meier curve of the IDR_model. The clinical endpoint that was tested was the time to prostate cancer specific death (PCa Death) after the start of salvage radiation therapy (SRT) due to post-surgical disease recurrence. Patients were stratified into two cohorts (low vs. high) according to their risk to experience the clinical endpoint as predicted by the IDR regression model using the value -2.3 as cut-off (logrank p=0.0001). The following supplementary list indicate the number of patients at risk for the TCR_signaling_model classes analyzed, i.e., the patients at risk at any time interval +20 months after surgery are shown: Low risk: 78, 77, 71, 68, 60, 46, 43, 22, 6, 3, 0; High risk: 73, 67, 59, 46, 35, 30, 28, 15, 0, 0, 0.

The Kaplan-Meier curve analysis as shown in Fig. 3 demonstrates the presence of different patient risk groups. The risk group of a patient is determined by the probability to suffer from the respective clinical endpoint (e.g., prostate cancer specific death) as calculated by the risk model IDR model. Depending on the predicted risk of a patient (i.e., depending on in which risk group 1 or 2 the patient may belong) different types of interventions might be indicated.

**Immune Defense Response Score**

As an alternative to the regression model (IDR model), we employed a combination of the gene expression profiles using a sum function (IDR_score). We extensively tested the model in Kaplan-Meier survival analysis.

The sum function was derived as follows:

```
IDR score:
    For each immune defense response gene i
      if(EL(i) < cut-off(i))
         BEL(i) = 0
      else
         BEL(i) = 1
    if (sum(BEL(i) < thresh)
      IDR score = 0
    else
      IDR score = 1
```

The details for the cut-offs cut-off(i) and the threshold thresh are shown in the following TABLE 2.

TABLE 2: Variables, cut-offs and threshold for the sum model, i.e., the immune defense response score (IDR score).

| Variable | Cut-off | Threshold |
|---|---|---|
| **AIM2** | 33,44 | 1 |
| **APOBEC3A** | 8,55 | |
| **CIAO1** | 64,49 | |
| **DDX58** | 45,87 | |
| **DHX9** | 230,03 | |
| **IFI16** | 53,56 | |
| **IFIH1** | 99,63 | |
| **IFIT1** | 14,57 | |
| **IFIT3** | 11,75 | |
| **LRRFIP1** | 283,82 | |
| **MYD88** | 23,03 | |
| **OAS1** | 33,93 | |
| **TLR8** | 9,89 | |
| **ZBP1** | 20,16 | |

**Kaplan-Meier Survival Analysis**

For Kaplan-Meier curve analysis, the performance of the immune defense response score (IDR_score) was compared to the clinical risk score CAPRA-S (see Cooperberg M.R. et al., "The CAPRA-S score: A straightforward tool for improved prediction of outcomes after radical prostatectomy", Cancer, Vol. 117, No. 22, pages 5039-5046, 2011) and the EAU BCR risk groups (see Tilki D. et al., "External validation of the European Association of Urology Biochemical Recurrence Risk groups to predict metastasis and mortality after radical prostatectomy in a European cohort", Eur Urol, Vol. 75, No. 6, pages 896-900, 2019) for different patient groups and clinical endpoints.

Fig. 4 shows a Kaplan-Meier curve analysis for men with post-surgical recurrent prostate cancer undergoing salvage radiation therapy (SRT). The total SRT patient sub-cohort of 151 men was stratified according to the level of the immune defense response score (IDR score) into two cohorts (low score vs. high score). The clinical endpoint that was tested was prostate cancer specific death (PCa Death) after the start of SRT (logrank p<0.0001). The following supplementary lists indicate the number of patients at risk for the IDR_score classes analyzed, i.e., the patients at risk at any time interval +20 months after surgery are shown: High score: 76, 74, 70, 66, 59, 45, 41, 21, 3, 2, 0; Low score: 75, 70, 60, 48, 36, 31, 30, 16, 3, 1, 0.

Fig. 5 shows a Kaplan-Meier curve analysis for men with post-surgical recurrent prostate cancer undergoing salvage radiation therapy (SRT). The total SRT patient sub-cohort of 151 men was stratified according to the level of the CARPA-S score categories (low, intermediate, high risk) into three cohorts. The clinical endpoint that was tested was prostate cancer specific death (PCa Death) after the start of SRT (logrank p=0.003). The following supplementary lists indicate the number of patients at risk for the CAPRA-S classes analyzed, i.e., the patients at risk at any time interval +20 months after surgery are shown: Low risk: 23, 23, 23, 23, 20, 18, 18, 12, 1, 1, 0; Intermediate risk: 76, 73, 66, 58, 50, 41, 38, 19, 4, 2, 0; High risk: 52, 48, 41, 33, 25, 17, 15, 6, 1, 0, 0.

Fig. 6 shows a Kaplan-Meier curve analysis for men with post-surgical recurrent prostate cancer undergoing salvage radiation therapy (SRT). The total SRT patient sub-cohort of 151 men was stratified according to the level of the EAU BCR risk groups (EAU_BCR_Risk) into two cohorts (low risk vs. high risk). The clinical endpoint that was tested was prostate cancer specific death (PCa Death) after the start of SRT (logrank p=0.0001). The following supplementary lists indicate the number of patients at risk for the EAU_BCR_Risk classes analyzed, i.e., the patients at risk at any time interval +20 months after surgery are shown: Low risk: 72, 72, 69, 66, 58, 48, 45, 25, 6, 3, 0; High risk: 79, 72, 61, 48, 37, 28, 26, 12, 0, 0, 0.

Fig. 7 shows a Kaplan-Meier curve analysis for men with post-surgical recurrent prostate cancer undergoing salvage androgen deprivation therapy (SADT). The total SADT patient sub-cohort of 106 men was stratified according to the level of the immune defense response score (IDR_score) into two cohorts (low score vs. high score). The clinical endpoint that was tested was prostate cancer specific death (PCa Death) after the start of SADT (logrank p<0.0001).

The following supplementary lists indicate the number of patients at risk for the IDR_score classes analyzed, i.e., the patients at risk at any time interval +20 months after surgery are shown: High score: 48, 47, 46, 43, 38, 29, 27, 17, 3, 2, 0; Low score: 58, 52, 43, 33, 24, 21, 19, 13, 2, 1, 0.

Fig. 8 shows a Kaplan-Meier curve analysis for men with post-surgical recurrent prostate cancer undergoing salvage androgen deprivation therapy (SADT). The total SADT patient sub-cohort of 106 men was stratified according to the level of the CARPA-S score categories (low, intermediate, high risk) into three cohorts. The clinical endpoint that was tested was prostate cancer specific death (PCa Death) after the start of SADT (logrank p=0.05). The following supplementary lists indicate the number of patients at risk for the CAPRA-S classes analyzed, i.e., the patients at risk at any time interval +20 months after surgery are shown: Low risk: 11, 11, 11, 11, 11, 10, 9, 6, 1, 1, 0; Intermediate risk: 51, 48, 44, 38, 33, 28, 26, 18, 3, 2, 0; High risk: 44, 40, 34, 27, 18, 12, 11, 6, 1, 0, 0.

Fig. 9 shows a Kaplan-Meier curve analysis for men with post-surgical recurrent prostate cancer undergoing salvage androgen deprivation therapy (SADT). The total SADT patient sub-cohort of 106 men was stratified according to the level of the EAU BCR risk groups (EAU_BCR_Risk) into two cohorts (low risk vs. high risk). The clinical endpoint that was tested was prostate cancer specific death (PCa Death) after the start of SADT (logrank p=0.004). The following supplementary lists indicate the number of patients at risk for the EAU_BCR_Risk classes analyzed, i.e., the patients at risk at any time interval +20 months after surgery are shown: Low risk: 43, 43, 42, 40, 35, 29, 27, 19, 5, 3, 0; High risk: 63, 56, 47, 36, 27, 21, 19, 11, 0, 0, 0.

Fig. 10 shows a Kaplan-Meier curve analysis for men with post-surgical recurrent prostate cancer undergoing cytotoxic therapy (CTX). The total SADT patient sub-cohort of 10 men was stratified according to the level of the immune defense response score (IDR score) into two cohorts (low score vs. high score). The clinical endpoint that was tested was prostate cancer specific death (PCa Death) after the start of CTX (logrank p=0.005). The following supplementary lists indicate the number of patients at risk for the IDR_score classes analyzed, i.e., the patients at risk at any time interval +20 months after surgery are shown: High score: 2, 2, 2, 1, 1, 1, 0, 0, 0, 0, 0; Low score: 17, 6, 2, 1, 1, 1, 1, 1, 1, 1, 0.

Fig. 11 shows a Kaplan-Meier curve analysis for men with post-surgical recurrent prostate cancer undergoing cytotoxic therapy (CTX). The total SADT patient sub-cohort of 19 men was stratified according to the level of the CARPA-S score categories (low, intermediate, high risk) into three cohorts. (In this example, there were no patients in the low risk class.) The clinical endpoint that was tested was prostate cancer specific death (PCa Death) after the start of CTX (logrank p=0.38). The following supplementary lists indicate the number of patients at risk for the CAPRA-S classes analyzed, i.e., the patients at risk at any time interval +20 months after surgery are shown: Low risk: -; Intermediate risk: 10, 3, 2, 1, 1, 1, 1, 1, 1, 1, 0; High risk: 9, 5, 2, 1, 1, 1, 0, 0, 0, 0, 0.

Fig. 12 shows a Kaplan-Meier curve analysis for men with post-surgical recurrent prostate cancer undergoing cytotoxic therapy (CTX). The total SADT patient sub-cohort of 19 men was stratified according to the level of the EAU BCR risk groups (EAU_BCR_Risk) into two cohorts (low risk vs. high risk). The clinical endpoint that was tested was prostate cancer specific death (PCa Death) after the start of CTX (logrank p=0.4). The following supplementary lists indicate the number of patients at risk for the EAU_BCR_Risk classes analyzed, i.e., the patients at risk at any time interval +20 months after surgery are shown: Low risk: 5, 3, 2, 1, 1, 1, 1, 1, 1, 1, 0; High risk: 14, 5, 2, 1, 1, 1, 0, 0, 0, 0, 0.

Fig. 13 shows a Kaplan-Meier curve analysis for men with post-surgical recurrent prostate cancer. The total patient cohort of 130 men was stratified according to the level of the immune defense response score (IDR score) into two cohorts (low score vs. high score). The clinical endpoint that was tested was metastases-free survival after primary treatment (logrank p=0.0004). The following supplementary lists indicate the number of patients at risk for the IDR_score classes analyzed, i.e., the patients at risk at any time interval +20 months after surgery are shown: High score: 87, 81, 66, 36, 19, 11, 2, 1, 0; Low score: 43, 37, 29, 17, 8, 5, 2, 0, 0.

Fig. 14 shows a Kaplan-Meier curve analysis for men with post-surgical recurrent prostate cancer. The total patient cohort of 130 men was stratified according to the level of the CARPA-S score categories (low, intermediate, high risk) into three cohorts. The clinical endpoint that was tested was metastases-free survival after primary treatment (logrank p=0.04). The following supplementary lists indicate the number of patients at risk for the CAPRA-S classes analyzed, i.e., the patients at risk at any time interval +20 months after surgery are shown: Low risk: 64, 61, 46, 22, 12, 6, 2, 0, 0; Intermediate risk: 42, 37, 31, 21, 10, 6, 1, 1, 0; High risk: 23, 20, 18, 10, 5, 4, 1, 0, 0.

Fig. 15 shows a Kaplan-Meier curve analysis for men with post-surgical recurrent prostate cancer. The total patient cohort of 130 men was stratified according to the level of the immune defense response score (IDR_score) into two cohorts (low score vs. high score). The clinical endpoint that was tested was metastases-free survival after post-surgical biochemical recurrence (BCR) (logrank p=0.004). The following supplementary lists indicate the number of patients at risk for the IDR_score classes analyzed, i.e., the patients at risk at any time interval +20 months after surgery are shown: High score: 15, 14, 12, 10, 6, 4, 1, 0; Low score: 11, 10, 9, 6, 3, 1, 0, 0.

Fig. 16 shows a Kaplan-Meier curve analysis for men with post-surgical recurrent prostate cancer. The total patient cohort of 130 men was stratified according to the level of the CARPA-S score categories (low, intermediate, high risk) into three cohorts. The clinical endpoint that was tested was metastases-free survival after post-surgical biochemical recurrence (BCR) (logrank p=0.39). The following supplementary lists indicate the number of patients at risk for the CAPRA-S classes analyzed, i.e., the patients at risk at any time interval +20 months after surgery are shown: Low risk: 4, 4, 3, 3, 2, 2, 0, 0; Intermediate risk: 11, 10, 9, 7, 4, 1, 0, 0; High risk: 11, 10, 9, 6, 3, 2, 1, 0.

Fig. 17 shows a Kaplan-Meier curve analysis for men with post-surgical recurrent prostate cancer. The total patient cohort of 56 men was stratified according to the level of the immune defense response score (IDR score) into two cohorts (low score vs. high score). The clinical endpoint that was tested was overall survival after primary treatment (logrank $p<0.0001$). The following supplementary lists indicate the number of patients at risk for the IDR_score classes analyzed, i.e., the patients at risk at any time interval +20 months after surgery are shown: High score: 42, 41, 39, 37, 37, 35, 26, 15, 9, 8, 5, 4, 1, 0; Low score: 14, 14, 14, 12, 11, 9, 7, 4, 2, 0, 0, 0, 0, 0.

Fig. 18 shows a Kaplan-Meier curve analysis for men with post-surgical recurrent prostate cancer. The total patient cohort of 56 men was stratified according to the level of the pathology Gleason grade group into two cohorts (pGGG≤2 vs. pGGG≥3). The clinical endpoint that was tested was overall survival after primary treatment (logrank $p<0.02$). The following supplementary lists indicate the number of patients at risk for the pGGG classes analyzed, i.e., the patients at risk at any time interval +20 months after surgery are shown: pGGG≤2: 42, 42, 40, 38, 37, 35, 26, 18, 10, 7, 5, 4, 1, 0; pGGG≥3: 14, 13, 13, 11, 11,9, 7, 1, 1, 1, 0, 0, 0, 0.

Fig. 19 shows a Kaplan-Meier curve analysis for men with post-surgical recurrent prostate cancer. The total patient cohort of 56 men was stratified according to the level of the immune defense response score (IDR_score) into two cohorts (low score vs. high score). The clinical endpoint that was tested was overall survival after post-surgical biochemical recurrence (BCR) (logrank $p<0.0001$). The following supplementary lists indicate the number of patients at risk for the IDR_score classes analyzed, i.e., the patients at risk at any time interval +20 months after surgery are shown: High score: 23, 22, 21, 20, 20, 18, 14, 10, 5, 5, 4, 3, 0; Low score: 11, 10, 8, 8, 7, 6, 4, 2, 1, 0, 0, 0, 0.

Fig. 20 shows a Kaplan-Meier curve analysis for men with post-surgical recurrent prostate cancer. The total patient cohort of 56 men was stratified according to the level of the pathology Gleason grade group into two cohorts (pGGG≤2 vs. pGGG≥3). The clinical endpoint that was tested was overall survival after post-surgical biochemical recurrence (BCR) (logrank $p=0.13$). The following supplementary lists indicate the number of patients at risk for the pGGG classes analyzed, i.e., the patients at risk at any time interval +20 months after surgery are shown: pGGG≤2: 22, 21, 20, 19, 18, 17, 13, 12, 6, 5, 4, 3, 0; pGGG≥3: 12, 11, 9, 9, 9, 7, 5, 0, 0, 0, 0, 0, 0.

The data presented in Figs. 2 to 20 demonstrate that the measurement and combination of immune defense response genes provide a means to define patient groups with different risks to die from prostate cancer after post-surgical disease recurrence and start of secondary therapies like radiation therapy. Furthermore, the presented data shows that a selection of 14 of these immune defense genes can separate patient groups with different risks of progressive disease in multiple independent data sets.

**Discussion**

The effectiveness of both radical RT and SRT for localized prostate cancer is limited, resulting in disease progression and ultimately death of patients, especially for those at high risk of recurrence. The prediction of the therapy outcome is very complicated as many factors play a role in therapy effectiveness and disease recurrence. It is likely that important factors have not yet been identified, while the effect of others cannot be determined precisely. Multiple clinico-pathological measures are currently investigated and applied in a clinical setting to improve response prediction and therapy selection, providing some degree of improvement. Nevertheless, a strong need remains for better prediction of the response to radical RT and to SRT, in order to increase the success rate of these therapies.

We have identified molecules of which expression shows a significant relation to mortality after radical RT and SRT and therefore are expected to improve the prediction of the effectiveness of these treatments. An improved prediction of effectiveness of RT for each patient be it in the radical or the salvage setting, will improve therapy selection and potentially survival. This can be achieved by 1) optimizing RT for those patients where RT is predicted to be effective (e.g. by dose escalation or a different starting time) and 2) guiding patients where RT is predicted not to be effective to an alternative, potentially more effective form of treatment. Further, this would reduce suffering for those patients who would be spared ineffective therapy and would reduce cost spent on ineffective therapies.

Other variations to the disclosed realizations can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality.

One or more steps of the method illustrated in Fig. 1 may be implemented in a computer program product that may be executed on a computer. The computer program product may comprise a non-transitory computer-readable recording medium on which a control program is recorded (stored), such as a disk, hard drive, or the like. Common forms of non-transitory computer-readable media include, for example, floppy disks, flexible disks, hard disks, magnetic tape, or any other magnetic storage medium, CD-ROM, DVD, or any other optical medium, a RAM, a PROM, an EPROM, a FLASH-EPROM, or other memory chip or cartridge, or any other non-transitory medium from which a computer can read and use.

Alternatively, the one or more steps of the method may be implemented in transitory media, such as a transmittable carrier wave in which the control program is embodied as a data signal using transmission media, such as acoustic or light waves, such as those generated during radio wave and infrared data communications, and the like.

The exemplary method may be implemented on one or more general purpose computers, special purpose computer(s), a programmed microprocessor or microcontroller and peripheral integrated circuit elements, an ASIC or other integrated circuit, a digital signal processor, a hardwired electronic or logic circuit such as a discrete element circuit, a programmable logic device such as a PLD, PLA, FPGA, Graphical card CPU (GPU), or PAL, or the like. In general, any device, capable of implementing a finite state machine that is in turn capable of implementing the flowchart shown in Fig. 1, can be used to implement one or more steps of the method of risk stratification for therapy selection in a patient with prostate cancer is illustrated. As will be appreciated, while the steps of the method may all be computer implemented, in some embodiments one or more of the steps may be at least partially performed manually.

The computer program instructions may also be loaded onto a computer, other programmable data processing apparatus, or other devices to cause a series of operational steps to be performed on the computer, other programmable apparatus or other devices to produce a computer implemented process such that the instructions which execute on the computer or other programmable apparatus provide processes for implementing the functions/acts specified herein.

Any reference signs in the claims should not be construed as limiting the scope.

The invention relates to a method of predicting a response of a prostate cancer subject to radiotherapy, comprising determining or receiving the result of a determination of a gene expression profile for each of one or more, for example, 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60 or all, immune defense response genes selected from the group consisting of: APOBEC3A, AIM2, APOBEC3B, CASP1, CIAO1, CTNNB1, CXCL10, CXCL9, DDX41, DDX58, DDX60, DHX36, DHX58, DHX9, GBP1, HERC5, HIST2H2BE, IFI16, IFI44L, IFIH1, IFIT1, IFIT1B, IFIT2, IFIT3, IFITM1, IFITM3, IL1B, IRF3, IRF5, IRF7, ISG15, ITGAX, LILRB1, LRRFIP1, MAVS, MB21D1, MX1, MX2, MYD88, NLRP3, NOD2, OAS1, OAS3, OASL, OPRK1, POLR3A, PTPRC, PYCARD, RSAD2, STAT1, TBK1, TICAM1, TLR2, TLR3, TLR4, TLR7, TLR8, TLR9, TMEM173, TRIM22, and ZBP1, preferably, for each of one or more, for example, 1, 2, 3, 4, 5, 6, 7, 8, 9, 10, 11, 12, 13 or all, immune defense response genes selected from the group consisting of: AIM2, APOBEC3A, CIAO1, DDX58, DHX9, IFI16, IFIH1, IFIT1, IFIT3, LRRFIP1, MYD88, OAS1, TLR8, and ZBP1, said gene expression profile(s) being determined in a biological sample obtained from the subject, determining the prediction of the radiotherapy response based on the gene expression profile(s) for the one or more immune defense response genes, and optionally, providing the prediction or a therapy recommendation based on the prediction to a medical caregiver or the subject. Since the status of the immune system and of the immune microenvironment have an impact on therapy effectiveness, the ability to identify markers predictive for this effect might help to be better able to predict overall RT response. Immune defense response genes play a central role in the regulation of immune activity. The identified immune defense response genes were found to exhibit a significant correlation with outcome after RT, wherefore we expect that they will provide predictive value with regard to the effectiveness of radical RT and/or SRT.

**The attached Sequence Listing, entitled 2019PF00711_Sequence Listing_ST25 is incorporated herein by reference, in its entirety.**

## Claims

1. A method of predicting a response of a prostate cancer subject to radiotherapy, comprising:

   - determining or receiving the result of a determination of a gene expression profile for each of one or more, for example, 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60 or all, immune defense response genes selected from the group consisting of: APOBEC3A, AIM2, APOBEC3B, CASP1, CIAO1, CTNNB1, CXCL10, CXCL9, DDX41, DDX58, DDX60, DHX36, DHX58, DHX9, GBP1, HERC5, HIST2H2BE, IFI16, IFI44L, IFIH1, IFIT1, IFIT1B, IFIT2, IFIT3, IFITM1, IFITM3, IL1B, IRF3, IRF5, IRF7, ISG15, ITGAX, LILRB1, LRRFIP1, MAVS, MB21D1, MX1, MX2, MYD88, NLRP3, NOD2, OAS1, OAS3, OASL, OPRK1, POLR3A, PTPRC, PYCARD, RSAD2, STAT1, TBK1, TICAM1, TLR2, TLR3, TLR4, TLR7, TLR8, TLR9, TMEM173, TRIM22, and ZBP1, preferably, for each of one or more, for example, 1, 2, 3, 4, 5, 6, 7, 8, 9, 10, 11, 12, 13 or all, immune defense response genes selected from the group consisting of: AIM2, APOBEC3A, CIAO1, DDX58, DHX9, IFI16, IFIH1, IFIT1, IFIT3, LRRFIP1, MYD88, OAS1, TLR8, and ZBP1, said gene expression profile(s) being determined in a biological sample obtained from the subject,
   - determining the prediction of the radiotherapy response based on the gene expression profile(s) for the one or more immune defense response genes, and
   - optionally, providing the prediction or a therapy recommendation based on the prediction to a medical caregiver or the subject.

2. The method as defined in claim 1, wherein the one or more immune defense response genes comprise three or more of the immune defense response genes selected from the group consisting of: AIM2, APOBEC3A, CIAO1, DDX58, DHX9, IFI16, IFIH1, IFIT1, IFIT3, LRRFIP1, MYD88, OAS1, TLR8, and ZBP1.

3. The method as defined in claim 1 or 2, wherein the one or more immune defense response genes comprise six or more of the immune defense response genes selected from the group consisting of: AIM2, APOBEC3A, CIAO1, DDX58, DHX9, IFI16, IFIH1, IFIT1, IFIT3, LRRFIP1, MYD88, OAS1, TLR8, and ZBP1.

4. The method as defined in any of claims 1 to 3, wherein the one or more immune defense response genes comprise nine or more, preferably, all of the immune defense response genes selected from the group consisting of: AIM2, APOBEC3A, CIAO1, DDX58, DHX9, IFI16, IFIH1, IFIT1, IFIT3, LRRFIP1, MYD88, OAS1, TLR8, and ZBP1.

5. The method as defined in any of claims 1 to 4, wherein the determining of the prediction of the radiotherapy response comprises combining the gene expression profiles for two or more, for example, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60 or all, of the immune defense response genes selected from the group consisting of: APOBEC3A, AIM2, APOBEC3B, CASP1, CIAO1, CTNNB1, CXCL10, CXCL9, DDX41, DDX58, DDX60, DHX36, DHX58, DHX9, GBP1, HERC5, HIST2H2BE, IFI16, IFI44L, IFIH1, IFIT1, IFIT1B, IFIT2, IFIT3, IFITM1, IFITM3, IL1B, IRF3, IRF5, IRF7, ISG15, ITGAX, LILRB1, LRRFIP1, MAVS, MB21D1, MX1, MX2, MYD88, NLRP3, NOD2, OAS1, OAS3, OASL, OPRK1, POLR3A, PTPRC, PYCARD, RSAD2, STAT1, TBK1, TICAM1, TLR2, TLR3, TLR4, TLR7, TLR8, TLR9, TMEM173, TRIM22, and ZBP1, preferably, for example, 2, 3, 4, 5, 6, 7, 8, 9, 10, 11, 12, 13 or all, of the immune defense response genes selected from the group consisting of: AIM2, APOBEC3A, CIAO1, DDX58, DHX9, IFI16, IFIH1, IFIT1, IFIT3, LRRFIP1, MYD88, OAS1, TLR8, and ZBP1, with a regression function that had been derived from a population of prostate cancer subjects.

6. The method as defined in claim 1, wherein the determining of the prediction of the radiotherapy response comprises combining the gene expression profiles for two or more, for example, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60 or all, of the immune defense response genes selected from the group consisting of: APOBEC3A, AIM2, APOBEC3B, CASP1, CIAO1, CTNNB1, CXCL10, CXCL9, DDX41, DDX58, DDX60, DHX36, DHX58, DHX9, GBP1, HERC5, HIST2H2BE, IFI16, IFI44L, IFIH1, IFIT1, IFIT1B, IFIT2, IFIT3, IFITM1, IFITM3, IL1B, IRF3, IRF5, IRF7, ISG15, ITGAX, LILRB1, LRRFIP1, MAVS, MB21D1, MX1, MX2, MYD88, NLRP3, NOD2, OAS1, OAS3, OASL, OPRK1, POLR3A, PTPRC, PYCARD, RSAD2, STAT1, TBK1, TICAM1, TLR2, TLR3, TLR4, TLR7, TLR8, TLR9, TMEM173, TRIM22, and ZBP1, preferably, for example, 2, 3, 4, 5, 6, 7, 8, 9, 10, 11, 12, 13 or all, of the immune defense response genes selected from the group consisting of: AIM2, APOBEC3A, CIAO1, DDX58, DHX9, IFI16, IFIH1, IFIT1, IFIT3, LRRFIP1, MYD88, OAS1, TLR8, and ZBP1, with a sum function that sums discretized gene expression profiles of the two or more immune defense response genes, wherein the gene expression profiles are discretized using respective cut-offs that had been derived from a population of prostate cancer subjects.

**7.** The method as defined in any of claims 1 to 6, wherein the biological sample is obtained from the subject before the start of the radiotherapy.

**8.** The method as defined in any of claims 1 to 7, wherein the radiotherapy is radical radiotherapy or salvage radiotherapy.

**9.** The method as defined in any of claims 1 to 8, wherein the prediction of the radiotherapy response is negative or positive for the effectiveness of the radiotherapy, wherein a therapy is recommended based on the prediction and, if the prediction is negative, the recommended therapy comprises one or more of: (i) radiotherapy provided earlier than is the standard; (ii) radiotherapy with an increased radiation dose; (iii) an adjuvant therapy, such as androgen deprivation therapy; and iv) an alternative therapy that is not a radiation therapy.

**10.** An apparatus for predicting a response of a prostate cancer subject to radiotherapy, comprising:

- an input adapted to receive data indicative of a gene expression profile for each of one or more, for example, 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60 or all, immune defense response genes selected from the group consisting of: APOBEC3A, AIM2, APOBEC3B, CASP1, CIAO1, CTNNB1, CXCL10, CXCL9, DDX41, DDX58, DDX60, DHX36, DHX58, DHX9, GBP1, HERC5, HIST2H2BE, IFI16, IFI44L, IFIH1, IFIT1, IFIT1B, IFIT2, IFIT3, IFITM1, IFITM3, IL1B, IRF3, IRF5, IRF7, ISG15, ITGAX, LILRB1, LRRFIP1, MAVS, MB21D1, MX1, MX2, MYD88, NLRP3, NOD2, OAS1, OAS3, OASL, OPRK1, POLR3A, PTPRC, PYCARD, RSAD2, STAT1, TBK1, TICAM1, TLR2, TLR3, TLR4, TLR7, TLR8, TLR9, TMEM173, TRIM22, and ZBP1, preferably, for each of one or more, for example, 1, 2, 3, 4, 5, 6, 7, 8, 9, 10, 11, 12, 13 or all, immune defense response genes selected from the group consisting of: AIM2, APOBEC3A, CIAO1, DDX58, DHX9, IFI16, IFIH1, IFIT1, IFIT3, LRRFIP1, MYD88, OAS1, TLR8, and ZBP1, said gene expression profile(s) being determined in a biological sample obtained from the subject,
- a processor adapted to determine the prediction of the radiotherapy response based on the gene expression profile(s) for the one or more immune defense response signaling genes, and
- optionally, a providing unit adapted to provide the prediction or a therapy recommendation based on the prediction to a medical caregiver or the subject.

**11.** A computer program product comprising instructions which, when the program is executed by a computer, cause the computer to carry out a method comprising:

- receiving data indicative of a gene expression profile for each of one or more, for example, 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60 or all, immune defense response genes selected from the group consisting of: APOBEC3A, AIM2, APOBEC3B, CASP1, CIAO1, CTNNB1, CXCL10, CXCL9, DDX41, DDX58, DDX60, DHX36, DHX58, DHX9, GBP1, HERC5, HIST2H2BE, IFI16, IFI44L, IFIH1, IFIT1, IFIT1B, IFIT2, IFIT3, IFITM1, IFITM3, IL1B, IRF3, IRF5, IRF7, ISG15, ITGAX, LILRB1, LRRFIP1, MAVS, MB21D1, MX1, MX2, MYD88, NLRP3, NOD2, OAS1, OAS3, OASL, OPRK1, POLR3A, PTPRC, PYCARD, RSAD2, STAT1, TBK1, TICAM1, TLR2, TLR3, TLR4, TLR7, TLR8, TLR9, TMEM173, TRIM22, and ZBP1, preferably, for each of one or more, for example, 1, 2, 3, 4, 5, 6, 7, 8, 9, 10, 11, 12, 13 or all, immune defense response genes selected from the group consisting of: AIM2, APOBEC3A, CIAO1, DDX58, DHX9, IFI16, IFIH1, IFIT1, IFIT3, LRRFIP1, MYD88, OAS1, TLR8, and ZBP1, said gene expression profile(s) being determined in a biological sample obtained from the subject,
- determining the prediction of the radiotherapy response based on the gene expression profile(s) for the one or more immune defense response genes, and
- optionally, providing the prediction or a therapy recommendation based on the prediction to a medical caregiver or the subject.

**12.** A diagnostic kit, comprising: at least one primer and/or probe for determining the gene expression profile for each of one or more, for example, 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60 or all, immune defense response genes selected from the group consisting of: APOBEC3A, AIM2, APOBEC3B, CASP1, CIAO1, CTNNB1, CXCL10, CXCL9, DDX41, DDX58, DDX60, DHX36, DHX58, DHX9, GBP1, HERC5, HIST2H2BE, IFI16, IFI44L, IFIH1, IFIT1, IFIT1B, IFIT2, IFIT3, IFITM1, IFITM3, IL1B, IRF3, IRF5, IRF7, ISG15, ITGAX, LILRB1, LRRFIP1, MAVS, MB21D1, MX1, MX2, MYD88, NLRP3, NOD2, OAS1, OAS3, OASL, OPRK1, POLR3A, PTPRC, PYCARD, RSAD2, STAT1, TBK1, TICAM1, TLR2, TLR3, TLR4, TLR7, TLR8, TLR9, TMEM173, TRIM22, and ZBP1, preferably, for each of one or more, for example, 1, 2, 3, 4, 5, 6, 7, 8, 9, 10, 11, 12, 13 or all, immune defense response genes selected from the group consisting of: AIM2, APOBEC3A, CIAO1, DDX58, DHX9, IFI16, IFIH1, IFIT1, IFIT3, LRRFIP1, MYD88, OAS1, TLR8, and ZBP1, in a biological sample obtained from the subject, and

- optionally, an apparatus as defined in claim 10 or a computer program product as defined in claim 11.

**13.** Use of the kit as defined in claim 12.

**14.** The use of the kit as defined in claim 13 in a method of predicting a response of a prostate cancer subject to radiotherapy.

**15.** A method, comprising:

- receiving a biological sample obtained from a prostate cancer subject,
- using the kit as defined in claim 12 to determine a gene expression profile for each of one or more, for example, 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60 or all, immune defense response genes selected from the group consisting of: APOBEC3A, AIM2, APOBEC3B, CASP1, CIAO1, CTNNB1, CXCL10, CXCL9, DDX41, DDX58, DDX60, DHX36, DHX58, DHX9, GBP1, HERC5, HIST2H2BE, IFI16, IFI44L, IFIH1, IFIT1, IFIT1B, IFIT2, IFIT3, IFITM1, IFITM3, IL1B, IRF3, IRF5, IRF7, ISG15, ITGAX, LILRB1, LRRFIP1, MAVS, MB21D1, MX1, MX2, MYD88, NLRP3, NOD2, OAS1, OAS3, OASL, OPRK1, POLR3A, PTPRC, PYCARD, RSAD2, STAT1, TBK1, TICAM1, TLR2, TLR3, TLR4, TLR7, TLR8, TLR9, TMEM173, TRIM22, and ZBP1, preferably, for each of one or more, for example, 1, 2, 3, 4, 5, 6, 7, 8, 9, 10, 11, 12, 13 or all, immune defense response genes selected from the group consisting of: AIM2, APOBEC3A, CIAO1, DDX58, DHX9, IFI16, IFIH1, IFIT1, IFIT3, LRRFIP1, MYD88, OAS1, TLR8, and ZBP1, in the biological sample obtained from the subject.

**16.** Use of a gene expression profile for each of one or more, for example, 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60 or all, immune defense response genes selected from the group consisting of: APOBEC3A, AIM2, APOBEC3B, CASP1, CIAO1, CTNNB1, CXCL10, CXCL9, DDX41, DDX58, DDX60, DHX36, DHX58, DHX9, GBP1, HERC5, HIST2H2BE, IFI16, IFI44L, IFIH1, IFIT1, IFIT1B, IFIT2, IFIT3, IFITM1, IFITM3, IL1B, IRF3, IRF5, IRF7, ISG15, ITGAX, LILRB1, LRRFIP1, MAVS, MB21D1, MX1, MX2, MYD88, NLRP3, NOD2, OAS1, OAS3, OASL, OPRK1, POLR3A, PTPRC, PYCARD, RSAD2, STAT1, TBK1, TICAM1, TLR2, TLR3, TLR4, TLR7, TLR8, TLR9, TMEM173, TRIM22, and ZBP1, preferably, for each of one or more, for example, 1, 2, 3, 4, 5, 6, 7, 8, 9, 10, 11, 12, 13 or all, immune defense response genes selected from the group consisting of: AIM2, APOBEC3A, CIAO1, DDX58, DHX9, IFI16, IFIH1, IFIT1, IFIT3, LRRFIP1, MYD88, OAS1, TLR8, and ZBP1, in a method of predicting a response of a prostate cancer subject to radiotherapy, comprising:

- determining the prediction of the radiotherapy response based on the gene expression profile(s) for the one or more immune defense response genes, and
- optionally, providing the prediction or a therapy recommendation based on the prediction to a medical caregiver or the subject.

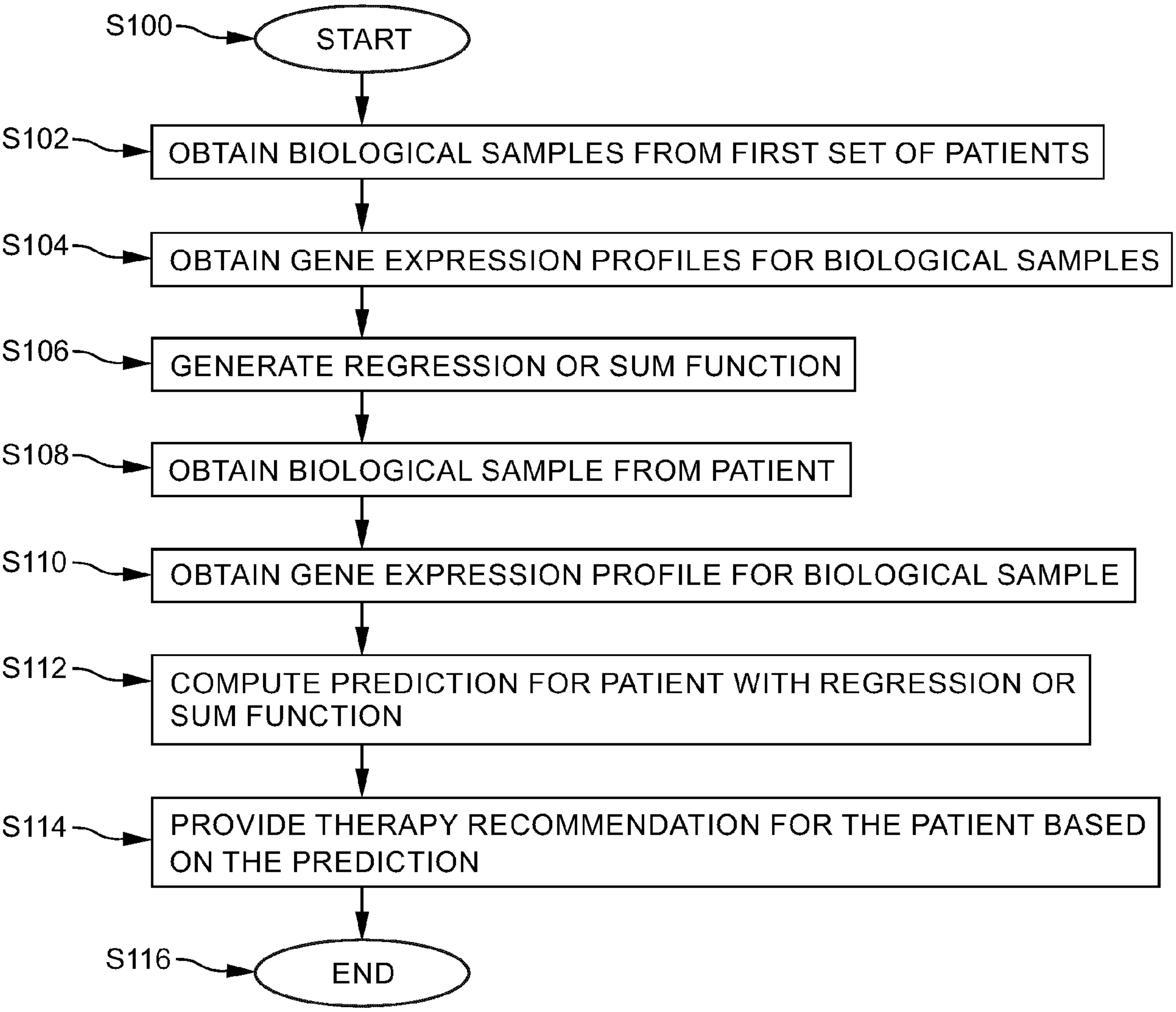
S100
START
S102
OBTAIN BIOLOGICAL SAMPLES FROM FIRST SET OF PATIENTS
S104
OBTAIN GENE EXPRESSION PROFILES FOR BIOLOGICAL SAMPLES
S106
GENERATE REGRESSION OR SUM FUNCTION
S108
OBTAIN BIOLOGICAL SAMPLE FROM PATIENT
S110
OBTAIN GENE EXPRESSION PROFILE FOR BIOLOGICAL SAMPLE
S112
COMPUTE PREDICTION FOR PATIENT WITH REGRESSION OR SUM FUNCTION
S114
PROVIDE THERAPY RECOMMENDATION FOR THE PATIENT BASED ON THE PREDICTION
S116
END

FIG. 1

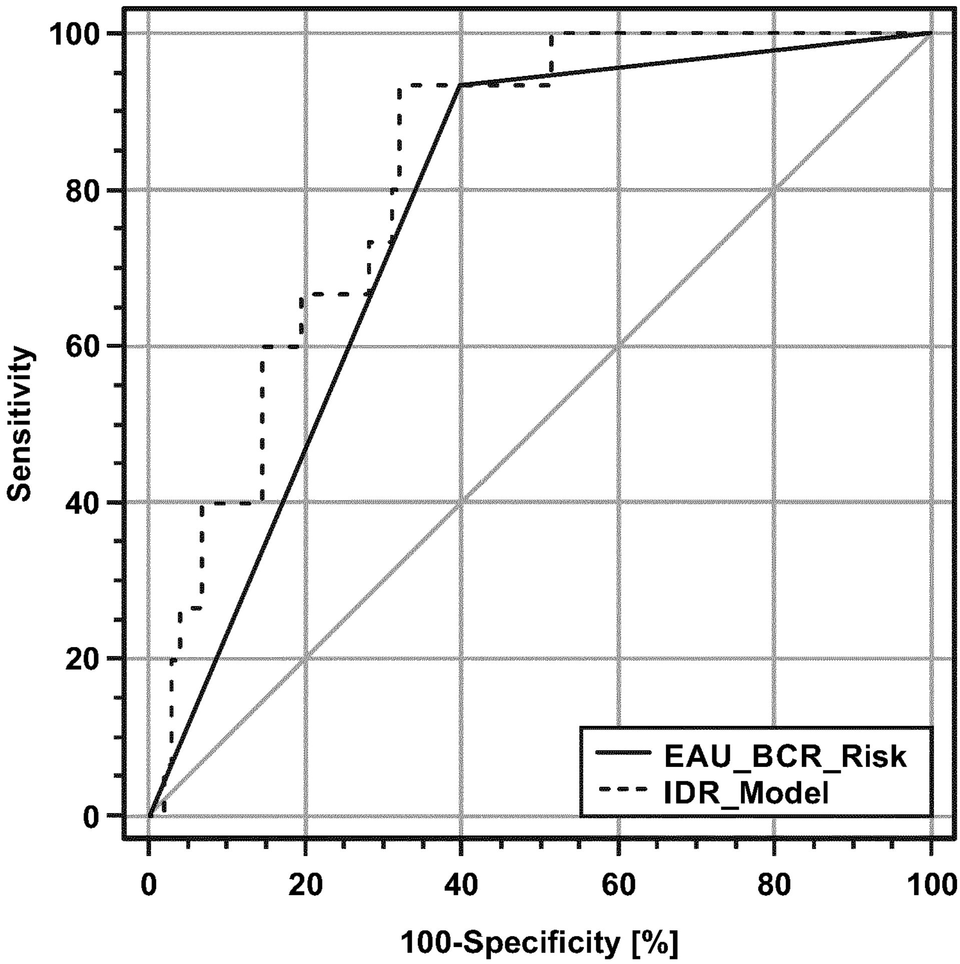
100
80
60
40
20
0
Sensitivity
0
20
40
60
80
100
100-Specificity [%]
EAU_BCR_Risk
IDR_Model

FIG. 2

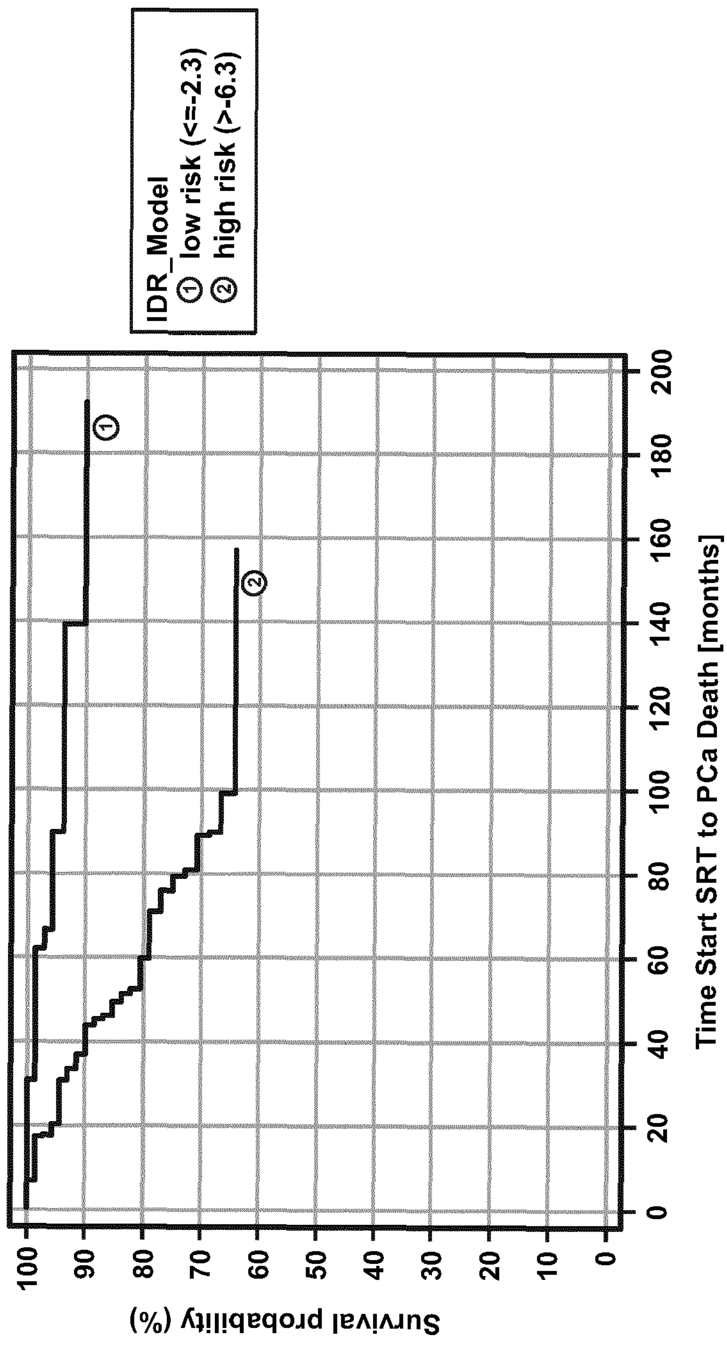
IDR_Model
① low risk (<=-2.3)
② high risk (>-6.3)
Survival probability (%)
100
90
80
70
60
50
40
30
20
10
0
0
20
40
60
80
100
120
140
160
180
200
Time Start SRT to PCa Death [months]
①
②

FIG. 3

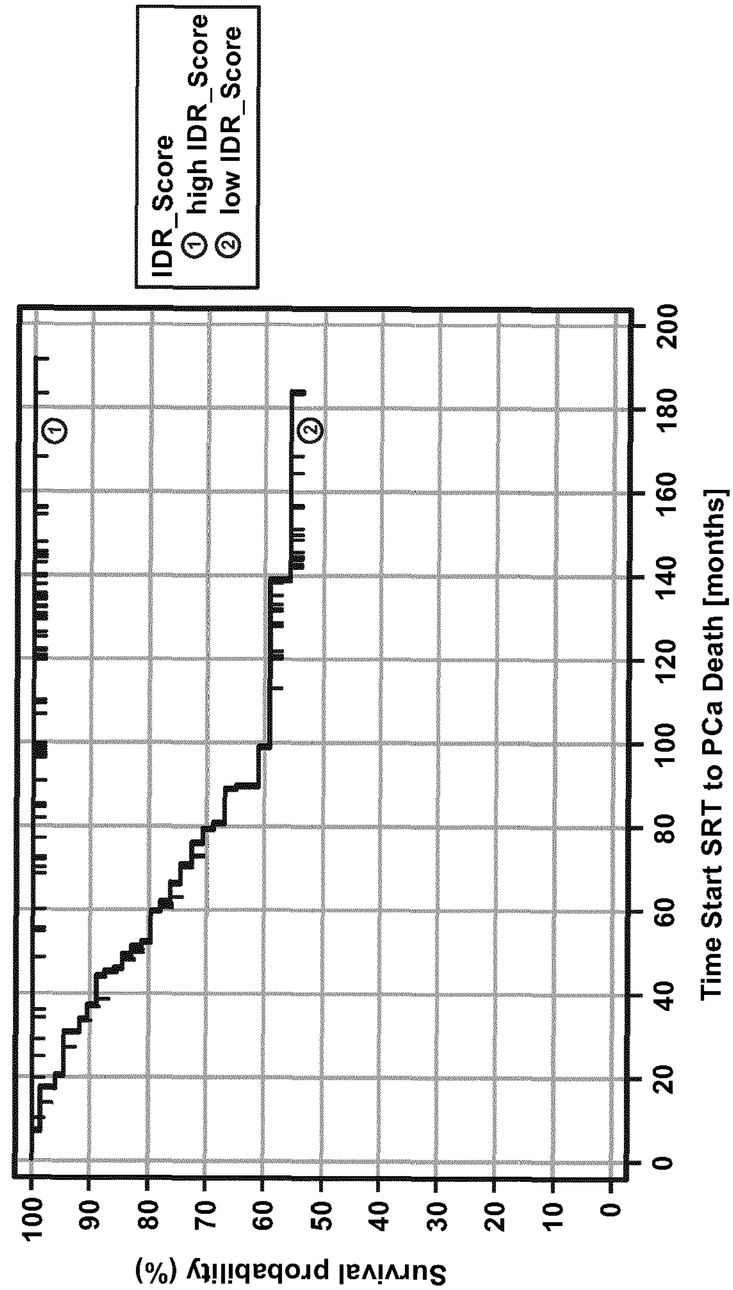
IDR_Score
① high IDR_Score
② low IDR_Score
Survival probability (%)
100
90
80
70
60
50
40
30
20
10
0
0
20
40
60
80
100
120
140
160
180
200
Time Start SRT to PCa Death [months]
①
②

FIG. 4

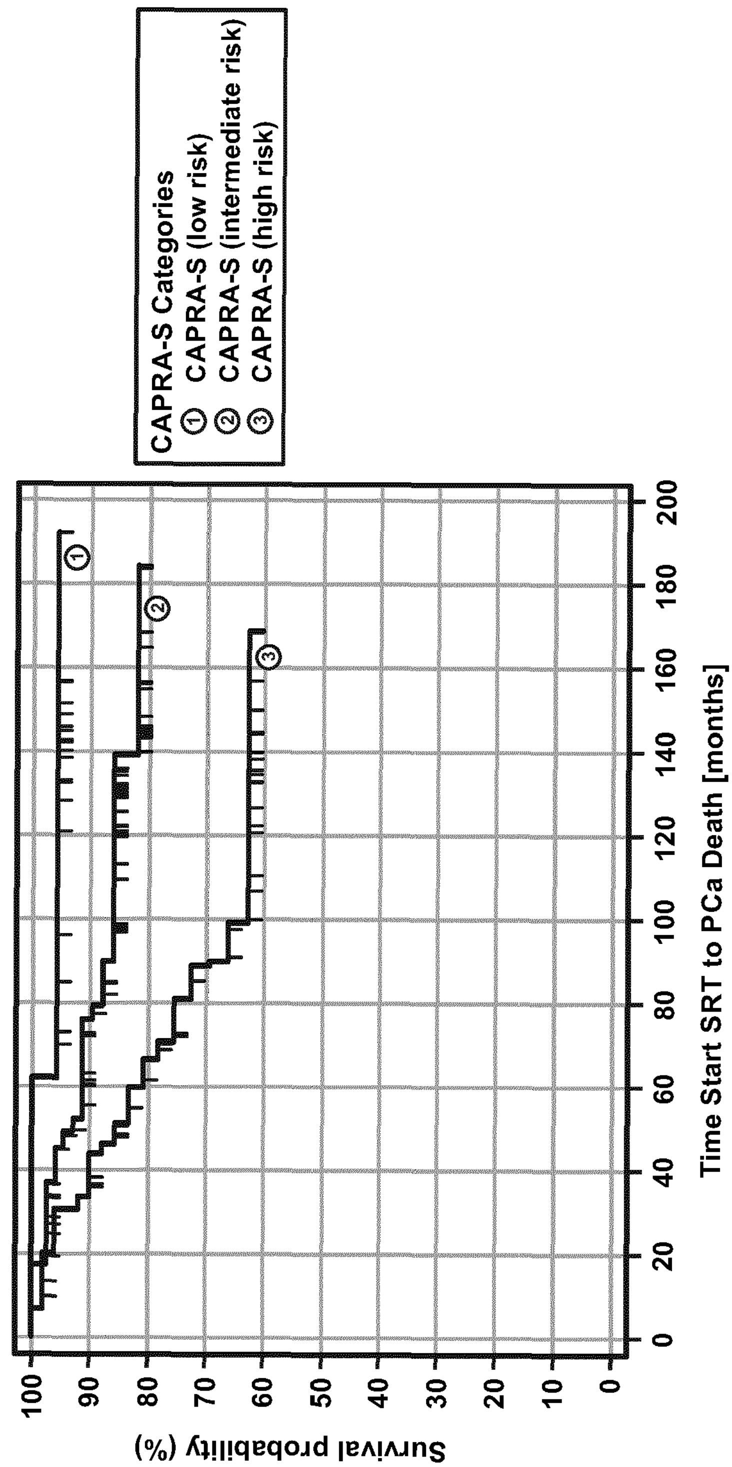
CAPRA-S Categories
① CAPRA-S (low risk)
② CAPRA-S (intermediate risk)
③ CAPRA-S (high risk)
Survival probability (%)
100
90
80
70
60
50
40
30
20
10
0
0
20
40
60
80
100
120
140
160
180
200
Time Start SRT to PCa Death [months]
1
2
3

FIG. 5

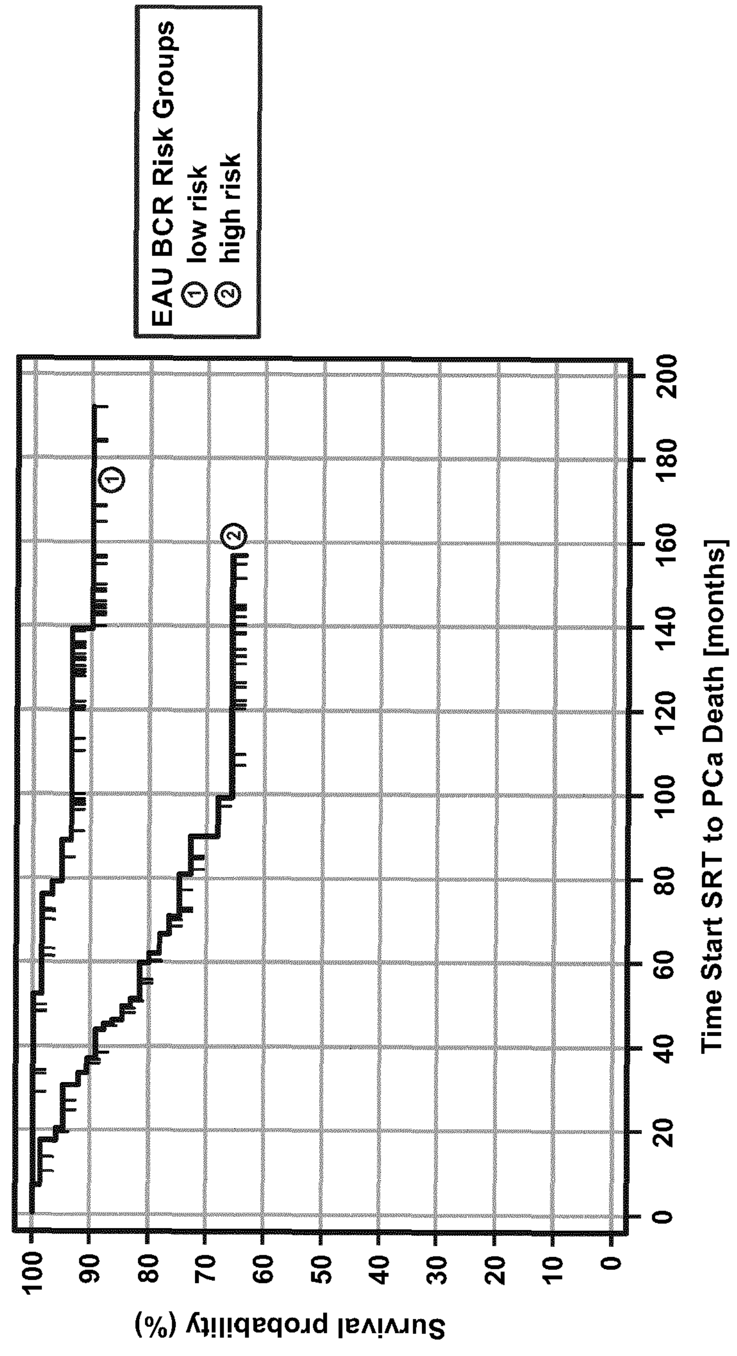
EAU BCR Risk Groups
① low risk
② high risk
Survival probability (%)
100
90
80
70
60
50
40
30
20
10
0
Time Start SRT to PCa Death [months]
0
20
40
60
80
100
120
140
160
180
200
①
②

FIG. 6

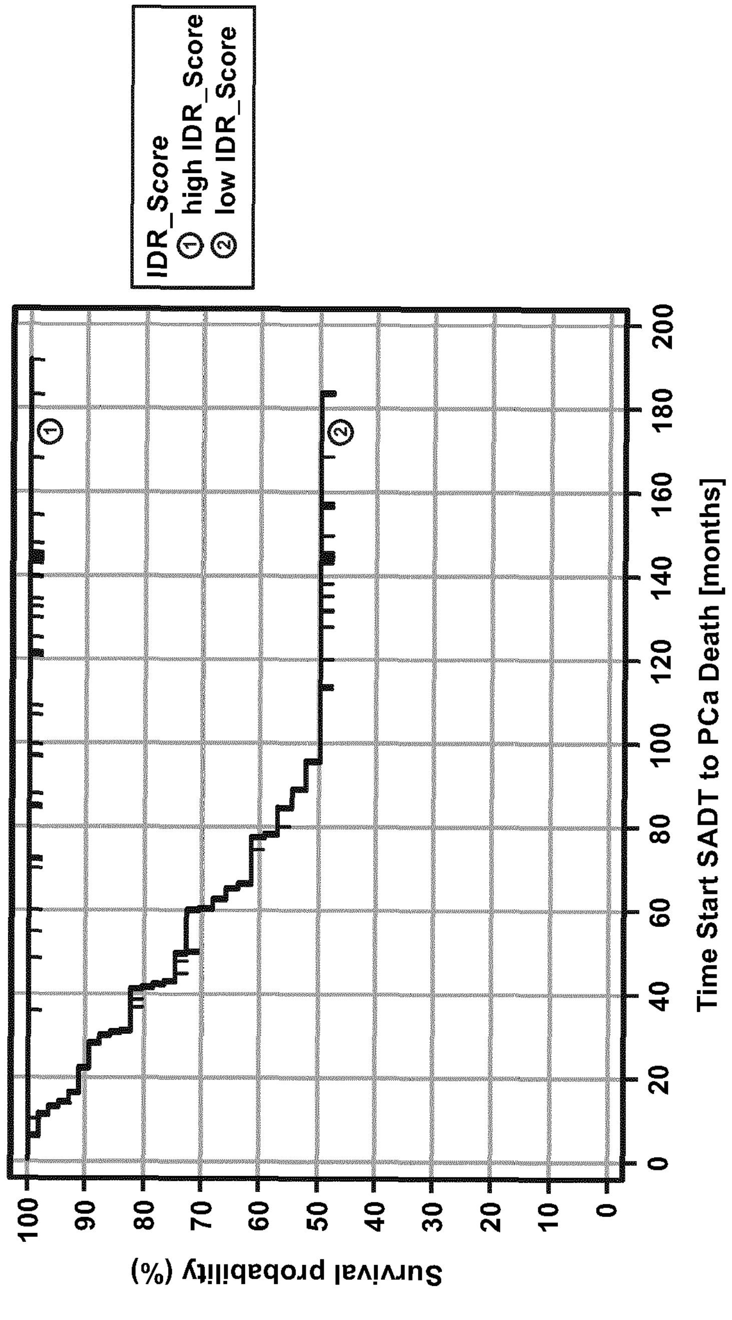
IDR_Score
① high IDR_Score
② low IDR_Score
Survival probability (%)
100
90
80
70
60
50
40
30
20
10
0
0
20
40
60
80
100
120
140
160
180
200
Time Start SADT to PCa Death [months]
①
②

FIG. 7

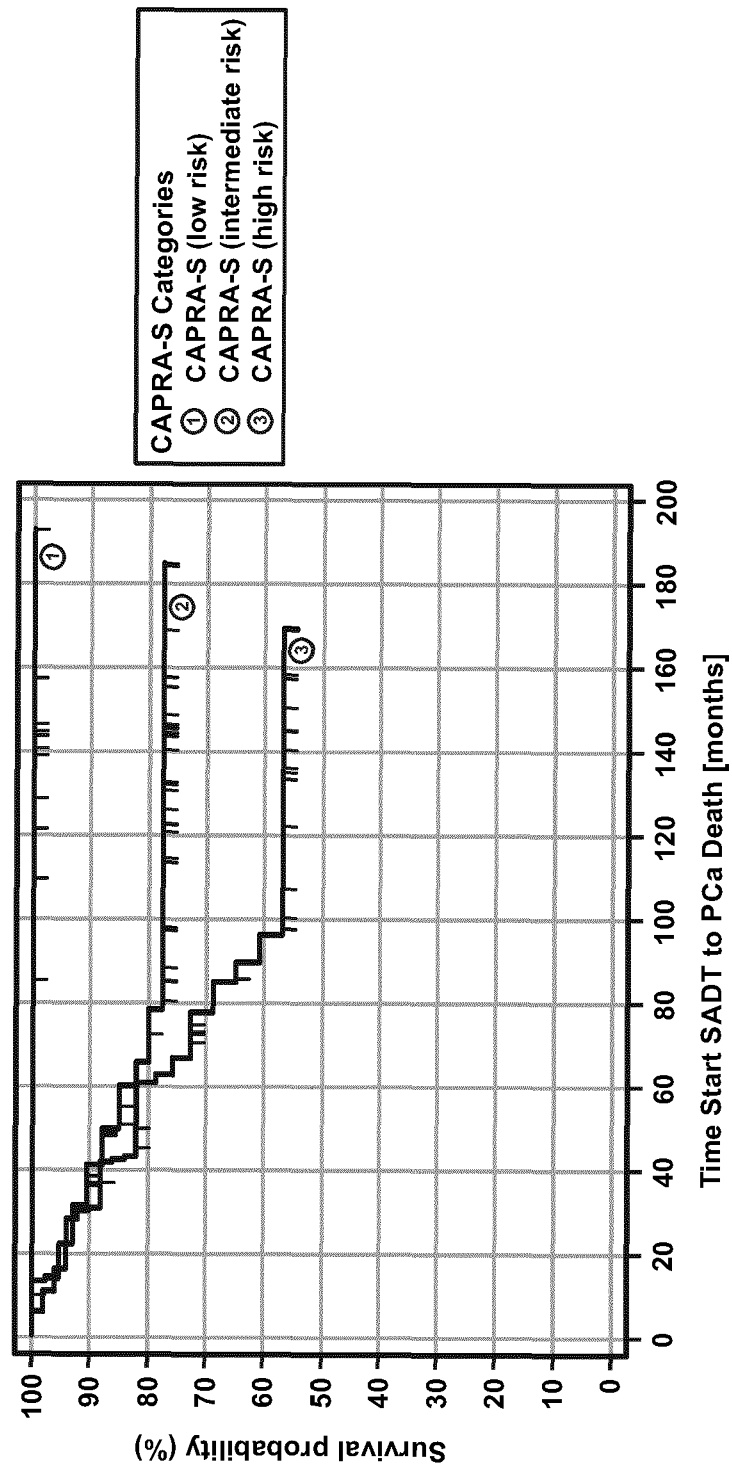
CAPRA-S Categories
① CAPRA-S (low risk)
② CAPRA-S (intermediate risk)
③ CAPRA-S (high risk)
Survival probability (%)
100
90
80
70
60
50
40
30
20
10
0
0
20
40
60
80
100
120
140
160
180
200
Time Start SADT to PCa Death [months]
①
②
③

FIG. 8

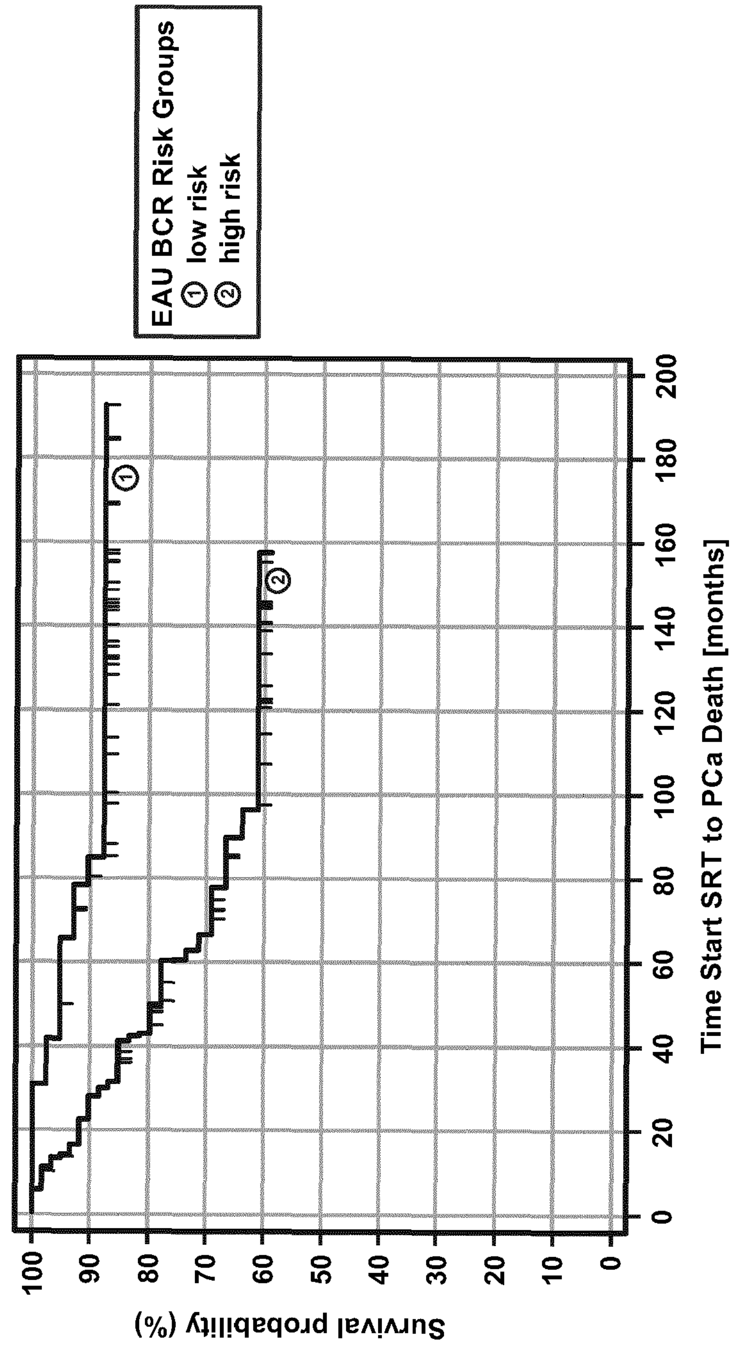
EAU BCR Risk Groups
① low risk
② high risk
Survival probability (%)
100
90
80
70
60
50
40
30
20
10
0
Time Start SRT to PCa Death [months]
0
20
40
60
80
100
120
140
160
180
200
①
②

FIG. 9

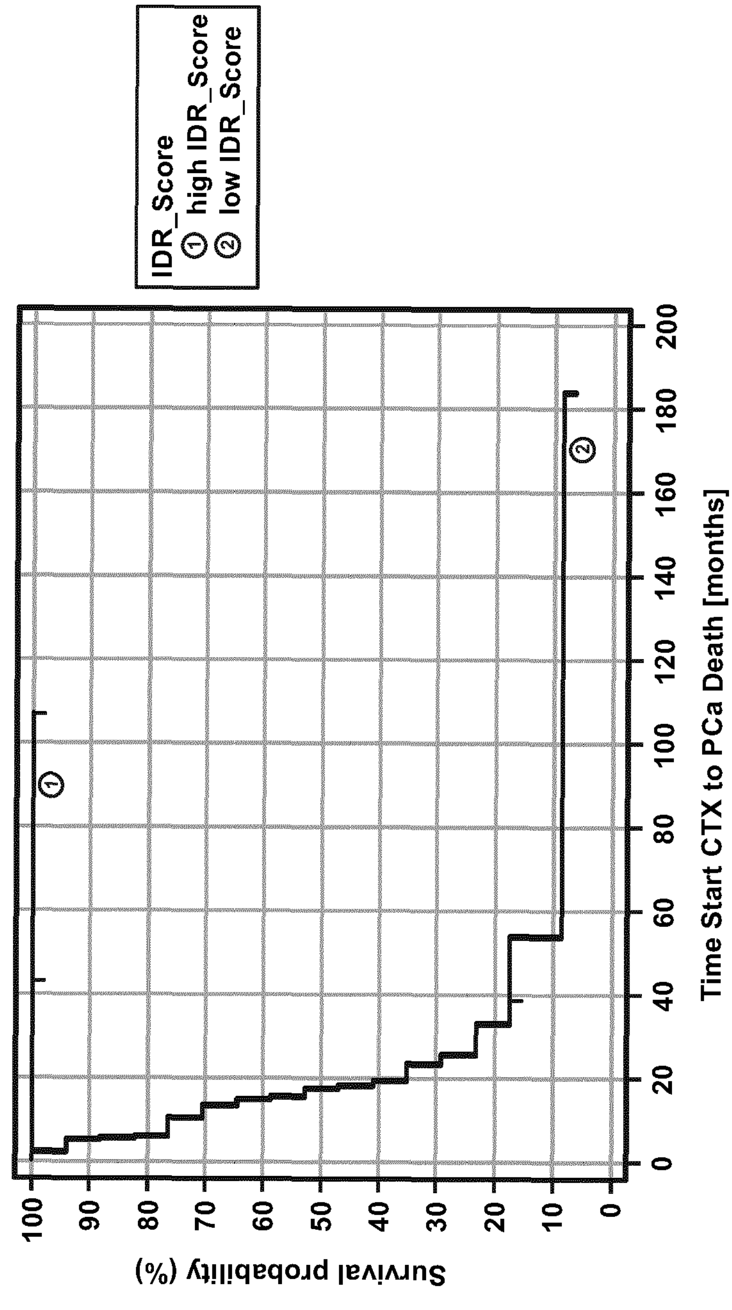

IDR_Score
① high IDR_Score
② low IDR_Score
Survival probability (%)
100
90
80
70
60
50
40
30
20
10
0
0
20
40
60
80
100
120
140
160
180
200
Time Start CTX to PCa Death [months]
①
②


FIG. 10

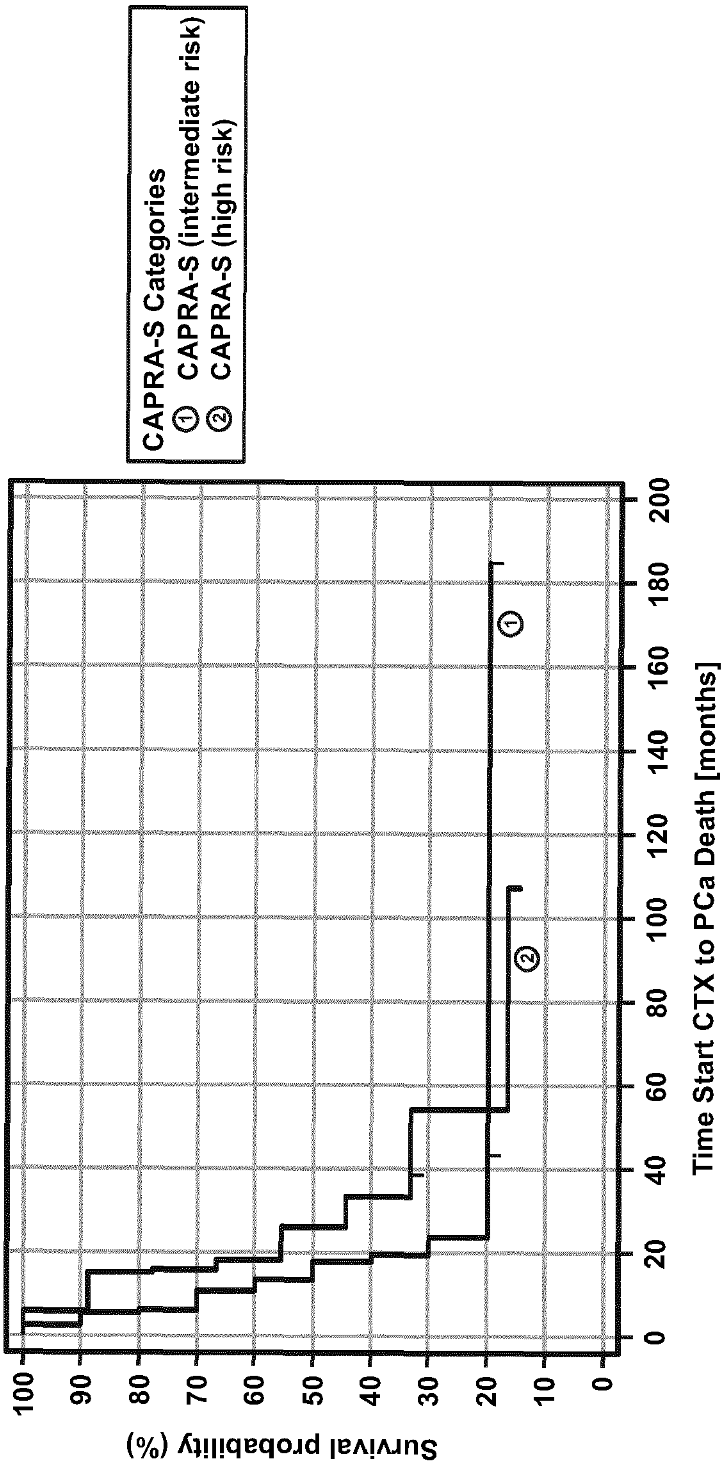

CAPRA-S Categories
① CAPRA-S (intermediate risk)
② CAPRA-S (high risk)
Survival probability (%)
100
90
80
70
60
50
40
30
20
10
0
Time Start CTX to PCa Death [months]
0
20
40
60
80
100
120
140
160
180
200
①
②


FIG. 11

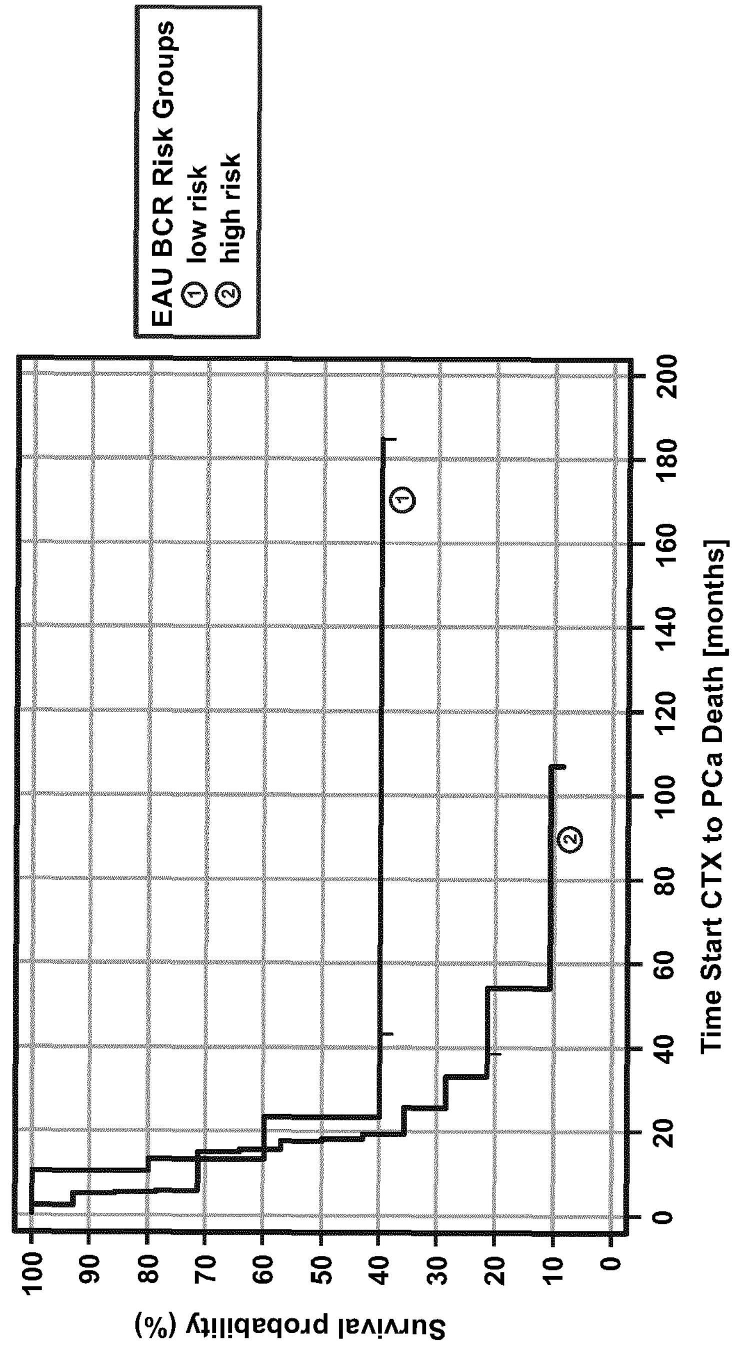
EAU BCR Risk Groups
① low risk
② high risk
Survival probability (%)
100
90
80
70
60
50
40
30
20
10
0
Time Start CTX to PCa Death [months]
0
20
40
60
80
100
120
140
160
180
200
①
②

FIG. 12

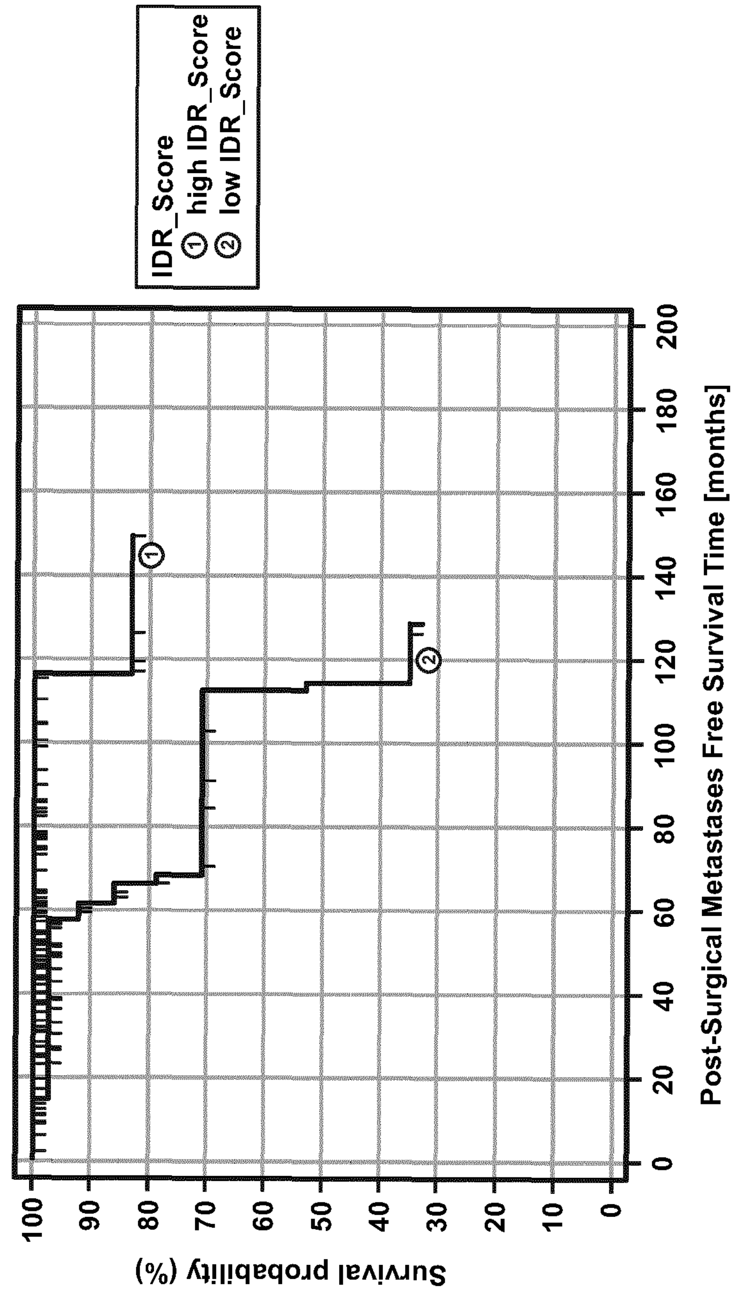
IDR_Score
① high IDR_Score
② low IDR_Score
Survival probability (%)
100
90
80
70
60
50
40
30
20
10
0
0
20
40
60
80
100
120
140
160
180
200
Post-Surgical Metastases Free Survival Time [months]
①
②

FIG. 13

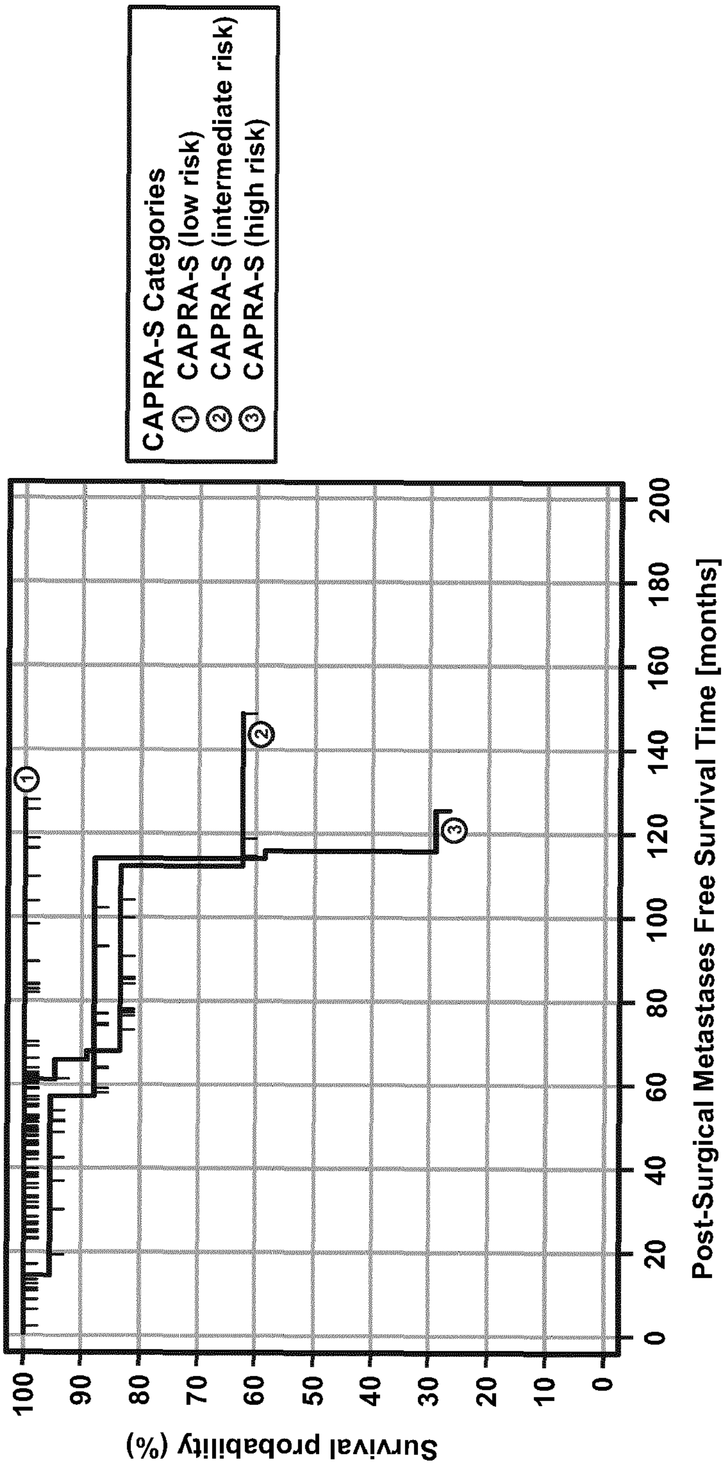
CAPRA-S Categories
① CAPRA-S (low risk)
② CAPRA-S (intermediate risk)
③ CAPRA-S (high risk)
Survival probability (%)
100
90
80
70
60
50
40
30
20
10
0
0
20
40
60
80
100
120
140
160
180
200
Post-Surgical Metastases Free Survival Time [months]

FIG. 14

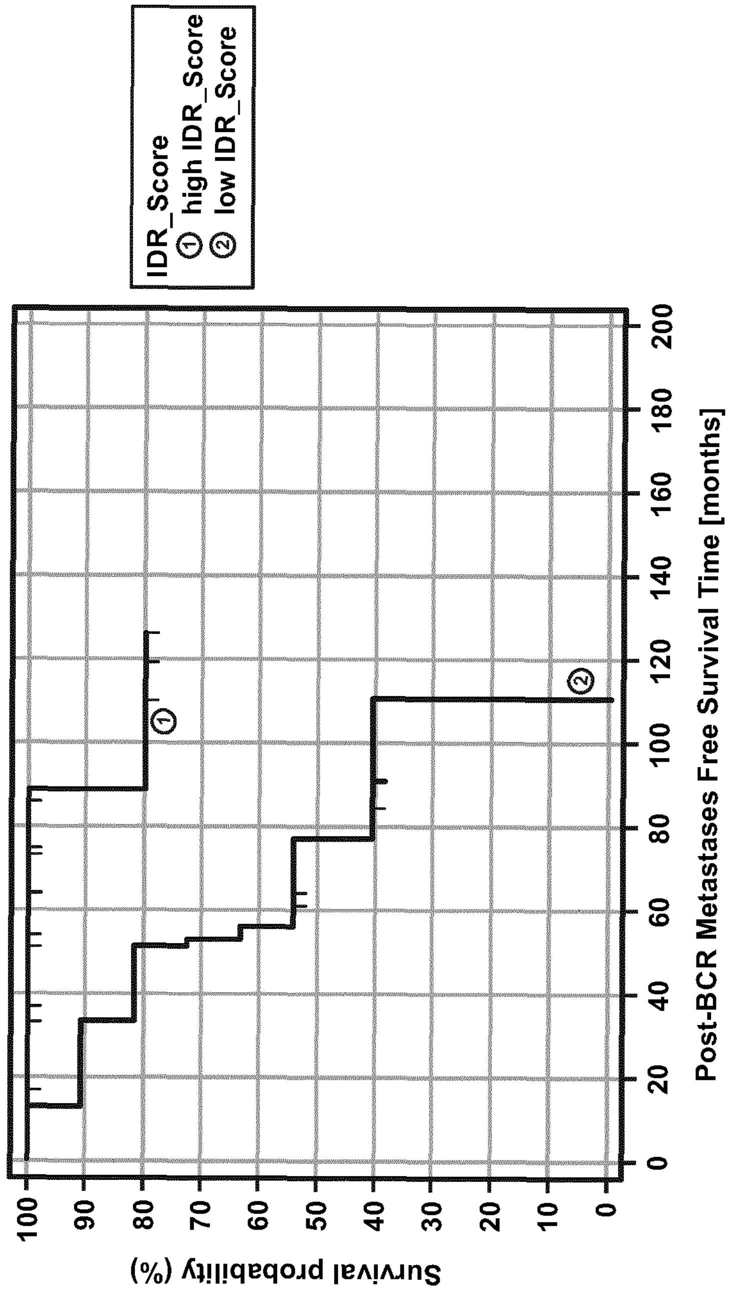
IDR_Score
① high IDR_Score
② low IDR_Score
Survival probability (%)
100
90
80
70
60
50
40
30
20
10
0
0
20
40
60
80
100
120
140
160
180
200
Post-BCR Metastases Free Survival Time [months]
①
②

FIG. 15

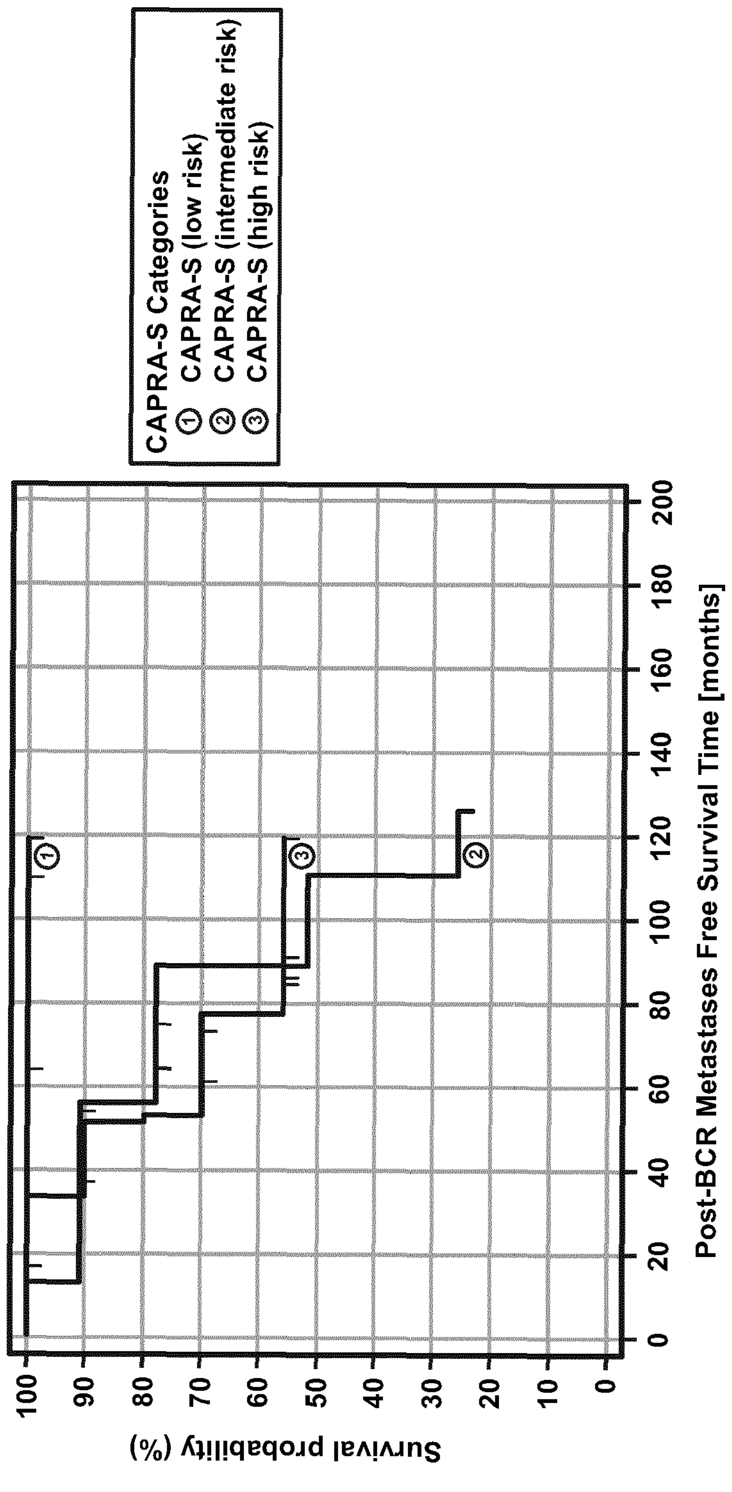
CAPRA-S Categories
① CAPRA-S (low risk)
② CAPRA-S (intermediate risk)
③ CAPRA-S (high risk)
Survival probability (%)
100
90
80
70
60
50
40
30
20
10
0
Post-BCR Metastases Free Survival Time [months]
0
20
40
60
80
100
120
140
160
180
200
①
②
③

FIG. 16

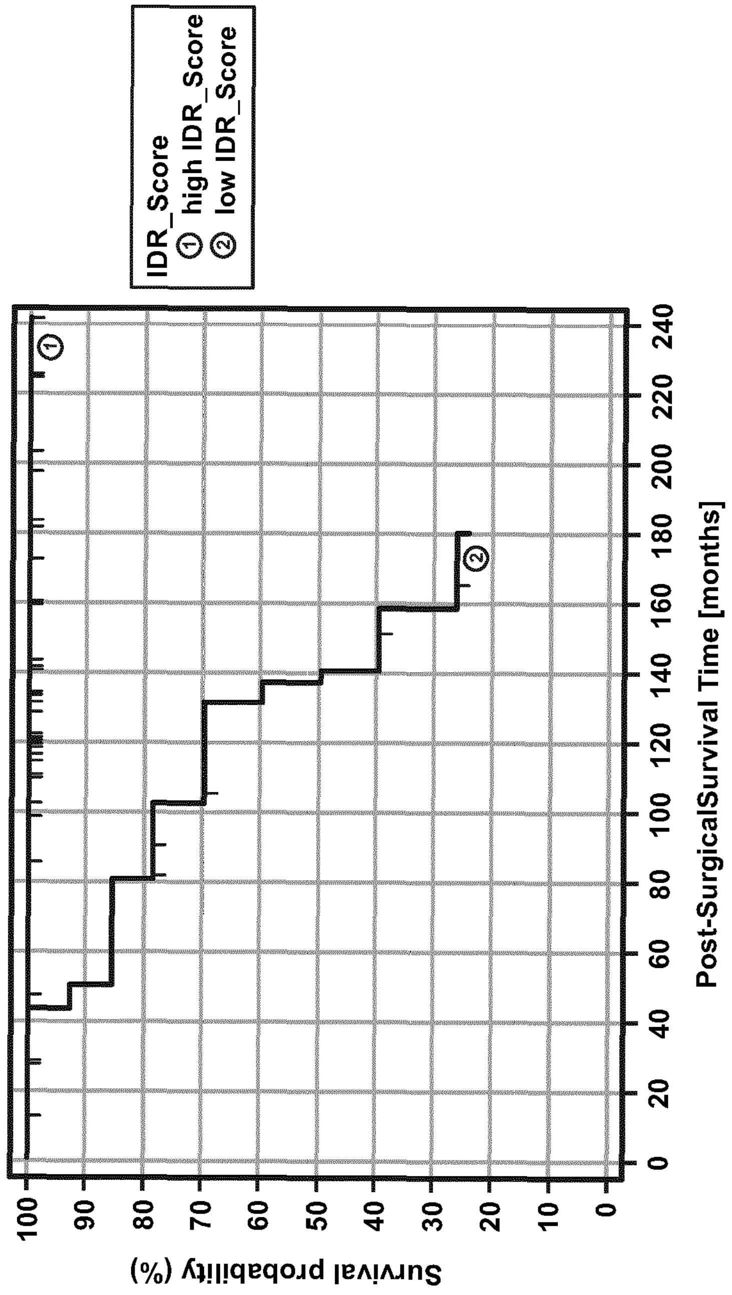
IDR_Score
① high IDR_Score
② low IDR_Score
Survival probability (%)
100
90
80
70
60
50
40
30
20
10
0
0
20
40
60
80
100
120
140
160
180
200
220
240
Post-SurgicalSurvival Time [months]
①
②

FIG. 17

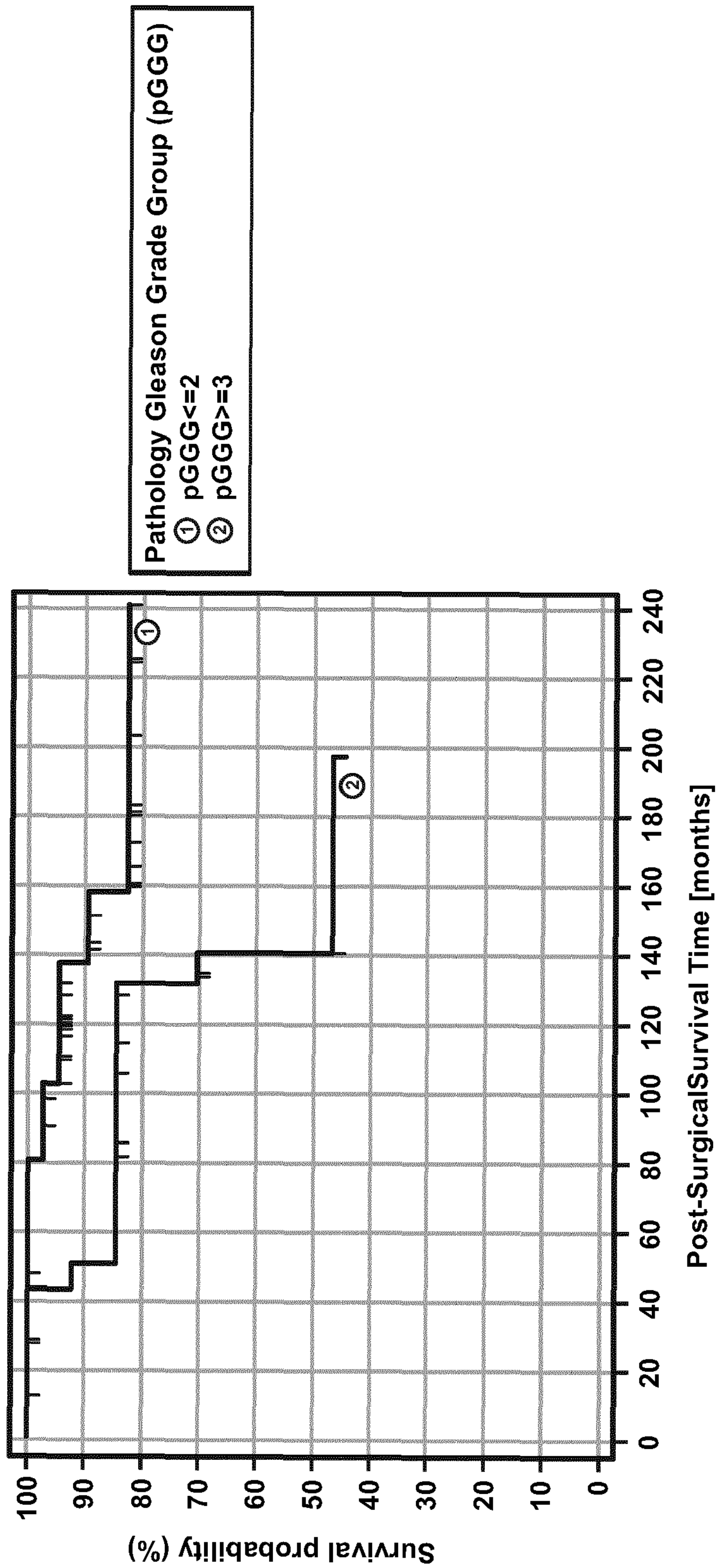
Pathology Gleason Grade Group (pGGG)
① pGGG<=2
② pGGG>=3
Survival probability (%)
100
90
80
70
60
50
40
30
20
10
0
Post-SurgicalSurvival Time [months]
0
20
40
60
80
100
120
140
160
180
200
220
240
①
②

FIG. 18

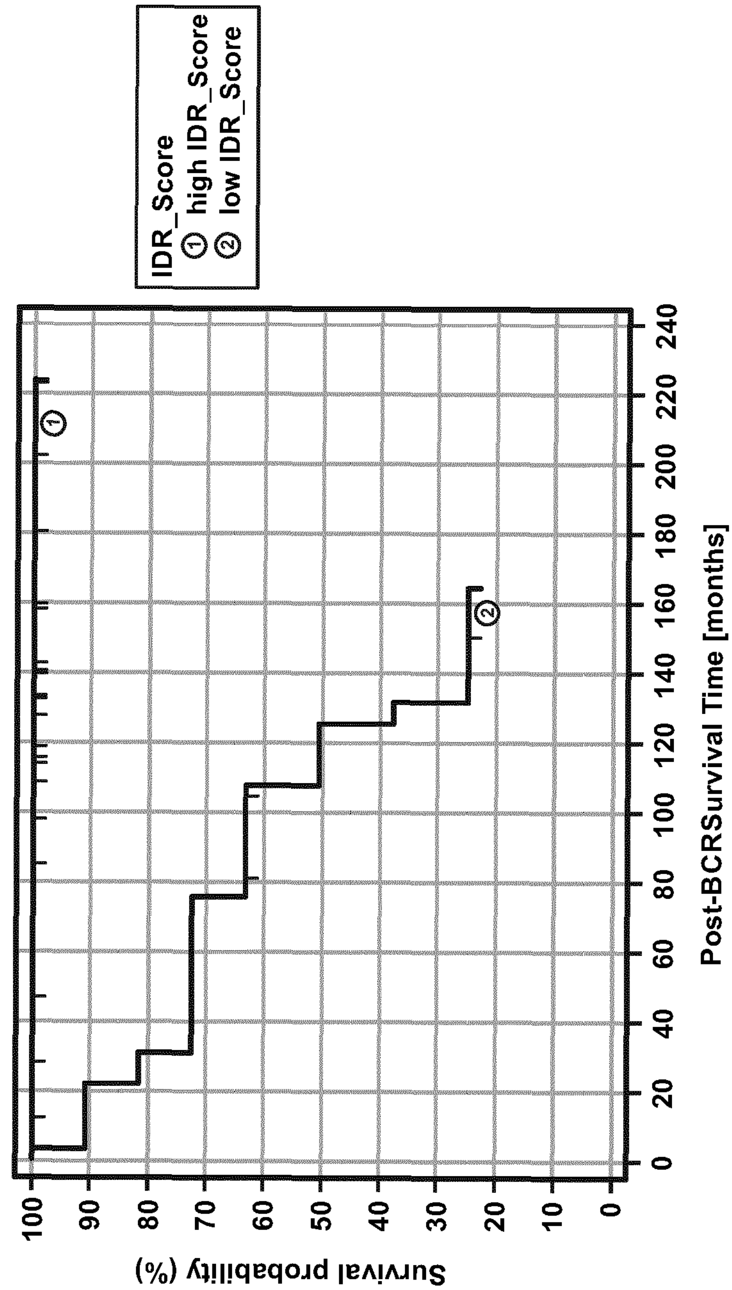
IDR_Score
① high IDR_Score
② low IDR_Score
Survival probability (%)
100
90
80
70
60
50
40
30
20
10
0
0
20
40
60
80
100
120
140
160
180
200
220
240
Post-BCRSurvival Time [months]
①
②

FIG. 19

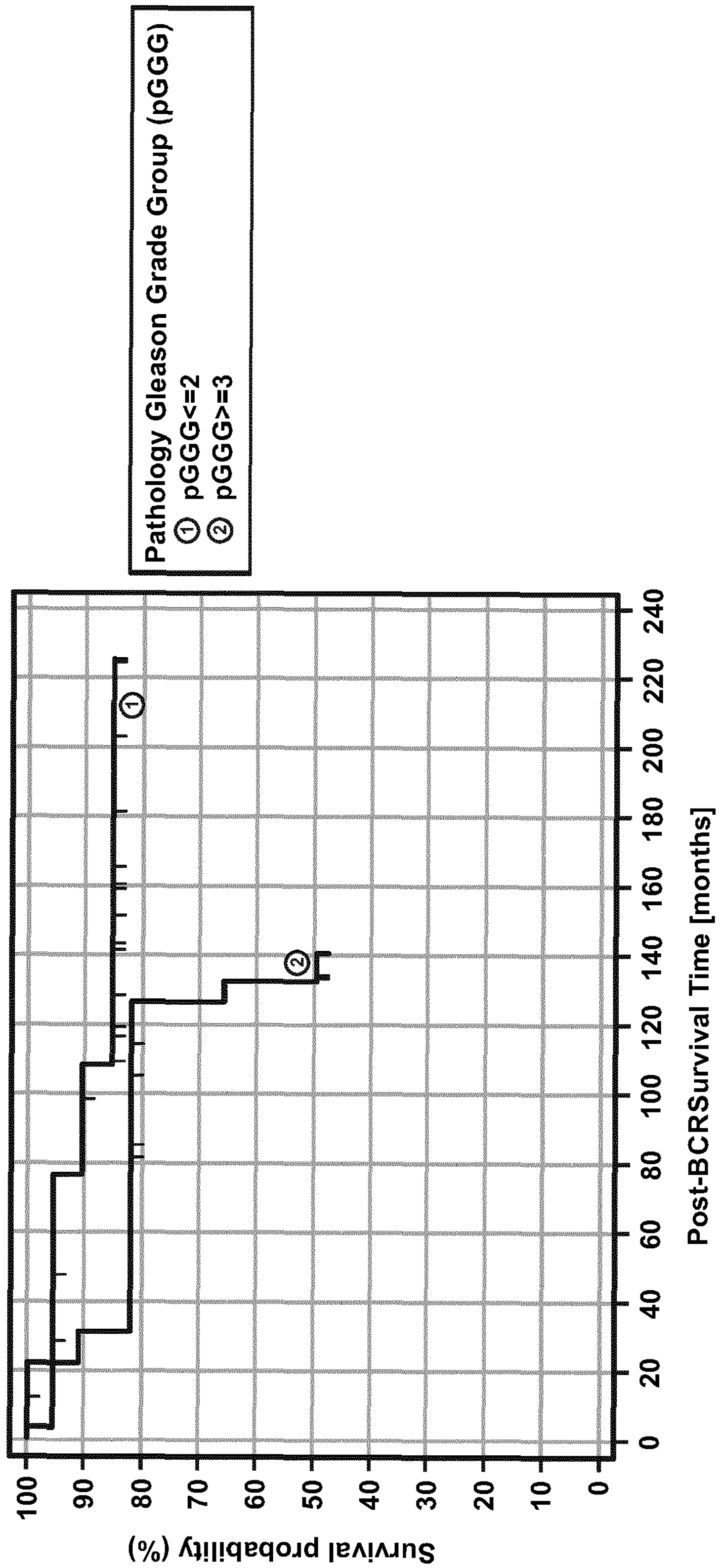
Pathology Gleason Grade Group (pGGG)
① pGGG<=2
② pGGG>=3
Survival probability (%)
100
90
80
70
60
50
40
30
20
10
0
0
20
40
60
80
100
120
140
160
180
200
220
240
Post-BCRSurvival Time [months]
①
②

FIG. 20

Europäisches Patentamt
European Patent Office
Office européen des brevets

# EUROPEAN SEARCH REPORT

Application Number
EP 20 16 1176

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2019/028285 A2 (DECIPHER BIOSCIENCES INC) 7 February 2019 (2019-02-07) * page 3 - page 4; claim 40 * ----- | 1-3,5-16 | INV. C12Q1/6886 |
| A | ZHAO SHUANG G ET AL: "Development and validation of a 24-gene predictor of response to postoperative radiotherapy in prostate cancer: a matched, retrospective analysis", THE LANCET ONCOLOGY, ELSEVIER, AMSTERDAM, NL, vol. 17, no. 11, 12 October 2016 (2016-10-12), pages 1612-1620, XP029794201, ISSN: 1470-2045, DOI: 10.1016/S1470-2045(16)30491-0 * the whole document * ----- | 1-16 | |

TECHNICAL FIELDS SEARCHED (IPC)

C12Q

~~The present search report has been drawn up for all claims~~

2

| Place of search | Date of completion of the search | Examiner |
|---|---|---|
| The Hague | 1 September 2020 | Gabriels, Jan |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

Application Number

EP 20 16 1176

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

see sheet B

☐ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☒ None of the further search fees have been paid within the fixed time limit. The present European search report has been drawn up for those parts of the European patent application which relate to the invention first mentioned in the claims, namely claims:

1-16(partially)

☐ The present supplementary European search report has been drawn up for those parts of the European patent application which relate to the invention first mentioned in the claims (Rule 164 (1) EPC).

Europäisches Patentamt
European Patent Office
Office européen des brevets

# LACK OF UNITY OF INVENTION SHEET B

Application Number
EP 20 16 1176

The Search Division considers that the present European patent application does not comply with the requirements of unity of invention and relates to several inventions or groups of inventions, namely:

1. claims: 1-16(partially)

A method of predicting a response of a prostate cancer subject to radiotherapy, comprising: determining or receiving the result of a determination of a gene expression profile for the immune defense response genes APOBEC3A and apparatuses, for performing such prediction, compute program products therefore, diagnostic kits comprising at least one primer/probe for determining said expression profile, and uses thereof.

---

2-61. claims: 1-16(partially)

The same as for invention 1 but limited to the next immune response defense gene, wherein invention 2 corresponds to AIM2, invention 3 corresponds to APOBEC3B, ..., invention 60 corresponds to TRIM22, and invention 61 corresponds to ZBP1.

---

# ANNEX TO THE EUROPEAN SEARCH REPORT ON EUROPEAN PATENT APPLICATION NO. EP 20 16 1176

This annex lists the patent family members relating to the patent documents cited in the above-mentioned European search report. The members are as contained in the European Patent Office EDP file on
The European Patent Office is in no way liable for these particulars which are merely given for the purpose of information.

01-09-2020

| Patent document cited in search report | Publication date | Patent family member(s) | Publication date |
|---|---|---|---|
| WO 2019028285 A2 | 07-02-2019 | AU 2018310987 A1 | 27-02-2020 |
| | | CA 3072061 A1 | 07-02-2019 |
| | | EP 3662082 A2 | 10-06-2020 |
| | | WO 2019028285 A2 | 07-02-2019 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **BRAY F. et al.** Global cancer statistics 2018: GLOBOCAN estimates of incidence and mortality worldwide for 36 cancers in 185 countries. *CA Cancer J Clin,* 2018, vol. 68 (6), 394-424
- Cancer Facts & Figures 2010. ACS (American Cancer Society), 2010
- **GRIMM P. et al.** Comparative analysis of prostate-specific antigen free survival outcomes for patients with low, intermediate and high risk prostate cancer treatment by radical therapy. Results from the Prostate Cancer Results Study Group. *BJU Int,* 2012, 22-29
- **DAL PRA A. et al.** Contemporary role of postoperative radiotherapy for prostate cancer. *Transl Androl Urol,* 2018, vol. 7 (3), 399-413
- **HERRERA F.G. ; BERTHOLD D.R.** Radiation therapy after radical prostatectomy: Implications for clinicians. *Front Oncol,* 2016, vol. 6 (117
- **PISANSKY T.M. et al.** Salvage radiation therapy dose response for biochemical failure of prostate cancer after prostatectomy - A multi-institutional observational study. *Int J Radiat Oncol Biol Phys,* 2016, vol. 96 (5), 1046-1053
- **RESNICK M.J. et al.** Long-term functional outcomes after treatment for localized prostate cancer. *N Engl J Med,* 2013, vol. 368 (5), 436-445
- **HEGARTY S.E. et al.** Radiation therapy after radical prostatectomy for prostate cancer: Evaluation of complications and influence of radiation timing on outcomes in a large, population-based cohort. *PLoS One,* 2015, vol. 10 (2
- **PARAVATI A.J. et al.** Variation in the cost of radiation therapy among medicare patients with cancer. *J Oncol Pract,* 2015, vol. 11 (5), 403-409
- **HALL W.A. et al.** Biomarkers of outcome in patients with localized prostate cancer treated with radiotherapy. *Semin Radiat Oncol,* 20 November 2016, vol. 27
- **RAYMOND E. et al.** An appraisal of analytical tools used in predicting clinical outcomes following radiation therapy treatment of men with prostate cancer: A systematic review. *Radiat Oncol,* 2017, vol. 12 (1), 56
- **MANTOVANI A. et al.** Cancer-related inflammation. *Nature,* 2008, vol. 454 (7203), 436-444
- **GIRALDO N.A. et al.** The clinical role of the TME in solid cancer. *Br J Cancer,* 2019, vol. 120 (1), 45-53
- **SHIAO S.L. et al.** Regulation of prostate cancer progression by tumor microenvironment. *Cancer Lett,* 2016, vol. 380 (1), 340-348
- **BARKER H.E. et al.** The tumor microenvironment after radiotherapy: Mechanisms of resistance or recurrence. *Nat Rev Cancer,* 2015, vol. 15 (7), 409-425
- **GASSER S. et al.** Sensing of dangerous DNA. *Mechanisms of Aging and Development,* 2017, vol. 165, 33-46
- **ALVES DE INDA M. et al.** Validation of Cyclic Adenosine Monophosphate Phosphodiesterase-4D7 for its Independent Contribution to Risk Stratification in a Prostate Cancer Patient Cohort with Longitudinal Biological Outcomes. *Eur Urol Focus,* 2018, vol. 4 (3), 376-384
- **TILKI D. et al.** External validation of the European Association of Urology Biochemical Recurrence Risk groups to predict metastasis and mortality after radical prostatectomy in a European cohort. *Eur Urol,* 2019, vol. 75 (6), 896-900
- **COOPERBERG M.R. et al.** The CAPRA-S score: A straightforward tool for improved prediction of outcomes after radical prostatectomy. *Cancer,* 2011, vol. 117 (22), 5039-5046